(19) Europäisches Patentamt
European Patent Office
Office européen des brevets

(11) EP 4 529 551 B1

(12) **EUROPEAN PATENT SPECIFICATION**

(45) Date of publication and mention of the grant of the patent:
**01.07.2026 Bulletin 2026/27**

(21) Application number: **23731095.8**

(22) Date of filing: **23.05.2023**

(51) International Patent Classification (IPC):
***C09B 23/01*** *(2006.01)* ***C09B 23/10*** *(2006.01)*
***C09B 23/08*** *(2006.01)* ***A61K 49/00*** *(2006.01)*
***C07K 7/64*** *(2006.01)* ***C07K 7/02*** *(2006.01)*

(52) Cooperative Patent Classification (CPC):
**C09B 23/086; A61K 49/0032; A61K 49/0052; A61K 49/0056; C07K 7/02; C09B 23/0058; C09B 23/107**

(86) International application number:
**PCT/EP2023/063787**

(87) International publication number:
**WO 2023/227601 (30.11.2023 Gazette 2023/48)**

(54) **NEAR-INFRARED FLUORESCENT HEPTAMETHINE DYE CONJUGATES WITH FAVORABLE BLEACHING PROPERTIES**

IM NAHEN INFRAROT FLUORESZIERENDE HEPTAMETHIN-FARBSTOFFKONJUGATE MIT GÜNSTIGEN BLEICHEIGENSCHAFTEN

CONJUGUÉS DE COLORANT HEPTAMÉTHINE FLUORESCENTS DANS LE PROCHE INFRAROUGE AYANT DES PROPRIÉTÉS DE BLANCHIMENT FAVORABLES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **23.05.2022 EP 22174958**

(43) Date of publication of application:
**02.04.2025 Bulletin 2025/14**

(73) Proprietors:
- **Helmholtz Zentrum München - Deutsches Forschungszentrum für Gesundheit und Umwelt (GmbH)**
  **85764 Neuherberg (DE)**
- **Universität zu Köln**
  **50923 Köln (DE)**

(72) Inventors:
- **PLETTENBURG, Oliver**
  **30167 Hannover (DE)**
- **WEITZENBERG, Merle Marie**
  **30167 Hannover (DE)**
- **BRUNS, Oliver**
  **85764 Neuherberg (DE)**
- **BISCHOF, Thomas**
  **85764 Neuherberg (DE)**
- **ARUS, Bernardo**
  **85764 Neuherberg (DE)**
- **FÜRTJES, Gina**
  **85764 Neuherberg (DE)**
- **NEUSCHMELTING, Volker**
  **50937 Köln (DE)**

(74) Representative: **Schiweck Weinzierl Koch Patentanwälte Partnerschaft mbB**
**Ganghoferstraße 68 B**
**80339 München (DE)**

(56) References cited:
**US-B1- 10 441 607**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 529 551 B1

## Description

### TECHNICAL FIELD OF THE INVENTION

The present invention relates to near-infrared fluorescent heptamethine dyes and conjugates of heptamethine dyes with favourable biodistribution and bleaching properties.

### BACKGROUND ART

Target-specific contrast agents enable the non-invasive visualization of molecular structures and processes in cells, tissues or living organisms and therefore are valuable tools for biomolecular imaging. They compose of a targeted ligand that is bound to the label via a linker. In the case of optical imaging, the label is a fluorescent dye with a) a conjugation handle and b) peak emission > 780 nm, which allows imaging in the Near and Shortwave Infrared. In particular, the latter allows comparatively deep penetration into tissue.

Applications for fluorescent target-specific contrast agents range from medical diagnostics and surgical navigation to basic biomedical research and all require a favourable target to background ratio. In vivo, this results from the combination of specific binding of the probe to its target structure and efficient clearance of free or unspecifically bound probe. Many applications furthermore require imaging over a prolonged period. Continuous irradiation is accompanied by photobleaching, the gradual decrease in emitted fluorescence due to photochemical alteration of the fluorophore.

Indocyanine green (ICG, NIR dye, gold standard) and 5-aminolevulinic acid (5-ALA, converted to protoporphyrin IX, VIS dye) are routinely used in the clinic for optical imaging. Neither of them has a conjugation handle and they thus cannot be used to label specific target tissues. Besides this, photostability poses a significant problem for in vivo experiments. For ICG this is the case when it is not used as a contrast agent for purely intravascular FI angiography, but plasma protein-bound as a contrast agent via EPR for tumour imaging (1). The lack of photostability of 5-ALA (PPIX) is a well-known problem in clinical research (2).

The majority of targeted fluorescent contrast agents currently propelling clinical trials consist of IRDye®800CW.

Štacková et al. disclosed an approach to a substituted heptamethine cyanine chain by the ring opening of Zincke Salts (3). However, in contrast to the present invention, the compounds of Štacková et al. do not disclose compounds which exhibit additional sulfonate groups on both aromatic rings and an electron withdrawing amide group attached to a polyene scaffold in combination which relate to different improved properties of the inventive compounds, as discussed below.

IRDye®800CW is a commercially available heptamethine cyanine dye that comes with a conjugation handle. Its conjugates are currently propelling more than 20 Phase I and Phase II clinical trials (4), (5). The compounds of the present invention and IRDye®800CW are of similar molecular weight and have the same degree of sulfonation/net charge which results in similar *in vivo* behaviour. In difference to the compounds of the present invention, IRDye®800CW has an electron donating phenol attached to the polyene scaffold that increases the nucleophilicity of the conjugated pi-system and makes it more prone to singlet oxygen driven photobleaching. IRDye®800CW is also characterized by long unspecific tissue half-live, limiting imaging applications shortly after injection.

There is a variety of conjugateable NIR-fluorescent dyes known in the prior art of which publications (6)-(14) represent a selection. However, there is no conjugateable NIR-fluorescent dye with an electron withdrawing amide group attached to a polyene scaffold in combination with a high degree of sulfonic acid substituents at the indocyanine backbone as described in the present invention.

Thus, there is a need for further fluorescent dyes.

### SUMMARY OF THE INVENTION

A compound according to formula (I),

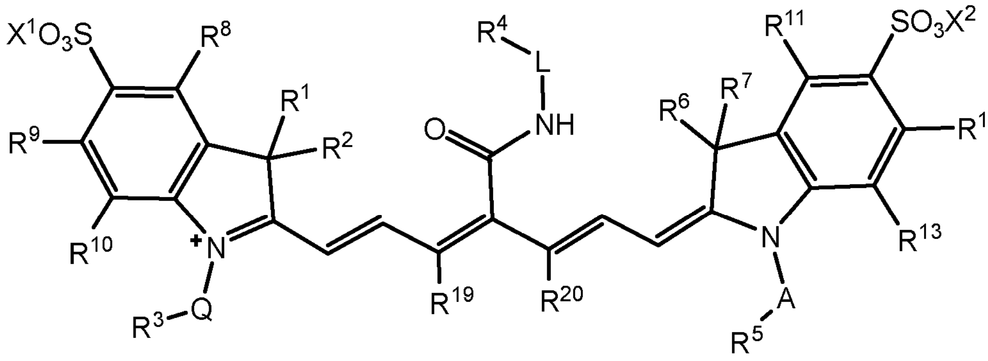

(I)

wherein

$R^1$, $R^2$, $R^6$, $R^7$, $R^{14}$, $R^{15}$, $R^{16}$, $R^{17}$, and $R^{18}$ are independently selected from the group consisting of H and $(C_1-C_6)$alkyl, preferably methyl;

$R^3$ is $-SO_3X^3$, or COOH;

$R^4$ is selected from the group consisting of -ethinyl, -azide,

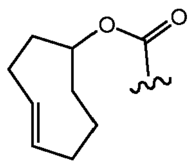 , 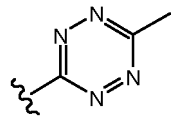 ,

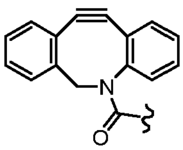 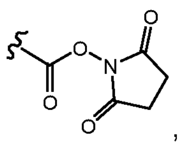 , 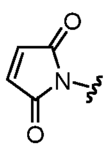 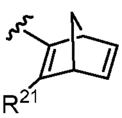 , 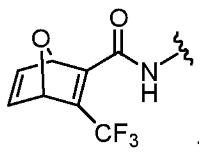 .

-COOH, $NH_2$, Cl, Br, I, - $OSO_2CF_3$, $-COSO_2CH_3$, -OH, vinyl, -SH;

$R^5$ is $-SO_3X^4$;

$R^8$, $R^9$, $R^{10}$, $R^{11}$, $R^{12}$ and $R^{13}$ are independently selected from the group consisting of H, CN, - $COO(C_1-C_6)$alkyl, $-CONH(C_1-C_6)$alkyl, Cl, Br, I, F, $-CF_3$, $-O(C_1-C_6)$, $-NR^{14}R^{15}$, $-NHC(O)(C_1-C_6)$alkyl, $-NHC(O)NH(C_1-C_6)$alkyl),$-SO_3H$, $-SO_2NH_2$, $-(C_1-C_6)$alkyl, preferably H;

$R^{19}$, $R^{20}$ are independently selected from the group consisting of H, $-(C_1-C_6)$alkyl , $-O(C_1-C_6)$alkyl, Cl, F, Br, CN; C(O) $NH_2$, preferably H;

$R^{21}$ is H, $-(C_1-C_{10})$alkyl;

L is a group consisting of one or more and/or one or more of each of the elements selected from the group: $-(CH_2)_{n1}-$, $-(C_1-C_6)$cycloalkylene-, $-(C_1-C_6)$heterocycloalkylene-, -O-, $-NR^{16}-$, -NH-, - NHC(O)-, $-C(O)NR^{17}-$, -CH(OH)-, and $-CH(OR^{18})-$;

$n^1$ is an integer between 1 to 30, preferably 1 to 20, more preferably 1 to 10;

$X^1$, $X^2$, $X^3$, and $X^4$ are independently selected from a cation, a proton or absent, preferably the cation is a monovatent cation or a divalent cation, more preferably the cation is a monovalent cation most preferably an alkali metal cation or an ammonium cation, particularly preferred the cation is independently selected from the group consisting of $Na^+$, $K^+$;

and $NH_4^+$.

Q is $-(CH_2)_{n2}-$,

wherein optionally one or more of each H in $-(CH_2)_{n2}-$ may be replaced by $-(C_1-C_{10})$alkyl;

$n^2$ is an integer between 1 to 10, preferably 1 to 6, more preferably 1 to 4;

A is $-(CH_2)_{n3}-$,

wherein optionally one or more of each H in $-(CH_2)_{n3}-$ may be replaced by $-(C_1-C_{10})$alkyl;

$n^3$ is an integer between 1 to 10, preferably 1 to 6, more preferably 1 to 4.

The invention is further directed to a compound according to formula (II)

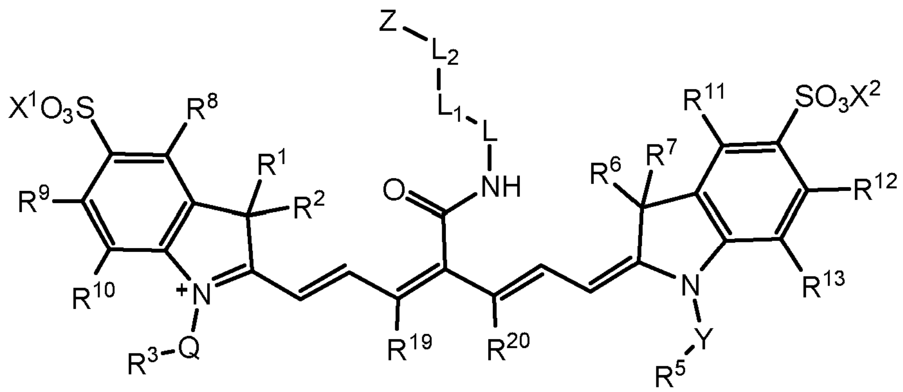

(II)

wherein

$R^1$, $R^2$, $R^6$, $R^7$, $R^{14}$, $R^{15}$, $R^{16}$, $R^{17}$, and $R^{18}$ are independently selected from the group consisting of H and $(C_1-C_6)$alkyl, preferably methyl;
$R^3$ is $-SO_3X^3$, or COOH;
Z is a protein, mono- or polyvalent saccharide, peptide, small molecule, antibody, oligonucleotide, nanobody, polymer;
$R^5$ is $-SO_3X^4$;
$R^8$, $R^9$, $R^{10}$, $R^{11}$, $R^{12}$ and $R^{13}$ are independently selected from the group consisting of H, CN, - $COO(C_1-C_6)$alkyl, $-CONH(C_1-C_6)$alkyl, Cl, Br, I, F, $-CF_3$, $-O(C_1-C_6)$, $-NR^{14}R^{15}$, $-NHC(O)(C_1-C_6)$alkyl, $-NHC(O)NH(C_1-C_6)$alkyl), $-(C_1-C_6)$ alkyl, $-SO_3H$, $-SO_2NH_2$, preferably H;
$R^{19}$, $R^{20}$ are independently selected from the group consisting of H, $-(C_1-C_6)$alkyl , $-O(C_1-C_6)$alkyl, Cl, F, Br, CN; C(O) $NH_2$, preferably H;
$R^{21}$ is H, $-(C_1-C_{10})$alkyl;
$X^1$, $X^2$, $X^3$, and $X^4$ are independently selected from a cation, a proton or absent, preferably the cation is a monovalent cation or a divalent cation, more preferably the cation is a monovalent cation, most preferably an alkali metal cation or an ammonium cation, particularly preferred the cation is independently selected from the group consisting of $Na^+$, $K^+$; and $NH_4^+$;
Q is $-(CH_2)_{n2}-$,
wherein optionally one or more of each H in $-(CH_2)_{n2}-$ may be replaced by $-(C_1-C_{10})$alkyl;
$n^2$ is an integer between 1 to 10, preferably 1 to 6, more preferably 1 to 4;
Y is $-(CH_2)_{n3}-$,
wherein optionally one or more of each H in $-(CH_2)_{n2}-$ may be replaced by $-(C_1-C_{10})$alkyl;
$n^3$ is an integer between 1 to 10, preferably 1 to 6, more preferably 1 to 4;
L is a group consisting of one or more and/or one or more of each of the elements selected from the group: $-(CH_2)_{n1}-$, $-(C_1-C_6)$cycloalkylene-, $-(C_1-C_6)$heterocycloalkylene-, -O-, $-NR^{16}-$, -NH-, - NHC(O)-, $-C(O)NR^{17}-$, -CH(OH)-, and $-CH(OR^{18})-$;
$n^1$ is an integer between 1 to 30, preferably 1 to 20, more preferably 1 to 10;

$L_1$ is selected from the group consisting of

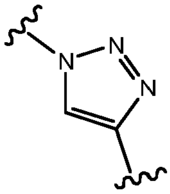, 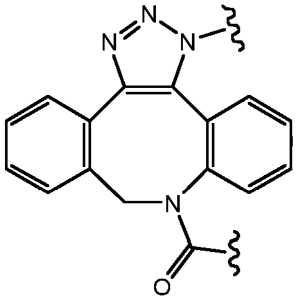, 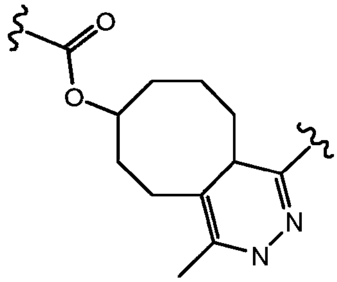, 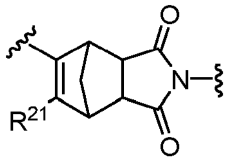, ,

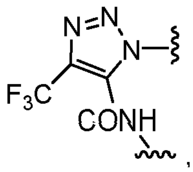, 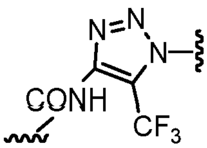, –CO-;

$L_2$ is is a group consisting of one or more and/or one or more of each of the elements selected from the group: $-(CH_2)_{n1}-$, $-(C_1-C_6)$cycloalkylene-, $-(C_1-C_6)$heterocycloalkylene-, -O-, $-NR^{16}-$, -NH-, -NHC(O)-, $-C(O)NR^{17}-$, -CH(OH)-, and $-CH(OR^{18})-$;

The invention is further directed to the use of the compounds as fluorescent dye.

The invention is furher directed to the use of the compounds in diagnosis, or for intraoperative surgery, in particular for use as contrast agent.

In a further aspect, the indication to be diagnosed or treated by intraoperative surgery is selected from the group consisting of inflammatory diseases, infectious diseases and cancer.

In a further aspect of the invention, the inflammatory disease is selected from the group consisting of otitis media, cholesteatoma, allergic rhinitis and chronic rhinosinusitis, chronic inflammation of tonsils, adenoids, and laryngitis.

In another aspect of the invention, the infectious disease is selected from the group consisting of implant infection (e.g. hip, dental), secondary infections of the inner ear, e.g. accompanying cholesteatoma. This comprises infections caused by gram positive or gram negative bacteria, responding to antibiotic treatment, resistant or multi-drug resistant, in particular by ESKAPE strains *Enterococcus faecium, Staphylococcus aureus, Klebsiella pneumoniae, Acinetobacter baumannii, Pseudomonas aeruginosa,* and *Enterobacter spp.*

In a further aspect, diagnosing of cancer or treating cancers by intraoperative surgery relates to visualizing the primary tumor and/or metastatic lesions in situ during surgery selected from the group meningioma, glioblastoma, NSCLC, pancreatic cancer, neuroendocrine tumors, lung cancer, breast cancer, meningiomas.

Another aspect is the visualization of risk structures during surgery to isolate or protect them. Such risk structures include nerves, blood vessels, lymphatic vessels, ureter, adrenals, pancreatic islets, pituitary gland, thyroid gland, parathyroid gland among others.

The compounds of the present invention, if used as fluorescent dyes and contrast agents, show a fast clearance and therefore favourable target to background ratios compared to reference conjugates using the known dye IRDye800. Moreover, the compounds of the present invention also exhibit a higher photostability which allow their improved application in fluorescence optical imaging, especially those that require laser exposure, over a prolonged period. The higher photostability is in particular beneficial for application of the inventive compounds in fluorescence-guided surgery. It has particularly been found that surprisingly the combination of a sulfonated aromatic system and an electron withdrawing amide attached to the polyene backbone is required for providing superior bleaching properties (see Fig. 3).

## BRIEF DESCRIPTION OF THE FIGURES

**Fig. 1:** Tissue and blood clearance of TOC-sNIR **(sstr2click1)** and TOC-IRDye®800CW **(sstr2click2).** For the data acquisition, mice were imaged during i.v. injection (5 nmol in 100 uL PBS). To avoid bleaching effects, the laser was triggered and only one image was acquired every 20 sec. power density was 50 $mW/cm^2$.

**Fig. 2:** Bleaching kinetics of TOC-sNIR **(sstr2click1),** TATE-sNIR **(sstr2click4)** and TOC-IRDye®800CW **(sstr2click2)** in vivo. For the data acquisition, mice were i.v. injected with 5 nmol dye conjugate in 100 μL PBS. The bleaching experiment was performed 3 h after injection. Two 785 nm lasers were used, differing in intensity and the size of the illuminated area. For local bleaching of the kidney, the kidney was irradiated with a power density of 200 $mW/cm^2$ over a period of 30 min. The whole-body images were taken with a power density of 50 $mW/cm^2$ before and after bleaching. After completion of the experiment, we noticed that TOC-sNIR (green curve), had not yet reached its end point (slope = 0) like TOC-IRDye (orange curve). For this reason, we performed another bleaching experiment with TATE-sNIR, for which we used a local power density of 450 $mW/cm^2$. To compare the data with those from TOC-IRDye, we multiplied the time axis of TATE-sNIR by a factor of (450/200). Since both TOC-IRDye and TATE-sNIR reached their endpoints, the data were normalized to maximum and minimum. The peptides TOC and TATE differ only with respect to the oxidation state of the C-terminus.

**Fig. 3:** Bleaching studies in vitro have been performed.
3A: Structural formulas of the alkyne-functionalized versions of NIR **(dyealkin2)** and sNIR **(dyealkin5)** as well as IRDye®800CW carboxylate as they were used for the bleaching experiments. Bleaching kinetics of NIR, sNIR and IRDye®800CW are shown in 3B) water and 3C) FBS. Under continuous 785 nm irradiation, sNIR is bleaching 3.5-times slower than NIR, ICG and IRDye®800CW in water. In fetal bovine serum (FBS), sNIR is 2.5-times more photostable than NIR, IRDye®800CW and 1.6-times more stable than ICG. Overall, sNIR has favourable photobleaching properties in comparison to its less sulfonated analogue NIR, Indocyanine Green (ICG, clinically approved dye without conjugation handle) and IRDye®800CW. These results prove that surprisingly the combination of the sulfonated aromatic system and the electron withdrawing amide attached to the polyene backbone is required for providing superior bleaching properties.

**Fig. 4:** Bleaching kinetics of sNIR, TOC-sNIR, IRDye®800CW and TOC-IRDye®800CW in A) water and B) FBS. Conjugation of sNIR and IRDye®800CW to the TOC-peptide improved photostability of the probe, but did not change the general trend of photobleaching described above for Fig. 3. 4C): Bleaching *in vitro:* Capillaries filled with 2.5 μM solutions in serum were continuously irradiated using a 785 nm laser (100 mW/cm^2) and frames were acquired for 20 minutes. Bleaching kinetics were extracted by analyzing the mean counts in an ROI over time

**Fig. 5:** Comparing Pan-sNIR **(NIR-fluorescent Panitumumab)** and Pan-IRDye800CW in tumor bearing mice post intraveneous injection. Mice were generated by subcutaneous implantation of MDA-MB-468 cells. A) *In vivo* distribution of monoclonal antibody Panitumumab conjugated to sNIR dye showed linear correlation between tumor signal and injected dose (100 μg-1 μg). 0.1 μg dose could not be detected at the tumor. B) Mice injected with 3 μg (2 animals/group) or 100 μg of Pan-sNIR (1 animal/group) showed ~3.1-3.2X tumor signal than the liver 24 hrs after injection. Instead, same doses of Pan-800CW showed ~1.4-1.8X tumor signal over the liver.

**Fig. 6:** Cell uptake assay using NIR-fluorescent KUE-ligands I and II on PSMA-positive C4-2 and PSMA-negative PC3 cells.

## DETAILED DESCRIPTION OF THE INVENTION

The solution of the present invention is described in the following, exemplified in the appended examples, illustrated in the Figures and reflected in the claims.

## Definitions

It is noted that as used herein, the singular forms Z"a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

The term "and/or" wherever used herein includes the meaning of "and", "or" and "all or any other combination of the elements connected by said term".

For example, less than 20 means less than the number indicated. Similarly, more than or greater than means more than or greater than the indicated number, f.e. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

The term "alkyl" refers in context of the present invention to a monoradical of a saturated straight or branched hydrocarbon. Preferably, the alkyl group comprises from 1 to 10 carbon atoms, i.e., 1, 2, 3, 4, 5, 6, 7, 8, 9, or 10, carbon atoms, more preferably 1 to 8 carbon atoms, such as 1 to 6 or 1 to 4 carbon atoms. Exemplary alkyl groups include methyl, ethyl, propyl, isopropyl, butyl, iso-butyl, tert-butyl, n-pentyl, iso-pentyl, sec-pentyl, neo-pentyl, 1,2-dimethylpropyl, iso-amyl, n-hexyl, iso-hexyl, sec-hexyl, n-heptyl, iso-heptyl, n-octyl, 2-ethyl-hexyl, n-nonyl, n-decyl, and the like.

The term "cycloalkyl" or "cycloaliphatic" represents cyclic non-aromatic versions of "alkyl" and "alkenyl" with preferably 3 to 14 carbon atoms, such as 3 to 10 carbon atoms, i.e., 3, 4, 5, 6, 7, 8, 9, or 10 carbon atoms, more preferably 3 to 6 carbon atoms, even more preferably 6 carbon atoms. Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, and adamantyl. The term "cycloalkyl" is also meant to include bicyclic and tricyclic versions thereof. If bicyclic rings are formed it is preferred that the respective rings are connected to each other at two adjacent carbon atoms, however, alternatively the two rings are connected via the same carbon atom, i.e., they form a spiro ring system or they form "bridged" ring systems. Preferred examples of cycloalkyl include $C_3$-$C_8$-cycloalkyl, in particular cyclopropyl, cyclobutyl, cyclopentyl, cyclohexyl, cycloheptyl, cyclooctyl, spiro[3,3]heptyl, spiro[3,4]octyl, spiro[4,3]octyl, bicyclo[4.1.0]heptyl, bicyclo[3.2.0]heptyl, bicyclo[2.2.1]heptyl, bicyclo[2.2.2]octyl, bicyclo[5.1.0]octyl, and bicyclo[4.2.0]octyl.

The term "cycloalkylene" means a cycloalkyl group as defined above in which one hydrogen atom has been removed resulting in a diradical. The cycloalkylene may link two atoms or moieties via the same carbon atom (1,1-cycloalkylene, i.e., a geminal diradical) or via two carbon atoms (1,2-cycloalkylene).

The term "heterocyclyl" means a cycloalkyl group as defined above in which from 1, 2, 3, or 4 carbon atoms in the cycloalkyl group are replaced by heteroatoms of O, S, or N. Preferably, in each ring of the heterocyclyl group the maximum number of O atoms is 1, the maximum number of S atoms is 1, and the maximum total number of O and S atoms is 2. The term "heterocyclyl" is also meant to encompass partially or completely hydrogenated forms (such as dihydro, tetrahydro or perhydro forms) of the above-mentioned heteroaryl groups. Exemplary heterocyclyl groups include morpholino, isochromanyl, chromanyl, pyrrolidinyl, imidazolidinyl, pyrazolidinyl, piperidinyl, piperazinyl, indolinyl, isoindolinyl, di- and tetrahydrofuranyl, di- and tetrahydrothienyl, di- and tetrahydrooxazolyl, di- and tetrahydroisoxazolyl, di- and tetrahydrooxadiazolyl (1,2,5- and 1,2,3-), dihydropyrrolyl, dihydroimidazolyl, dihydropyrazolyl, di-and tetrahydrotriazolyl (1,2,3- and 1,2,4-), di- and tetrahydrothiazolyl, di- and tetrahydrothiazolyl, di- and tetrahydrothiadiazolyl (1,2,3- and 1,2,5-), di- and tetrahydropyridyl, di- and tetrahydropyrimidinyl, di- and tetrahydropyrazinyl, di- and tetrahydrotriazinyl (1,2,3-, 1,2,4-, and 1,3,5-), di- and tetrahydrobenzofuranyl (1- and 2-), di- and tetrahydroindolyl, di- and tetrahydroisoindolyl, di- and tetrahydrobenzothienyl (1- and 2), di- and tetrahydro-1H-indazolyl, di- and tetrahydrobenzimidazolyl, di- and tetrahydrobenzoxazolyl, di- and tetrahydroindoxazinyl, di- and tetrahydrobenzisoxazolyl, di- and tetrahydrobenzothiazolyl, di- and tetrahydrobenzisothiazolyl, di- and tetrahydrobenzotriazolyl, di- and tetrahydroquinolinyl, di- and tetrahydroisoquinolinyl, di- and tetrahydrobenzodiazinyl, di- and tetrahydroquinoxalinyl, di- and tetrahydroquinazolinyl, di- and tetrahydrobenzotriazinyl (1,2,3- and 1,2,4-), di- and tetrahydropyridazinyl, di- and tetrahydrophenoxazinyl, di- and tetrahydrothiazolopyridinyl (such as 4,5,6-7-tetrahydro[1,3]thiazolo[5,4-c]pyridinyl or 4,5,6-7-tetrahydro[1,3]thiazolo[4,5-c]pyridinyl, e.g., 4,5,6-7-tetrahydro[1,3]thiazolo[5,4-c]pyridin-2-yl or 4,5,6-7-tetrahydro[1,3]thiazolo[4,5-c]pyridin-2-yl), di- and tetrahydropyrrolothiazolyl (such as 5,6-dihydro-4H-pyrrolo[3,4-d][1,3]thiazolyl), di- and tetrahydrophenothiazinyl, di- and tetrahydroisobenzofuranyl, di- and tetrahydrochromenyl, di- and tetrahydroxanthenyl, di- and tetrahydrophenoxathiinyl, di- and tetrahydropyrrolizinyl, di- and tetrahydroindolizinyl, di- and tetrahydroindazolyl, di- and tetrahydropurinyl, di- and tetrahydroquinolizinyl, di- and tetrahydrophthalazinyl, di- and tetrahydronaphthyridinyl (1,5-, 1,6-, 1,7-, 1,8-, and 2,6-), di- and tetrahydrocinnolinyl, di- and tetrahydropteridinyl, di- and tetrahydrocarbazolyl, di- and tetrahydrophenanthridinyl, di- and tetrahydroacridinyl, di- and tetrahydroperimidinyl, di- and tetrahydrophenanthrolinyl (1,7-, 1,8-, 1,10-, 3,8-, and 4,7-), di- and tetrahydrophenazinyl, di- and tetrahydrooxazolopyridinyl, di- and tetrahydroisoxazolopyridinyl, di- and tetrahydropyrrolooxazolyl, and di- and tetrahydropyrrolopyrrolyl. Exemplary 5- or 6-memered heterocyclyl groups include morpholino, pyrrolidinyl, imidazolidinyl, pyrazolidinyl, piperidinyl, piperazinyl, di- and tetrahydrofuranyl, di- and tetrahydrothienyl, di- and tetrahydrooxazolyl, di-and tetrahydroisoxazolyl, di- and tetrahydrooxadiazolyl (1,2,5- and 1,2,3-), dihydropyrrolyl, dihydroimidazolyl, dihydropyrazolyl, di- and tetrahydrotriazolyl (1,2,3- and 1,2,4-), di- and tetrahydrothiazolyl, di- and tetrahydroisothiazolyl, di- and tetrahydrothiadiazolyl (1,2,3- and 1,2,5-), di- and tetrahydropyridyl, di- and tetrahydropyrimidinyl, di- and tetrahydropyrazinyl, di-and tetrahydrotriazinyl (1,2,3-, 1,2,4-, and 1,3,5-), and di- and tetrahydropyridazinyl.

The term "heterocycloalkylene" as used herein means a heterocyclyl group as defined above and in which one hydrogen atom has been removed from a nitrogen atom, the same carbon atom or different carbon atoms, resulting in a

diradical. The heterocycloalkylene may link two atoms or moieties via the same carbon atom or via two carbon atoms.

*Compounds*

The invention is directed to a compound according to formula (I),

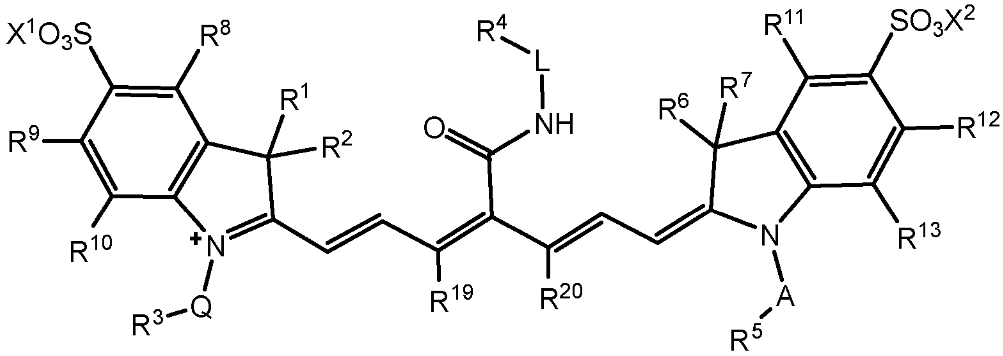

(I)

wherein

$R^1$, $R^2$, $R^6$, $R^7$, $R^{14}$, $R^{15}$, $R^{16}$, $R^{17}$, and $R^{18}$ are independently selected from the group consisting of H and $(C_1-C_6)$alkyl, preferably methyl.

$R^3$ is $-SO_3X^3$, or COOH.

$R^4$ is selected from the group consisting of -ethinyl, -azide,

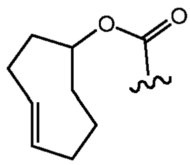 , 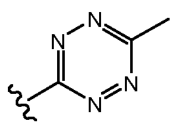 ,

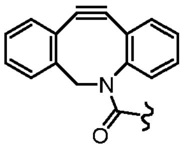 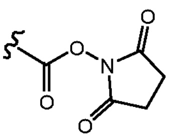 , 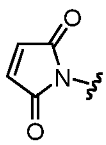 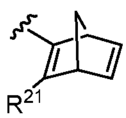 , 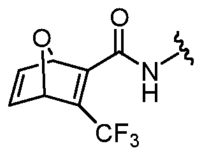

, -COOH, $NH_2$, Cl, Br, I, $-OSO_2CF_3$, $-COSO_2CH_3$, -OH, vinyl, -SH.

$R^5$ is $-SO_3X^4$.

$R^8$, $R^9$, $R^{10}$, $R^{11}$, $R^{12}$ and $R^{13}$ are independently selected from the group consisting of H, CN, $-COO(C_1-C_6)$alkyl, $-CONH(C_1-C_6)$alkyl, Cl, Br, I, **F,** $-CF_3$, $-O(C_1-C_6)$, $-NR^{14}R^{15}$, $-NHC(O)(C_1-C_6)$alkyl, $-NHC(O)NH(C_1-C_6)$alkyl),- $SO_3H$, $-SO_2NH_2$, $-(C_1-C_6)$alkyl, preferably H.

$R^{19}$, $R^{20}$ are independently selected from the group consisting of H, $-(C_1-C_6)$alkyl , $-O(C_1-C_6)$alkyl, Cl, **F,** Br, CN; C(O) $NH_2$, preferably H.

$R^{21}$ is H, $-(C_1-C_{10})$alkyl.

L is a group consisting of one or more and/or one or more of each of the elements selected from the group: $-(CH_2)_{n1}-$, $-(C_1-C_6)$cycloalkylene-, $-(C_1-C_6)$heterocycloalkylene-, -O-, - $NR^{16}$-, -NH-, -NHC(O)-, $-C(O)NR^{17}$-, -CH(OH)-, and $-CH(OR^{18})$-.

Preferably L is selected from the group consisting of $-CH_2(CH_2OCH_2)_{n1}CH_2-$, $-(CH_2)_{n2}-$, $-CH_2(CH_2NHCH_2)_{n1}CH_2-$, $-CH_2(CH_2NR^{16}CH_2)_{n1}CH_2-$, $-CH_2(CH_2OCH_2)_{n1}CH_2C(O)(CH_2)_{n4}-$, $-CH_2(CH_2NHCH_2)_{n1}CH_2C(O)(CH_2)_{n4}-$, $-CH_2(CH_2NR^{16}CH_2)_{n1}CH_2C(O)(CH_2)_{n4}-$.

$n^1$ is an integer between 1 to 30, preferably 1 to 20, more preferably 1 to 10.

$n^2$ is an integer between 1 to 10, preferably 1 to 6, more preferably 1 to 4.

$n^4$ is an integer between 1 to 10, preferably 1 to 6, more preferably 1 to 4.

$X^1$, $X^2$, $X^3$, and $X^4$ are independently selected from a cation, a proton or absent, preferably the cation is a monovalent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$;

If $X^1$, $X^2$, $X^3$, and/or $X^4$ are absent the underlying sulfonyl group is present as anion.

Q is $-(CH_2)_{n2}-$,

wherein optionally one or more of each H in $-(CH_2)_{n2}-$ may be replaced by $-(C_1-C_{10})$alkyl.

A is $-(CH_2)_{n3}-$,

wherein optionally one or more of each H in $-(CH_2)_{n3}-$ may be replaced by $-(C_1-C_{10})$alkyl.

$n^3$ is an integer between 1 to 10, preferably 1 to 6, more preferably 1 to 4.

In a further embodiment, in formula (I),

$R^1$, $R^2$, $R^6$, $R^7$ are Methyl.
$R^3$ is $-SO_3X^3$.
$R^4$ is selected from the group consisting of -ethinyl, -azide,

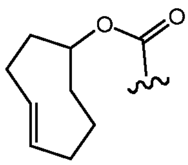 , 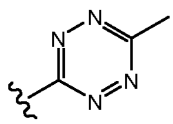 ,

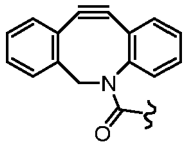 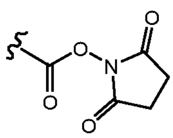 ., 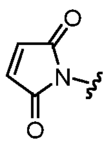 , 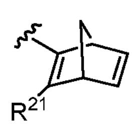 , 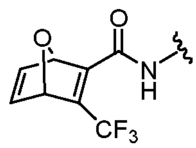

-COOH, and $NH_2$, preferably ethinyl, -azide,

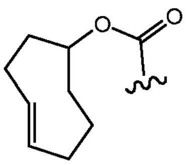 , 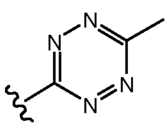 , 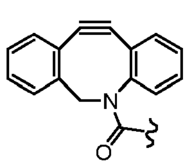 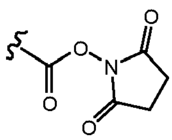 ;

$R^5$ is $-SO_3X^4$.
$R^8$, $R^9$, $R^{10}$, $R^{11}$, $R^{12}$ and $R^{13}$ are H.
$R^{16}$, $R^{17}$ and $R^{18}$ are independently selected from the group consisting of H and $(C_1-C_6)$alkyl, preferably methyl.

$R^{19}$ and $R^{20}$ are H.
$R^{21}$ is H, $-(C_1-C_{10})$alkyl.
L is a group consisting of one or more and/or one or more of each of the elements selected from the group: $-(CH_2)_{n1}-$, -O-, $-NR^{16}-$, -NHC(O)-, $-C(O)NR^{17}-$, -CH(OH)-, and - $CH(OR^{18})-$; preferably $-(CH_2)_{n1}-$, and -O-.
$n^1$ is an integer between 1 to 30, preferably 1 to 20, more preferably 1 to 10.
$X^1$, $X^2$, $X^3$, and $X^4$ are independently selected from a cation, a proton or absent, preferably the cation is a monovatent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$.
Q is $-(CH_2)_{n2}-$.
wherein optionally one or more of each H in $-(CH_2)_{n2}-$ may be replaced by $-(C_1-C_{10})$alkyl.
$n^2$ is an integer between 1 to 10, preferably 1 to 6, more preferably 1 to 4.
Y is $-(CH_2)_{n3}-$,
wherein optionally one or more of each H in $-(CH_2)_{n3}-$ may be replaced by $-(C_1-C_{10})$alkyl;
$n^3$ is an integer between 1 to 10, preferably 1 to 6, more preferably 1 to 4.

In a further embodiment, the compound according to formula (I) is selected from the group consisting of

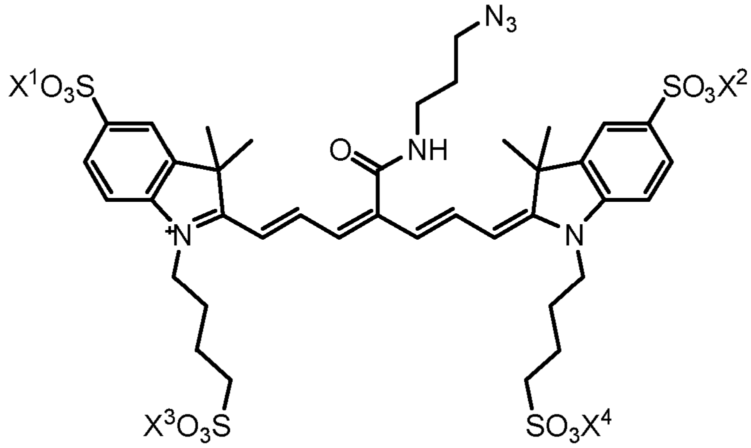

,

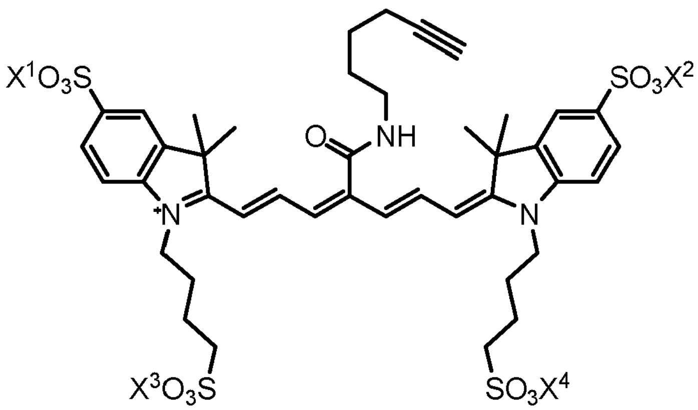

.

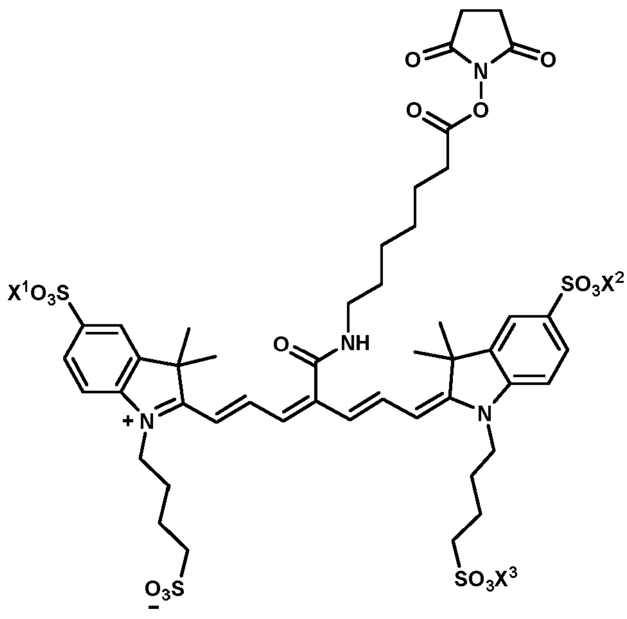

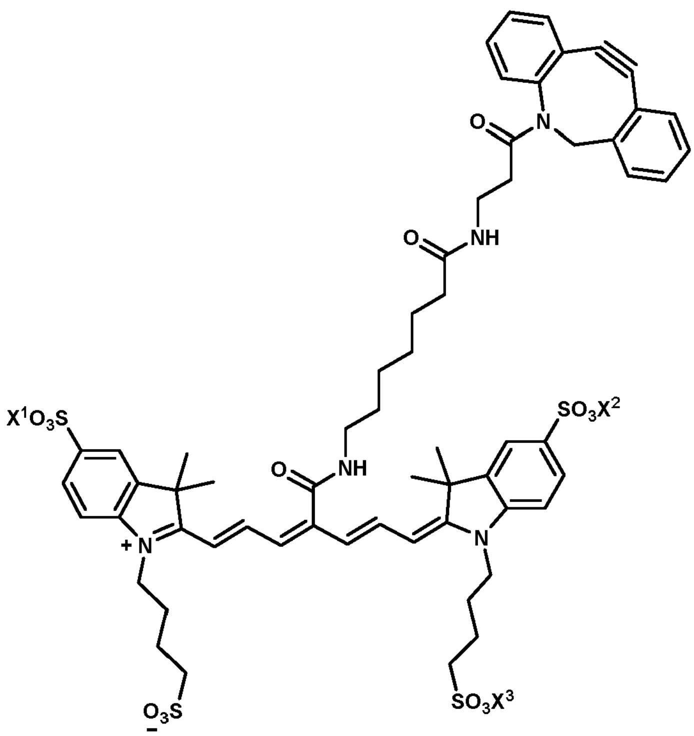

$X^1$, $X^2$, $X^3$, and $X^4$ are independently selected from a cation, a proton or absent, preferably the cation is a monovatent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$.

The compounds according to formula (I) may be generally prepared by following the approach of Štacková et al. by opening of Zincke Salts (3). An approach for the synthesis of compounds according to formula (I) is also outlined in more detail in the examples.

The compounds and dyes according to formula (I) may be further linked via a suitable linker, known to the person skilled in the art, for example with a biological active molecule such as protein, peptide, small molecule, antibody, oligonucleotide, nanobody, or polymer, resulting in compounds according to formula (II).

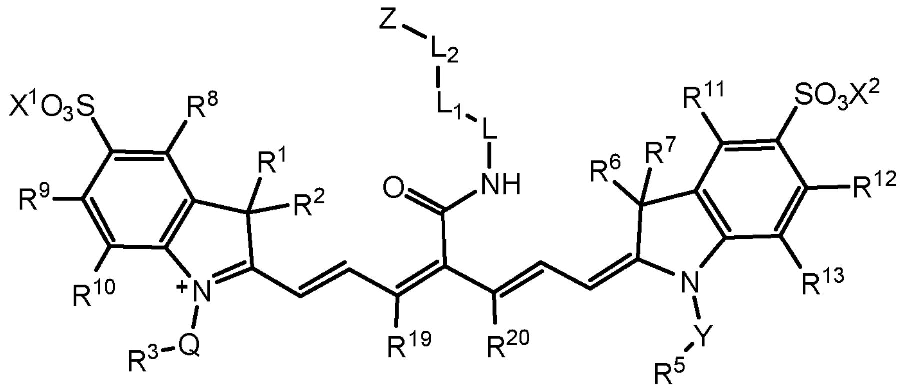

(II)

$R^1$, $R^2$, $R^6$, $R^7$, $R^{14}$, $R^{15}$, $R^{16}$, $R^{17}$, and $R^{18}$ are independently selected from the group consisting of H and $(C_1\text{-}C_6)$alkyl, preferably methyl.

$R^3$ is $-SO_3X^3$, or COOH.

Z is a protein, peptide, small molecule, antibody, oligonucleotide, nanobody, mono- or polyvalent saccharide or polymer, preferably a protein, a peptide, a small molecule, an antibody or a nanobody, preferably a protein, a peptide, an antibody or a nanobody.

Z may be an antibody such as Panitumumab (CAS-Nummer: 339177-26-3).

The protein or peptide may comprise proteinogenic amino acids and non-proteinogenic amino acids. Preferably the protein comprises only proteinogenic amino acids, selected from the group consisting of alanine, arginine, asparagine, asparaginic acid, cysteine, glutamine, glutaminic acid, glycine, histidine, isoleucine, leucine, lysine, methionine, phenylalanine, proline, serine, threonine, tryptophane, tryrosine, and valine. The peptide may be cyclic or acyclic.

Preferably, the protein has a molecular weight from 5000 Da to 400000 Da, more preferably 50000 to 300000 Da. Preferably, the protein is selected from the group consisting of leptin, insulin.

Preferably, the peptide comprises 2 to 45 amino acids, more preferably 2 to 20 amino acids as described above. Preferably, the peptide is selected from the group consisting of Octreotide, Octreotate, nerve binding peptide 41 (NP41), glucagon and derivatives thereof, glucagon like peptide 1 (GLP-1) and derivatives thereof like eGLP-1, Exendin-4, lixisenatide, octreotide analogues TOC ($[Tyr^3]$Octreotide), and TATE ($[Tyr^3, Thr^8]$Octreotide), methanobactin OB3b.

A small molecule is a molecule with a molecular weight below 900 g/mol, preferably a molecule that exhibits a pharmaceutical activity. Preferably, the small molecule is selected from the group consisting of prostate-specific membrane antigen (PSMA), PSMA binding ligands like Lysine-urea-glutamate (KUE),

An antibody also known as an immunoglobulin, is a large, Y-shaped protein used by the immune system to identify and neutralize foreign objects such as pathogenic bacteria and viruses. The antibody recognizes a unique molecule of the pathogen, called an antigen. Preferably, the antibody has a molecular weight of 10000 Da to 400000 Da, more preferably 50000 to 300000 Da. Preferably, the antibody is selected from the group consisting of EGFR targeting antibodies such as cetuximab, and panitutumab, HER2 targeting antibodies such as trastuzumab, carcino-embryonic antigen (CEA) targeting antibodies, and vascular endothelial growth factor receptor (VEGFR) targeting antibodies such as bevatuzumab.

An oligonucleotide is a short single or double stranded DNA or RNA molecule of preferably 10-100 nucleotides, such as an aptamer, small interfering RNA (siRNA), or short hairpin RNA (shRNA).

Nanobodies are antibodies which comprise just one domain compared to a regular antibody. This domain usually corresponds to the variable region of a conventional antibody. Nanobodies preferably have a size of 10 to 30 kDA, more preferably 12 to 20 kDa.

Saccharides are carbohydrates preferably mannose, glucose, galactose, N-acetyl galactosamine, galactosamine, gluconic acid, glucuronic acid or sialic acid, which can be monovalent or polyvalent, preferably coupled to each other by glycosidic bonds or employing a Tris(hydroxymethyl)aminomethane (TRIS) residue.

The Polymer may be any polymer known to the person skilled in the art which suitable for solving the require task, preferably oligosaccharide based polymers like dextran, chitosan, pullulan, alginate, lentinane, hyaluronic acid and combinations thereof, poly-lactic acid, poly-lysine, polyurethanes and polyhydroxyethylmethacrylates.

$R^5$ is $-SO_3X^4$.

$R^8$, $R^9$, $R^{10}$, $R^{11}$, $R^{12}$ and $R^{13}$ are independently selected from the group consisting of H, CN, $-COO(C_1-C_6)$alkyl, $-CONH(C_1-C_6)$alkyl, Cl, Br, I, F, $-CF_3$, $-O(C_{1-}C_6)$, $-NR^{14}R^{15}$, $-NHC(O)(C_1-C_6)$alkyl, $-NHC(O)NH(C_1-C_6)$alkyl), $-SO_3H$, $-SO_2NH_2$, $-(C_1-C_6)$alkyl, preferably H.

$R^{19}$, $R^{20}$ are independently selected from the group consisting of H, $-(C_1-C_6)$alkyl , $-O(C_1-C_6)$alkyl, Cl, F, Br, CN; C(O) $NH_2$, preferably H.

$R^{21}$ is H, $-(C_1-C_{10})$alkyl.

$X^1$, $X^2$, $X^3$, and $X^4$ are independently selected from a cation, a proton or absent, preferably the cation is a monovalent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$.

Q is $-(CH_2)_{n2}-$,

wherein optionally one or more of each H in $-(CH_2)_{n2}-$ may be replaced by $-(C_1-C_{10})$alkyl.

$n^2$ is an integer between 1 to 10, preferably 1 to 6, more preferably 1 to 4.

Y is $-(CH_2)_{n3}-$,

wherein optionally one or more of each H in $-(CH_2)_{n2}-$ may be replaced by $-(C_1-C_{10})$alkyl.

$n^3$ is an integer between 1 to 10, preferably 1 to 6, more preferably 1 to 4.

L and $L_2$ are each independently a group consisting of one or more and/or one or more of each of the elements selected from the group: $-(CH_2)_{n1}-$, $-(C_1-C_6)$cycloalkylene-, $-(C_1-C_6)$heterocycloalkylene-, -O-, $-NR^{16}-$, -NH-, -NHC(O)-, -C(O) $NR^{17}-$, -CH(OH)-, and $-CH(OR^{18})-$.

Preferably L and $L_2$ are independently selected from the group consisting of $CH_2(CH_2OCH_2)_{n1}CH_2-$, $-(CH_2)_{n2}-$, $-CH_2(CH_2NHCH_2)_{n1}CH_2-$, $-CH_2(CH_2NR^{16}CH_2)_{n1}CH_2-$, - $CH_2(CH_2OCH_2)_{n1}CH_2C(O)(CH_2)_{n5}-$, $-CH_2(CH_2NHCH_2)_{n1}CH_2C(O)(CH_2)_{n5}-$, - $CH_2(CH_2NR^{16}CH_2)_{n1}CH_2C(O)(CH_2)_{n5}-$.

$n^1$ is an integer between 1 to 30, preferably 1 to 20, more preferably 1 to 10.

$n^2$ is an integer between 1 to 10, preferably 1 to 6, more preferably 1 to 4.

$n^5$ is an integer between 1 to 10, preferably 1 to 6, more preferably 1 to 4.

n' is an integer between 1 to 30, preferably 1 to 20, more preferably 1 to 10.

$L_1$ is selected from the group consisting of ,

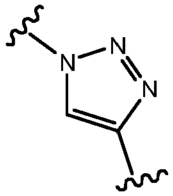 , 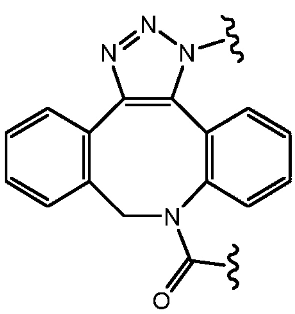 , 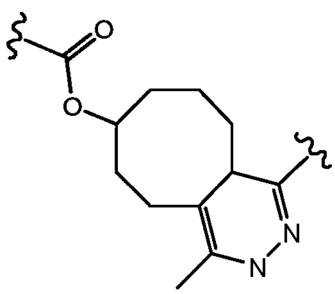 , 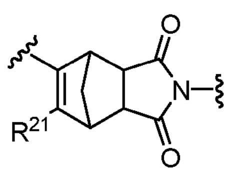 , ,

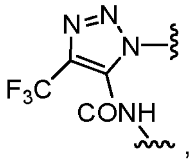, 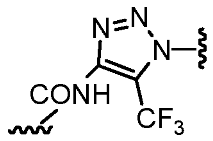,

and -CO-, preferably

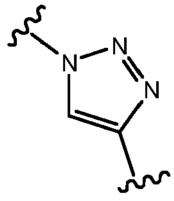, –CO-.

$R^{21}$ is H, -$(C_1\text{-}C_{10})$alkyl.

In a further embodiment, in formula (II)

$R^1$, $R^2$, $R^6$, $R^7$are Methyl.
$R^3$ is -$SO_3X^3$.
$R^5$ is -$SO_3X^4$.
$R^8$, $R^9$, $R^{10}$, $R^{11}$, $R^{12}$ and $R^{13}$ are H.
$R^{16}$, $R^{17,}$ and $R^{18}$ are independently selected from the group consisting of H and $(C_1\text{-}C_6)$alkyl, preferably methyl.
$R^{19}$ and $R^{20}$ are H.
$X^1$, $X^2$, $X^3$, and $X^4$ are independently selected from a cation, a proton or absent, preferably the cation is a monovalent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$.
Q is -$(CH_2)_{n2}$-,
wherein optionally one or more of each H in -$(CH_2)_{n2}$- may be replaced by -$(C_1\text{-}C_{10})$alkyl.
$n^2$ is an integer between 1 to 10, preferably 1 to 6, more preferably 1 to 4.
Y is -$(CH_2)_{n3}$-,
wherein optionally one or more of each H in -$(CH_2)_{n3}$- may be replaced by -$(C_1\text{-}C_{10})$alkyl.
$n^3$ is an integer between 1 to 10, preferably 1 to 6, more preferably 1 to 4.
L and $L_2$ are each independently a group consisting of one or more and/or one or more of each of the elements selected from the group: -$(CH_2)_{n1}$-, -$(C_1\text{-}C_6)$cycloalkylene-, -$(C_1\text{-}C_6)$heterocycloalkylene-, -O-, -$NR^{16}$-, -NH-, -NHC(O)-, -C(O)$NR^{17}$-, -CH(OH)-, and -CH($OR^{18}$)-.
Preferably L and $L_2$ are independently selected from the group consisting of $CH_2(CH_2OCH_2)_{n1}CH_2$-, -$(CH_2)_{n2}$-, -$CH_2(CH_2NHCH_2)_{n1}CH_2$-, -$CH_2(CH_2NR^{16}CH_2)_{n1}CH_2$-, - $CH_2(CH_2OCH_2)_{n1}CH_2C(O)(CH_2)_{n5}$-, -$CH_2(CH_2NHCH_2)_{n1}CH_2C(O)(CH_2)_{n5}$-, - $CH_2(CH_2NR^{16}CH_2)_{n1}CH_2C(O)(CH_2)_{n5}$-.
$n^1$ is an integer between 1 to 30, preferably 1 to 20, more preferably 1 to 10.
$n^4$ is an integer between 1 to 10, preferably 1 to 6, more preferably 1 to 4.
$L_1$ is selected from the group consisting of

, 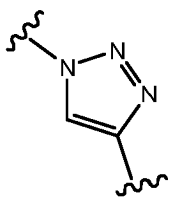, 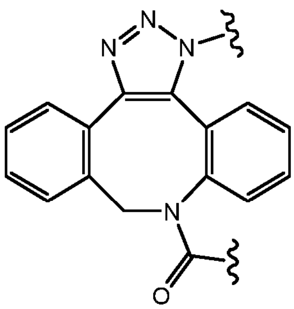, 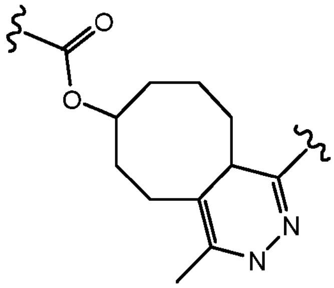, 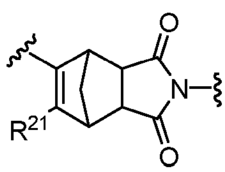,

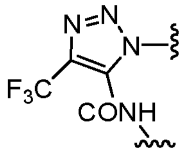, 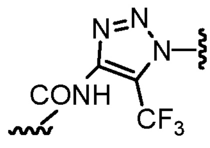, –CO-, `

$R^{21}$ is H, -($C_1$-$C_{10}$)alkyl;

In a further embodiment, the compound according to formula (II) is selected from the group consisting of

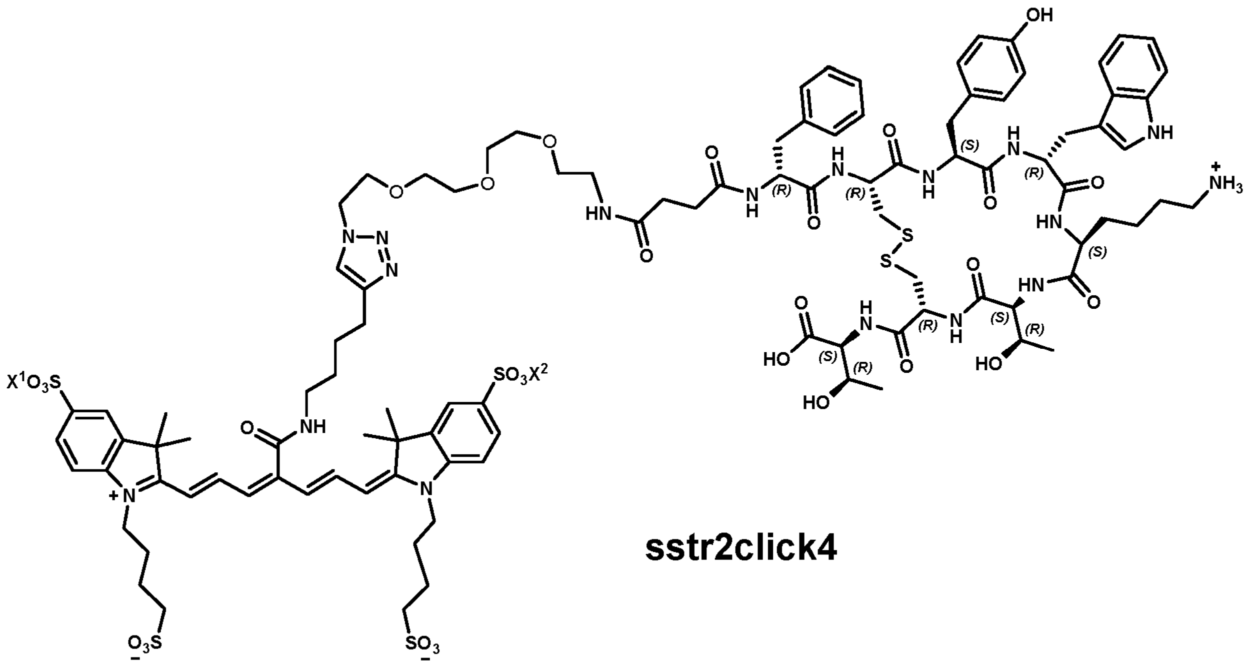

**sstr2click4**

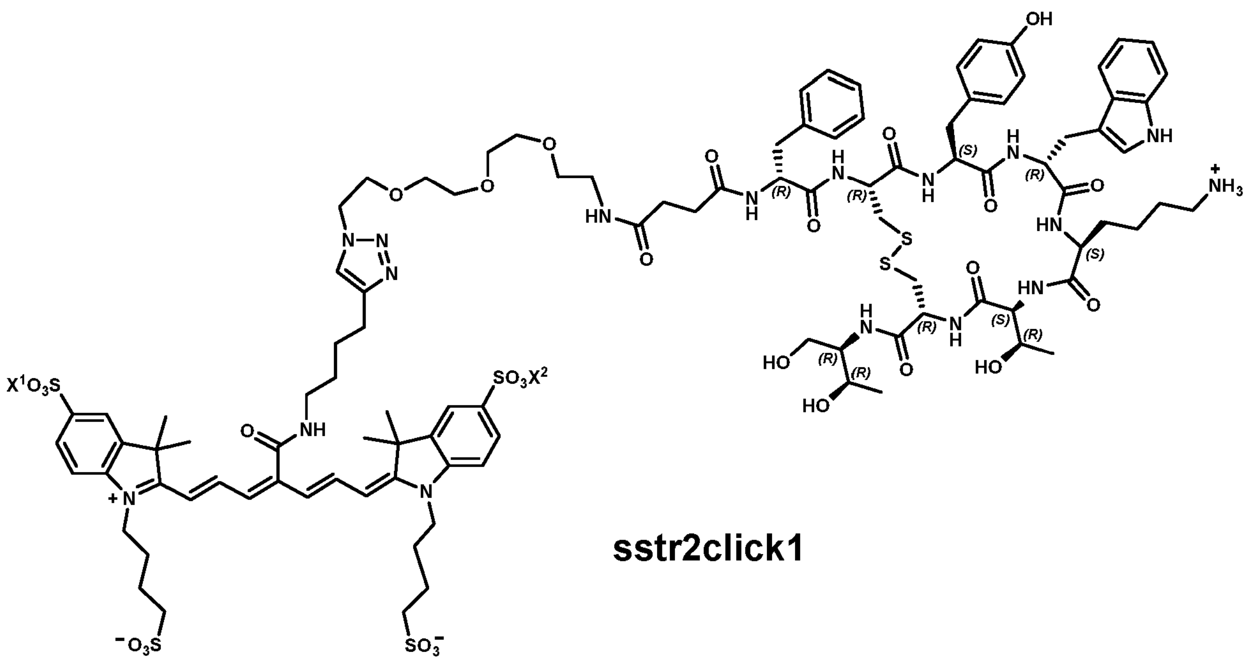

**sstr2click1**

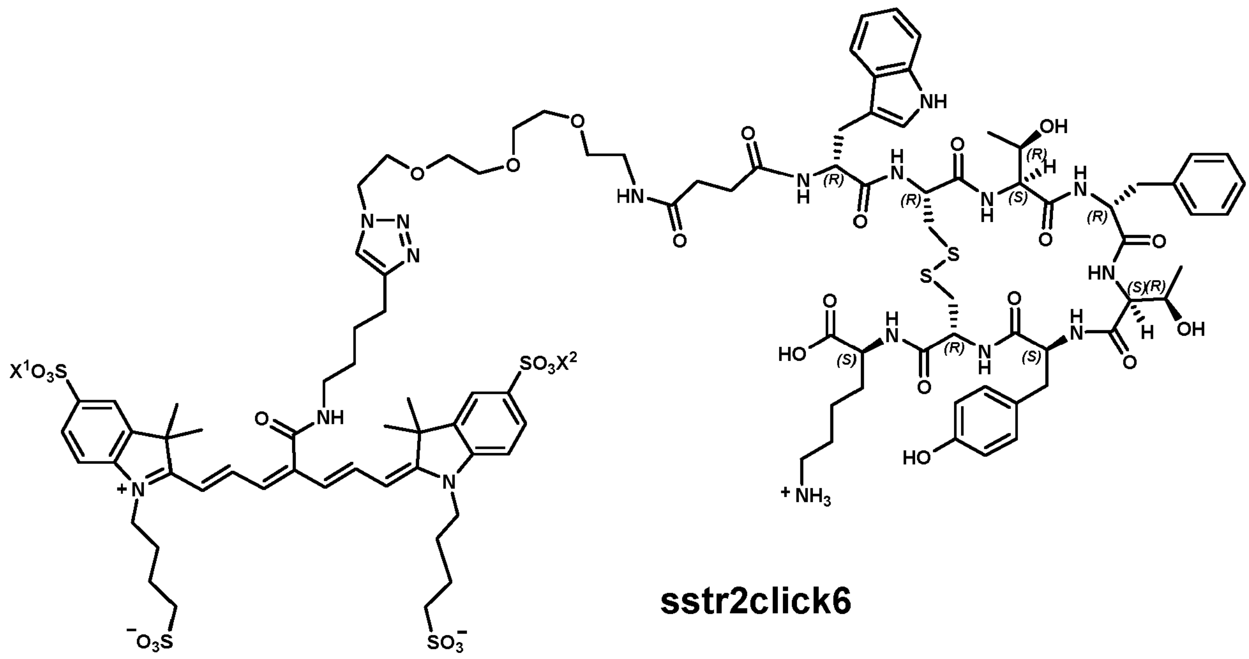

sstr2click6

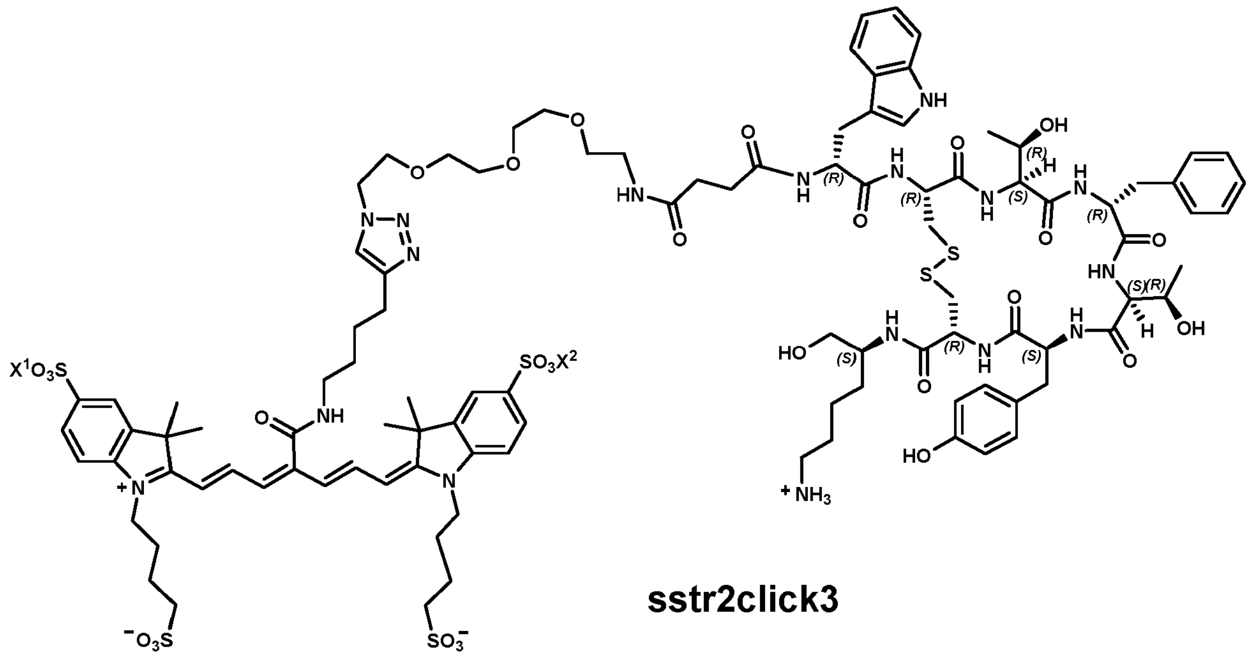

sstr2click3

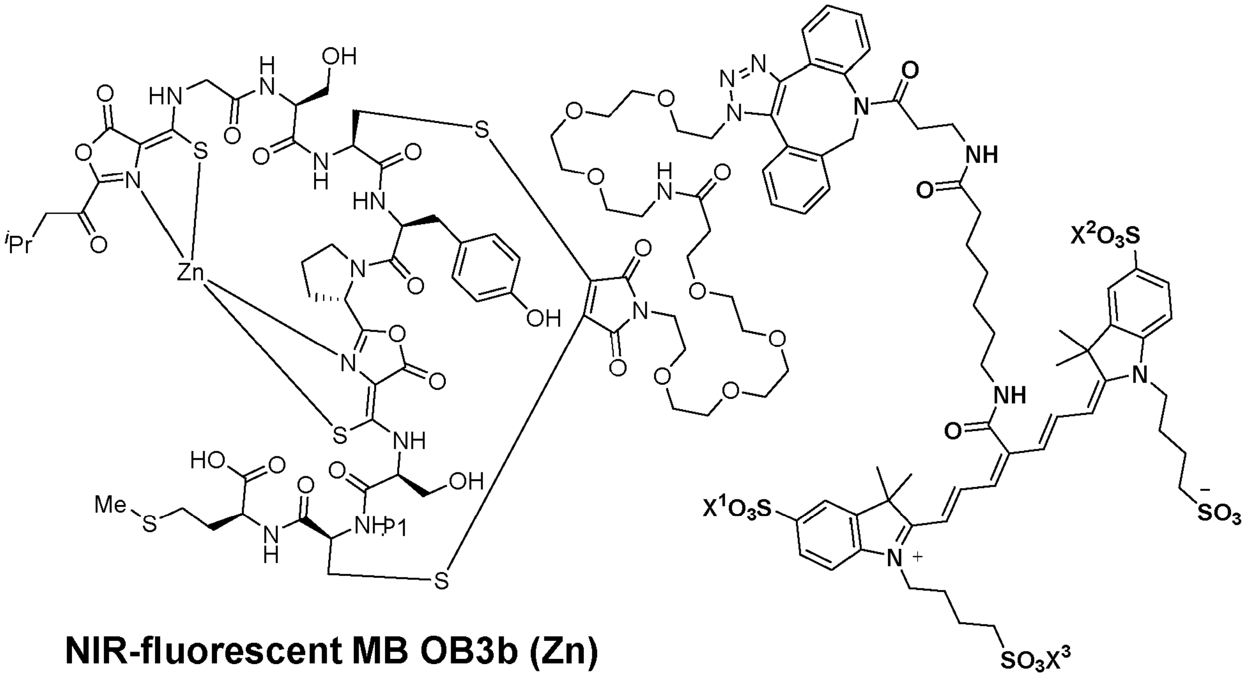

NIR-fluorescent MB OB3b (Zn)

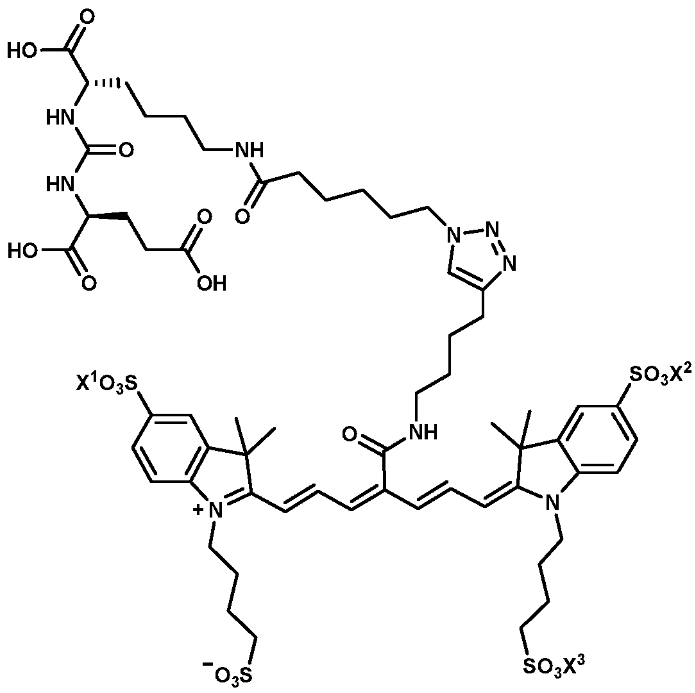

NIR-fluorescent KUE Ligand I

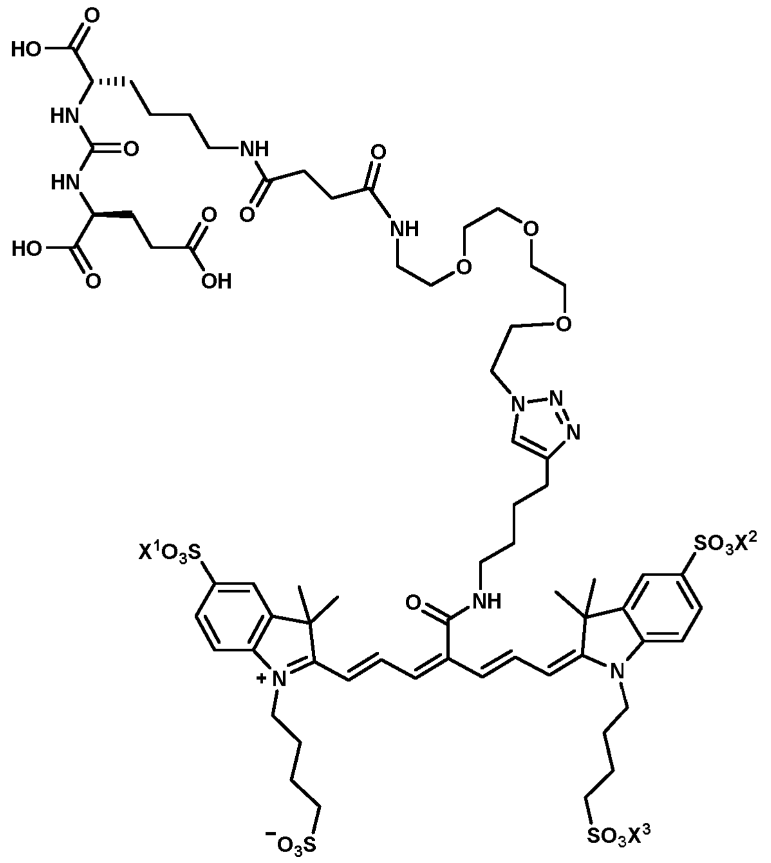

**NIR-fluorescent KUE Ligand II**

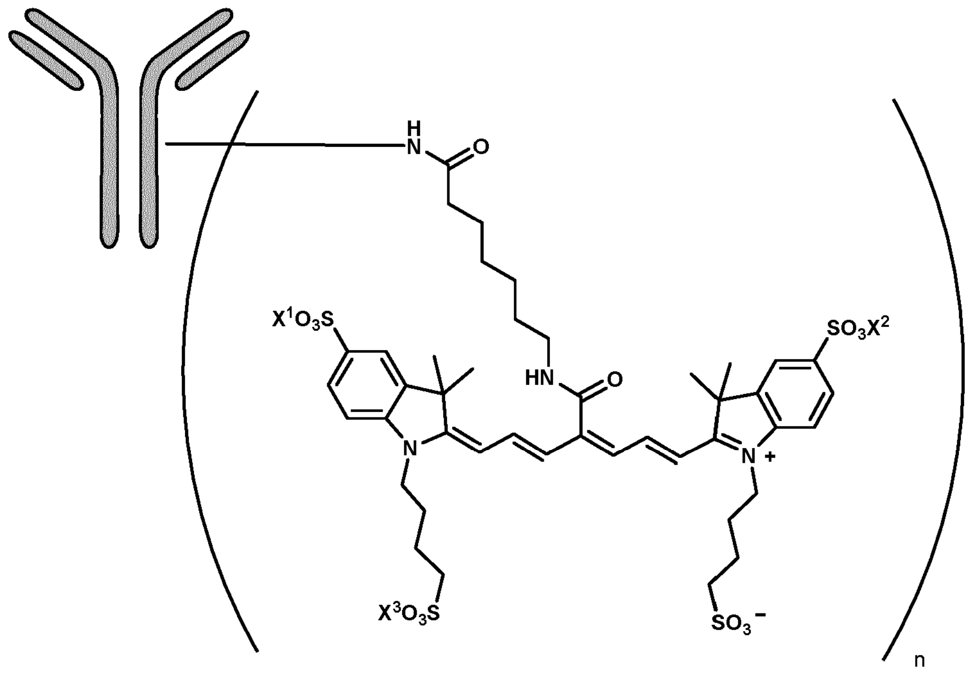

**NIR-fluorescent Panitumumab**

Wherein the antibody shown symbolically in the formula is Panitumumab.

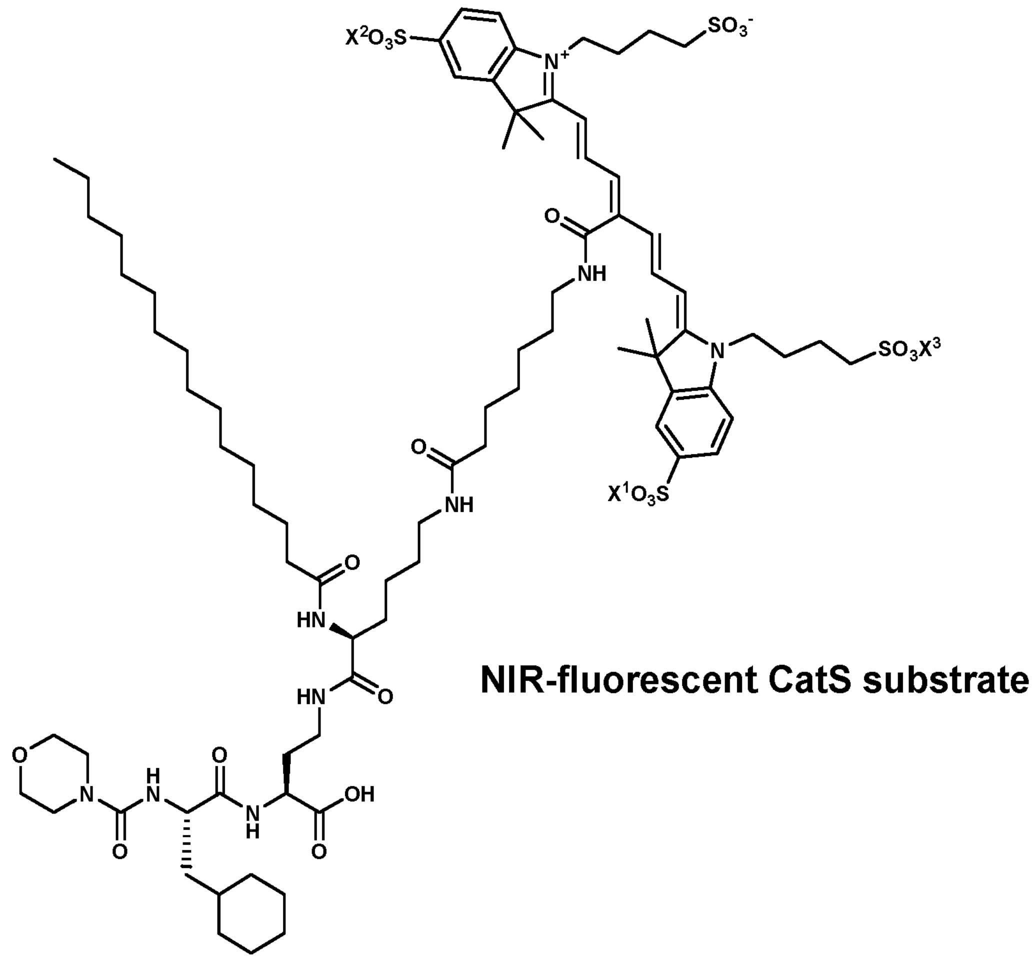

NIR-fluorescent CatS substrate

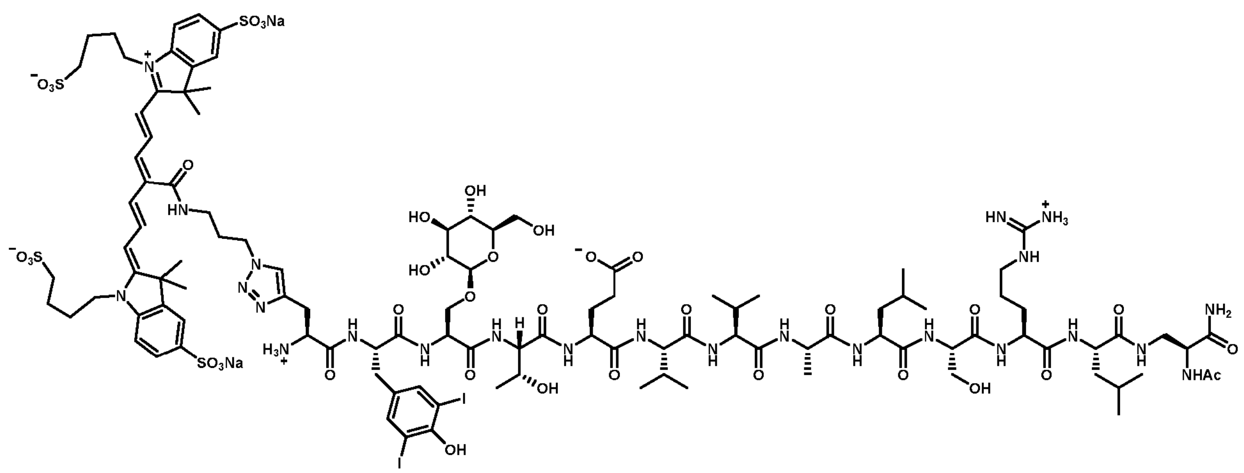

NIR-fluorecent leptin-receptor-agonist-I

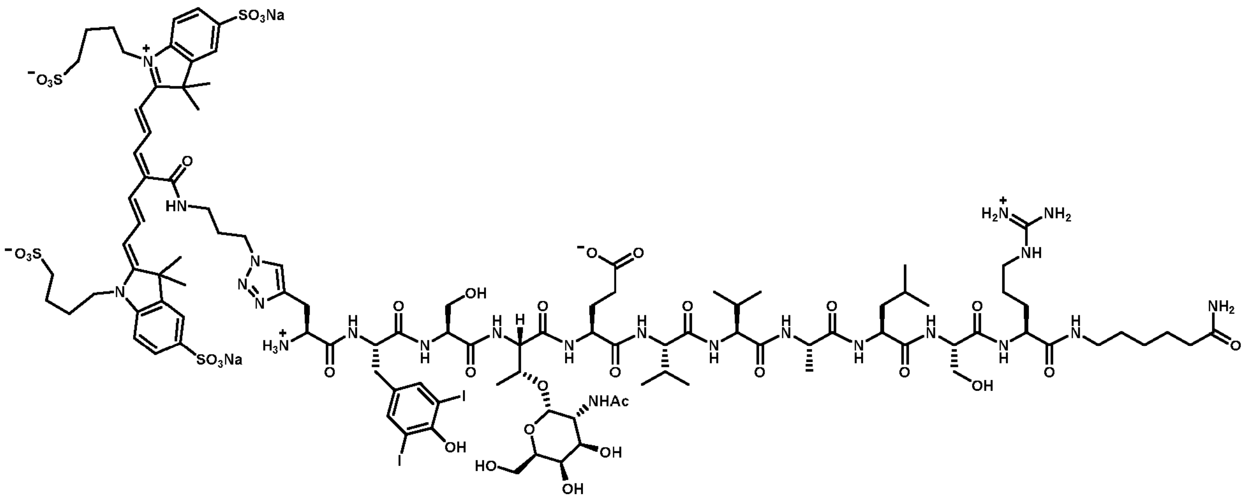

**NIR-fluorecent leptin-receptor-agonist-II**

*Applications*

The compounds of the present invention as described above, preferably according to formula (I) may be used as fluorescent dye.

Preferably, the peak emission in PBS of the compounds as described above is between 400 nm to 1200 nm, ,more preferably from 500 nm to 900 nm, most preferably from 790 to 820 nm.

Preferably, the absorption crosssection in PBS of the compounds as described above is > 5,000 1/(M*cm), more preferably > 50,000 1/(M*cm), most preferably >200,000 1/(M*cm).

Preferably, the peak absorption in PBS of the compounds as described above is from 400 nm to 1200 nm, more preferably from 600 nm to 1000 nm, most preferably from 750 nm to 850 nm.

Moreover, the compounds of the present invention as described above, preferably according to formula (II) may be used *in vivo* or *ex vivo* preferably in *vivo* as contrast agent. Thus, the invention is also directed to the compounds according to formula (I) or (II) preferably according to formula (II) for use as contrast agent *in vivo.*

Further, the compounds as described above, preferably according to formula (II), are for use in diagnosis or for intraoperative surgery. Preferably, wherein the indication to be diagnosed or treated by intraoperative surgery is selected from the group consisting of inflammatory diseases, infectious diseases and cancer.

Preferably, the inflammatory disease is selected from the group consisting of otitis media, allergic rhinitis and chronic rhinosinusitis, chronic inflammation of tonsils, adenoids, and laryngitis.

Preferably, the infectious disease is selected from the group consisting of implant infection (e.g. hip, dental), secondary infections of the inner ear, e.g. accompanying cholesteatoma. This comprises infections caused by gram positive or gram negative bacteria, responding to antibiotic treatment, resistant or multi-drug resistant, in particular by ESKAPE strains *Enterococcus faecium, Staphylococcus aureus, Klebsiella pneumoniae, Acinetobacter baumannii, Pseudomonas aeruginosa,* and *Enterobacter spp..*

Preferably, diagnosing of cancer or treating cancers by intraoperative surgery relates to visualizing the primary tumor and/or metastatic lesions in situ during surgery selected from the group meningioma, glioblastoma, NSCLC, and pancreatic cancer.

A better understanding of the present invention and of its advantages will be had from the following examples, offered for illustrative purposes only.

## EXAMPLES OF THE INVENTION

### 1.1 Material und Methods

#### General Notes

Solvents and reagents were purchased from ACROS, ALFA AESAR, CARL ROTH, COMBI-BLOCKS, ENAMINE, FISHER SCIENTIFIC, HONEYWELL, MERCK, SIGMA ALDRICH, TCI CHEMICALS and VWR and used as received. Moisture- and air-sensitive reactions were performed in oven-dried glassware under argon atmosphere with

anhydrous solvents. For oxygen-sensitive reactions, the solvent was degassed by passing an argon stream for ten minutes or by freeze-pump-thaw method. Reactions with light-sensitive substances were carried out in the absence of light as far as possible. Aqueous solutions used were based on demineralized water and were saturated unless otherwise described.

Solvent was removed under reduced pressure with a BÜCHI Rotavapor with a VACUUBRAND CVC 3000 vacuum pump. Aqueous solvent was removed by lyophilization with a CHRIST Alpha 1-4 LDplus Freeze Dryer with a VACUUBRAND RZ6 vacuum pump. Bath sonication was performed using a BANDELIN Sonorex.

**Thin Layer Chromatography (TLC)**

Thin layer chromatography was performed using MERK'S silica gel-coated glass plates with fluorescence indicator DC Kieselgel 60 $F_{254}$. Visualization was performed using UV light ($\lambda$ = 254 or 366 nm) and/or by treating the plate with suitable stain (potassium permanganate, ceric ammonium molybdate, ninhydrin and bromcresol green) and subsequent heating.

**Column Chromatography**

Flash column Chromatography was performed on a BÜCHI Reveleris® Prep system with UV and evaporative light scattering detector at variable flow rate. Silica, amino-, C8- and C18- modified silica cartridges manufactured by BÜCHI, AXEL SEMRAU and MACHERY-NAGEL were used. The particle size was 40-63 $\mu$m irregular or 20-40 $\mu$m spherical. Used solvents and gradients are listed in **1.2 Synthetic Procedures.**

**NMR Spectroscopy**

$^{1}$H and $^{13}$C NMR spectroscopic measurements were performed on BRUKER'S Ascend-400, Avance-400, Avance-500 and Avance-600 at 25 °C. Calibration was performed to the residual proton signal of the deuterated solvent. The chemical shift $\delta$ is given in parts per million (ppm) and the coupling constant *J* in Hz. For the multiplicities the following abbreviations are used: singlet (s), doublet (d), triplet (t), quartet (q), quintet (quint.), broad singlet (bs), and multiplet (m). For a complete characterization, additional $^{1}$H-$^{1}$H correlation spectra (COSY) and $^{1}$H- $^{13}$C correlation spectra (HMBC, HSQC) were acquired. The numbering of the carbon atoms corresponds to an arbitrary determination and does not follow the IUPAC rules.

**Infrared Spectroscopy**

IR spectra were acquired on a SHIMADZU IRAffinity-1S.

**Mass Spectrometry**

Analytical LC-MS measurements of molecules with M < 1000 g/mol were performed using a WATERS Acquity UPLC H-Class System utilizing a QDa Mass Detector, a PDA e$\lambda$ Detector and a UPLC BEH C18-column (50 x 2.1 mm, particle size: 1.7 $\mu$m). Runs **(05-95_3.5 min)** employed a standard gradient as listed in **Table 1** at a flow rate of 0.8 mL/min. Electrospray ionization (ESI) was used and M/z range of 50-1250 was detected. WATERS MassLynx 4.1 software was used for analysis.

Table 1: **Solvent gradient used for WATERS Acquity UPLC H-Class System.**

| time [min] | $H_2O$ + 0.1% FA | MeCN + 0.1% FA |
|---|---|---|
| 0.00 | 95% | 5% |
| 1.00 | 5% | 95% |
| 2.25 | 5% | 95% |
| 2.27 | 95% | 5% |
| 3.50 | 95% | 5% |

Analytical LC-MS measurements of molecules with M >1000 g/mol were performed using a WATERS 2795 Separation Module connected to a WATERS LCT Premier Mass Spectrometer, a HP 1050 UV multiwavelength detector and a PHENOMENEX Aeris™ Widepore XB-C18 (50 x 2.1 mm, particle size: 3.6 $\mu$m) column. Runs **(01-90_5 min)** employed a standard gradient as listed in **Table 2** at a flow rate of 0.6 mL/min. Electrospray ionization (ESI) was used for

ionization and M/z range of 100-2500 was detected. WATERS MassLynx 4.1 software was used for analysis.

Table 2: **Solvent gradient used for composite HPLC system.**

| time [min] | $H_2O$ + 0.1% FA | MeCN + 0.1% FA |
|---|---|---|
| 0.00 | 99% | 1% |
| 4.00 | 10% | 90% |
| 5.00 | 10% | 90% |

Alternatively, analytical LC-MS measurements of molecules with M >1000 g/mol were performed using a THERMO FISHER SCIENTIFIC UltiMate 3000 UHPLC System utilizing a ISQ EC Mass Spectrometer, a Variable Wavelength Detector and a MaCHERY-NAGEL Nucleodur PFP column (50 x 2.0 mm, particle size: 3.0 µm) with the corresponding precolumn cartridge. Runs **(05-95_13 min)** followed on an equilibration (5 min) and employed a standard gradient as listed in **Table 3** at a flow rate of 0.3 mL/min. Electrospray ionization (ESI) was used for ionization and M/z range of 100-1250 was detected. THERMO FISHER SCIENTIFIC's Chromeleon CDS software was used for analysis.

Table 3: **Solvent gradient used for the THERMO FISHER SCIENTIFIC UltiMate 30000 UHPLC System.**

| time [min] | $H_2O$ + 0.1% FA | MeCN + 0.1% FA |
|---|---|---|
| -5 | 5% | 95% |
| 0 | 5% | 95% |
| 8 | 95% | 5% |
| 13 | 95% | 5% |

**Microwave Assisted Synthesis**

Reactions under microwave irradiation were performed using the BIOTAGE Initiator+.

**Solid Phase Peptide Synthesis**

Peptide synthesis was either performed automatically or manually. (Amino) acids used are listed in **Table 4.** L-amino acids are labeled with capital and D-amino acids with small letters.

Table 4: **Overview of (amino) acids used for solid phase peptide synthesis.**

| (Amino) acid | Three / single letter code | Reagent |
|---|---|---|
| Alanine | Ala / A | Fmoc-Ala-OH |
| Arginine | Arg / R | Fmoc-Arg(Pbf)-OH |
| Asparagine | Asn / N | Fmoc-Asn(Trt)-OH |
| Aspartic acid | Asp / D | Fmoc-Arg(OtBu)-OH |
| Cysteine | Cys / C | Fmoc-Cys(Trt)-OH |
| Glutamine | Gln / Q | Fmoc-Gln(Trt)-OH |
| Glutamic acid | Glu / E | Fmoc-Glu(OtBu)-OH |
| Glycine | Gly / G | Fmoc-Gly-OH |
| Histidine | His / H | Fmoc-His(Trt)-OH |
| Isoleucine | Ile / I | Fmoc-Ile-OH |
| Leucine | Leu / L | Fmoc-Leu-OH |
| Lysine | Lys / K | Fmoc-Lys(Boc)-OH |
| Methionine | Met / M | Fmoc-Met-OH |
| Phenylalanine | Phe / F | Fmoc-Phe-OH |
| Proline | Pro / P | Fmoc-Pro-OH |
| Serine | Ser / S | Fmoc-Ser(tBu)-OH |
| Threonine | Thr / T | Fmoc-Thr(tBu)-OH |
| Tryptophan | Trp / W | Fmoc-Trp(Boc)-OH |
| Tyrosine | Tyr / Y | Fmoc-Tyr(tBu)-OH |

(continued)

| (Amino) acid | Three / single letter code | Reagent |
|---|---|---|
| Valine | Val / V | Fmoc-Val-OH |
| Propargylglycine | Pra / - | Fmoc-Pra-OH |
| Threonol | - | Fmoc-Thr(tBu)-ol |
| Lysinol | - | Fmoc-Lys(Boc)-ol |
| 6-Azidohexanoic acid | - | - |
| $N_3$-PEG(3)-$CO_2$H | - | - |

Automated SSPS

Automated peptide synthesis was performed on a CEM Liberty BlueTM Automated Mirowave Peptide Synthesizer following a standard Fmoc protocol at 0.1 mmol scale. MERCK's Rink Amide AM resin LL (100 200 mesh; loading 0.40 mmol/g), BACHEM's 2-chlorotrityl chloride resin (200-400 mesh, loading 1.5-1.9 mmol/g) and MERCK's DHP HM resin (100 - 200 mesh, 1.17 mmol/g) were used as solid support. In some cases, manually preloaded resin were used. Therefore, the following procedure was followed:

- 2-chlorotrityl chloride resin
  - Swelling ($CH_2Cl_2$, 0.5 h)
  - Activation (dest. AcCl (1 mL/g resin), toluene, 60°C, 3 h, Ar)
  - Loading (2.0 eq protected aminoacid, 5.0 eq dest. DIPEA, $CH_2Cl_2$, rt, 2.0 h)
  - 2 x Capping (5 mL $CH_2Cl_2$/MeOH/DIPEA (8/1.5/0.5), rt, 0.5 h)
  - Drying to constant mass in order to determine the loading efficacy
- DHP HM resin
  - ◦ Swelling (DCE, 1 h)
  - ◦ Loading (3.5 eq protected aminoalcohol, 1.8 eq PPTS, DCE, 80°C, on, Ar)
  - ◦ Capping (addition of pyridine, 15 min, rt)
  - ◦ Drying to constant mass in order to determine the loading efficacy

Stock solutions of (amino) acids (0.2 M in DMF), DIC (0.5 M in DMF), Oxyma® (1 M in DMF), and a deprotection solution (10% (w/v) piperazine in EtOH:NMP (1:9) or 10% (v/v) piperidine in DMF) were prepared and provided to the instrument. Couplings were performed with a fivefold excess (compared to the resin) of (amino) acids as well as coupling and deprotection reagents. Instrument settings for deprotection and coupling are summarized in **Table 5.**

Table 5: **Overview of the deprotection and coupling settings used for automated peptide synthesis.**

| name | temperature [°C] | performance [W] | hold time [s] | $\Delta$T [°C] |
|---|---|---|---|---|
| standard deprotection | 75 | 155 | 15 | 2 |
| | 85 | 30 | 50 | 1 |
| conventional deprotection | 25 | 0 | 900 | 2 |
| standard coupling | 75 | 160 | 15 | 2 |
| | 90 | 3 | 110 | 1 |
| conventional coupling | 25 | 2 | 3600 | 2 |
| 50 °C coupling | 25 | 0 | 120 | 2 |
| | 50 | 35 | 240 | 1 |

Manual SSPS

Manual peptide synthesis was performed following a standard Fmoc protocol at a 0.3 mmol scale. BACHEM's 2-chlorotrityl chloride resin (200-400 mesh, loading 1.5-1.9 mmol/g) and MERCK's DHP HM resin (100 - 200 mesh, 1.17 mmol/g) were used as solid support. To a suspension of the resin (200 mg) in $CH_2Cl_2$ (2 mL) was added pyridine (0.1 mL) and $SO_2Cl_2$ (0.05 mL) at 0 °C. The mixture was heated to reflux for 4 h without using a stirring bar, cooled to rt and then filtered using a 20 mL syringe reactor (equipped with a filter). The activated resin was washed with $CH_2Cl_2$ (3x 4 mL) and dried. The resin was transferred into a 10 mL flask and $CH_2Cl_2$ (4 mL), the C-terminal amino alcohol (0.6 mmol, 2 equiv.) and distilled DIPEA (0.2 mL) were added. The flask was flushed with argon, closed with a glass stopper, placed into a beaker and shaken with 300 rpm on HEIDOLPH's Rotamax 120 at rt for 18 h. The mixture was transferred into a 20 mL syringe reactor and the resin was washed with DMF (3x 4 mL) and $CH_2Cl_2$ (3x 4 mL).

- For deprotection 20% piperidine in DMF (4 mL) were drawn into the syringe reactor and the mixture was shaken with 300 rpm on HEIDOLPH's Rotamax 120 at rt for 15 min. The supernatant was filtered off and the deproctection procedure was performed a second time.

    - In the meanwhile, coupling was prepared by dissolving the following amino acid (1.2 mmol, 4 equiv.) in DMF/$CH_2Cl_2$ (1/1, v/v, 4 mL) in a 10 mL flask. HATU (456 mg, 1.2 mmol, 4 equiv.) and DIPEA (0.4 mL, 2.4 mmol, 8 equiv.) were added and the reaction mixture was stirred at rt for 20 min which resulted in a yellow coloration of the solution. The stirring bar was removed and the washed and dried resin was added to the flask. The flask was flushed with argon, closed with a glass stopper, placed into a beaker and shaken with 300 rpm on HEIDOLPH's Rotamax 120 at rt for 1-18 h. The mixture was transferred into a 20 mL syringe reactor and the resin was washed with DMF (3x 4 mL), $CH_2Cl_2$ (3x 4 mL).

All following (amino) acids were coupled following the iterative sequence of deprotection, and coupling.

After completed (automated or manual) peptide synthesis, the suspension was transferred to a 20 mL syringe reactor. The resin-bound peptide was filtered and washed with DMF (3x 5 mL) and $CH_2Cl_2$ (3x 5 mL). For resin-cleavage and removal of the protecting groups, the resin-bound peptide was incubated with 10 mL TFA/$H_2O$/DODT/TIS (94%/2.5%/2.5%/1%, v/v/v/v) at rt and 300 rpm for a minimum of 4 h. The solution was filtered off and portioned onto two 40 mL Greiner tubes filled with 35 mL of ice cold diethyl ether. The tubes were centrifuged (4000 rpm, 4 °C, 4 min), the supernatants discarded and the precipitates washed three times with diethylether. The two precipitates were dissolved in MeCN, combined, diluted with $H_2O$ and lyophilized. Further purification is described in in **1.2 Synthetic Procedures.**

**Absorbance and Emission Measurements**

Quartz cuvettes (HELLMA 115B-QS, HELLMA 105.202-QS) were used for absorbance and emission measurements. All spectra were obtained at ambient temperature. Normalized spectra are displayed for clarity.

Absorbance spectra were obtained at a PERKIN ELMER Lambda 1050+ with solvent in the reference beam path. Spectrophotometer operated by PERKIN ELMER'S UV WinLab software.

Emission spectra were recorded using custom-built PRINCETON INSTRUMENT'S system. Emission in the visible wavelength range was detected with a Pixis:400BR camera (cooled to -70 °C) attached to a SpectraPro HRS-300-SS spectrograph. Emission in the Near and Shortwave Infrared wavelength range was detected with a Pylon-IR:1024 line-camera (cooled to -100 °C) attached to the same spectrograph. A grating with density 150 g/nm, a blaze of 800 nm was used. PRINCETON INSTRUMENTs LightField software was used to control acquisition.

Excitation wavelength were 635 nm (THORLABS CPS635R with THORLABS'S FL-635-10 clean-up filter) and 785 nm (THORLABS Fiber-Coupled Laser S4FC785 with THORLABS FL780-10 clean-up filter). For the excitation wavelengths, THORLABS FELH0650 and THORLABS FELH0800 were used as emission filters, respectively.

The exposure times for both detectors were set to 1000 ms. Five frames per detector per spectrum were acquired. The frames were background-, flatfiled-corrected and averaged. The sum of the rows of frames acquired with the Pixis:400BR was taken. The resulting spectra were intensity corrected (THORLABS SLS201L/M). The intensity corrected spectra from both detectors were stitched resulting in the final spectrum.

**Imaging**

Imaging was performed on a custom-built setup. LUMICS laser unit LU0785DLU250-S70AN03 (25 W) "785 nm" was used for excitation. Laser was externally controlled using a LUMICS laser diode control system. Laser output was coupled in a 4x1 fan-out fiber-optic bundle (THORLABS BF46LS01) of 600 μm core diameter for each optical path. The

output from the fiber was fixed in an excitation cube (THORLABS KCB1EC/M), reflected off of a mirror (THORLABS BBE1-E03), and passed through a positive achromat (THORLABS AC254-050-B), SP filter (THORLABS FESH800) and an engineered diffuser (THORLABS ED1-S20-MD or ED1-S50-MD) to provide uniform illumination over the working area. In a typical experiment, the excitation flux in the field of view was adjusted to be close to 100 $mWcm^{-2}$. Power density was determined using a THORLABS energy meter console PM100D in combination with a THORLABS S130C sensor (measurement uncertainty of ± 3%). The working area was covered by blackout fabric (THORLABS BK5). Emitted light was directed through two filters (THORLABS FELH800) screwed onto a EDMUND OPTICS Acktar Hexa-Black 25 mm noise reduction extension tube, via a C-mount camera lens (NAVITAR SWIR-35) onto an NIR-camera (PCO Edge 4.2 or Prime BSI). The software μManager was used to externally control the camera. The assembly was partially enclosed to avoid excess light while enabling manipulation of the field of view during operation.

**Procedure for Determining Photostability**

For each dye (conjugate), solutions in $H_2O$ and FBS with an OD (785 nm) < 1.0 in a 1 cm cuvette (HELLMA 115B-QS) were prepared. Solutions were transferred to HIRSCHMANN mini caps (Code-Nr: 9000110, V = 10 μL, d = 0.0652 cm) and closed with wax. The inclusion of bubbles was avoided. Filled capillaries were placed on a homemade capillary holder made from acryl. Samples were continuously irradiated using the imaging setup described above. Data was acquired with 1 fps until samples were completely bleached. Images were processed using the software Fiji (ImageJ). All images were dark subtracted and corrected with a flatfield, that itself was dark-corrected, Gaussian-blurred and normalized to the maximum. In order to be able to make useful comparison between different samples and experiments, the time axis was converted into a number of absorbance events by multiplication with the "absorptions per molecule per second".

**Imaging in vivo**

**Procedure for Measuring Tissue and Blood Clearance**

The anesthetized mice were injected with 5nmol of each dye (conjugate) dissolved in PBS under continuous imaging in a custom build imaging setup for one hour. For excitation a 785 nm laser (Fa. Lumics) with a power density of 50mW/$cm^2$, an engineered diffuser (ED1-S20-MD, Fa. Thorlabs) and an 800 nm shortpass filter (FESH 800, Fa. Thorlabs) was used. On the emission site an 825-50 bandpass filter (Fa. Edmund Optics) and two 800 longpass filters (FELH 800, Fa. Thorlabs) were mounted in a zoom lens system (Optem Fusion Zoom lens, Fa. Qioptic) to optimize the signal for the used dyes and images were acquired using MicroManager and a NIR camera (pco edge 4.2, Fa. PCO). To reduce the bleaching effect, the laser was triggered by the camera so that it was turned on only during image acquisition every 10 to 20 s. The vital parameters of the mice were monitored during the continuous imaging, so that the anesthesia and temperature could be adjusted to the mice needs. In the end of the experiment the mice were sacrificed and after necropsy the organs were imaged to estimate biodistribution. Image processing (dark and flatfield correction) was performed in image j, then a ROI (region of interest) was drawn on the tissue of the front limb and the tail vain respectively, by using the "plot Z-axis profile" the mean value of the ROI was measured in all of the images. The blood clearance was corrected for the tissue clearance by subtracting the values of a ROI on the tail next to the tail vein.

**Bleaching in vivo**

Mice were injected with 5For image acquisition the same custom built camera setup was used as mentioned above. For this experiment a second laser beam with the same 785 nm excitation but without engineered diffusor and shortpass filter was added to the setup. For this bleaching laser beam the power density was increased to 200-450mW/$cm^2$ to cause a quick, continuous bleaching in a small area, here we chose one ear, kidney and leg for bleaching. The bleaching was stopped, when a steady state of the signal in the bleaching area was reached. Whole mouse images were acquired before and after the bleaching under the same conditions mentioned above with the bleaching laser beamed turned off. In the end of the experiment the mice were sacrificed and after necropsy, organs were imaged. Image processing was performed as already mentioned and the bleaching kinetic was measured using the "plot Z-axis profile" tool for the ROI in the bleached area.

**1.2 Synthetic Procedures**

**1.2.1 Conjugateable Heptamethine Cyanine Dyes**

**2,4-dinitrophenyltrifluoromethanesulfonate (zi1)**

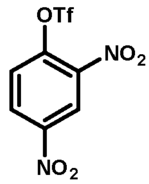

$C_7H_3F_3N_2O_7S$
Exact Mass: 315,9613

2,4-dinitrophenol (sb1, 70% in $H_2O$, 800 mg, 3.04 mmol, 1.00 equiv.) and distilled triethylamine (422 μL, 3.04 mmol, 1.00 equiv.) were dissolved in $CH_2Cl_2$ (12.2 mL, 0.25 M). The solution was cooled to 0 °C and $Tf_2O$ (1.02 mL, 6.08 mmol, 2.00 equiv.) was added. The reaction mixture was stirred at 0 °C for 20 min. The mixture was diluted with $CH_2Cl_2$ (10 mL), quenched with saturated aqueous $NaHCO_3$ (10 mL) and extracted with $CH_2Cl_2$ (3 x 100 mL). The combined organic layers were dried over $Na_2SO_4$, filtered and evaporated onto silica gel. Purification by chromatography (silica, 0% - 20% Ethyl acetate/PE, main separation step at 2%) afforded triflate **zi1** (718 mg, 2.27 mmol, 75%) as a yellow oil. The analytical data are in agreement with those reported in the literature (15).

$\mathbf{R_f}$ (50% Ethyl acetate/PE) = 0.75; **$^1$H NMR** (400 MHz, $CDCl_3$): δ = 9.03 (d, *J* = 2.7 Hz, 1H), 8.62 (dd, *J* = 9.0, 2.7 Hz, 1H), 7.76-7.69 (m, 1H) ppm; **$^{13}$C NMR** (101 MHz, $CDCl_3$): δ = 146.6, 145.1, 129.6, 125.7, 122.5, 120.1, 117.0 ppm.

***N*-(hex-5-yn-1-yl)isonicotinamide (zi2)**

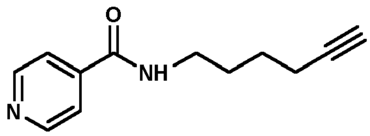

$C_{12}H_{14}N_2O$
Exact Mass: 202,1106

To an oven-dried two-neck flask was added isonicotinic acid **(sb2,** 750 mg, 6.10 mmol, 1.00 equiv.), a cat. amount of dry DMF (five drops) and dry $CH_2Cl_2$ (12.2 mL, 0.5 M) under argon. The solution was cooled to 0 °C, oxalylchloride (627 μL, 7.32 mmol, 1.20 equiv.) was added dropwise and the reaction mixture was stirred at rt for 1 h. The volatiles were removed at reduced pressure and the residue was dissolved in $CH_2Cl_2$ (30.5 mL, 0.2 M). The solution was cooled to 0 °C and hexynamine (826 μL, 7.32 mmol, 1.20 equiv.) and distilled triethylamine (2.55 mL, 18.3 mmol, 3.00 equiv.) were added. After 5 min, the ice bath was removed and stirring was continued at rt for 1 h. The reaction mixture was quenched by the addition of saturated aqueous $NaHCO_3$ (5 mL), washed with saturated aqueous $NaHCO_3$ (25 mL) and extracted with $CH_2Cl_2$ (3 x 20 mL). The combined organic layers were dried over $Na_2SO_4$, filtered, concentrated under reduced pressure, and the crude was purified by column chromatography (silica, 0% - 2% MeOH/$CH_2Cl_2$, main separation step at 2%). Alkyne zi2 (1.16 g, 5.72 mmol, 93%) was obtained as a brown oil (16).

$\mathbf{R_f}$ (10% MeOH/$CH_2Cl_2$ + 0.5% TEA) = 0.6; **$^1$H NMR** (400 MHz, $CDCl_3$): δ = 8.61 (d, *J* = 5.9 Hz, 2H), 7.58 (dd, *J* = 4.5, 1.6 Hz, 2H), 7.16 (s, 1H), 3.44-3.36 (m, 2H), 2.17 (td, *J* = 6.9, 2.6 Hz, 2H), 1.92 (t, *J* = 2.7 Hz, 1H), 1.69 (ddd, *J* = 14.6, 9.8, 7.1 Hz, 2H), 1.55 (ddd, *J= 10.6,* 7.2, 2.2 Hz, 2H) ppm; **$^{13}$C NMR** (101 MHz, $CDCl_3$): δ = 165.8, 150.3, 141.9, 121.1, 83.9, 69.0, 39.7, 28.4, 25.7, 18.1 ppm; **LC-MS** (05-95_3.5 min): *m/z* 203 $[M+H^+]^+$, $t_R$ = 0.99 min; **HRMS (ESI+):** calculated for $C_{12}H_{15}N_2O^+$ $[M+H^+]^+$ 203.1184, observed 203.1186.

**1-(2,4-dinitrophenyl)-4-(hex-5-yn-1-ylcarbamoyl)pyridin-1-iumtriflat (zi3)**

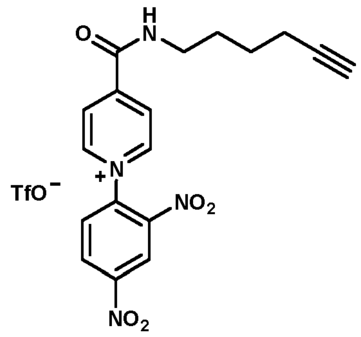

$CF_3O_3S^-$ Exact Mass: 148,9526 $C_{18}H_{17}N_4O_5^+$ Exact Mass: 369,1193

Triflate **zi1** (172 mg, 0.54 mmol, 1.10 equiv.) and alkyne **zi2** (100 mg, 0.49 mmol, 1.00 equiv.) were dissolved in toluene (3.5 mL, 0.15 M). The reaction mixture was refluxed for 16 h and left to cool to rt. The resulting precipitate was filtered, washed with toluene (5 mL) and Et2O (5 mL) and dissolved in MeOH (10 mL). The solvent was removed under reduced pressure. Zincke salt **zi3** (258 mg, 0.49 mmol, quant.) was obtained as a brown solid and used without any further purification.

**$R_f$** (20% MeOH/$CH_2Cl_2$) = 0.7; **$^1$H NMR** (400 MHz, $CD_3OD$): $\delta$ = 9.44-9.41 (m, 2H), 9.29 (d, *J* = 2.5 Hz, 1H), 8.93 (dd, *J* = 8.7, 2.5 Hz, 1H), 8.67-8.63 (m, 2H), 8.32-8.28 (m, 1H), 3.57-3.49 (m, 2H), 2.31-2.21 (m, 3H), 1.88-1.77 (m, 2H), 1.70-1.60 (m, 2H) ppm; **$^{13}$C NMR** (101 MHz, CD3OD): $\delta$ = 163.6, 153.4, 151.3, 148.4, 144.5, 139.9, 132.5, 131.2, 127.2, 123.2, 84.6, 69.9, 41.2, 29.2, 27.0, 18.7 ppm; **LC-MS** (05-95_3.5 min): *m/z* 369 [M-OTf$^-$]$^+$, $t_R$ = 1.01 min; **HRMS (ESI+):** calculated for $C_{18}H_{17}N_4O_5^+$ [M-OTf$^-$]$^+$ 369.1187, observed 369.1199.

**3-azidopropan-1-amine hydrochloride (zi4)**

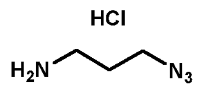

$C_3H_8N_4$
Exact Mass: 100,07

To a solution of 3-azidopropan-1-amine hydro bromide **(sb4,** 1.00 g, 4.57 mmol, 1.00 equiv.) in water (3.1 mL, 0.15 M) was added aqueous $NaN_3$ (3.3 M, 6.9 mL, 23.1 mmol, 5.00 equiv.). The reaction mixture was refluxed for 20 h, left to cool to rt, basified with aqueous KOH (10%, 10 mL) and extracted with $Et_2O$ (3x 20 mL). The combined organic layers were washed with aqueous HCl (1 M, 3x 20 mL). The combined aqueous layers were lyophilized to afford azide **zi4** (449 mg, 3.29 mmol, 72%) as a white solid, which was used without any further purification.

**$^1$H NMR** (400 MHz, $D_2O$) $\delta$ = 3.52 (t, *J* = 6.5 Hz, 2H), 3.11 (t, *J* = 7.5 Hz, 2H), 2.00-1.92 (m, 2H) ppm; **$^{13}$C NMR** (101 MHz, $D_2O$) $\delta$ 48.2, 37.3, 26.1 ppm; **HRMS (ESI+):** calculated for $C_3H_{10}N_4^+$ [M+H$^+$]$^+$ 101.0822, observed 101.0815; **IR:** $\tilde{\nu}$ [cm$^{-1}$] = 2094.69 (azide).

***N*-(3-azidopropyl)isonicotinamide (zi5)**

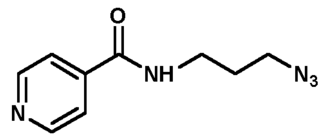

$C_9H_{11}N_5O$
Exact Mass: 205,0964

To a solution of azide **zi4** (449 mg, 3.30 mmol, 1.00 equiv.) in dry $CH_2Cl_2$/DMF (1/1, 16.5 mL, 0.2 M) were added isonicotinic acid **(sb2,** 406 mg, 3.30 mmol, 1.00 equiv.), EDC-HCl (696 mg, 3.64 mmol, 1.10 equiv.) and $NaHCO_3$ (1.38 g, 16.5 mmol, 5.00 equiv.). The suspension was stirred at rt for 16 h. The volatiles were removed at reduced pressure and the residue was diluted with saturated aqueous $NaHCO_3$ (10 mL) and extracted with $Et_2O$ (3x 20 mL). The combined organic layers were

washed with brine (30 mL), dried over $Na_2SO_4$, filtered and evaporated onto silica gel. Purification by chromatography (silica, 0% - 10% $MeOH/CH_2Cl_2$, main separation step at 3%) afforded azide zi5 (620 mg, 3.03 mmol, 92%) as a brown solid.

$\mathbf{R_f}$ (10% $MeOH/CH_2Cl_2$) = 0.4, **$^1$H NMR** (400 MHz, $CDCl_3$) $\delta$ = 8.70-8.64 (m, 2H), 7.63-7.57 (m, 2H), 3.53 (q, *J* = 6.4 Hz, 2H), 3.43 (t, *J* = 6.4 Hz, 2H), 1.88 (p, *J* = 6.5 Hz, 2H) ppm; **$^{13}$C NMR** (101 MHz, $CDCl_3$) $\delta$ = 165.9, 150.5, 141.7, 121.1, 49.6, 38.1, 28.6 ppm; **LC-MS** (05-95_3.5 min): *m/z* 206 $[M+H^+]^+$, $t_R$ = 0.70 min; **HRMS (ESI+):** calculated for $C_9H_{11}N_5ONa^+$ $[M+Na^+]^+$ 228.0862, observed 228.0861; **IR:** $\tilde{\nu}$ [$cm^{-1}$] = 2090.84 (azide).

**4-((3-azidopropyl)carbamoyl)-1-(2,4-dinitrophenyl)pyridin-1-ium triflate (zi6)**

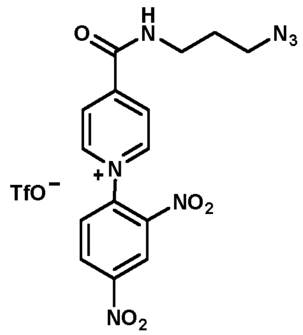

$CF_3O_3S^-$ Exact Mass: 148,9526

$C_{15}H_{14}N_7O_5^+$ Exact Mass: 372,1051

Triflate **zi1** (804 mg, 2.54 mmol, 1.10 equiv.) and azide **zi5** (475 mg, 2.31 mmol, 1.00 equiv.) were dissolved in toluene (21 mL, 0.15 M). The reaction mixture was refluxed for 18 h and left to cool to rt. The resulting precipitate was filtered, washed with toluene (10 mL) and $Et_2O$ (10 mL) and dissolved in MeOH (20 mL). The solvent was removed under reduced pressure. Zincke salt **zi6** (1.14 g, 2.20 mmol, 86%) was obtained as a brown solid and used without any further purification.

**$^1$H NMR** (400 MHz, $CD_3OD$) $\delta$ = 9.46-9.40 (m, 2H), 9.29 (d, *J* = 2.4 Hz, 1H), 8.93 (dd, *J* = 8.7, 2.5 Hz, 1H), 8.69-8.63 (m, 2H), 8.30 (d, *J* = 8.7 Hz, 1H), 3.59 (t, *J* = 6.8 Hz, 2H), 3.49 (t, *J* = 6.6 Hz, 2H), 1.96 (p, *J*= 6.7 Hz, 2H) ppm; **$^{13}$C NMR** (101 MHz, $CD_3OD$) $\delta$ = 163.8, 153.3, 151.3, 148.4, 144.5, 139.9, 132.5, 131.2, 127.3, 123.2, 50.2, 39.2, 29.4 ppm; **LC-MS** (05-95_3.5 min): *m/z* 372 $[M-OTf^-]^+$, $t_R$ = 0.71 min; **HRMS (ESI+):** calculated for $C_{15}H_{14}N_7O_5^+$ $[M-OTF^-]^+$ 372.1051, observed 372.1056; **IR:** $\tilde{\nu}$ [$cm^{-1}$] = 2098.55 (azide).

**Methyl 7-(isonicotinamido)heptanoate (zi7)**

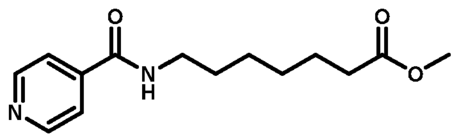

$C_{14}H_{20}N_2O_3$
Exact Mass: 264,1474

To an an oven-dried two-neck flask were added isonicotinic acid **(sb2,** 1.00 g, 8.13 mmol, 1.00 equiv.) a cat. amount of dry DMF (five drops) and dry $CH_2Cl_2$ (16.3 mL, 0.5 M). The solution was cooled to 0 °C, oxalylchloride (0.84 mL, 9.75 mmol, 1.20 equiv.) was added dropwise and the reaction mixture was stirred at rt for 1 h. The volatiles were removed at reduced pressure and the residue was dissolved in $CH_2Cl_2$ (40.6 mL, 0.2 M). The solution was cooled to 0 °C and amine **sb5** (2.22 mL, 9.75 mmol, 1.20 equiv.) and distilled triethylamine (3.39 mL, 18.3 mmol, 3.00 equiv.) were added. After 5 min, the ice bath was removed and stirring was continued at rt for 1 h. The reaction mixture was quenched by the addition of saturated aqueous $NaHCO_3$ (10 mL), washed with saturated aqueous $NaHCO_3$ (30 mL) and extracted with $CH_2Cl_2$ (3 x 40 mL). The combined organic layers were dried over $Na_2SO_4$, filtered, concentrated under reduced pressure, and the crude was purified by column chromatography (silica, 0% - 2% $MeOH/CH_2Cl_2$, main separation step at 2%). Ester **zi7** (1.88 g, 7.11 mmol, 87%) was obtained as a brown oil.

**$R_f$**(10% MeOH/$CH_2Cl_2$ + 0.5% TEA) = 0.25; **$^1$H NMR** (400 MHz, $CDCl_3$) $\delta$ = 8.75-8.68 (m, 2H), 7.64-7.55 (m, 2H), 3.48-3.42 (m, 2H), 2.31 (t, *J* = 7.4 Hz, 2H), 1.68-1.57 (m, 4H), 1.44-1.31 (m, 4H) ppm; **$^{13}$C NMR** (101 MHz, $CDCl_3$) $\delta$ = 174.3, 165.7, 150.7, 142.0, 121.0, 51.7, 40.2, 34.0, 29.4, 28.8, 26.6, 24.8 ppm; **LC-MS** (05-95_3.5 min): *m/z* 265 $[M+H^+]^+$, $t_R$ = 1.12; **HRMS (ESI+):** calculated for $C_{14}H_{20}N_2O_3Na^+$ $[M+Na^+]^+$ 287.1372, observed 287.1386.

**1-(2,4-dinitrophenyl)-4-((7-methoxy-7-oxoheptyl)carbamoyl)pyridin-1-ium triflate (zi8)**

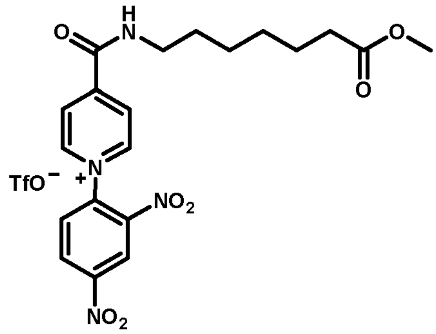

$CF_3O_3S^-$
Exact Mass: 148,95

$C_{20}H_{23}N_4O_7^+$
Exact Mass: 431,16

Triflate **zi1** (864 mg, 2.73 mmol, 1.10 equiv.) und ester **zi7** (656 mg, 2.48 mmol, 1.00 equiv.) were dissolved in toluene (21 mL, 0.15 M). The reaction mixture was refluxed for 18 h and left to cool to rt. The resulting precipitate was filtered, washed with toluene (10 mL) and $Et_2O$ (10 mL) and dissolved in MeOH (20 mL). The solvent was removed under reduced pressure. Zincke salt **zi8** (1.42 g, 2.73 mmol, 99%) was obtained as a brown solid and used without any further purification.

**$^1$H NMR** (400 MHz, $CD_3OD$) $\delta$ = 9.45-9.41 (m, 2H), 9.29 (d, *J* = 2.4 Hz, 1H), 8.93 (dd, *J* = 8.7, 2.5 Hz, 1H), 8.68-8.62 (m, 2H), 8.30 (d, *J* = 8.7 Hz, 1H), 3.66 (s, 3H), 3.50 (t, *J* = 7.2 Hz, 2H), 2.35 (t, *J* = 7.4 Hz, 2H), 1.76-1.59 (m, 4H), 1.51-1.36 (m, 4H) ppm; **$^{13}$C NMR** (101 MHz, $CD_3OD$) $\delta$ = 175.9, 163.6, 153.5, 151.3, 148.3, 144.5, 139.9, 132.5, 131.2, 127.2, 123.2, 52.0, 41.6, 34.7, 29.9, 29.8, 27.7, 25.9 ppm; **LC-MS** (05-95_3.5 min): *m/z* 431 $[M-OTf^-]^+$, $t_R$ = 1.08; **HRMS (ESI+):** calculated for $C_{20}H_{23}N_4O_7^+$ $[M-OTf^-]^+$ 431.1567, observed 431.1516.

**4-(2,3,3-trimethyl-3H-indol-1-ium-1-yl)butan-1-sulfonate (hv2)**

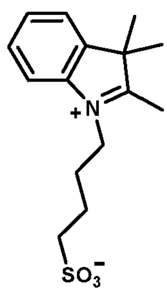

$C_{15}H_{21}NO_3S$
Exact Mass: 295,1242

A solution of indole **hve1** (1.00 g, 6.29 mmol, 1.00 equiv.) and 1,4-butane sultone (670 µL, 6.61 mmol, 1.05 equiv.) in toluene (10.5 mL, 0.6 M) was heated at 140 °C for 7 h in a microwave reactor. The reaction mixture was left to cool to rt and the supernatant was decanted into another microwave vial. The precipitate was washed with toluene (10 mL), petrolether (10 mL) and aceton (10 mL), dissolved in MeOH (20 mL) and concentrated under reduced pressure. Another portion 1,4-butane sultone (670 µL, 6.61 mmol, 1.05 equiv.) was added to the supernatant and the mixture was heated again at 140 °C for 7 h in a microwave reactor. The resulting precipitate was washed as previously described, combined with the other precipitate and purified by column chromatography (C18, 5% - 95% MeCN+0.05% TFA/$H_2O$+0.05% TFA, main separation step at 5%). Heterocycle **hv2** (931 mg, 3.16 mmol, 51%) was obtained as an off-white solid. The analytical data are in

agreement with those reported in the literature (17).

**$R_f$** (20% MeOH/$CH_2Cl_2$) = 0.1; **$^1$H NMR** (400 MHz, DMSO-*$d_6$*): δ = 8.05-8.00 (m, 1H), 7.85-7.80 (m, 1H), 7.64-7.58 (m, 2H), 4.49 (t, *J* = 7.9 Hz, 2H), 2.85 (s, 3H), 2.55-2.51 (m, 1H), 1.96 (q, *J* = 7.7 Hz, 2H), 1.75 (q, *J* = 7.5 Hz, 2H), 1.53 (s, 5H) ppm; **$^{13}$C NMR** (101 MHz, DMSO-*$d_6$*): δ = 196.5, 141.6, 141.2, 129.4, 129.0, 123.4, 115.6, 74.4, 54.1, 50.2, 47.3, 26.0, 22.0, 13.9.ppm; **LC-MS** (05-95_3.5 min): *m/z* 296 $[M+H^+]^+$, $t_R$ = 0.68 min; **HRMS (ESI+):** calculated for $C_{15}H_{21}NO_3SNa^+$ $[M+Na^+]^+$ 318.1140, observed 318.1132.

**4-hydrazineylbenzenesulfonic acid (hv5)**

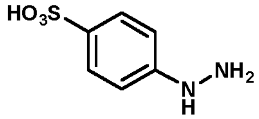

$C_6H_8N_2O_3S$
Exact Mass: 188,0256

In a two-neck flask 4-aminobenzenesulfonic acid **(hve5,** 3.00 g, 17.3 mmol, 1.00 equiv.) was suspended in $H_2O$ (11.5mL, 1.5 M). The suspension was heated to 85 °C, followed by the portion wise addition of $Na_2CO_3$ (973 mg, 9.18 mmol, 0.53 equiv.). The reaction mixture was cooled to 5 °C and $H_2SO_4$ (1.15 mL, 1.25 mmol, 1.25 equiv.) was added dropwise. Aqueous $NaNO_2$ (6 M, 2.89 mL, 17.3 mmol, 1.00 equiv.) was added at 5 °C, causing a brownish coloration of the solution and precipitation of a white solid. The diazo-compound was filtered, washed with $H_2O$, added to a solution of $Na_2SO_3$ (4.91 g, 39.0 mmol, 2.25 equiv.) in $H_2O$ (14.4 mL, 2.7 M) at 5 °C and stirring at this temperature was continued for 1 h. The reaction mixture was heated to reflux followed by the addition of conc. HCl (11.6 mL, 141 mmol, 8.16 equiv.). The reaction mixture was left to cool to rt and the resulting precipitate was filtered and washed with $H_2O$ (20 mL). Hydrazine hv5 (2.11 g, 11.2 mmol, 65%) was obtained as a white solid and used without any further purification.

**$R_f$**(10% MeOH/$CH_2Cl_2$) = 0.4; **$^1$H NMR** (400 MHz, DMSO-*$d_6$*): δ = 7.51 (d, *J* = 8.3 Hz, 2H), 8.38 (d, *J* = 6.6 Hz, 2H) ppm; **$^{13}$C NMR** (101 MHz, DMSO-*$d_6$*): δ = 145.7, 141.4, 126.2, 113.0 ppm; **HRMS (ESI-):** calculated for $C_6H_7N_2O_3S^-$ $[M-H^+]^-$ 187.0177, observed 187.0172.

**potassium 2,3,3-trimethyl-3H-indole-5-sulfonate (hv6)**

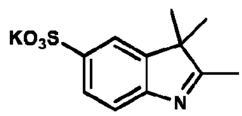

$C_{11}H_{12}KNO_3S$
Exact Mass: 277,0175

To a suspension of hydrazine **hv5** (2.11 g, 11.2 mmol, 1.00 equiv.) in acetic acid (11.2 mL, 1 M) was added 3-methyl-2-butanone (3.6 mL, 33.7 mmol, 3.00 equiv.). The reaction mixture was refluxed for 3 h and then cooled to 5 °C. $Et_2O$ (10 mL) was added causing precipitation. The solid was filtered, washed with $Et_2O$ (10 mL), dissolved in MeOH (20 mL) and concentrated under reduced pressure. The ammonium salt was dissolved in MeOH/iPrOH (1/1, 40 mL, 1 M), followed by the addition of grounded KOH (0.71 g, 12.7 mmol, 1.18 equiv.). Sulfonated indole hv6 (1.98 g, 7.2 mmol, 67%) was obtained as a hygroscopic yellow solid and used without any further purification.

**$^1$H NMR** (400 MHz, $D_2O$): δ = 7.91 (d, *J* = 1.6 Hz, 1H), 7.85 (dd, 2H, *J* = 8.7, 1.7 Hz, 1H), 7.57 (d, *J* = 8.3 Hz, 1H), 1.39 (s, 6H) ppm; **$^{13}$C NMR** (101 MHz, $D_2O$): δ = 197.1, 151.0, 145.8, 140.8, 125.9, 119.7, 118.3, 54.5, 21.6 ppm; **LC-MS** (05-95_3.5 min): *m/z* 240 $[M-K^++2H^+]^+$, $t_R$ = 0.45 min; **HRMS (ESI-):** calculated for $C_{11}H_{12}NO_3^-$ $[M-K^+]^-$ 238.0538, observed 238.0529.

**potassium 2,3,3-trimethyl-1-(4-sulfonatobutyl)-3*H*-indol-1-ium-5-sulfonate (hv7)**

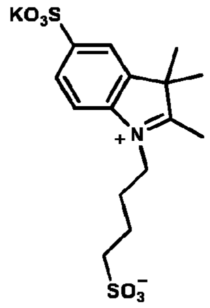

$C_{15}H_{20}KNO_6S_2$
Exact Mass: 413,0369

Sulfonated indole **hv6** (2.20 g, 7.9 mmol, 1.00 equiv.) was suspended in 1,4-butane sultone (16.1mL, 158.8mmol, 20.0 Äq.) and heated to 120 °C. The solution was stirred at this temperature for 12 h. The reaction mixture was left to cool to rt and the resulting precipitate was filtered, washed with Ethyl acetate (10 mL), dissolved in MeOH (20 mL) and concentrated under reduced pressure. Purification by column chromatography (C8, 2% - 95% MeCN+0.05% TFA/$H_2O$+0.05% TFA, main separation step at 2%) afforded heterocycle **hv7** (1.14 g, 3.0 mmol, 38%) as a gray-brown solid.

**$^1$H NMR** (400 MHz, DMSO-$d_6$): δ = 8.01 (s, 1H), 7.96 (d, *J* = 8.4 Hz, 1H), 7.81 (dd, *J* = 8.4, 1.6 Hz, 1H), 4.46 (t, *J* = 7.9 Hz, 2H), 2.84 (s, 3H), 2.53 (t, *J* = 7.3 Hz, 2H), 1.95 (p, *J* = 7.8 Hz, 2H), 1.75 (p, *J* = 7.3 Hz, 2H), 1.54 (s, 6H) ppm; **$^{13}$C NMR** (101 MHz, DMSO-$d_6$): δ = 197.2, 149.5, 141.5, 141.0, 126.3, 120.7, 115.0, 69.8, 54.2, 50.1, 26.0, 22.1, 21.91, 13.9 ppm; **LC-MS** (05-95_3.5 min): *m/z* 376 [M-$K^+$+2$H^+$]$^+$, $t_R$ = 0.2 min; **HRMS (ESI-):** calculated for $C_{15}H_{20}NO_6S_2^-$ [M-$K^+$]$^-$ 374.0732, observed 374.0719.

**sodium 4-((*E*)-2-((2*E*,4*Z*,6*E*)-7-(3,3-dimethyl-1-(4-sulfonatobutyl)-3*H*-indol-1-ium-2-yl)-4-(hex-5-yn-1-ylcarbamoyl)hepta-2,4,6-trien-1-ylidene)-3,3-dimethylindolin-1-yl)butane-1-sulfonate (dyealkin2)**

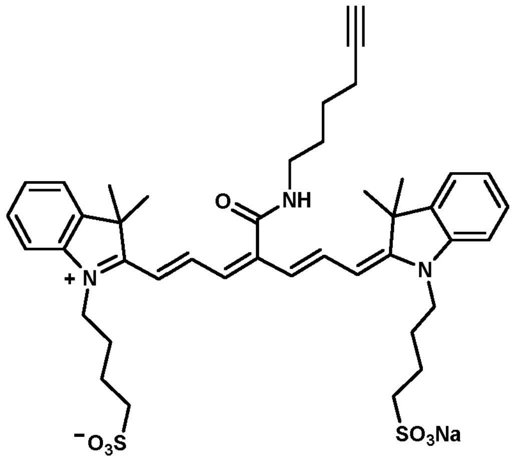

$C_{42}H_{52}N_3NaO_7S_2$
Exact Mass: 797,3144

To a solution of Zincke salt **zi3** (150 mg, 0.29 mmol, 1.00 equiv.) in MeOH (2.1 mL, 0.14 M) was added 4-bromoaniline (59.4 mg, 0.35 mmol, 1.20 equiv.). The reaction mixture was stirred at rt for 30 min. Heterocycle **hv2** (256 mg, 0.87 mmol, 3.00 equiv.) and NaOAc (143 mg, 1.73 mmol, 6.00 equiv.) were added and the reaction mixture was stirred at rt for 1 h under light exclusion. Afterwards, $Et_2O$ (6 mL) was added and the mixture was placed at -20 °C for 16 h. The resulting precipitate was filtered, washed with $Et_2O$ (10 mL) and $CH_2Cl_2$ (10 mL), dissolved in MeOH (20 mL) and concentrated under reduced pressure. Purification by column chromatography (C18 gold, 5% - 95% MeCN+0.05% TFA/$H_2O$+0.05% TFA, main separation step at 35%) afforded cyanine **dyeaklin2** (147 mg, 0.18 mmol, 64%) as a green solid.

**$R_f$** (20% MeOH/$CH_2Cl_2$) = 0.08; **$^1$H NMR** (400 MHz, DMSO-$d_6$): δ = 8.92 (bt, *J* = 5.9 Hz, 1H), 7.74-7.62 (m, 2H), 7.57 (d, *J* = 7.4 Hz, 2H), 7.41 (dt, *J* = 15.2, 7.9 Hz, 4H), 7.23 (t, *J* = 7.3 Hz, 2H), 6.53 (d, *J* = 13.6 Hz, 2H), 6.46 (d, *J* = 13.2 Hz, 2H), 4.10 (t, *J* = 7.6 Hz, 4H), 3.34 (q, *J* = 6.8 Hz, 2H), 2.87 (d, *J* = 2.6 Hz, 1H), 2.59 (t, *J* = 7.1 Hz, 4H), 2.25 (td, *J* = 7.0, 2.5 Hz, 2H), 1.85-1.63 (m, 10H), 1.56 (s, 14H) ppm; **$^{13}$C NMR** (101 MHz DMSO-$d_6$): δ = 171.3, 166.8, 158.8, 146.4, 142.1, 141.0,

128.6, 124.9, 122.5, 121.8, 111.5, 104.7, 84.3, 71.7, 50.8, 48.7, 43.6, 38.6, 28.9, 27.5, 26.2, 25.6, 22.3, 17.6 ppm; **LC-MS** (05-95_3.5 min): *m/z* 777 $[M-Na^{+}+2H^{+}]^{+}$, $t_R$ = 1.1 min; **HRMS (ESI-):** calculated for $C_{42}H_{52}N_3O_7S_2^-$ $[M-Na^{+}]^-$ 774.3247, observed 774.3256.

**sodium 2-((1*E*,3*Z*,5*E*)-7-((*E*)-3,3-dimethyl-5-sulfonato-1-(4-sulfonatobutyl)indolin-2-ylidene)-4-(hex-5-yn-1-ylcarbamoyl)hepta-1,3,5-trien-1-yl)-3,3-dimethyl-1-(4-sulfonatobutyl)-3*H*-indol-1-ium-5-sulfonate (dyealkin5)**

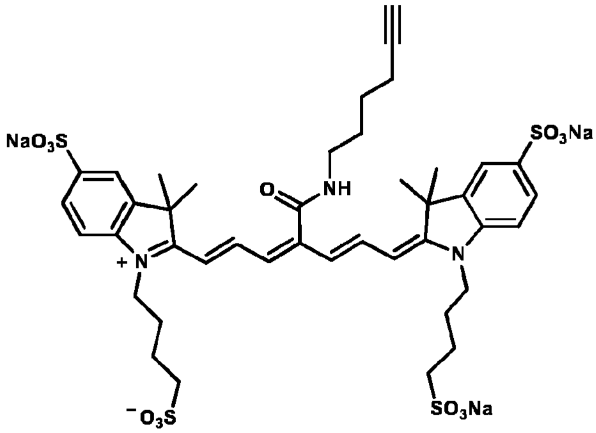

$C_{42}H_{50}N_3Na_3O_{13}S_4$
Exact Mass: 1001,1920

To a solution of Zincke salt **zi3** (150 mg, 0.29 mmol, 1.00 equiv.) in MeOH (2.1 mL, 0.14 M) was added 4-bromoaniline (59.4 mg, 0.35 mmol, 1.20 equiv.). The reaction mixture was stirred at rt for 30 min. Heterocycle **hv7** (326 mg, 0.87 mmol, 3.00 equiv.) and NaOAc (143 mg, 1.73 mmol, 6.00 equiv.) were added and the reaction mixture was stirred at rt for 1 h under light exclusion. Afterwards, $Et_2O$ (6 mL) was added and the mixture was placed at -20 °C for 16 h. The resulting precipitate was filtered, washed with $Et_2O$ (10 mL) and $CH_2Cl_2$ (10 mL), dissolved in MeOH (20 mL) and concentrated under reduced pressure. Purification by column chromatography (C18 gold, 5% - 95% MeCN+0.05% TFA/$H_2O$+0.05% TFA, main separation step at 25%) afforded cyanine **dyeaklin5** (88 mg, 0.09 mmol, 32%) as a green solid.

$\mathbf{R_f}$ (20% MeOH/$CH_2Cl_2$) = 0.00; **$^1$H NMR** (400 MHz, $D_2O$): δ = 7.83-7.70 (m, 4H), 7.63 (bd, *J* = 11.1 Hz, 2H), 7.28 (d, *J* = 8.2 Hz, 2H), 6.38 (bd, *J* = 12.3 Hz, 2H), 3.98 (s, 4H), 3.47 (t, *J* = 7.2 Hz, 2H), 2.89 (t, *J* = 7.3 Hz, 5H), 2.40 (t, *J* = 2.6 Hz, 1H), 2.27 (td, *J* = 6.9, 2.6 Hz, 2H), 1.93-1.70 (m, 10H), 1.65-1.57 (m, 2H), 1.54 (s, 12H) ppm; **$^{13}$C NMR** (101 MHz, $D_2O$): δ = 172.7, 169.5, 146.7, 144.2, 141.6, 139.0, 126.6, 122.8, 119.7, 111.1, 85.5, 69.8, 50.3, 49.0, 43.7, 39.5, 28.4, 27.1, 25.5, 25.3, 21.6, 17.4 ppm*; **LC-MS** (01-95_5 min): *m/z* 934 $[M-2Na^{+}]^-$, $t_R$ = 3.22 min; **HRMS (ESI-):** calculated for $C_{42}H_{50}N_3Na_2O_{13}S_4^-$ $[M-Na^{+}]^-$ 978.2022, observed 978.2070. *only one of three polyene carbon atoms visible in $^{13}$C NMR

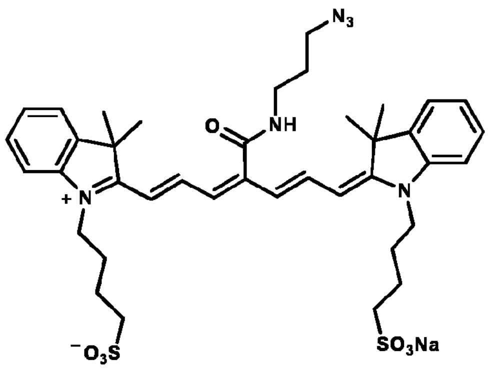

$C_{39}H_{49}N_6NaO_7S_2$
Exact Mass: 800,3002

**sodium 4-(2-((1*E*,3*Z*,5*E*)-4-((3-azidopropyl)carbamoyl)-7-((*E*)-3,3-dimethyl-1-(4-sulfonatobutyl)indolin-2-ylidene)hepta-1,3,5-trien-1-yl)-3,3-dimethyl-3*H*-indol-1-ium-1-yl)butane-1-sulfonate (dyeazid2)**

To a solution of Zincke salt **zi6** (150 mg, 0.29 mmol, 1.00 equiv.) in MeOH (2.1 mL, 0.14 M) was added 4-bromoaniline (59.4 mg, 0.35 mmol, 1.20 equiv.). The reaction mixture was stirred at rt for 30 min. Heterocycle **hv2** (255 mg, 0.87 mmol, 3.00 equiv.) and NaOAc (143 mg, 1.73 mmol, 6.00 equiv.) were added and the reaction mixture was stirred at rt for 1 h under light exclusion. Afterwards, $Et_2O$ (6 mL) was added and the mixture was placed at -20 °C for 16 h. The resulting precipitate was filtered, washed with $Et_2O$ (10 mL) and $CH_2Cl_2$ (10 mL), dissolved in MeOH (20 mL) and concentrated under reduced pressure. Purification by column chromatography (C18 gold, 5% - 95% MeCN+0.05% TFA/$H_2O$+0.05% TFA, main separation step at 35%) afforded cyanine **dyeazid2** (157 mg, 0.20 mmol, 68%) as a green solid.

**$R_f$** (20% MeOH/$CH_2Cl_2$) = 0.08; **$^1$H NMR** (400 MHz, DMSO-$d_6$): δ = 8.97 (bt, *J* = 5.7 Hz, 1H), 7.68 (t, *J* = 13.4 Hz, 2H), 7.56 (d, *J* = 7.4 Hz, 2H), 7.41 (dt, *J* = 15.3, 7.9 Hz, 4H), 7.24 (t, *J* = 7.4 Hz, 2H), 6.54 (d, *J* = 13.6 Hz, 2H), 6.48 (d, *J* = 9.8 Hz, 2H), 4.11 (d, *J* = 7.4 Hz, 4H), 3.49 (t, *J* = 6.8 Hz, 2H), 3.41 (q, *J* = 6.7 Hz, 2H), 2.60 (t, *J* = 7.0 Hz, 4H), 1.90-1.67 (m, 11H), 1.55 (s, 14H) ppm; **$^{13}$C NMR** (101 MHz, DMSO): δ = 171.3, 167.1, 158.3, 146.3, 142.1, 140.9, 128.6, 124.9, 122.4, 121.8, 111.5, 104.7, 50.8, 48.7, 48.5, 43.7, 36.4, 28.9, 27.5, 26.2, 22.2 ppm; **LC-MS** (05-95_3.5 min): *m*/*z* 780 [M-$Na^+$+$2H^+$]$^+$, $t_R$ = 1.08 min; **HRMS (ESI-):** calculated for $C_{39}H_{49}N_6O_7S_2^-$ [M-$Na^+$]$^-$ 777.3104, observed 777.3110.

**sodium 2-((1*E*,3*Z*,5*E*)-4-((3-azidopropyl)carbamoyl)-7-((*E*)-3,3-dimethyl-5-sulfonato-1-(4-sulfonatobutyl)indolin-2-ylidene)hepta-1,3,5-trien-1-yl)-3,3-dimethyl-1-(4-sulfonatobutyl)-3*H*-indol-1-ium-5-sulfonate (dyeazid3)**

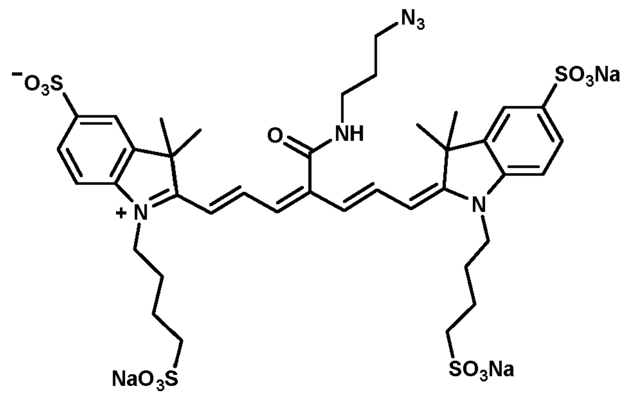

$C_{39}H_{47}N_6Na_3O_{13}S_4$
Exact Mass: 1004,1777

To a solution of Zincke salt **zi6** (150 mg, 0.29 mmol, 1.00 equiv.) in MeOH (2.1 mL, 0.14 M) was added 4-bromoaniline (59.4 mg, 0.35 mmol, 1.20 equiv.). The reaction mixture was stirred at rt for 30 min. Heterocycle **hv7** (326 mg, 0.87 mmol, 3.00 equiv.) and NaOAc (143 mg, 1.73 mmol, 6.00 equiv.) were added and the reaction mixture was stirred at rt for 1 h under light exclusion. Afterwards, $Et_2O$ (6 mL) was added and the mixture was placed at -20 °C for 16 h. The resulting precipitate was filtered, washed with $Et_2O$ (10 mL) and $CH_2Cl_2$ (10 mL), dissolved in MeOH (20 mL) and concentrated under reduced pressure. Purification by column chromatography (C18 gold, 5% - 95% MeCN+0.05% TFA/$H_2O$+0.05% TFA, main separation step at 25%) afforded cyanine **dyeazid3** (112 mg, 0.12 mmol, 41%) as a green solid.

**$R_f$** (20% MeOH/$CH_2Cl_2$) = 0.00; **$^1$H NMR** (400 MHz, $D_2O$): δ = 7.86-7.79 (m, 4H), 7.67 (t, *J* = 13.2 Hz, 2H), 7.33 (d, *J* = 8.4 Hz, 2H), 6.43 (d, *J* = 13.3 Hz, 2H), 6.31 (d, *J* = 13.5 Hz, 2H), 4.03 (s, 4H), 3.59 (t, *J* = 7.0 Hz, 2H), 3.52 (t, *J* = 6.7 Hz, 2H), 2.98-2.91 (m, 4H), 2.03-1.95 (m, 2H), 1.95-1.79 (m, 8H), 1.59 (s, 12H) ppm; **$^{13}$C NMR** (101 MHz, $D_2O$): δ = 172.8, 169.6, 146.7, 144.1, 141.7, 139.2, 126.6, 123.1, 119.7, 117.8, 111.1, 105.4, 50.3, 49.0, 48.7, 43.8, 37.3, 28.4, 27.1, 25.6, 21.6 ppm; **LC-MS** (05-95_3.6 min): *m*/*z* 939 [M-3$Na^+$+$4H^+$]$^+$, $t_R$ = 0.74 min; **HRMS (ESI-):** calculated for $C_{39}H_{48}N_6NaO_{13}S_4^{2-}$ [M-2$Na^+$]$^{2-}$ 479.0991, observed 479.0990.

**sodium 4-((*E*)-2-((2*E*,4*Z*,6*E*)-7-(3,3-dimethyl-1-(4-sulfonatobutyl)-3*H*-indol-1-ium-2-yl)-4-((7-methoxy-7-oxoheptyl)carbamoyl)hepta-2,4,6-trien-1-ylidene)-3,3-dimethylindolin-1-yl)butane-1-sulfonate (dyecarb1)**

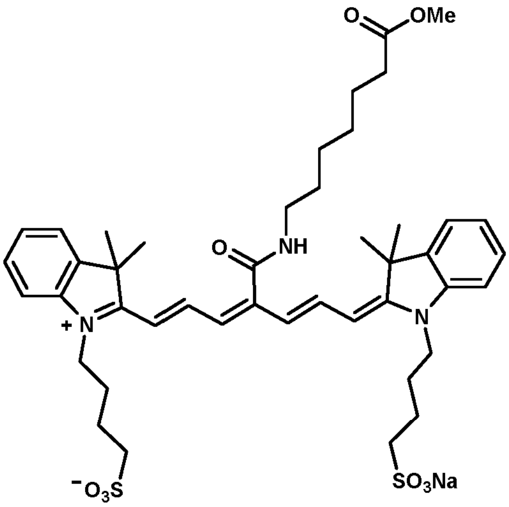

$C_{44}H_{58}N_3NaO_9S_2$
Exact Mass: 859,3512

To a solution of Zincke salt **zi8** (100 mg, 0.17 mmol, 1.00 equiv.) in MeOH (1.2 mL, 0.14 M) was added 4-bromoaniline (35.5 mg, 0.21 mmol, 1.20 equiv.). The reaction mixture was stirred at rt for 30 min. Heterocycle **hv2** (153 mg, 0.52 mmol, 3.00 equiv.) and NaOAc (84.7 mg, 1.03 mmol, 6.00 equiv.) were added and the reaction mixture was stirred at rt for 1 h under light exclusion. Afterwards, $Et_2O$ (4 mL) was added and the mixture was placed at -20 °C for 16 h. The resulting precipitate was filtered, washed with $Et_2O$ (10 mL) and $CH_2Cl_2$ (10 mL), dissolved in MeOH (20 mL) and concentrated under reduced pressure. Purification by column chromatography (C18 gold, 5% - 95% MeCN+0.05% TFA/$H_2O$+0.05% TFA, main separation step at 40%) afforded cyanine **dyecarb1** (83 mg, 0.10 mmol, 56%) as a green solid.

$\mathbf{R_f}$ (20% MeOH/$CH_2Cl_2$) = 0.15; **$^1$H NMR** (400 MHz, DMSO-$d_6$): δ = 8.88 (t, *J* = 5.7 Hz, 1H), 7.68 (t, *J* = 13.4 Hz, 2H), 7.58 (dd, *J* = 7.2, 1.0 Hz, 2H), 7.48-7.36 (m, 4H), 7.24 (td, *J* = 7.3, 1.2 Hz, 2H), 6.53 (d, *J* = 13.6 Hz, 2H), 6.46 (d, *J* = 13.2 Hz, 2H), 4.11 (t, *J* = 7.3 Hz, 4H), 3.58 (s, 3H), 3.31 (q, *J* = 6.8 Hz, 2H), 2.53 (t, *J* = 7.2 Hz, 4H), 2.34 (t, *J* = 7.4 Hz, 2H), 1.84-1.66 (m, 8H), 1.55 (s, 14H), 1.41-1.31 (m, 4H) ppm; **$^{13}$C NMR** (101 MHz, DMSO-$d_6$): δ = 173.4, 171.3, 166.8, 146.4, 142.1, 140.9, 128.6, 124.8, 122.4, 121.7, 111.4, 104.6, 51.2, 50.8, 48.6, 43.6, 39.0, 33.3, 29.5, 28.2, 27.4, 26.3, 26.2, 24.3, 22.4 ppm; **LC-MS** (05-95_3.5 min): *m/z* 838 $[M-Na^++2H^+]^+$, $t_R$ = 1.06 min; **HRMS (ESI-):** calculated for $C_{44}H_{58}N_3O_9S_2^-$ $[M-Na^+]^-$ 837.3693, observed 837.3705.

**sodium 4-(2-((1*E*,3*Z*,5*E*)-4-((6-carboxyhexyl)carbamoyl)-7-((*E*)-3,3-dimethyl-1-(4-sulfonatobutyl)indolin-2-ylidene)hepta-1,3,5-trien-1-yl)-3,3-dimethyl-3*H*-indol-1-ium-1-yl)butane-1-sulfonate (dyecarb2)**

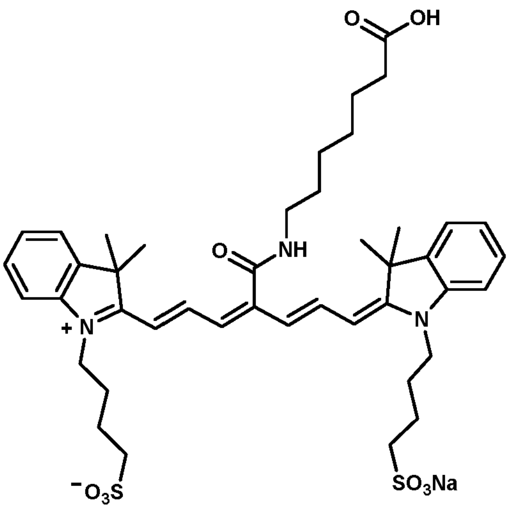

$C_{43}H_{56}N_3NaO_9S_2$
Exact Mass: 845,3356

**Dyecarb1** (18.5 mg, 21.5 μmol, 1.00 equiv.) was dissolved in THF/$H_2O$ (3/1, v/v, 4.9 mL, 5 mM) under light exclusion. An aqueous solution of LiOH*$H_2O$ (0.6 M, 0.3 mL, 172 μmol, 8.00 equiv.) was added to the reaction mixture, causing an orange coloration of the green solution. The reaction mixture was stirred at rt for 2 h and neutralized by the addition of acetic acid (57 μL, 172 μmol, 8.00 equiv.), which caused a green coloration. The volatiles were removed at reduced pressure, water (2 mL) was added and the solution was lyophilized. Purification by column chromatography (C18 gold, 5% - 95% MeCN+0.05% TFA/H2O+0.05% TFA, main separation step at 35%) afforded cyanine **dyecarb2** (9.10 mg, 10.8 μmol, 50%) as a green solid.

**LC-MS** (05-95_3.5 min): *m/z* 823 $[M-Na^+ +2H^+]^+$, $t_R$ = 1.04 min; **HRMS (ESI-):** calculated for $C_{43}H_{56}N_3O_9S_2^-$ $[M-Na^+]^-$ 822.3458, observed 822.3465.

**sodium 4-((*E*)-2-((2*E*,4*Z*,6*E*)-7-(3,3-dimethyl-1-(4-sulfonatobutyl)-3*H*-indol-1-ium-2-yl)-4-((7-((2,5-dioxopyrrolidin-1-yl)oxy)-7-oxoheptyl)carbamoyl)hepta-2,4,6-trien-1-ylidene)-3,3-dimethylindolin-1-yl)butane-1-sulfonate (dyecarb3)**

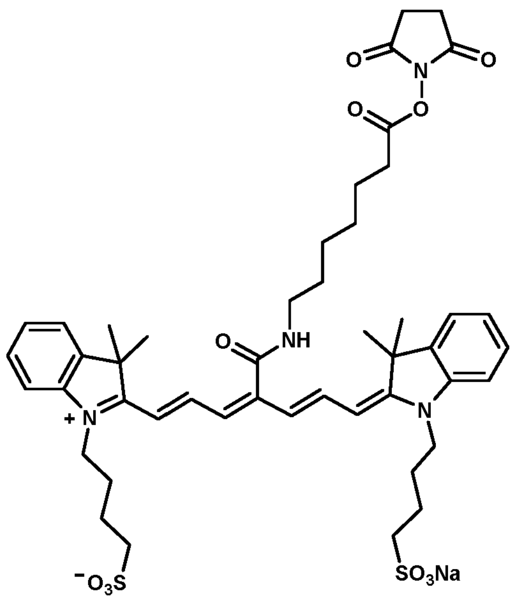

$C_{47}H_{59}N_4NaO_{11}S_2$
Exact Mass: 942,3519

To a solution of **dyecarb2** (5.7 mg, 6.74 μmol, 1.00 equiv.) in DMF (2.7 mL, 2.5 mM) were added DIPEA (4.6 μL, 27.0 μmol, 4.00 eqiv) and TSTU (8.1 mg, 27.0 μmol, 4.00 eqiv). The reaction was sonicated and stirred at rt for 1 h under light exclusion. The volatiles were removed at reduced pressure and the residue was diluted with $H_2O$/MeCN (4/1, v/v, 0.5 mL) and purified by column chromatography (C18 gold, 5% - 95% MeCN+0.05% TFA/H2O+0.05% TFA, main separation step at 35%). **Dyecarb3** (5.00 mg, 5.31 μmol, 79%) was obtained as a green solid. **LC-MS** (05-95_3.5 min): *m/z* 921 $[M-Na^+ +2H^+]^+$, $t_R$ = 1.08 min; **HRMS (ESI-):** calculated for $C_{47}H_{59}N_4O_{11}S_2^-$ $[M-Na^+]^-$ 919.3622, observed 919.3660.

**sodium 2-((1*E*,3*Z*,5*E*)-7-((E)-3,3-dimethyl-5-sulfonato-1-(4-sulfonatobutyl)indolin-2-ylidene)-4-((7-methoxy-7-oxoheptyl)carbamoyl)hepta-1,3,5-trien-1-yl)-3,3-dimethyl-1-(4-sulfonatobutyl)-3*H*-indol-1-ium-5-sulfonate (dyecarb5)**

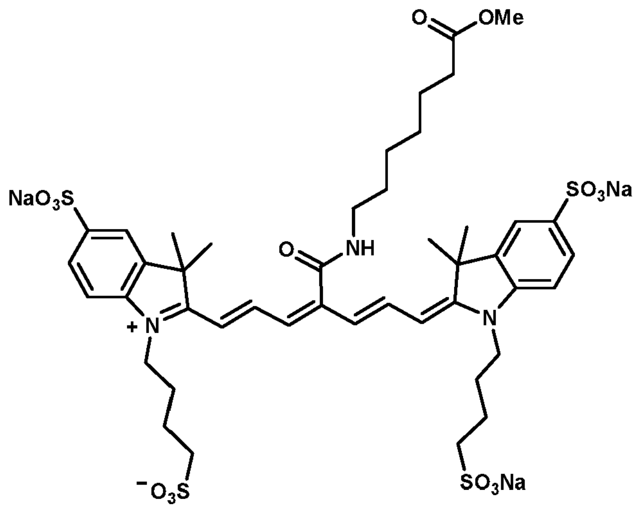

$C_{44}H_{56}N_3Na_3O_{15}S_4$
Exact Mass: 1063,2287

To a solution of Zincke salt **zi8** (270 mg, 0.47 mmol, 1.00 equiv.) in MeOH (3.3 mL, 0.14 M) was added 4-bromoaniline (95.9 mg, 0.56 mmol, 1.20 equiv.). The reaction mixture was stirred at rt for 30 min. Heterocycle **hv7** (553 mg, 1.39 mmol, 3.00 equiv.) and NaOAc (229 mg, 2.79 mmol, 6.00 equiv.) were added and the reaction mixture was stirred at rt for 1 h under light exclusion. Afterwards, $Et_2O$ (15 mL) was added and the mixture was placed at -20 °C for 16 h. The resulting precipitate was filtered, washed with $Et_2O$ (10 mL) and $CH_2Cl_2$ (10 mL), dissolved in MeOH (20 mL) and concentrated under reduced pressure. Purification by column chromatography (C18 gold, 1% - 95% MeCN+0.05% TFA/$H_2O$+0.05% TFA, main separation step at 10%) afforded cyanine **dyecarb5** (60.5 mg, 0.06 mmol, 13%) as a green solid.

**HRMS (ESI-):** calculated for $C_{44}H_{56}N_3NaO_{15}S_4^{2-}$ $[M-2Na^+]^{2-}$ 508.6246, observed 508.6247.

**sodium 2-((1*E*,3*Z*,5*E*)-4-((6-carboxyhexyl)carbamoyl)-7-((*E*)-3,3-dimethyl-5-sulfonato-1-(4-sulfonatobutyl)indolin-2-ylidene)hepta-1,3,5-trien-1-yl)-3,3-dimethyl-1-(4-sulfonatobutyl)-3*H*-indol-1-ium-5-sulfonate (dyecarb6)**

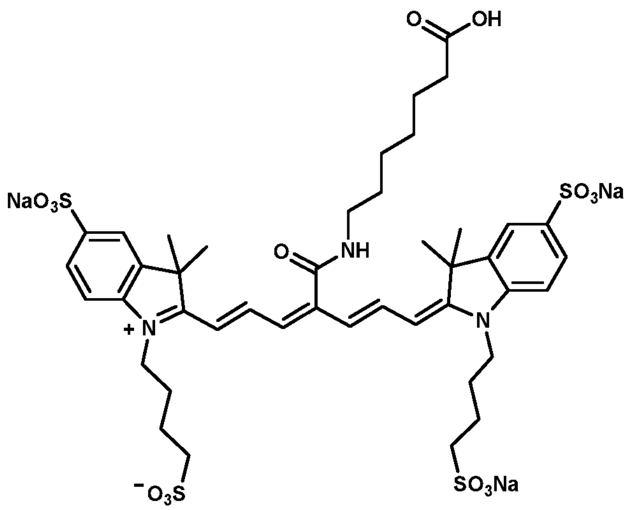

$C_{43}H_{54}N_3Na_3O_{15}S_4$
Exact Mass: 1049,2131

**Dyecarb5** (30.0 mg, 28.2 µmol, 1.00 equiv.) was dissolved in THF/$H_2O$ (3/1, v/v, 3.5 mL, 8 mM) under light exclusion. An aqueous solution of LiOH*$H_2O$ (0.6 M, 0.47 mL, 282 µmol, 10.0 equiv.) was added to the reaction mixture, causing an orange coloration of the green solution. The reaction mixture was stirred at rt for 2 h and neutralized by the addition of acetic acid (57 µL, 282 µmol, 8.00 equiv.), which caused a green coloration. The volatiles were removed at reduced pressure, water (2 mL) was added and the solution was lyophilized. Purification by column chromatography (C18 gold, 5% - 95%

MeCN+0.05% TFA/H2O+0.05% TFA, main separation step at 10%) afforded cyanine **dyecarb6** (21.8 mg, 20.8 µmol, 74%) as a green solid.

**LC-MS** (05-95_3.5 min): *m/z* 982 [M-3Na$^+$+2H$^+$]$^-$, $t_R$ = 1.07 min; **HRMS (ESI-):** calculated for $C_{44}H_{54}N_3NaO_{15}S_4{}^{2-}$ [M-2Na$^+$]$^{2-}$ 501.6168, observed 501.6172.

**sodium 2-((1*E*,3*Z*,5*E*)-7-((*E*)-3,3-dimethyl-5-sulfonato-1-(4-sulfonatobutyl)indolin-2-ylidene)-4-((7-((2,5-dioxopyrrolidin-1-yl)oxy)-7-oxoheptyl)carbamoyl)hepta-1,3,5-trien-1-yl)-3,3-dimethyl-1-(4-sulfonatobutyl)-3*H*-indol-1-ium-5-sulfonate (dyecarb7)**

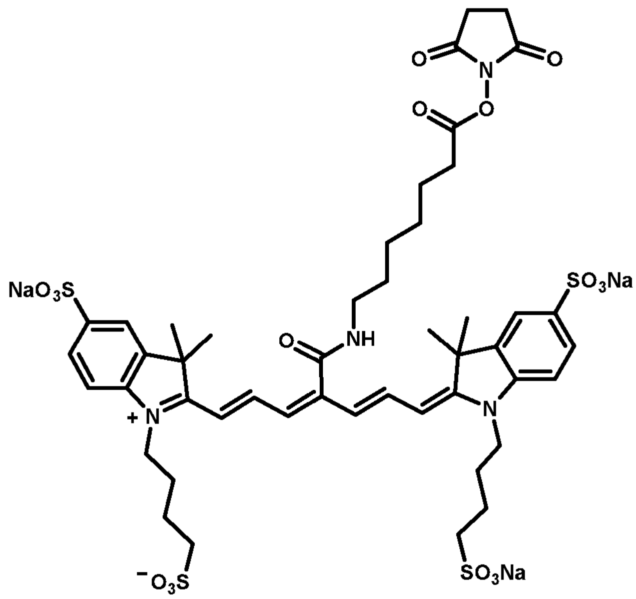

$C_{47}H_{57}N_4Na_3O_{17}S_4$
Exact Mass: 1146,2295

To a solution of **dyecarb6** (21.5 mg, 20.0 µmol, 1.00 equiv.) in DMF (8.2 mL, 2.5 mM) were added DIPEA (13.9 µL, 820 µmol, 4.00 eqiv) and TSTU (24.7 mg, 82.0 µmol, 4.00 eqiv). The reaction was sonicated and stirred at rt for 1 h under light exclusion. The reaction mixture was inversely added to ice cold diethyl ether and the precipitate was purified by column chromatography (C18 gold, 5% - 95% MeCN+0.05% TFA/H2O+0.05% TFA, main separation step at 15%). **Dyecarb7** (19.0 mg, 17.0 µmol, 81%) was obtained as a green solid.

**HRMS (ESI-):** calculated for $C_{47}H_{57}N_4NaO_{17}S_4{}^{2-}$ [M-2Na$^+$]$^{2-}$ 550.1250, observed 550.1249.

**Dyecarb8**

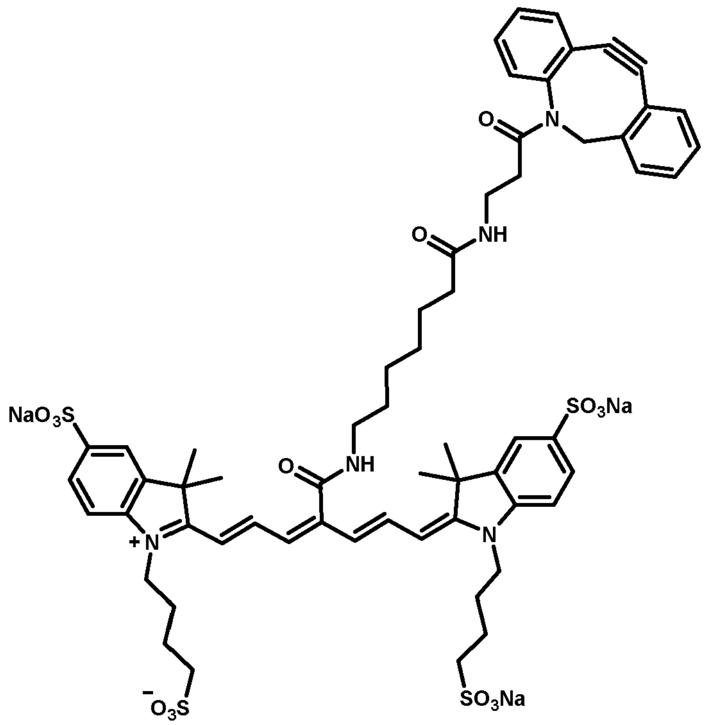

$C_{61}H_{68}N_5Na_3O_{15}S_4$
M [g/mol] = 1308,44

**Dyecarb7** (8.00 mg, 6.98 μmol, 1.00 equiv.) was dissolved in DMSO/$H_2O$ (1/1, v/v, 2.0 mL, 3.5 mM). DBCO-Amin (2.1 μL, 7.68 μmol, 1.10 equiv.) and DIPEA (5.9 μL, 34.9 μmol, 5.0 equiv.) were added and the reaction mixture was stirred at rt for 30 min. The reaction mixture was directly purified by column chromatography (C18 gold, 1% - 95% MeCN+0.05% TFA/$H_2O$+0.05% TFA, main separation step at 30%). Cyanine **dyecarb8** (3.00 mg, 2.42 μmol, 35%) was obtained as a green solid.

**sodium 2-((*E*)-2-((*E*)-3-(2-((*E*)-3,3-dimethyl-5-sulfonato-1-(4-sulfonatobutyl)indolin-2-ylidene)ethylidene)-2-(4-sulfonatophenoxy)cyclohex-1-en-1-yl)vinyl)-1-(6-(hex-5-yn-1-ylamino)-6-oxohexyl)-3,3-dimethyl-3*H*-indol-1-ium-5-sulfonate (dye1)**

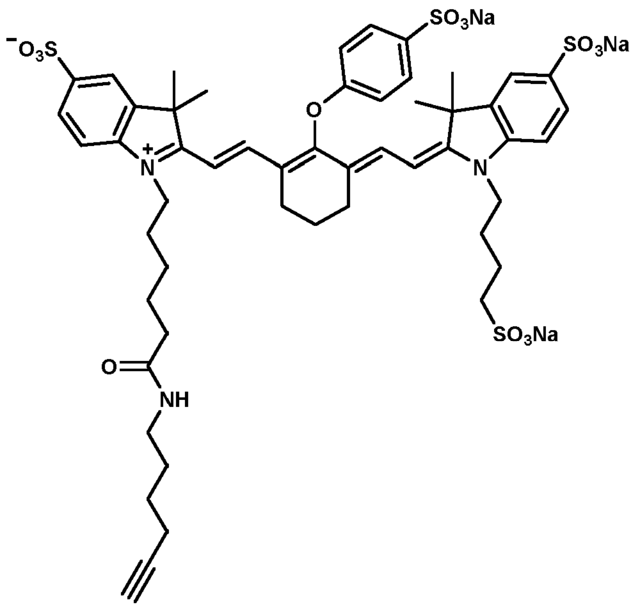

$C_{52}H_{60}N_3Na_3O_{14}S_4$
Exact Mass: 1147,2651

To a solution of IRDye® 800CW Carboxylate **(dye1,** 10 mg, 9.36 μmol, 1.00 equiv.) in DMF (1.9 mL, 5 mM) were added DIPEA (3.2 μL, 18.7 μmol, 2.00 equiv.) and TSTU (5.6 mg, 18.7 μmol, 2.00 equiv.). The solution was stirred under light exclusion at rt for 30 min, until LCMS indicated complete conversion. The reaction mixture was inversely added to ice cold Et2O (2 mL) resulting in a green precipitate. After centrifugation the supernatant was discarded and the pellet was dissolved in PBS (1.9 mL, 5 mM). Hexynamine (1 μL, 10.3 μmol, 1.10 equiv.) was added and the reaction mixture was stirred at rt for 30 min. The reaction mixture was directly purified by column chromatography (C18 gold, 1% - 95% MeCN+0.05% TFA/$H_2O$+0.05% TFA, main separation step at 15%). Cyanine **dye2** (11.0 mg, 9.36 μmol, 100%) was obtained as a green solid.

**LC-MS** (05-95_3.5 min): *m/z* 539 $[M-3Na^{+}+H^{+}]^{2-}$, $t_R$ = 0.68 min; **HRMS (ESI-):** calculated for $C_{52}H_{60}N_3O_{14}S_4^{3-}$ $[M-3Na^{+}]^{3-}$ 359.4319, observed 359.4319.

**1.2.2 SSTR2-targeting peptides**

**1-azido-13-oxo-3,6,9-trioxa-12-azahexadecan-16-oic acid (linker2)**

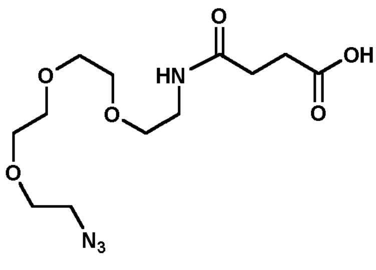

$C_{12}H_{22}N_4O_6$
Exact Mass: 318,1539

2-(2-(2-(2-azidoethoxy)ethoxy)ethoxy)ethan -1-amine (300 mg, 1.375 mmol, 1.00 equiv.) and dihydrofuran-2,5-dione (151 mg, 1.513 mmol, 1.10 equiv.) were dissolved in $CH_2Cl_2$ (3.8 mL, 0.4 M) and heated to reflux for 1.5 h. The reaction mixture was cooled to rt, concentrated under reduced pressure and the crude was purified by column chromatography (silica, 0% - 20% MeOH/CH2Cl2, main separation step at 10%). Azide **linker2** (320 mg, 1.01 mmol, 73%) was obtained as a colorless oil.

**$R_f$** (10% MeOH/$CH_2Cl_2$) = 0.2; **$^1$H NMR** (400 MHz, $CDCl_3$): δ = 6.81 (t, *J* = 5.5 Hz, 1H), 3.56-3.47 (m, 10H), 3.42 (dd, *J* = 5.7, 4.7 Hz, 2H), 3.32-3.24 (m, 4H), 2.52 (t, *J* = 7.0 Hz, 2H), 2.39 (t, *J* = 6.6 Hz, 2H) ppm; **$^{13}$C NMR** (101 MHz, $CDCl_3$):

δ = 175.2, 172.4, 70.2, 70.2, 70.1, 69.8, 69.7, 69.3, 50.3, 39.2, 30.4, 29.4 ppm; **LC-MS** (05-95_3.5 min): *m/z* 317 [M-H+]-, $t_R$ = 0.8 min; **HRMS (ESI-):** calculated for $C_{12}H_{21}N_4O_6^-$ [M-H+]- 317.1416, observed 317.1456.

**(9*H*-Fluoren-9-yl)methyl ((2R,3R)-3-(tert-butoxy)-1-hydroxybutan-2-yl)carbamate (toc1)**

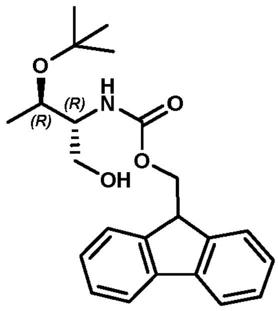

$C_{23}H_{29}NO_4$
Exact Mass: 383,2097

A solution of Fmoc-Thr(tBu)-OH (1.39 g, 3.5 mmol. 1.00 equiv.) in dry THF (30 mL, 0.12 M) was cooled to -10 °C. *N*-Methylmorpholine (389 μL, 3.5 mmol, 1.00 equiv.) and ethyl chloroformate (333 μL, 3.5 mmol, 1.00 equiv.) were added successively. The reaction mixture was stirred at -10 °C for 30 min, followed by the addition of $NaBH_4$ (398 mg, 10.5 mmol, 3.00 equiv.). Over a period of 30 min, MeOH (60 mL) was slowly added to the reaction mixture, which was then stirred at 0 °C for 3 h. The reaction was ended by the addition of 1 M HCl (90 mL). The volatiles were removed at reduced pressure and the aqueous solution was extracted with CH2Cl2 (3x 60 mL). The combined organic layers were dried over Na2SO4, filtered, concentrated under reduced pressure, and the crude was purified by column chromatography (C18, 5% - 95% MeCN/$H_2O$, main separation step at 60%). Fmoc-Thr(tBu)-ol **(toc1,** 1,2 g, 3.13 mmol, 90%) was obtained as a white solid. The analytical data are in agreement with those reported in the literature (18).

$\mathbf{R_f}$(Ethyl acetate) = 0.73; **$^1$H NMR** (400 MHz, $CDCl_3$): δ = 7.77 (d, *J* = 7.5 Hz, 2H), 7.61 (d, *J* = 7.4 Hz, 2H), 7.41 (t, *J* = 7.3 Hz, 2H), 7.3 (t, *J* = 7.3 Hz, 2H), 5.27 (m, *J* = 8.2 Hz, 1H), 4.48-4.37 (m, 2H), 4.24 (t, *J* = 6.8 Hz, 1H), 3.99-3.92 (m, 1H), 3.76-3.67 (m, 2H), 3.67-3.57 (m, 1H), 1.22 (s, 9 H), 1.18 (d, *J* = 6.3 Hz, 3H) ppm; **$^{13}$C NMR** (101 MHz, $CDCl_3$): δ = 157.2, 144.1, 141.5, 127.8, 127.2, 125.2, 120.1, 67.5, 67.0, 64.1, 57.2, 47.4, 28.8, 20.2 ppm; **LC-MS** (05-95_3.5 min): *m/z* 406 [M+Na+]+, $t_R$ = 1.34 min; **HRMS (ESI+):** calculated for $C_{23}H_{29}NO_4Na^+$ [M+Na+]+ 406.1994, observed 406.1990.

**(9*H*-fluoren-9-yl)methyl tert-butyl (6-hydroxyhexane-1,5-diyl)(S)-dicarbamate** (toc2)

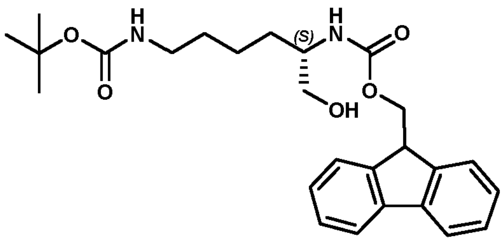

$C_{26}H_{34}N_2O_5$
M [g/mol] = 454,57

A solution of Fmoc-Lys(Boc)-OH (3.00 g, 6.4 mmol. 1.00 equiv.) in dry THF (32 mL, 0.2 M) was cooled to - 10 °C. DIPEA (1.11 mL, 6.5 mmol, 1.02 equiv.) and isobutyl chloroformate (831 μL, 6.4 mmol, 1.00 equiv.) were added successively. The reaction mixture was stirred at -10 °C for 30 min and the resulting precipitate was filtered and washed with THF (2x 10 mL). The filtrate was cooled to -10 °C. After addition of $NaBH_4$ (484 mg, 12.8 mmol, 2.00 equiv.) the solution was warmed to rt and stirred for 30 min. The volatiles were removed at reduced pressure and Ethyl acetate (100 mL) was added. The organic layer was washed with 1 M HCl (2x 50 mL), saturated aqueous $NaHCO_3$ (2x 50 mL) and brine (2x 50 mL), concentrated under reduced pressure, and the crude was purified by column chromatography (C18, 30% - 95% MeCN/$H_2O$, main separation step at 60%). %). Fmoc-Lys(Boc)-ol **(toc2,** 1.50 g, 3.3 mmol, 52%) was obtained as a white solid. The analytical

data are in agreement with those reported in the literature (19).

**$R_f$** (10% MeOH/$CH_2Cl_2$) = 0.56; **$^1$H NMR** (400 MHz, $CDCl_3$): δ = 7.76 (d, *J* = 7.6 Hz, 2H), 7.60 (d, *J* = 7.5 Hz, 2H), 7.40 (t, *J* = 7.5 Hz, 2H), 7.32 (td, *J* = 7.5, 1.2 Hz, 2H), 5.05 (bs, 1H), 4.58 (bs, 1H), 4.41 (bs, 2H), 4.21 (t, *J* = 6.8 Hz, 1H), 3.65 (d, *J* = 12.8 Hz, 3H), 3.25-3.02 (m, 2H), 1.54-1.31 (m, 15H) ppm; **$^{13}$C NMR** (101 MHz, $CDCl_3$): δ = 156.8, 156.5, 144.0, 141.5, 127.8, 127.2, 125.2, 120.1, 79.5, 66.7, 64.8, 53.1, 47.4, 39.7, 30.5, 30.1, 28.54, 22.7 ppm; **LC-MS** (05-95_3.5 min): *m*/*z* 477 $[M+Na^+]^+$, $t_R$ = 1.31 min; HRMS (ESI+): calculated for $C_{26}H_{34}N_2O_5Na^+$ $[M+Na^+]^+$ 477.2365, observed 477.2359.

**(1-azido-13-oxo-3,6,9-trioxa-12-azahexadecan-16-oyl)-phe-cyclo(Cys-Tyr-trp-Lys-Thr-Cys)-Thr(ol) (toc4)**

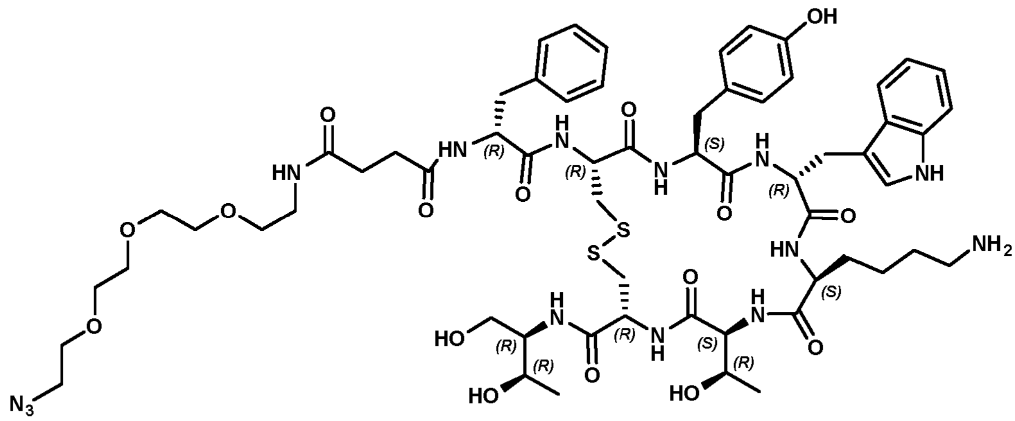

$C_{61}H_{86}N_{14}O_{16}S_2$
Exact Mass: 1334,5788

Automated peptide synthesis at 100 μmol scale with preloaded DHP-HM resin, cleavage and precipitation was performed as described in 1.1 and afforded linear peptide **toc3.** The crude peptide was purified by column chromatography (C18 gold, 1% - 95% MeCN+0.05% TFA/$H_2O$+0.05% TFA, main separation step at 40%). For disulfide cyclization, linear peptide **toc3** (40 mg) was dissolved in 40 mL of THF/5 mM ammonium acetate (4/1). The solution was buffered to pH = 8 with aqueous $NaHCO_3$ and 4 μL 30% H2O2 were added. The reaction mixture was stirred 1 h, THF was removed at reduced pressure and the solution was lyophilized. The residue was purified by column chromatography (C18 gold, 1% - 95% MeCN+0.05% TFA/$H_2O$+0.05% TFA, main separation step at 40%). Cyclized peptide **toc4** (X) was obtained as a white solid. Low yield was accepted in favor of purity.

**LC-MS** (01-95_5 min): *m*/*z* 1335 $[M+H^+]^+$, $t_R$ = 2.62 min, **HRMS (ESI+):** calculated for $C_{61}H_{87}N_{14}O_{16}S_2^+$ $[M+H^+]^+$ 1335.5866, observed 1335.5880.

**(1-azido-13-oxo-3,6,9-trioxa-12-azahexadecan-16-oyl)-trp-cyclo(Cys-Thr-phe-Thr-Tyr-Cys)-Lys(ol) (toc6)**

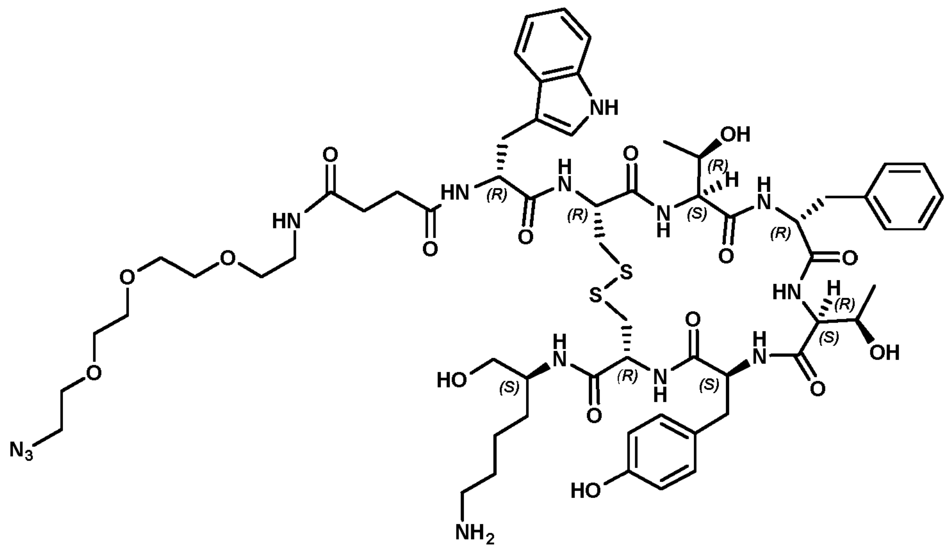

$C_{61}H_{86}N_{14}O_{16}S_2$
Exact Mass: 1334,5788

Automated peptide synthesis at 100 μmol scale with preloaded DHP-HM resin, cleavage and precipitation was performed as described in **1.1** and afforded linear peptide **toc5.** The crude peptide was purified by column chromatography (C18 gold, 1% - 95% MeCN+0.05% TFA/$H_2O$+0.05% TFA, main separation step at 40%). For disulfide cyclization, linear peptide **toc5** (40 mg) was dissolved in 40 mL of THF/5 mM ammonium acetate (4/1). The solution was buffered to pH = 8 with aqueous $NaHCO_3$ and 4 μL 30% H2O2 were added. The reaction mixture was stirred 1 h, THF was removed at reduced pressure and the solution was lyophilized. The residue was purified by column chromatography (C18 gold, 1% - 95% MeCN+0.05% TFA/$H_2O$+0.05% TFA, main separation step at 40%). Cyclized peptide **toc6** (X) was obtained as a white solid. Low yield was accepted in favor of purity.

**LC-MS** (01-95_5 min): *m/z* 1335 $[M+H^+]^+$, $t_R$ = 2.62 min, **HRMS (ESI+):** calculated for $C_{61}H_{86}N_{14}NaO_{16}S_2^+$ $[M+Na^+]^+$ 1357.5685, observed 1357.5688.

**(1-azido-13-oxo-3,6,9-trioxa-12-azahexadecan-16-oyl)-phe-cyclo(Cys-Tyr-trp-Lys-Thr-Cys)-Thr-OH (tate2)**

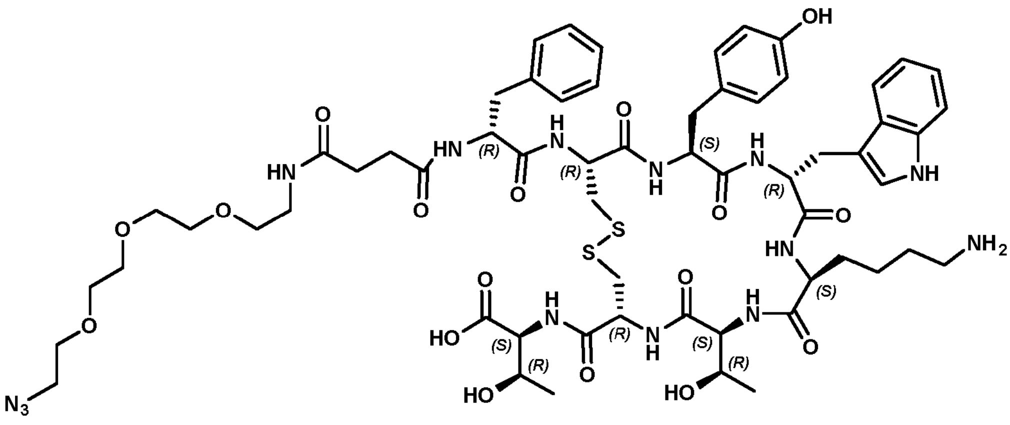

$C_{61}H_{84}N_{14}O_{17}S_2$
Exact Mass: 1348,5580

Automated peptide synthesis at 100 μmol scale with preloaded 2-chlorotrityl chloride resin, cleavage and precipitation was performed as described in **1.1** and afforded linear peptide **tate1.** The crude peptide was purified by column chromatography (C18 gold, 1% - 95% MeCN+0.05% TFA/$H_2O$+0.05% TFA, main separation step at 35%). For disulfide cyclization, linear peptide **tate1** (40 mg) was dissolved in 40 mL of THF/5 mM ammonium acetate (4/1). The solution was buffered to pH

= 8 with aqueous $NaHCO_3$ and 4 μL 30% $H_2O_2$ were added. The reaction mixture was stirred 1 h, THF was removed at reduced pressure and the solution was lyophilized. The residue was purified by column chromatography (C18 gold, 1% - 95% MeCN+0.05% TFA/$H_2O$+0.05% TFA, main separation step at 35%). Cyclized peptide **tate2** (X) was obtained as a white solid. Low yield was accepted in favor of purity.

**LC-MS:** *m/z* 1349 $[M+H^+]^+$, $t_R$ = 2.65 min, **HRMS (ESI-):** calculated for $C_{61}H_{83}N_{14}O_{17}S_2^-$ $[M-H^+]^-$ 1347.5502, observed 1347.5497.

**(1-azido-13-oxo-3,6,9-trioxa-12-azahexadecan-16-oyl)- trp-cyclo(Cys-Thr-phe-Thr-Tyr-Cys)-Lys-OH (tate4)**

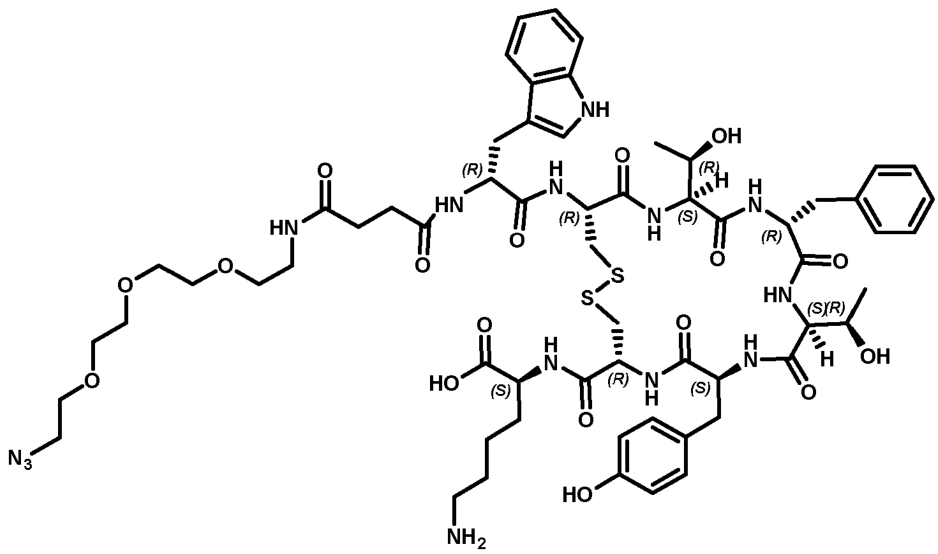

$C_{61}H_{84}N_{14}O_{17}S_2$
**Exact Mass: 1348,5580**

Automated peptide synthesis at 100 μmol scale with preloaded 2-chlorotrityl chloride resin, cleavage and precipitation was performed as described in **1.1** and afforded linear peptide **tate3.** The crude peptide was purified by column chromatography (C18 gold, 1% - 95% MeCN+0.05% TFA/$H_2O$+0.05% TFA, main separation step at 35%). For disulfide cyclization, linear peptide **tate3** (40 mg) was dissolved in 40 mL of THF/5 mM ammonium acetate (4/1). The solution was buffered to pH = 8 with aqueous $NaHCO_3$ and 4 μL 30% H2O2 were added. The reaction mixture was stirred 1 h, THF was removed at reduced pressure and the solution was lyophilized. The residue was purified by column chromatography (C18 gold, 1% - 95% MeCN+0.05% TFA/$H_2O$+0.05% TFA, main separation step at 35%). Cyclized peptide **tate4** (X) was obtained as a white solid. Low yield was accepted in favor of purity.

**LC-MS:** *m/z* 1349 $[M+H^+]^+$, $t_R$ = 2.65 min, **HRMS (ESI-):** calculated for $C_{61}H_{83}N_{14}O_{17}S_2^-$ $[M-H^+]^-$ 1347.5502, observed 1347.5497.

**1.2.3 SSTR2-targeting NIR-fluorescent probes**

**sstr2click1**

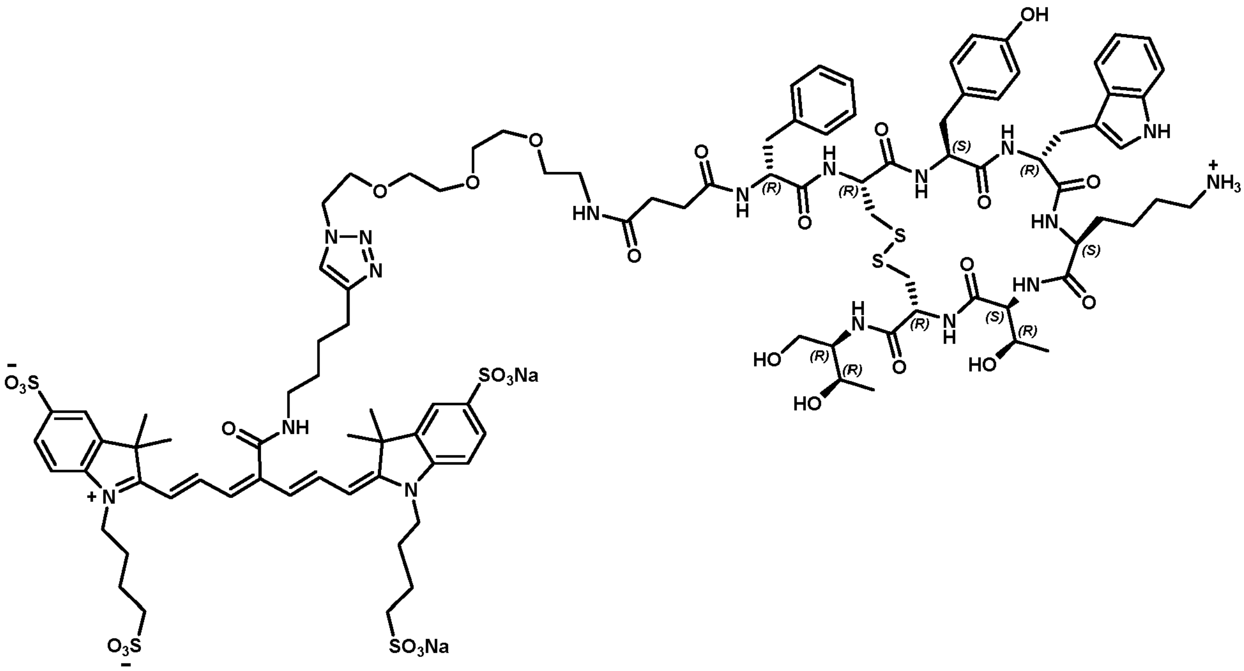

$C_{103}H_{137}N_{17}Na_2O_{29}S_6$
M [g/mol] = 2315,66

Cyclized peptide **toc4** (2.00 mg, 1.50 µmol, 1.00 equiv.) and cyanine **dyealkin5** (1.50 mg, 1.50 µmol, 1.00 equiv.) were under light exclusion dissolved in degassed MeOH (250 µL, 6 mM). In parallel, THPTA (2.60 mg, 5.99 µmol, 4.00 equiv.) and sodium ascorbate (0.89 mg, 4.50 µmol, 4.00 equiv.) were added to degassed aqueous $CuSO_4$ (50 mM, 120 µL, 5.99 µmol, 4.00 equiv.). The copper (I) containing solution was pipetted to the solution of azide and alkyne. The reaction mixture was flushed with argon, sonicated for 1 min, stirred at rt for 2 h and then directly purified by column chromatography (C18 gold, 5% - 95% MeCN+0.05% TFA/$H_2O$+0.05% TFA, main separation step at 25%). Probe **sstr2click1** (1.40 mg, 0.61 µmol, 40%) was obtained as a green solid.

**LC-MS** (05-95_13 min): *m/z* 1136 $[M-2Na^{+}+4H^{+}]^{2+}$, $t_R$ = 6.33 min; **HRMS (ESI-):** calculated for $C_{103}H_{136}N_{17}O_{29}S_2^{3-}$ $[M-2Na^{+}-H^{+}]^{3-}$ 755.6005, observed 755.6006.

**sstr2click2**

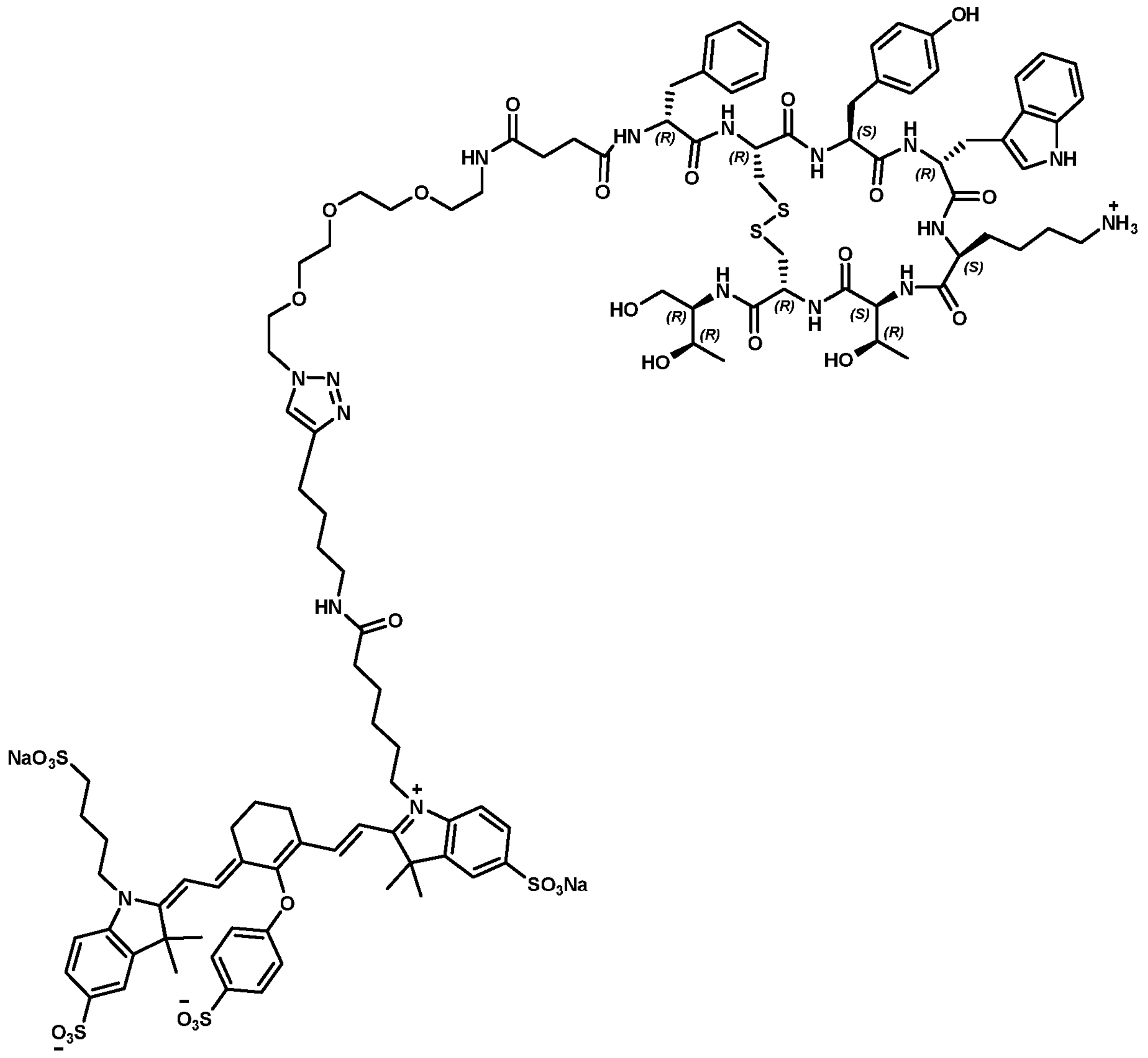

$C_{113}H_{147}N_{17}Na_2O_{30}S_6$
Exact Mass: 2459,8619

Cyclized peptide **toc4** (1.50 mg, 1.12 μmol, 1.00 equiv.) and cyanine **dye2** (1.23 mg, 1.50 μmol, 0.95 equiv.) were under light exclusion dissolved in degassed *tert*-BuOH (180 μL, 6 mM). In parallel, THPTA (1.95 mg, 4.50 μmol, 4.00 equiv.) and sodium ascorbate (0.89 mg, 4.50 μmol, 4.00 equiv.) were added to degassed aqueous $CuSO_4$ (50 mM, 45 μL, 2.25 μmol, 2.00 equiv.). The copper (I) containing solution was pipetted to the solution of azide and alkyne. The reaction mixture was flushed with argon, sonicated for 1 min, stirred at rt for 2 h and then directly purified by column chromatography (C18 gold, 5% - 95% MeCN+0.05% TFA/$H_2O$+0.05% TFA, main separation step at 25%). Probe sstr2click2 (1.26 mg, 0.52 μmol, 49%) was obtained as a green solid.

**LC-MS** (05-95_13 min): *m/z* 1209 $[M-2Na^{+}+4H^{+}]^{2+}$, $t_R$ = 6.62 min; **HRMS (ESI-):** calculated for $C_{113}H_{146}N_{17}O_{30}S_6^{3-}$ $[M-2Na^{+}-H^{+}]^{3-}$ 805.2977, observed 805.6284.

**sstr2click3**

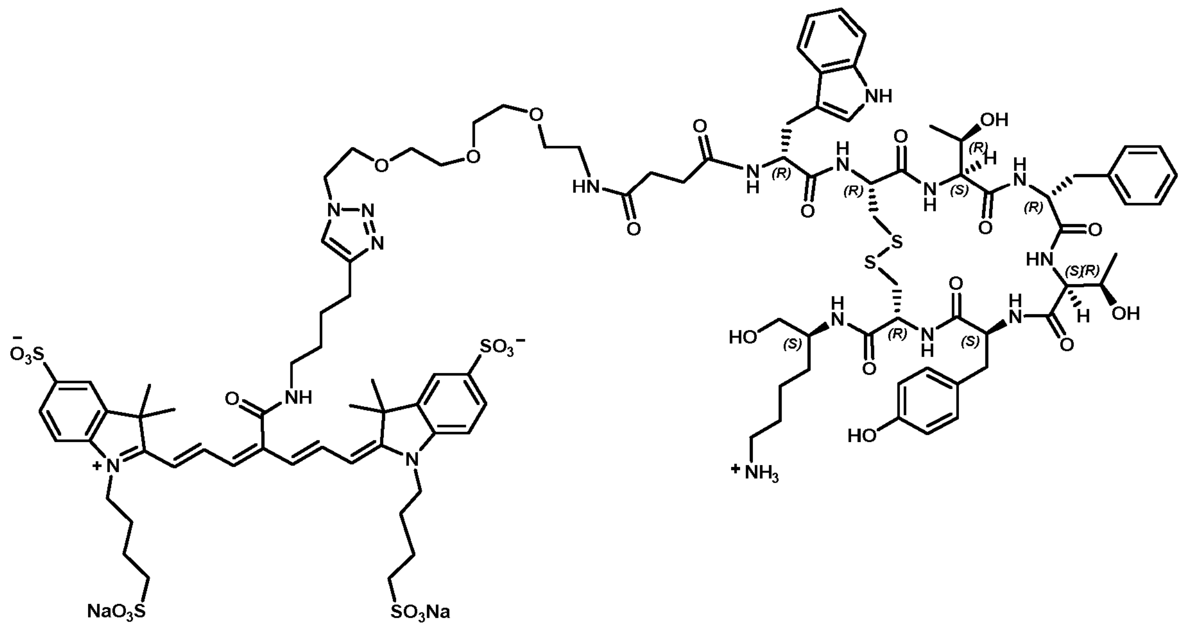

$C_{103}H_{137}N_{17}Na_2O_{29}S_6$
**Exact Mass: 2313,7888**

Cyclized peptide **toc6** (3.30 mg, 2.47 μmol, 1.00 equiv.) and cyanine **dyealkin5** (2.23 mg, 2.22 μmol, 0.90 equiv.) were under light exclusion dissolved in degassed water (370 μL, 6 mM). In parallel, THPTA (4.29 mg, 9.88 μmol, 4.00 equiv.) and sodium ascorbate (1.47 mg, 7.41 μmol, 3.00 equiv.) were added to degassed aqueous $CuSO_4$ (50 mM, 198 μL, 9.88 μmol, 4.00 equiv.). The copper (I) containing solution was pipetted to the solution of azide and alkyne. The reaction mixture was flushed with argon, sonicated for 1 min, stirred at rt for 2 h and then directly purified by column chromatography (C18 gold, 5% - 95% MeCN+0.05% TFA/$H_2O$+0.05% TFA, main separation step at 25%). Probe **sstr2click3** (2.30 mg, 1.01 μmol, 46%) was obtained as a green solid.

**LC-MS** (05-95_13 min): *m/z* 1136 $[M-2Na^{+}+4H^{+}]^{2+}$ $t_R$ = 6.56 min, **HRMS (ESI-):** calculated for $C_{103}H_{136}N_{17}O_{29}S_2^{3-}$ $[M-2Na^{+}-H^{+}]^{3-}$ 755.6005, observed 755.6006.

**sstr2click4**

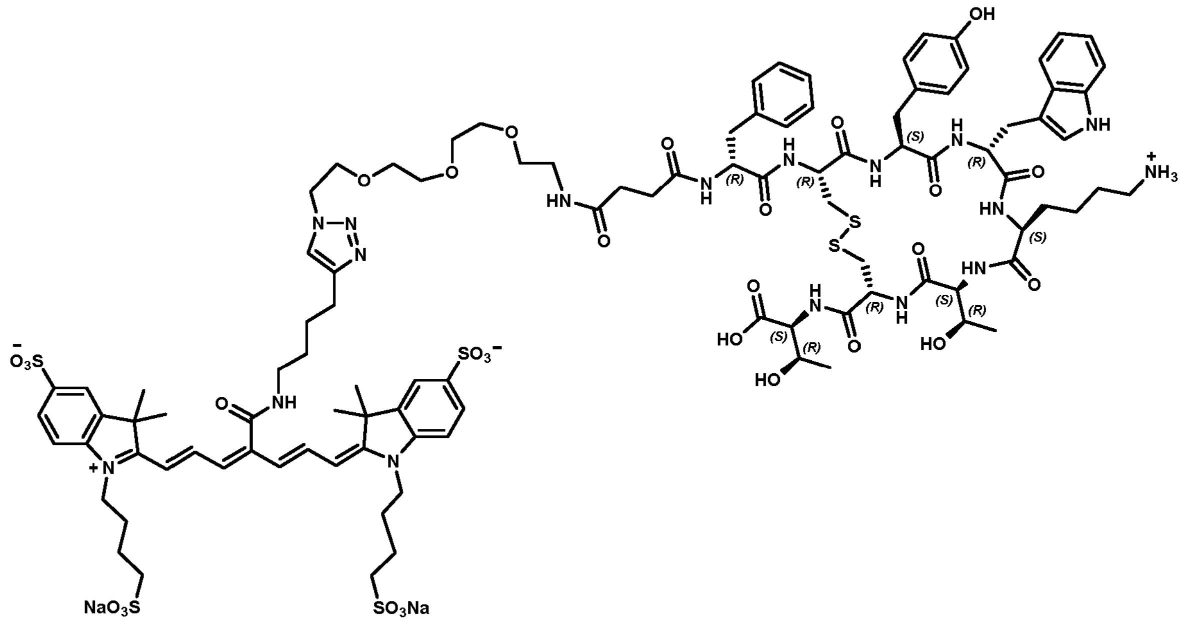

$C_{103}H_{135}N_{17}Na_2O_{30}S_6$
Exact Mass: 2327,7680

Cyclized peptide **tate2** (5.00 mg, 3.71 μmol, 1.00 equiv.) and cyanine **dyealkin5** (3.53 mg, 3.52 μmol, 0.95 equiv.) were under light exclusion dissolved in degassed water (590 μL, 6 mM). In parallel, THPTA (6.44 mg, 14.8 μmol, 4.00 equiv.) and sodium ascorbate (2.20 mg, 11.1 μmol, 3.00 equiv.) were added to degassed aqueous $CuSO_4$ (50 mM, 297 μL, 14.8 μmol, 4.00 equiv.). The copper (I) containing solution was pipetted to the solution of azide and alkyne. The reaction mixture was flushed with argon, sonicated for 1 min, stirred at rt for 2 h and then directly purified by column chromatography (C18 gold, 5% - 95% MeCN+0.05% TFA/$H_2O$+0.05% TFA, main separation step at 25%). Probe **sstr2click4** (1.98 mg, 0.81 μmol, 23%) was obtained as a green solid.

**LC-MS** (05-95_13 min): *m/z* 1143 $[M-2Na^{+}+4H^{+}]^{2+}$ $t_R$ = 7.62 min, **HRMS (ESI-):** calculated for $C_{103}H_{134}N_{17}O_{30}S_2^{3-}$ $[M-2Na^{+}-H^{+}]^{3-}$ 760.2602, observed 760.2604.

**sstr2click5**

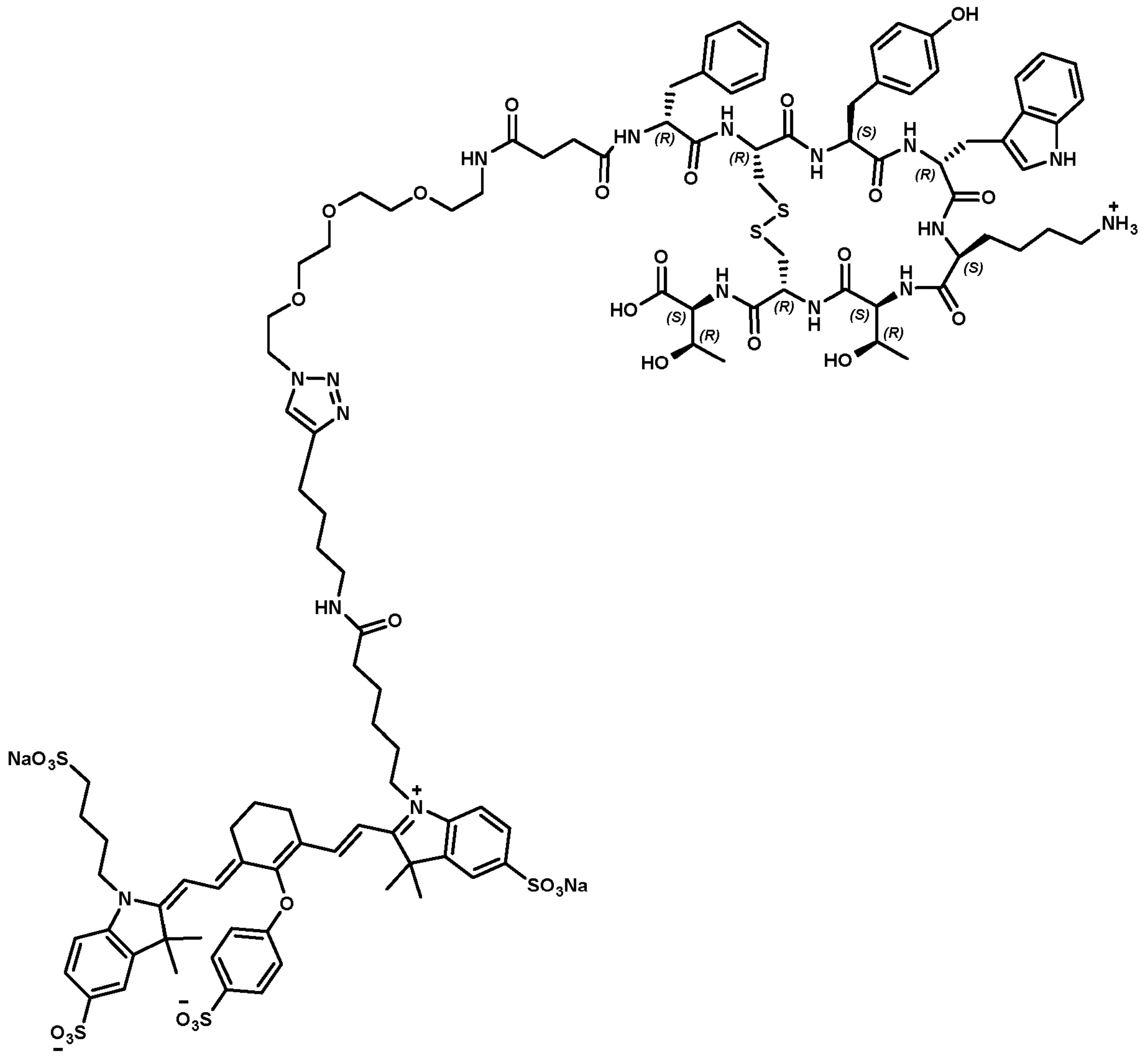

$C_{113}H_{145}N_{17}Na_2O_{31}S_6$
Exact Mass: 2473,8412

Probe **sstr2click5** was synthesized in a similar fashion as described for **sstr2click3.**

**LC-MS** (05-95_13 min): *m/z* 1216 $[M\text{-}2Na^{+}+4H^{+}]^{2+}$ $t_R$ = 7.68 min.

**sstr2click6**

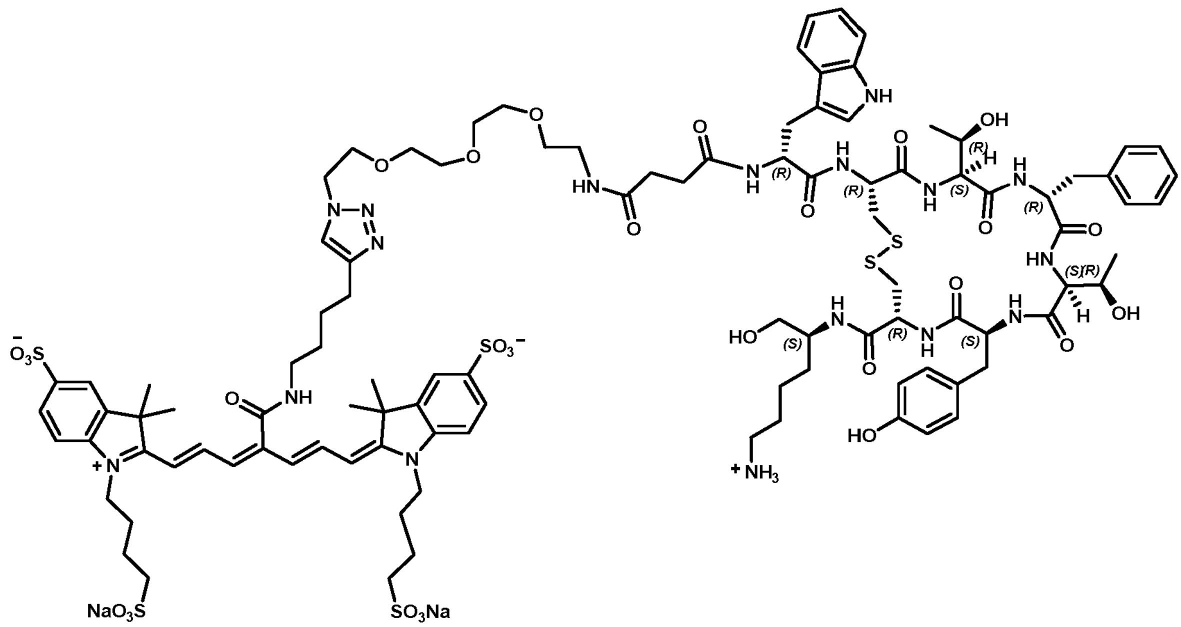

$C_{103}H_{137}N_{17}Na_2O_{29}S_6$
**Exact Mass: 2313,7888**

Probe sstr2click6 was synthesized in a similar fashion as described for **sstr2click2.**

**LC-MS** (05-95_13 min): *m/z* 1143 $[M-2Na^{+}+4H^{+}]^{2+}$ $t_R$ = 8.63 min, **HRMS (ESI-):** calculated for $C_{103}H_{135}N_{17}O_{30}S_2^{2-}$ $[M-2Na^{+}]^{2-}$ 1140.8943, observed 1140.8931.

PSMA0

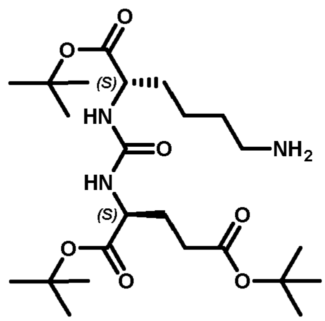

$C_{24}H_{45}N_3O_7$
Exact Mass: 487,3258

Urea **PSMA0** was synthesized according to literature (20, compound 3).

**PSMA1**

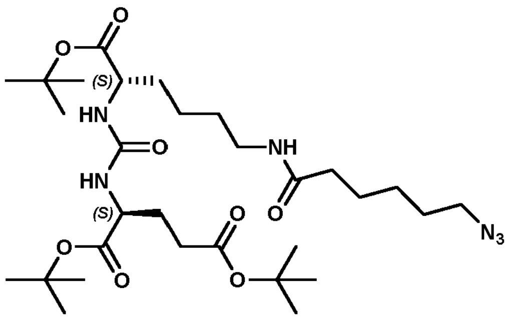

$C_{30}H_{54}N_6O_8$
Exact Mass: 626,4003

Amine **PSMA0** (190 mg, 0.39 mmol, 1.0 equiv.) and N3-$(CH_2)_5$-CO-OSu (100 mg, 0.39 mmol, 1.0 equiv.) were dissolved in $CHCl_3$ (2 mL, 0.1 M), stirred at rt for 3 h and concentrated under reduced pressure. After purification by column chromatography (C18 gold, $H_2O$/MeCN, 5%-95%, main separation step at 80%), urea **PSMA1** (223 mg, 0.36 mmol, 91%) was obtained as a colorless oil.

**LC-MS** (01-90_5min): *m/z* 628 $[M+H^+]^+$, $t_R$ = 3.80 min; **HRMS (ESI+):** calculated for $C_{30}H_{54}N_6O_8Na^+$ $[M+Na^+]^+$ 810.4589, observed 810.4564; **$^1$H NMR** (400 MHz, $CDCl_3$): δ = 4.34 - 4.24 (m, 2H), 3.27 (t, J = 6.5Hz, 3H), 2.39 - 2.26 (m, 2H), 2.20 (t, J = 7.7Hz, 2H), 2.12 - 2.03 (m, 1H), 1.90 - 1.73 (m, 2H), 1.69 - 1.58 (m, 5H), 1.54 - 1.36 (m, 33H) ppm; $^{13}$C NMR (400 MHz, $CDCl_3$): δ = 173.2, 172.5, 157.2, 128.6, 82.4, 81.9, 81.9, 80.8, 53.6, 53.3, 51.5, 39.1, 36.5, 32.7, 31.8, 28.9, 28.8, 28.3, 28.8, 28.8, 26.5, 25.7, 25.4, 24.3 ppm.

**PSMA2**

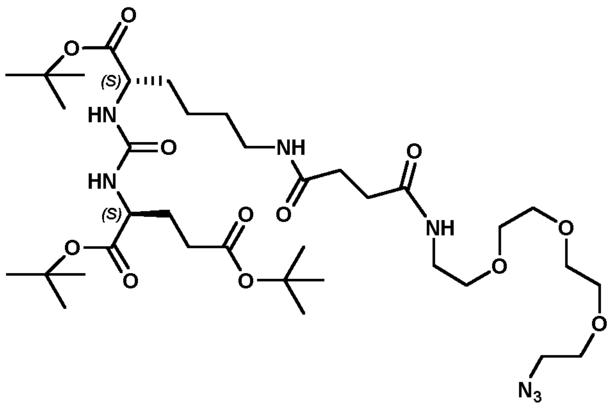

$C_{36}H_{65}N_7O_{12}$
Exact Mass: 787,4691

Amine **PSMA0** (100 mg, 0.24 mmol, 1.0 equiv.) and N3-PEG(3)-NHCO-$(CH_2)_2$-CO-OSu (117 mg, 0.24 mmol, 1.0 equiv.) were dissolved in $CHCl_3$ (2 mL, 0.1 M), stirred at rt for 3 h and concentrated under reduced pressure. After purification by column chromatography (C18 gold, $H_2O$/MeCN, 5%-95%, main separation step at 75%), urea **PSMA2** (91 mg, 0.12 mmol, 48%) was obtained as a colorless oil.

**LC-MS** (01-90_5min): *m/z* 798 $[M+H^+]^+$, $t_R$ = 3.64 min; **HRMS (ESI+):** calculated for $C_{36}H_{65}N_7O_{12}Na^+$ $[M+Na^+]^+$ 810.4589, observed 810.4564; **$^1$H NMR** (400 MHz, $CDCl_3$): δ = 7.03 (m, 2H), 6.83 (m, 2H), 6.08 (d, J = 8.6Hz, 2H), 5.86 (d, J = 8.9Hz, 2H), 5.45 (m, 7H), 4.33 - 4.25 (m, 13H), 3.65 - 3.56 (m, 25H), 3.52 - 3.43 (m, 8H), 3.37 - 3.34 (m, 10H), 2.69 - 2.59 (m, 11H), 4.33 - 4.25 (m, 13H), 2.43 - 2.21 (m, 19H), 2.07 - 1.97 (m, 12H), 1.83 - 1.66 (m, 12H) ppm; **$^{13}$C NMR** (400 MHz, $CDCl_3$): δ = 173.5, 172.8, 172.4, 172.4, 157.6, 157.1, 82.0, 81.6, 81.1, 80.5, 70.7, 70.3, 70.1, 69.8, 53.5, 53.0, 50.7, 41.8, 39.4, 38.7, 33.0, 31.8, 31.7, 31.5, 31.2, 28.1, 28.1, 22.5, 21.6 ppm.

**PSMA3**

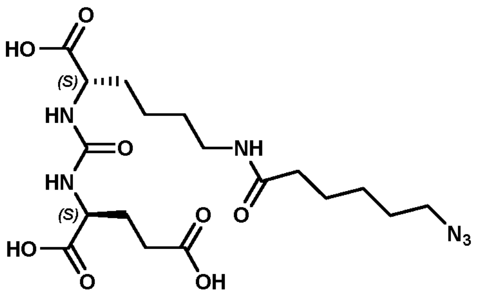

$C_{18}H_{30}N_6O_8$
Exact Mass: 458,2125

Urea **PSMA1** (100 mg, 0.16 mmol, 1.0 equiv.) was dissolved in $CH_2Cl_2$/TFA (1:1, 4 mL, 0.02 M), stirred over night at rt and concentrated under reduced pressure. After column chromatographic purification (C18 gold, $H_2O$ + 0.05% TFA/MeCN + 0.05% TFA, 5%-95%, main separation step at 42%) urea **PSMA3** (18 mg, 0.04 mmol, 24%) was obtained as a yellow oil.

**LC-MS** (01-90_5min): *m/z* 459 $[M+H^+]^+$, $t_R$ = 2.39 min; **HRMS (ESI+):** calculated for $C_{18}H_{30}N_6O_8Na^+$ $[M+Na^+]^+$ 481.2023, observed 481.2018; **$^1$H NMR** (400 MHz, MeOD): δ = 4.33 (q, J = 4.9Hz, 1H), 4.28 (q, J = 4.9Hz, 1H), 3.37 (s, 1H), 3.34 - 3.29 (m, 1H), 3.19 (t, J = 6.9Hz, 2H), 2.46 - 2.41 (m, 2H), 2.23 - 2.12 (m, 3H), 1.96 - 1.82 (m, 2H), 1.73 - 1.61 (m, 5H), 1.59 - 1.51 (m, 2H), 1.48 - 1.39 (m, 4H) ppm; **$^{13}$C NMR** (400 MHz, MeOD): δ = 176.4, 176.4, 176.0, 175.8, 160.1, 53.9, 53.5, 52.3, 40.1, 36.9, 33.2, 31.1, 29.9, 29.6, 28.9, 27.4, 26.5, 24.0 ppm.

**PSMA4**

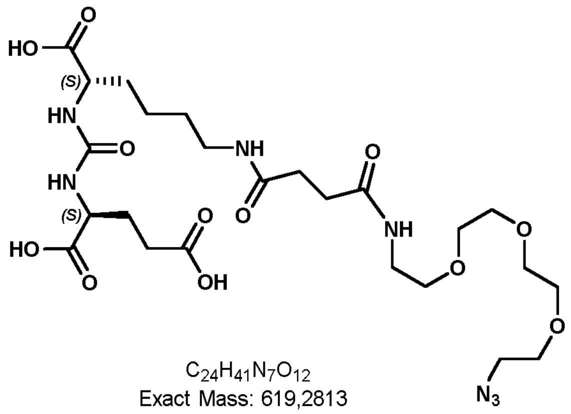

$C_{24}H_{41}N_7O_{12}$
Exact Mass: 619,2813

Urea **PSMA2** (100 mg, 0.13 mmol, 1.0 equiv.) was dissolved in $CH_2Cl_2$/TFA (1:1, 4 mL, 0.02 M), stirred over night at rt and concentrated under reduced pressure. After column chromatographic purification (C18 gold, $H_2O$ + 0.05% TFA/MeCN + 0.05% TFA, 5%-95%, main separation step at 30%), urea **PSMA4** (23 mg, 0.04 mmol, 29%) was obtained as a yellow oil.

**LC-MS** (01-90_5min): *m/z* 620 $[M+H^+]^+$, $t_R$ = 2.32 min; **HRMS (ESI-):** calculated for $C_{24}H_{40}N_7O_{12}$ $[M-H^+]^-$ 618.2735, observed 618.2725; **$^1$H NMR** (400 MHz, MeOD): δ = 4.31 (q, J = 5.3Hz, 1H), 4.26 (q, J = 4.8Hz, 1H), 3.68 - 3.60 (m, 8H), 3.55 - 3.53 (m, 2H), 3.39 - 3.35 (m, 3H), 3.23 - 3.10 (m, 2H), 2.50 - 2.39 (m, 5H), 2.19 - 2.10 (m, 1H), 1.94 - 1.79 (m, 2H), 1.70 - 1.61 (m, 1H), 1.57 - 1.39 (m, 4H) ppm; **$^{13}$C NMR** (400 MHz, MeOD): δ = 176.4, 176.4, 175.9, 174.8, 174.6, 160.1, 71.6, 71.6, 71.5, 71.3, 71.1, 70.5, 53.9, 53.5, 51.8, 40.4, 40.0, 33.1, 32.3, 32.3, 31.1, 29.8, 28.9, 23.8 ppm.

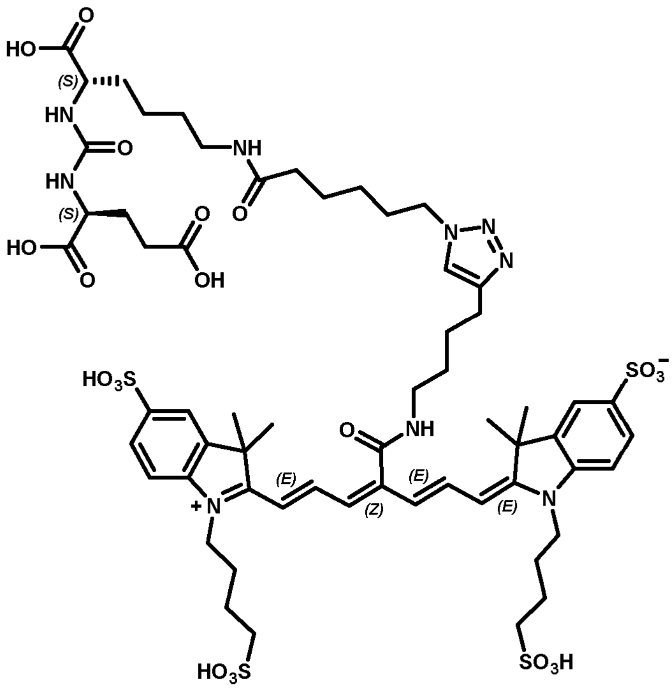

$C_{60}H_{83}N_9O_{21}S_4$
Exact Mass: 1393,4586

**NIR-fluorescent KUE Ligand I**

A solution of urea **PSMA3** (5.00 mg, 0.01 mmol, 1.0 equiv.) in tBuOH (1 mL, 0.01 M) was added to a solution of **dyealkin5** (10.0 mg, 0.01 mmol, 0.95 equiv.) in water (1 mL, 0.01 M). The mixture was combined with an aqueous solution of $CuSO_4$ (0.05 M, 872 µL, 0.04 mmol, 4.0 equiv.), THPTA (19.0 mg, 0.04 mmol, 4.0 equiv.) and sodium ascorbate (18.0 mg, 0.09 mmol, 8.0 equiv.) and stirred for 2 h at rt. The reaction solution was directly purified by column chromatography (C18 gold, $H_2O$ + 0.05% TFA/MeCN + 0.05% TFA, 5%-90%, main separation step at 20%), **NIR-fluorescent KUE Ligand I** (4.2 mg, 3.01 µmol, 29%) was obtained as a green solid.

**LC-MS** (05-95_5min): *m/z* 696 $[M-2H^+]^{2-}$, $t_R$ = 5.86 min.

**NIR-fluorescent KUE Ligand II**

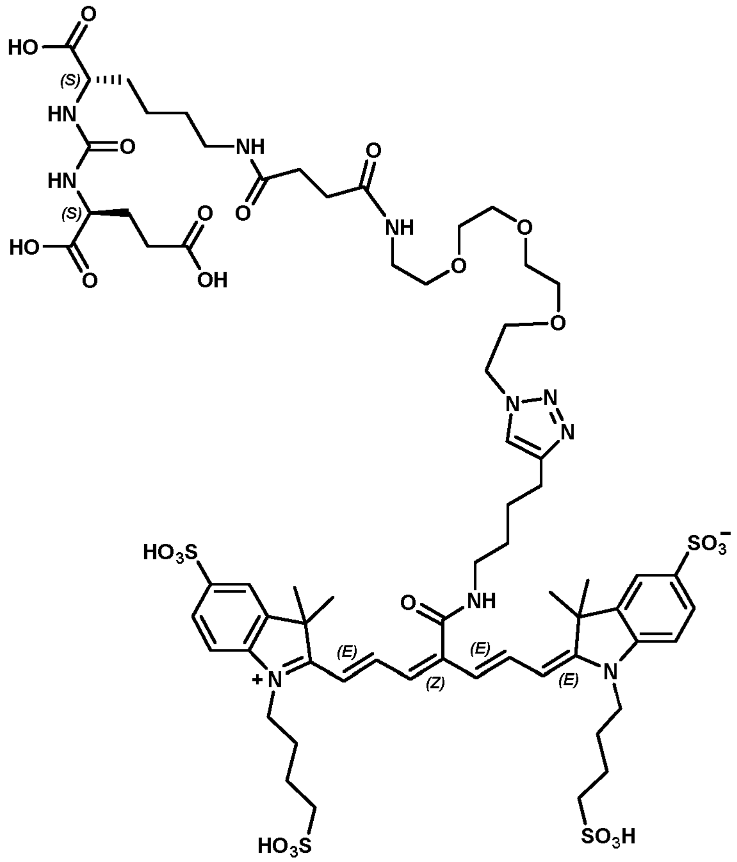

$C_{66}H_{94}N_{10}O_{25}S_4$
Exact Mass: 1554,5274

A solution of urea **PSMA4** (5.00 mg, 0.01 mmol, 1.0 equiv.) in tBuOH (1 mL, 0.01 M) was added to a solution of **dyealkin5** (8.0 mg, 0.01 mmol, 0.95 equiv.) in water (1 mL, 0.01 M). The mixture was combined with an aqueous solution of $CuSO_4$ (0.05 M, 872 μL, 0.04 mmol, 4.0 equiv.), THPTA (19.0 mg, 0.04 mmol, 4.0 equiv.) and sodium ascorbate (18.0 mg, 0.09 mmol, 8.0 equiv.) and stirred for 2 h at rt. The reaction solution was directly purified by column chromatography (C18 gold, $H_2O$ + 0.05% TFA/MeCN + 0.05% TFA, 5%-90%, main separation step at 20%), **NIR-fluorescent KUE Ligand II** (2.7 mg, 1.7 μmol, 17%) was obtained as a green solid.

**LC-MS** (05-95_5min): *m/z* 777 $[M-2H^+]^{2-}$, $t_R$ = 5.93 min.

**NIR-fluorescent Panitumumab**

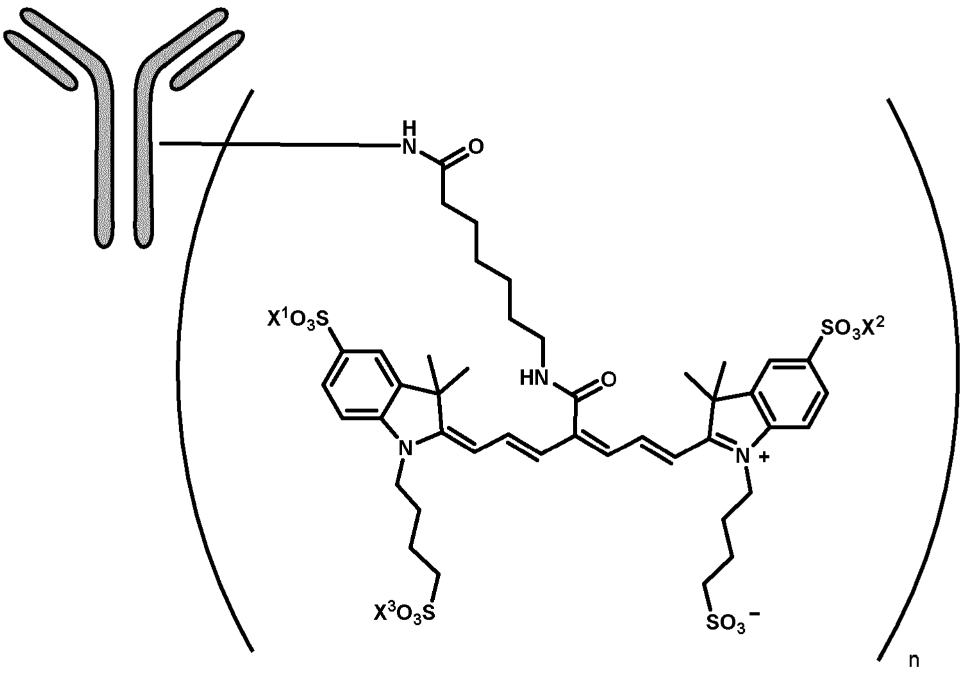

**Panitumumab** (20 mg/mL commercial stock solution, 50 μL, 7 nmol) was added to 25 μL of PBS (50 mM, pH 7.4) in a 1.5 mL microcentrifuge tube. Dyecarb7 (1 mM stock in DMSO, 50 μL, 7 equiv) was added to the Panitumumab solution, vortexed and incubated at room temperature in the dark for 100 min. The resulting solutions were eluted through a Zeba spin desalting column (7 kDa MWCO, Thermo Fisher Scientific) to remove unreacted free dye. UV/VIS of the filtrate dilution was acquired directly and used to calculate a density of labeling (DOL) of 1.6 using the following equation:

$$DOL = \frac{A_{785}}{\varepsilon_{sNIR\ 785}} \div \frac{A_{280} - 0.064 * A_{785}}{\varepsilon_{Protein}}$$

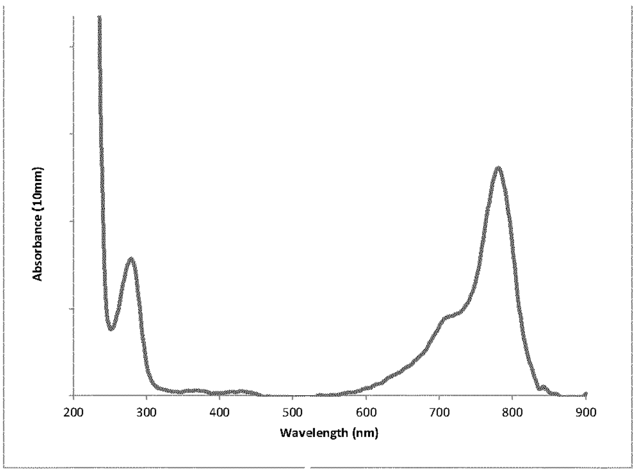


Antibody fluorophore conjugates were used for animal studies within 1 week after labeling. Panitumumab-IRDye800 was prepared in a similar fashion.

**Ber0**

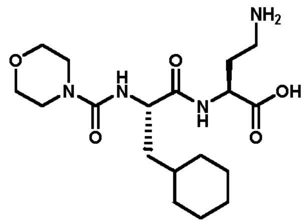

$C_{18}H_{32}N_4O_5$

Peptide **ber0** was synthesized according to literature (21, compound 3).

**Ber1**

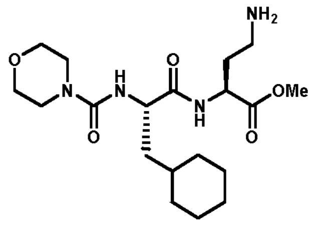

$C_{19}H_{34}N_4O_5$

A solution of peptide **ber0** (130 mg, 0.27 mmol, 1.0 equiv.) in MeOH (270 μL, 1.0 M) was cooled to 0°C and $SOCl_2$ (60 μL, 0.81 mmol, 3.0 equiv.) was added. The reaction was stirred for 2 h at room temperature. The solvent was removed in vacuo to afford the desired product ber1 (95 mg, 0.21 mmol, 78%) as a colorless solid.

**LCMS** (Agilent, unpol, C8): $t_R$ = 0.13, *m*/*z* = 421 $[M+Na]^+$; **ESI-HRMS:** *m*/*z* calc. for $C_{19}H_{34}N_4O_5Na$ $[M+Na]^+$: 421,2427 observed: 421.2427; **$^1$H-NMR** (400 MHz, MeOD) δ = 4.66-4.54 (m, 1H, CH), 4.30-4.14 (m, 1H, CH), 3.76 (s, 3H, $CH_3$), 3.70-3.61 (m, 4H, H-1,1'), 3.48-3.52 (m, 4H, **H-2,2'),** 3.07-3.01 (m, 2H, **H-13),** 2.33-2.21 (m, 1H, **H-12),** 2.10-1.97 (m, 1H, **H-12),** 1.81-1.19 (m, 11H, **H-5,6,7,8,9,10,4),** 1.03-0.92 (m, 2H, **H-5,6,7,8,9,10,4)** ppm; **$^{13}$C-NMR** (100 MHz, MeOD) δ = 177.2 ($C_q$), 172.5 **($C_q$),** 159.9 ($C_q$), 67.6 **(C-1,1'),** 54.4 (CH), 53.1 ($CH_3$), 50.9 (CH), 45.3 **(C-2,2'),** 40.2 **(C-13),** 37.7 **(C-4),** 35.3 **(C-6,7,8,9,10),** 34.7 **(C-6,7,8,9,10),** 33.7 **(C-12),** **30.6 (C-5),** 27.6 **(C-6,7,8,9,10),** 27.4 **(C-6,7,8,9,10),** 27.3 **(C-6,7,8,9,10)** ppm.

**Ber3**

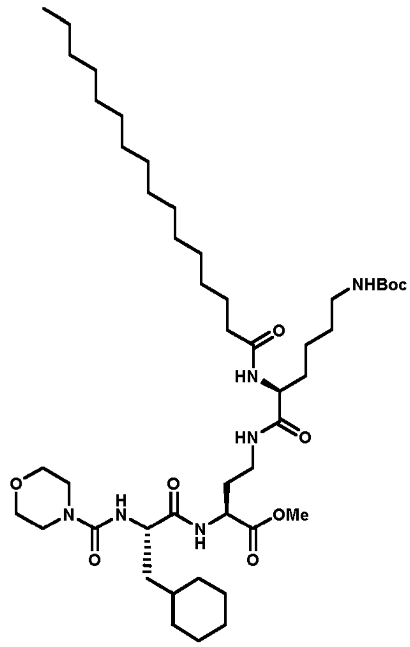

$C_{46}H_{84}N_6O_9$

A solution of peptide **ber1** (50 mg, 0.125 mmol, 1.0 equiv.) was dissolved in DMF (250 µL, 0.02 M) and mixed with side chain **ber2** (synthesized as described in literature (20, compound 8), 49 mg, 0.100 mmol, 0.8 equiv.), HATU (72 mg, 0.188 mmol, 1.5 equiv.) and DIPEA (110 µL, 0.627 mmol, 5.0 equiv.) and stirred for 20h at room temperature. Column chromatographic purification ($CH_2Cl_2$/MeOH = 0-10%) afforded the desired Boc-protected amine **ber3** (76 mg, 0.003 mmol, 88%) as a colorless solid.

**LCMS (Thermofisher, PFP):** $t_R$ = 10.09, *m/z* = 866 $[M+H]^+$; **ESI-HRMS:** *m/z* calc. für $C_{81}H_{117}N_8O_{20}S_4$ $[M-3K+4H]^+$: 1649.7267, gefunden: 1649.7327; $^1$H-NMR (600 MHz, $CDCl_3$) δ = 6.27-6.03 (m, 5H, **NH),** 3.76-3.66 (m, 10H, CH, **CH$_2$,** O-$CH_3$), 3.43-3.33 (m, 1H; $CH_2$)**,** 3.22-3.15 (m, 8H, C**H,** C**H**$_2$)**,** 3.12-3.04 (m, 1H, $CH_2$)**,** 3.01-2.95 (m, 1H, C**H**), 1.49-1.39 (m, 48H, C**H**, C**H$_2$,** C**H**$_3$), 1.30-1.22 (m, 9H, C**H**$_2$), 0.87 (t, 3H, *J* = 7.0 Hz, C**H**$_3$) ppm; **$^{13}$C-NMR** (150 MHz, $CDCl_3$) δ = 175.7 (**C**$_q$), 174.0 (**C**$_q$), 172.6 (**C**$_q$), 171.6 (**C**$_q$), 158.0 (**C**$_q$), 156.2 (**C**$_q$), 79.3 (**C**$_q$), 66.6 (**C**$H_2$), 55.9 (**C**$H_3$), 53.9 (**C**H), 52.6 (**C**H), 52.2 (**C**H), 43.8 (**C**$H_2$), 32.1 (**C**$H_2$), 29.8 (**C**$H_3$), 29.5 (**C**$H_3$), 28.6 (**C**H), 22.8 (**C**$H_2$), 18.7 (**C**$H_2$), 17.3 (**C**$H_2$), 12.7 (**C**$H_3$), ppm.

**Ber4**

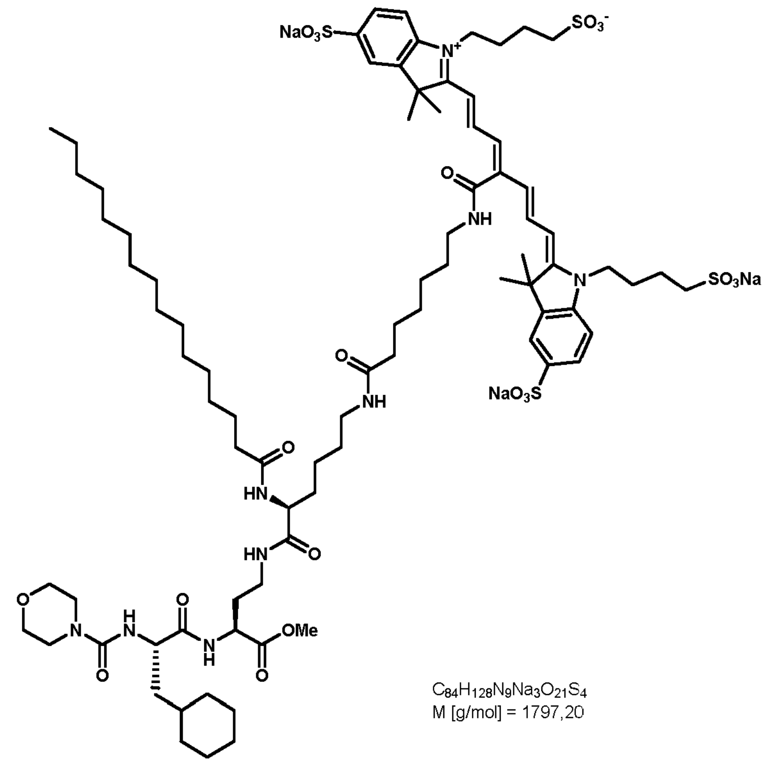

$C_{84}H_{128}N_9Na_3O_{21}S_4$
M [g/mol] = 1797,20

A solution of Boc-protected amine **ber3** (3 mg, 0.004 mmol, 1.0 equiv.) in TFA/$CH_2Cl_2$ (400 μL, 0.01 M) was stirred at rt for 30 min and then concentrated under reduced pressure. The residue was dissolved in DMF (400 μL, 0.01 M) and mixed with **dyecarb7** (3 mg, 0.004 mmol, 1.0 equiv.) and DIPEA (3 μL, 0.020 mmol, 5.0 equiv.) and stirred for 1h at room temperature. Column chromatographic purification (C18 WP, $H_2O$(TFA)/ACN(TFA) = 5-95%) afforded the desired methylester **ber4** (2 mg, 0.002 mmol, 42%) as a green solid.

**LCMS (Thermofisher PFP):** $t_R$ = 8.69, *m/z* = 866 $[M\text{-}3Na\text{+}5H]^{2+}$; **ESI-HRMS:** *m/z* calc. für $C_{84}H_{131}N_9O_{21}S_4Na$ $[M\text{-}2Na\text{+}3H]^+$: 1752.8240, gefunden: 1752.8240; **$^1$H-NMR** (600 MHz, MeOD) δ = 7.92-7.83 (m, 6H, $CH_{aromatisch}$), 7.34 (d, 2H, J = 8.3 Hz, $CH_{aromatisch}$), 6.53 (dd, 4H, *J* = 39.9, 13.4 Hz, C**H**$_{aromatisch}$), 4.46 (dd, 1H, *J* = 10.5, 4.3 Hz, C**H**), 4.30 (dd, 1H, *J* = 9.0, 6.3 Hz, CH), 4.21-4.14 (m, 5H, C**H**, $CH_2$), 3.71 (s, 3H, O-C**H**$_3$), 3.66-3.62 (m, 4H, $CH_2$), 3.51-3.47 (m, 2H, $CH_2$), 3.43-3.39 (m, 2H, $CH_2$), 3.26-3.22 (m, 2H, $CH_2$), 3.16 (t, 2H, *J* = 7.1 Hz, $CH_2$), 2.90 (t, 4H, *J* = 6.9 Hz, $CH_2$), 2.25 (t, 2H, *J* = 7.5 Hz, $CH_2$), 2.21 (t, 2H, *J* = 7.5 Hz, $CH_2$), 2.12-2.05 (m, 2H, $CH_2$), 1.99-1.92 (m, 8H, $CH_2$), 1.84-1.57 (m, 24H, **CH$_2$**, $CH_3$), 1.52-1.16 (m, 38H, C**H**, C**H**$_2$,), 1.00-0.87 (m, 7H, **CH$_2$**, $CH_3$) ppm; **$^{13}$C-NMR** (150 MHz, MeOD) δ = 176.6 (**C**$_q$), 176.4 (**C**$_q$), 175.9 (**C**$_q$), 174.8 (**C**$_q$), 174.0 (**C**$_q$), 173.6 (**C**$_q$), 169.9 (**C**$_q$), 159.8 (**C**$_q$), 148.8 (**C**H$_{aromatisch}$), 144.9 (**C**$_q$), 143.6 (**C**$_q$), 142.4 (**C**$_q$), 128.3 (**C**H$_{aromatisch}$), 124.5 (**C**H$_{aromatisch}$), 121.4 (**C**H$_{aromatisch}$), 111.9 (**C**H$_{aromatisch}$), 106.7 (**C**H$_{aromatisch}$), 67.7 (**C**H$_2$), 55.2 (**C**H), 54.2 (**C**H), 52.8 (O-**C**H$_3$), 51.7 (**C**H, **C**H$_2$), 50.4 (**C**$_q$), 45.4 (**C**H$_2$), 45.3 (**C**H$_2$), 41.0 (**C**H$_2$), 40.5 (**C**H$_2$), 40.1 (**C**H$_2$), 37.3 (**C**H$_2$), 37.1 (**C**H$_2$), 36.8 (**C**H$_2$), 35.4 (**C**H), 34.8 (**C**H$_2$), 33.7 (**C**H$_2$), 33.1 (**C**H$_2$), 32.5 (**C**H$_2$), 31.7 (**C**H$_2$), 31.2 (**C**H$_2$), 30.8 (**C**H$_2$), 30.7 (**C**H$_2$), 30.5 (**C**H$_2$), 30.4 (**C**H$_2$), 30.2 (**C**H$_2$), 30.1 (**C**H$_2$), 28.3 (**C**H$_3$), 28.1 (**C**H$_2$), 27.7 (**C**H$_2$), 27.4 (**C**H$_2$), 27.3 (**C**H$_2$), 26.9 (**C**H$_2$), 24.5 (**C**H$_2$), 23.7 (**C**H$_2$), 23.5 (**C**H$_2$), 14.5 (**C**H$_3$) ppm.

**NIR-fluorescent CatS substrate**

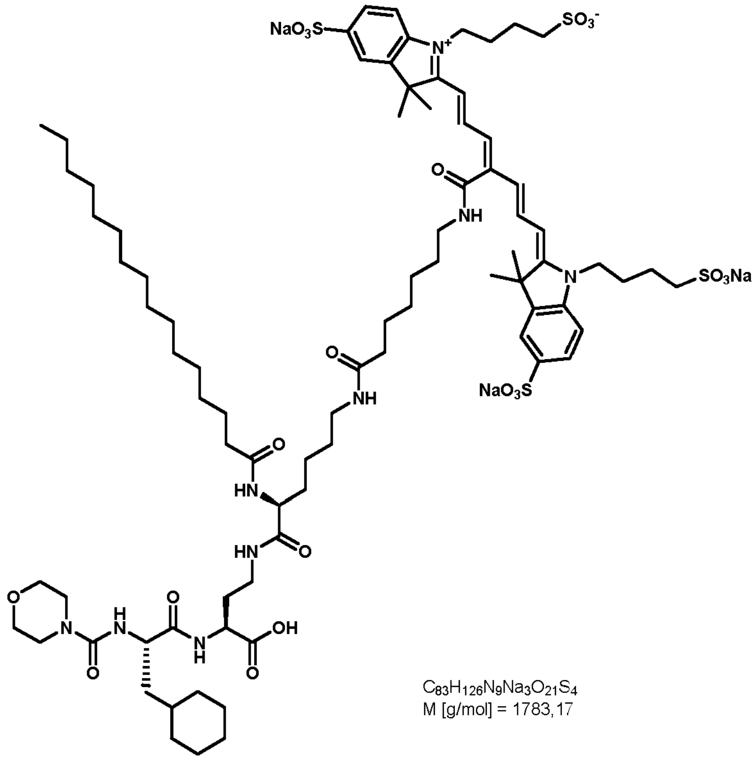

$C_{83}H_{126}N_9Na_3O_{21}S_4$
M [g/mol] = 1783,17

Methylester **ber4** was solved in THF/$H_2O$ (3:1, 800 μL, 0.003 M) and mixed with a 0.6 M solution of LiOH*$H_2O$ in $H_2O$ (24 μL, 0.014 mmol, 8.0 equiv.) and stirred for 2 h at room temperature and neutralized by the addition of acetic acid (814 μL, 0.014 mmol, 8.0 equiv.). The reaction solution was diluted with $H_2O$ and lyophilised. Column chromatographic purification (C18-WP, $H_2O$(TFA)/ACN(TFA) = 5-95%) afforded **NIR-fluorescent CatS substrate** (1.7 mg, 0.002 mmol, 86%) as a green solid.

**LCMS (Thermofisher PFP):** $t_R$ = 8.11, *m/z* = 859 $[M-3Na+5H]^{2+}$; **ESI-HRMS:** *m/z* calc. für $C_{83}H_{129}N_9O_{21}S_4Na$ $[M-2Na+3H]^+$: 1738.8084, gefunden: 1738.8113; **$^1$H-NMR** (500 MHz, MeOD) δ = 7.93-7.82 (m, 6H, C**H**$_{aromatisch}$), 7.40 (d, 2H, *J* = 8.3 Hz, C**H**$_{aromatisch}$), 6.53 (dd, 4H, *J* = 33.0, 13.4 Hz, C**H**$_{aromatisch}$), 4.43 (dd, 1H, *J* = 10.3, 4.3 Hz, **CH),** 4.31 (dd, 1H, *J* = 9.1, 6.2 Hz, C**H**), 4.23-4.11 (m, 5H, **CH,** $CH_2$), 3.67-3.60 (m, 4H, $CH_2$), 3.46-3.39 (m, 4H, $CH_2$), 3.19-3.14 (m, 2H, $CH_2$), 2.90 (t, 4H, *J* = 6.6 Hz, $CH_2$), 2.25-2.08 (m, 6H, $CH_2$), 2.01-1.92 (m, 8H, $CH_2$), 1.84-1.57 (m, 30H, C**H$_2$,** C**H**$_3$), 1.53-1.17 (m, 37H, **CH,** $CH_2$,), 0.99-0.87 (m, 7H, **CH$_2$,** $CH_3$) ppm; **$^{13}$C-NMR** (150 MHz, MeOD) δ = 176.5 (**C**$_q$), 176.4 (**C**$_q$), 176.0 (**C**$_q$), 174.7 (**C**$_q$), 174.0 (**C**$_q$), 169.9 (**C**$_q$), 159.8 (**C**$_q$), 148.8 (**C**H$_{aromatisch}$), 144.9 (**C**$_q$), 143.5 (**C**$_q$), 142.4 (**C**$_q$), 130.8 (**C**$_q$), 130.2 (**C**$_q$), 128.3 (**C**H$_{aromatisch}$), 127.9 (**C**H$_{aromatisch}$), 124.4 (**C**H$_{aromatisch}$), 111.9 (**C**H$_{aromatisch}$), 106.7 (**C**H$_{aromatisch}$), 67.7 (**C**H$_2$), 55.2 (**C**H), 54.2 (**C**H), 51.7 (**C**H$_2$), 51.6 (**C**H), 50.5 (**C**$_q$), 45.4 (**C**H$_2$), 45.3 (**C**H$_2$), 41.0 (**C**H$_2$), 40.4 (**C**H$_2$), 40.1 (**C**H$_2$), 37.3 (**C**H$_2$), 37.1 (**C**H$_2$), 36.8 (**C**H$_2$), 35.3 (**C**H), 34.9 (**C**H$_2$), 33.6 (**C**H$_2$), 33.1 (**C**H$_2$), 32.6 (**C**H$_2$), 31.7 (**C**H$_2$), 30.8 (**C**H$_2$), 30.7 (**C**H$_2$), 30.5 (**C**H$_2$), 30.2 (**C**H$_2$), 30.1 (**C**H$_2$), 28.3 (**C**H$_3$), 28.1 (**C**H$_2$), 27.7 (**C**H$_2$), 27.4 (**C**H$_2$), 27.3 (**C**H$_2$), 27.0 (**C**H$_2$), 26.9 (**C**H$_2$), 24.5 (**C**H$_2$), 23.7 (**C**H$_2$), 23.5 (**C**H$_2$), 14.5 (**C**H$_3$) ppm.

**Leptin-receptor-agonist-I**

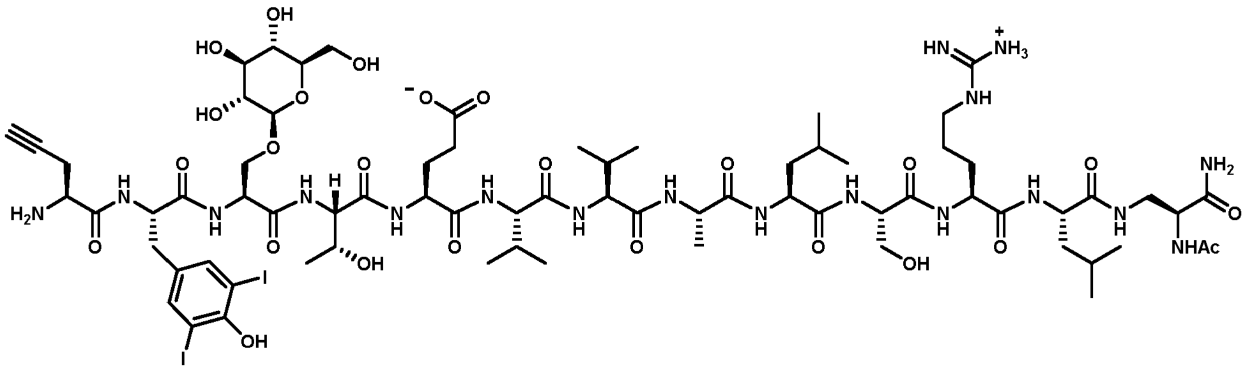

$C_{71}H_{114}I_2N_{18}O_{25}$
**M [g/mol] = 1873,60**

**Leptin-receptor-agonist-I** was synthesized according to literature (22), with the difference that an additional propargyl glycine was added at the N-terminus. Synthesis was performed using Fmoc solid phase synthesis.

| Sequence | modified residues | yield | $t_R$ [min] | m/z |
|---|---|---|---|---|
| **XYS**TEVVALSRL**Q 06-009** | **X =** propargylglycine<br>**Y =** Tyr(3,5-$I_2$)<br>**S =** *O*-β-Glucopyranosyl-serine<br>**Q =** 2-acetamido-3-amino propionic acid | 16% | 8.28 | 937.87 (2+)<br>calc: 937.32 (2+) |

**NIR-fluorecent leptin-receptor-agonist-I**

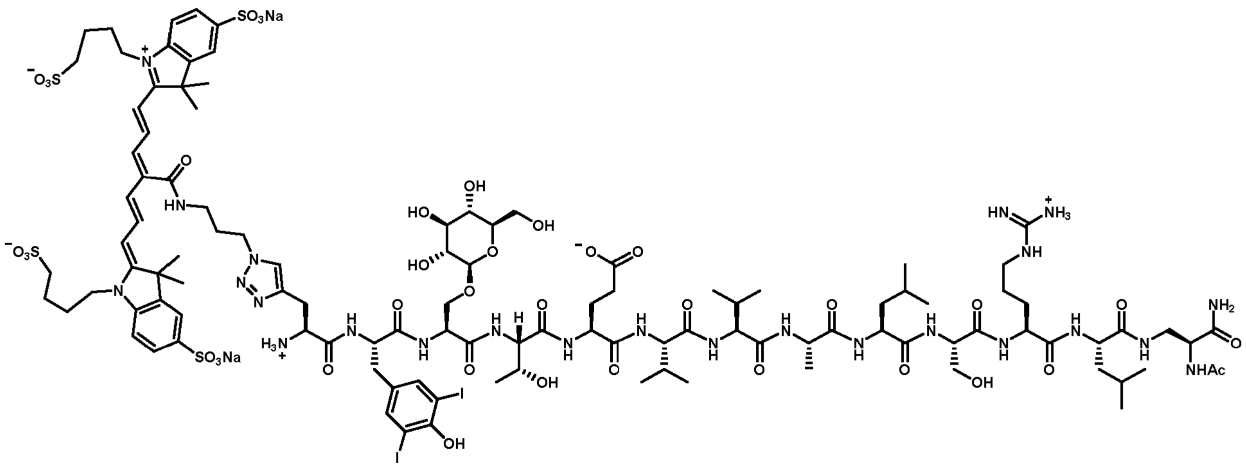

$C_{110}H_{162}I_2N_{24}Na_2O_{38}S_4$
M [g/mol] = 2856,66

**Leptin-receptor-agonist-I** (4.4 μmol) and **dyeazid3** (4.6 mg, 4.6 μmol, 1.1 eq) were dissolved in water (1.0 mL). Aminoguanidin-HCl (50 mM in water, 527 μL, 26.3 μmol, 6.0 eq), THPTA (50 mM in water, 263 μL, 13.2 μmol, 3.0 eq) was added, then $CuSO_4$ (20 mM in water, 658 μL, 13.2 μmol, 3.0 eq). Sodium ascorbate (500 mM in water, freshly dissolved, 53 μL, 26.3 μmol, 6.0 eq) was added and it was stirred until LCMS indicated completeness of reaction (1-2 h). The reaction mixture was loaded directly on a C18-Flash-Cartridge (4g) and chromatographed (Eluent: water/acetonitril, 0.05% TFA). **NIR-fluorescent leptin-receptor-agonist-I** was obtained as dark green solid.

| Sequence | modified residues | yield | $t_R$ [min] | m/z |
|---|---|---|---|---|
| **XYS**TEVVALSRL**Q** 06-011 | **X** = propargylglycine, clicked to dyeazid3<br>**Y** = Tyr(3,5-I2)<br>**S** = O-β-Glucopyranosyl-serine<br>**Q** = 2-acetamido-3-amino propionic acid | 57% | 9.3 | 1406.4362 (2+) calc: 1406.4384 (2+) |

**Leptin-receptor-agonist-II**

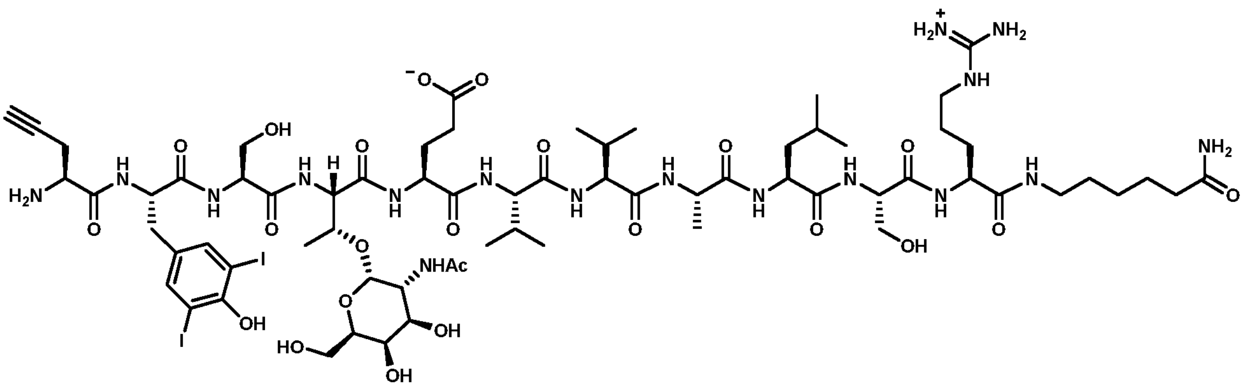

$C_{68}H_{109}I_2N_{17}O_{23}$
**M [g/mol] = 1786,5249**

**Leptin-receptor-agonist-II** was obtained in a similar manner. Therefore N-terminally propargyl glycine elongated derivative was synthesized according to literature (23).

| Sequence | modified residues | yield | $t_R$ [min] | m/z |
|---|---|---|---|---|
| **XYS**TEVVALSRL**Q** **06-077** | **X** = propargylglycine<br>**Y** = Tyr(3,5-$I_2$)<br>**T** = *O*-α-(2-acetamido-2-deoxy-Galactopyranosyl-threonine<br>**Q** = 6-aminocaproic acid | 38% | 6.38 | 893.82 (2+), 596.58 (3+) calc: 893.81 (2+), 596.21 (3+) |

**NIR-fluorescent leptin-receptor-agonist-II**

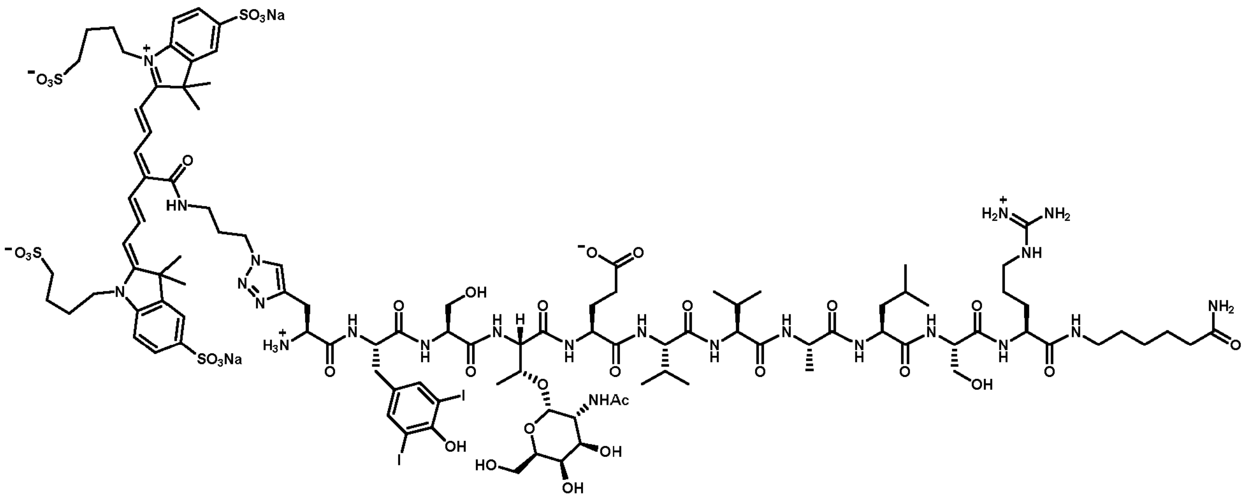

$C_{107}H_{157}I_2N_{23}Na_2O_{36}S_4$

**M [g/mol] = 2769,5865**

Conjugation of **dyeazid3** was performed in a similar manner as described above to yield **NIR-fluorescent leptin-receptor-agonist-II.**

| Sequence | modified residues | yield | $t_R$ [min] | m/z |
|---|---|---|---|---|
| **XYS**TEVVALSRL**Q**<br>06-080 | **X** = propargylglycine, clicked to dyeazid3<br>**Y** = Tyr(3,5-I2)<br>**T** = O-α-(2-acetamido-2-deoxy-Galactopyranosyl-threonine<br>**Q** = 6-aminocaproic acid | 60% | 6.44 | 908.77 (3+)<br>calc: 908.95 (3+) |

**REFERENCES**

1. ACS Nano. 2016 May 24; 10(5): 5015-5026, Figure 10
2. Clin Cancer Res; 18(20) October 15, 2012
3. Štacková L. et al.; J. Am. Chem. Soc. 2019, 141, 17, 7155-7162
4. https://www.licor.com/bio/targeted-therapeutics-development/clinical-applications
5. US0221512A1
6. Roger R. et al; Org. Lett. 2015, 17, 302-305
7. Li et al.; Angew. Chem. Int. Ed. 2020, 59, 12154-12161
8. Luciano et al.; ACS Chem. Biol. 2019, 14, 934-940
9. WO 2011/116142A1
10. WO 2020/020905 A1
11. WO 00/71162 A2
12. US 2008/0233050 A1
13. Vargas, S. H. et al.; Clin Cancer Res; 25(14) July 15, 201
14. US 10,441,607 B1
15. Dommerholt, J., Rutjes, F. P. J. T. & van Delft, F. L. Strain-Promoted 1,3-Dipolar Cycloaddition of Cycloalkynes and Organic Azides. Top. Curr. Chem. 374, 1-20 (2016).
16. Liu, Z., Derosa, J. & Engle, K. M. Palladium(II)-Catalyzed Regioselective syn-Hydroarylation of Disubstituted Alkynes Using a Removable Directing Group. J. Am. Chem. Soc. 138, 13076-13081 (2016).
17. Cao, H. et al. A red Cy3-based biarsenical fluorescent probe targeted to a complementary binding peptide. J. Am. Chem. Soc. 129, 8672-8673 (2007).
18. Schottelius, M., Wester, H. J., Reubi, J. C., Senekowitsch-Schmidtke, R. & Schwaiger, M. Improvement of pharmacokinetics of radioiodinated Tyr3-octreotide by conjugation with carbohydrates. Bioconjug. Chem. 13, 1021-1030 (2002).

19. Jadhav, S. V., Bandyopadhyay, A., Benke, S. N., Mali, S. M. & Gopi, H. N. A facile synthesis and crystallographic analysis of N-protected β-amino alcohols and short peptaibols. Org. Biomol. Chem. 9, 4182-4187 (2011).
20. Weineisen et al. EJNMMI Research 2014, 4:63
21. Angew. Chem. Int. Ed. 2014, 53, 7669 -7673.
22. Biochimica et Biophysica Acta 1783 (2008) 1745-1754.
23. Diabetes, Obesity and Metabolism 12: 393-402, 2010.

## Claims

**1.** A compound according to formula (I),

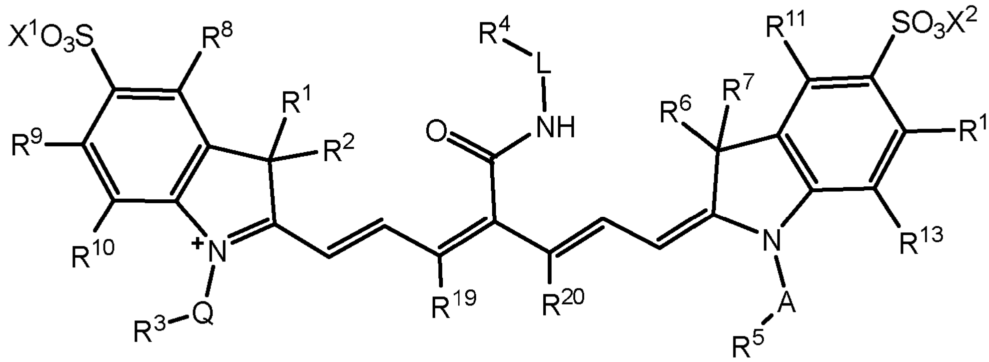

(I)

wherein

$R^1$, $R^2$, $R^6$, $R^7$, $R^{14}$, $R^{16}$, $R^{16}$, $R^{17,}$ and $R^{18}$ are independently selected from the group consisting of H and $(C_1\text{-}C_6)$ alkyl, preferably methyl;
$R^3$ is $-SO_3X^3$, or COOH;
$R^4$ is selected from the group consisting of -ethinyl, -azide,

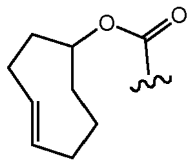
, 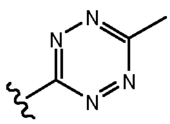
,

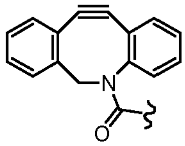 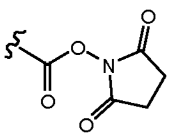 , 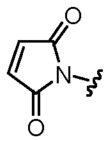 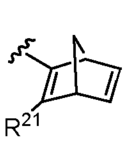 , 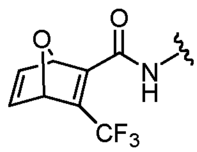

-COOH, $NH_2$, Cl, Br, I, - $OSO_2CF_3$, $-COSO_2CH_3$, -OH, vinyl, -SH;
$R^5$ is $-SO_3X^4$;
$R^8$, $R^9$, $R^{10}$, $R^{11}$, $R^{12}$ and $R^{13}$ are independently selected from the group consisting of H, CN, - $COO(C_1\text{-}C_6)$alkyl, $-CONH(C_1\text{-}C_6)$alkyl, Cl, Br, I, F, $-CF_3$, $-O(C_1\text{-}C_6)$, $-NR^{14}R^{15}$, $-NHC(O)(C_1\text{-}C_6)$alkyl, $-NHC(O)NH(C_1\text{-}C_6)$alkyl),-$SO_3H$, $-SO_2NH_2$, $-(C_1\text{-}C_6)$alkyl, preferably H;
$R^{19}$, $R^{20}$ are independently selected from the group consisting of H, $-(C_1\text{-}C_6)$alkyl , $-O(C_1\text{-}C_6)$alkyl, Cl, F, Br, CN; $C(O)NH_2$, preferably H;
$R^{21}$ is H, $-(C_1\text{-}C_{10})$alkyl;
L is a group consisting of one or more and/or one or more of each of the elements selected from the group: $-(CH_2)_{n1}$-, $-(C_1\text{-}C_6)$cycloalkylene-, $-(C_1\text{-}C_6)$heterocycloalkylene-, -O-, $-NR^{16}$-, - NH-, -NHC(O)-, $-C(O)NR^{17}$-, -CH(OH)-, and $-CH(OR^{18})$-;
$n^1$ is an integer between 1 to 30, preferably 1 to 20, more preferably 1 to 10;

$X^1$, $X^2$, $X^3$, and $X^4$ are independently selected from a cation, a proton or absent, preferably the cation is a monovalent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$;
Q is $-(CH_2)_{n2}-$,
wherein optionally one or more of each H in $-(CH_2)_{n2}-$ may be replaced by $-(C_1-C_{10})$alkyl;
$n^2$ is an integer between 1 to 10, preferably 1 to 6, more preferably 1 to 4;
A is $-(CH_2)_{n3}-$,
wherein optionally one or more of each H in $-(CH_2)_{n3}-$ may be replaced by $-(C_1-C_{10})$alkyl;
$n^3$ is an integer between 1 to 10, preferably 1 to 6, more preferably 1 to 4.

**2.** The compounds of claim 1, wherein

$R^1$, $R^2$, $R^6$, $R^7$ are Methyl;
$R^3$ is $-SO_3X^3$;
$R^4$ is selected from the group consisting of -ethinyl, -azide,

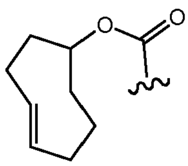, 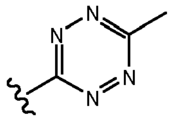,

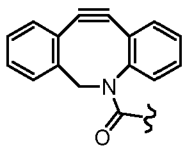 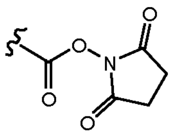., 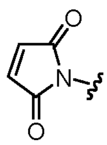, 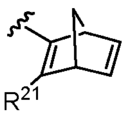, 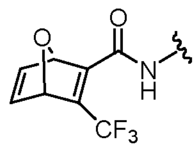

-COOH, and $NH_2$, preferably ethinyl, -azide,

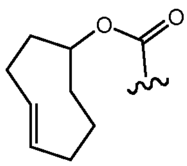, 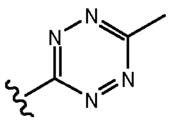, 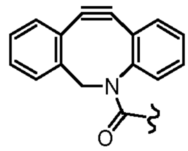 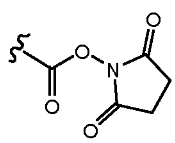;

$R^5$ is $-SO_3X^4$;
$R^8$, $R^9$, $R^{10}$, $R^{11}$, $R^{12}$ and $R^{13}$ are H;
$R^{16}$, $R^{17,}$ and $R^{18}$ are independently selected from the group consisting of H and $(C_1-C_6)$alkyl, preferably methyl;
$R^{19}$ and $R^{20}$ are H;
$R^{21}$ is H, $-(C_1-C_{10})$alkyl;
L is a group consisting of one or more and/or one or more of each of the elements selected from the group: $-(CH_2)_{n1}-$, -O-, $-NR^{16}-$, -NHC(O)-, $-C(O)NR^{17}-$, -CH(OH)-, and $-CH(OR^{18})-$; preferably $-(CH_2)_{n1}-$, and -O-;
$n^1$ is an integer between 1 to 30, preferably 1 to 20, more preferably 1 to 10;
$X^1$, $X^2$, $X^3$, and $X^4$ are independently selected from a cation, a proton or absent, preferably the cation is a monovalent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$;
Q is $-(CH_2)_{n2}-$,
wherein optionally one or more of each H in $-(CH_2)_{n2}-$ may be replaced by $-(C_1-C_{10})$alkyl;
$n^2$ is an integer between 1 to 10, preferably 1 to 6, more preferably 1 to 4;
A is $-(CH_2)_{n3}-$,
wherein optionally one or more of each H in $-(CH_2)_{n3}-$ may be replaced by $-(C_1-C_{10})$alkyl;
$n^3$ is an integer between 1 to 10, preferably 1 to 6, more preferably 1 to 4.

3. The compound of claim 1, wherein the compound is selected from

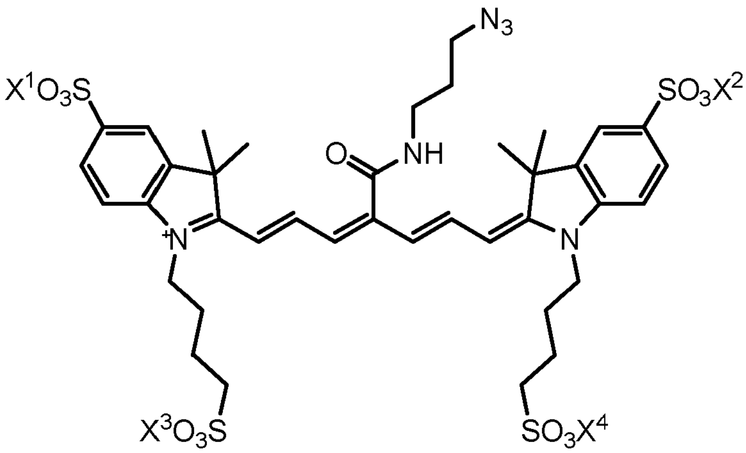

and ,

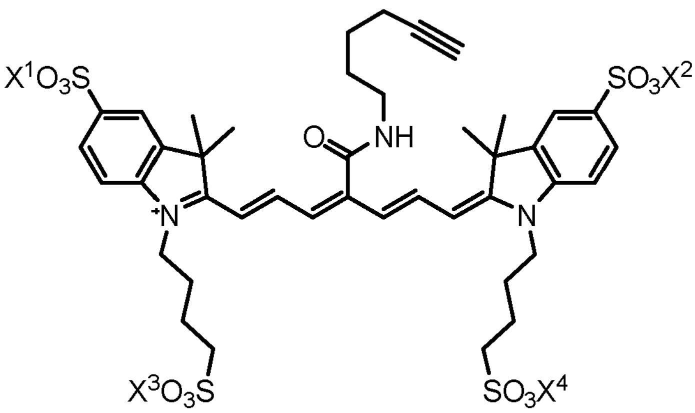

,

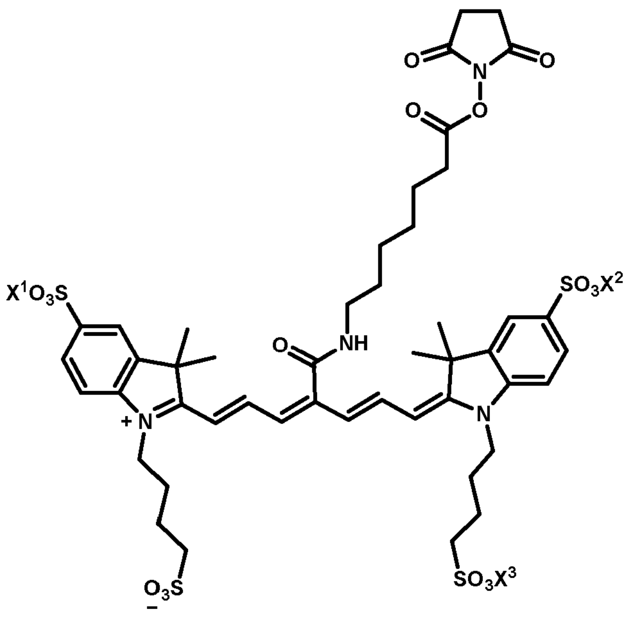

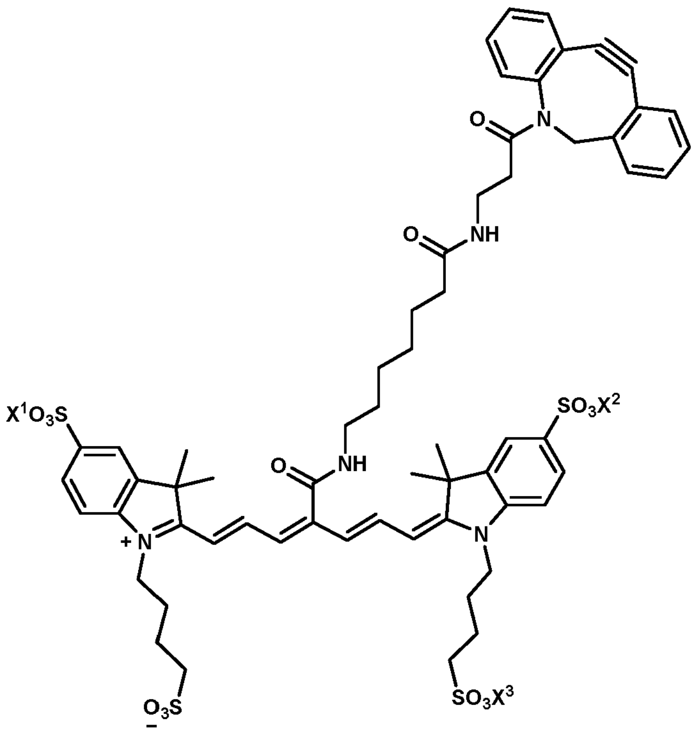

**4.** A compound according to formula (II)

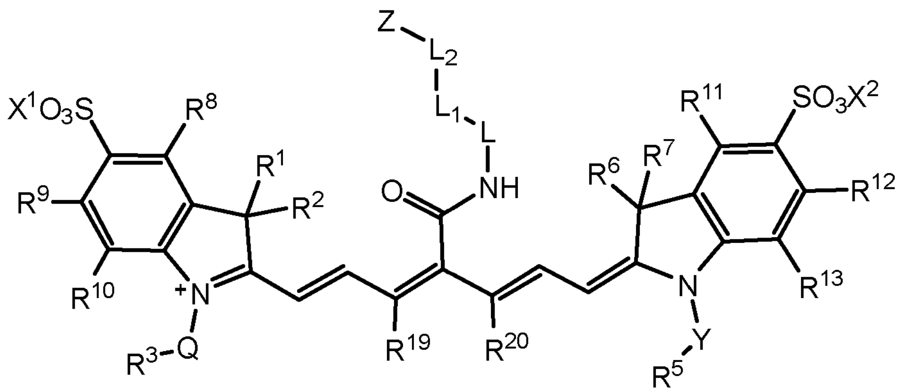

(II)

wherein

$R^1$, $R^2$, $R^6$, $R^7$, $R^{14}$, $R^{15}$, $R^{16}$, $R^{17,}$ and $R^{18}$ are independently selected from the group consisting of H and $(C_1\text{-}C_6)$ alkyl, preferably methyl;
$R^3$ is $-SO_3X^3$, or COOH;
Z is a protein, mono- or polyvalent saccharide, peptide, small molecule, antibody, oligonucleotide, nanobody, or polymer;
$R^5$ is $-SO_3X^4$;
$R^8$, $R^9$, $R^{10}$, $R^{11}$, $R^{12}$ and $R^{13}$ are independently selected from the group consisting of H, CN, - $COO(C_1\text{-}C_6)$alkyl, $-CONH(C_1\text{-}C_6)$alkyl, Cl, Br, I, F, $-CF_3$, $-O(C_{1\text{-}}C_6)$, $-NR^{14}R^{15}$, $-NHC(O)(C_1\text{-}C_6)$alkyl, $-NHC(O)NH(C_1\text{-}C_6)$alkyl), - $SO_3H$, $-SO_2NH_2$, $-(C_1\text{-}C_6)$alkyl, preferably H;
$R^{19}$, $R^{20}$ are independently selected from the group consisting of H, $-(C_1\text{-}C_6)$alkyl , $-O(C_1\text{-}C_6)$alkyl, Cl, F, Br, CN; $C(O)NH_2$, preferably H;
$R^{21}$ is H, $-(C_1\text{-}C_{10})$alkyl;
$X^1$, $X^2$, $X^3$, and $X^4$ are independently selected from a cation, a proton or absent, preferably the cation is a

monovalent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$;

Q is $-(CH_2)_{n2}-$,

wherein optionally one or more of each H in $-(CH_2)_{n2}-$ may be replaced by $-(C_1-C_{10})$alkyl;

$n^2$ is an integer between 1 to 10, preferably 1 to 6, more preferably 1 to 4;

Y is $-(CH_2)_{n3}-$,

wherein optionally one or more of each H in $-(CH_2)_{n3}-$ may be replaced by $-(C_1-C_{10})$alkyl;

$n^3$ is an integer between 1 to 10, preferably 1 to 6, more preferably 1 to 4;

L is a group consisting of one or more and/or one or more of each of the elements selected from the group: $-(CH_2)_{n1}-$, $-(C_1-C_6)$cycloalkylene-, $-(C_1-C_6)$heterocycloalkylene-, -O-, $-NR^{16}-$, - NH-, -NHC(O)-, $-C(O)NR^{17}-$, -CH(OH)-, and $-CH(OR^{18})-$;

$n^1$ is an integer between 1 to 30, preferably 1 to 20, more preferably 1 to 10;

$L_1$ is selected from the group consisting of

, 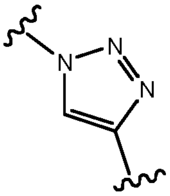, 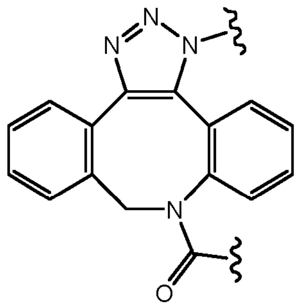, 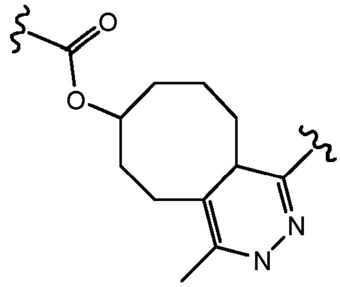, 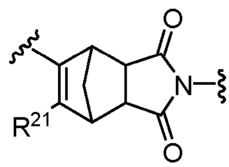, ,

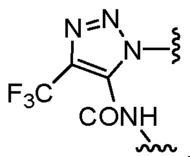, 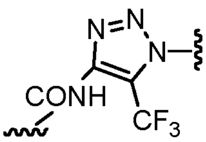, –CO-;

$L_2$ is a group consisting of one or more and/or one or more of each of the elements selected from the group: $-(CH_2)_{n1}-$, $-(C_1-C_6)$cycloalkylene-, $-(C_1-C_6)$heterocycloalkylene-, -O-, $-NR^{16}-$, - NH-, -NHC(O)-, $-C(O)NR^{17}-$, -CH(OH)-, and $-CH(OR^{18})-$.

**5.** The compound of claim 4, wherein

$R^1$, $R^2$, $R^6$, $R^7$ are Methyl;

$R^3$ is $-SO_3X^3$;

$R^5$ is $-SO_3X^4$;

$R^8$, $R^9$, $R^{10}$, $R^{11}$, $R^{12}$ and $R^{13}$ are H;

$R^{16}$, $R^{17,}$ and $R^{18}$ are independently selected from the group consisting of H and $(C_1-C_6)$alkyl, preferably methyl;

$R^{19}$ and $R^{20}$ are H;

$X^1$, $X^2$, $X^3$, and $X^4$ are independently selected from a cation, a proton or absent, preferably the cation is a monovalent cation or a divalent cation, more preferably the cation is a monovalent cation most preferably an alkali metal cation or an ammonium cation, particularly preferred the cation is independently selected from the group consisting of $Na^+$, $K^+$; and $NH_4^+$;

Q is $-(CH_2)_{n2}-$,

wherein optionally one or more of each H in $-(CH_2)_{n2}-$ may be replaced by $-(C_1-C_{10})$alkyl;

$n^2$ is an integer between 1 to 10, preferably 1 to 6, more preferably 1 to 4;

Y is $-(CH_2)_{n3}-$,

wherein optionally one or more of each H in $-(CH_2)_{n3}-$ may be replaced by $-(C_1-C_{10})$alkyl;

$n^3$ is an integer between 1 to 10, preferably 1 to 6, more preferably 1 to 4.

L is a group consisting of one or more and/or one or more of each of the elements selected from the group: $-(CH_2)_{n1}-$, -O-, $-NR^{16}-$, -NHC(O)-, $-C(O)NR^{17}-$, -CH(OH)-, and $-CH(OR^{18})-$; preferably $-(CH_2)_{n1}-$, and -O-;

$n^1$ is an integer between 1 to 30, preferably 1 to 20, more preferably 1 to 10;

$n^4$ is an integer between 1 to 10, preferably 1 to 6, more preferably 1 to 4
$L_1$ is selected from the group consisting of

, 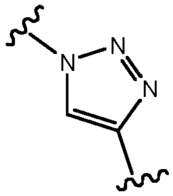, 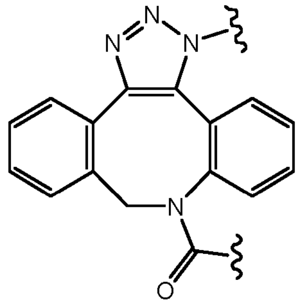, 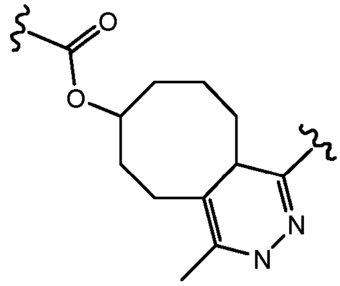, 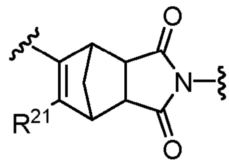, ,

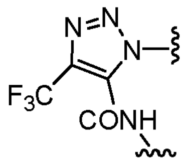, 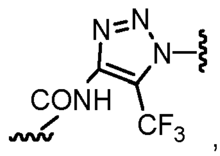, –CO-, `

$R^{21}$ is H, -($C_1$-$C_{10}$)alkyl.

6. The compound according to claim 4, wherein Z is selected from the group consisting of Octreotide, Octreotate, TOC, TATE, NP41, Leptin, Insulin, Glucagon, GLP-1, methanobactin OB3b, PSMA (Prostate-Specific Membrane Antigen), PSMA binding ligands like KUE, antibodies such as EGFR targeting antibodies like cetuximab, panitutumab, HER2 targeting antibodies such as trastuzumab, CEA targeting antibodies, VEGFR targeting antibodies such as bevatuzumab, $\alpha v \beta 6$-integrin binders like Trivehexin.

7. The compound according to claim 4 or 6, wherein $L_1$ is selected from the group consisting of

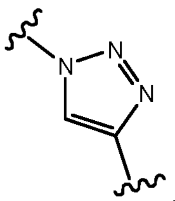, –CO-, `

$n^4$ is an integer between 1 to 10, preferably 1 to 6, more preferably 1 to 4.

8. The compound of claim 1 to 7, wherein L is selected from the group consisting of - $CH_2(CH_2OCH_2)_{n1}CH_2$-, -$(CH_2)_{n2}$-, -$CH_2(CH_2NHCH_2)_{n1}CH_2$-, -$CH_2(CH_2NR^{16}CH_2)_{n1}CH_2$-, - $CH_2(CH_2OCH_2)_{n1}CH_2C(O)(CH_2)_{n5}$-, -$CH_2(CH_2NHCH_2)_{n1}CH_2C(O)(CH_2)_{n5}$-, - $CH_2(CH_2NR^{16}CH_2)_{n1}CH_2C(O)(CH_2)_{n5}$-;

$n^1$ is an integer between 1 to 30, preferably 1 to 20, more preferably 1 to 10;
$n^2$ is an integer between 1 to 10, preferably 1 to 6, more preferably 1 to 4;
$n^5$ is an integer between 1 to 10, preferably 1 to 6, more preferably 1 to 4.

9. The compound of claim 4, wherein the compound is

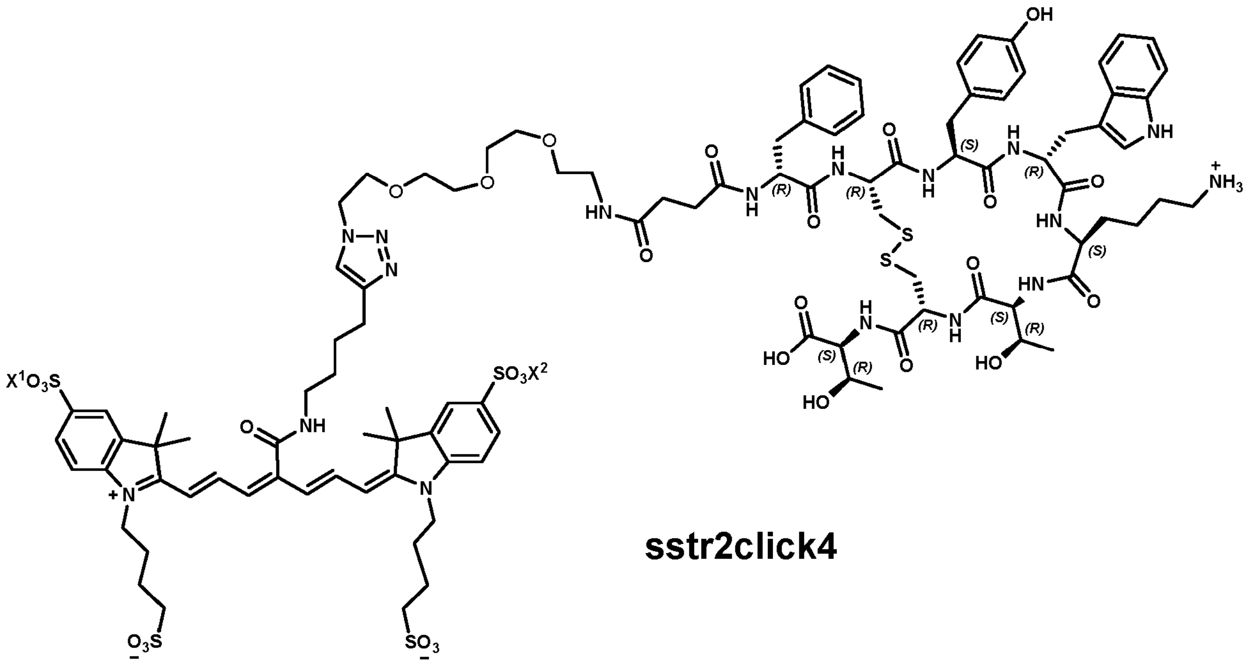

**sstr2click4**

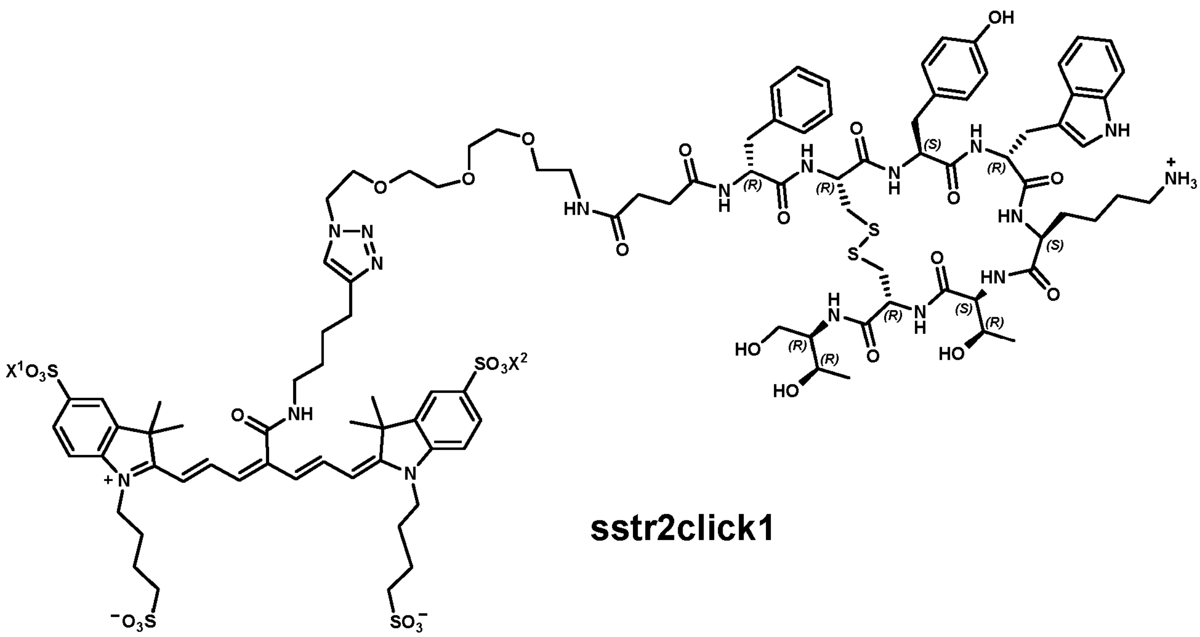

**sstr2click1**

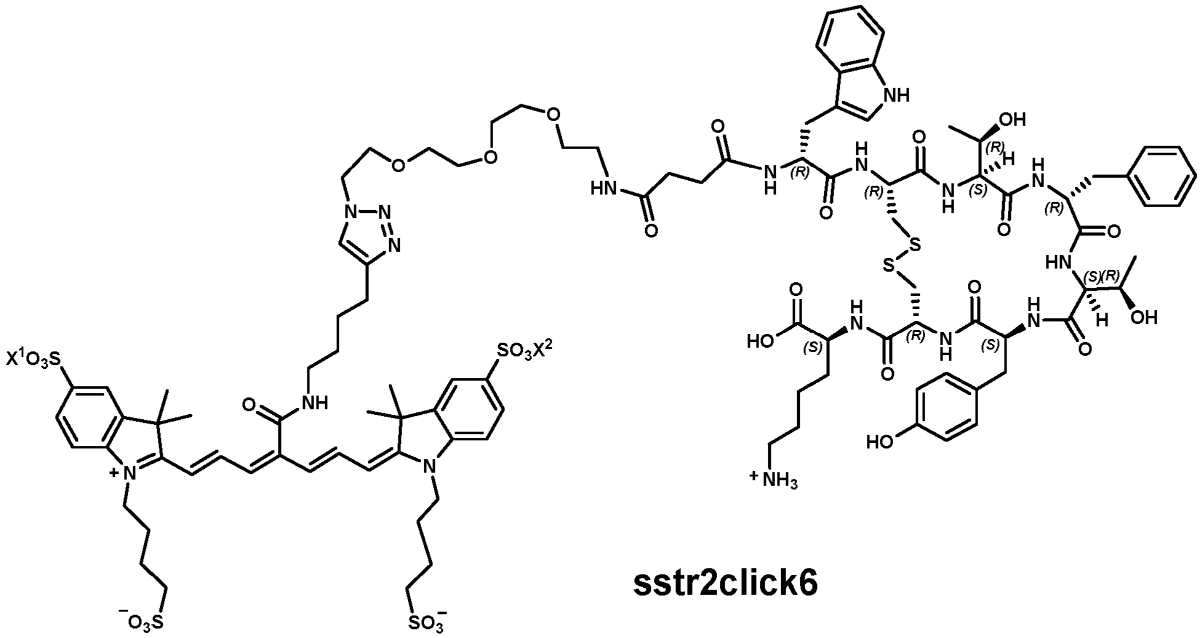

sstr2click6

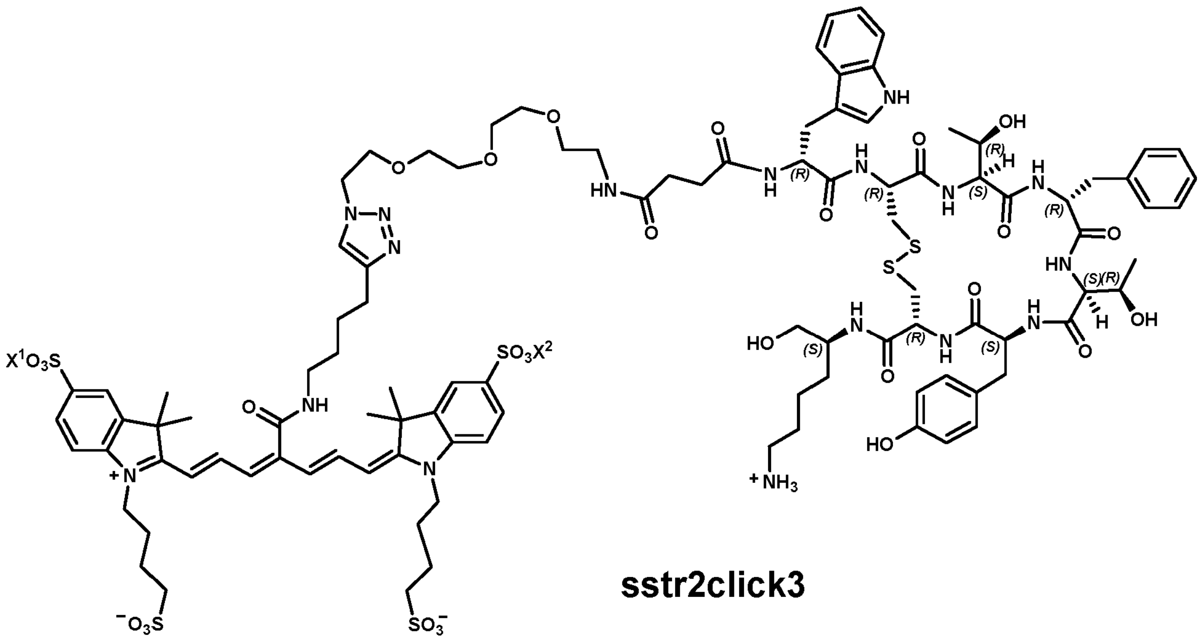

sstr2click3

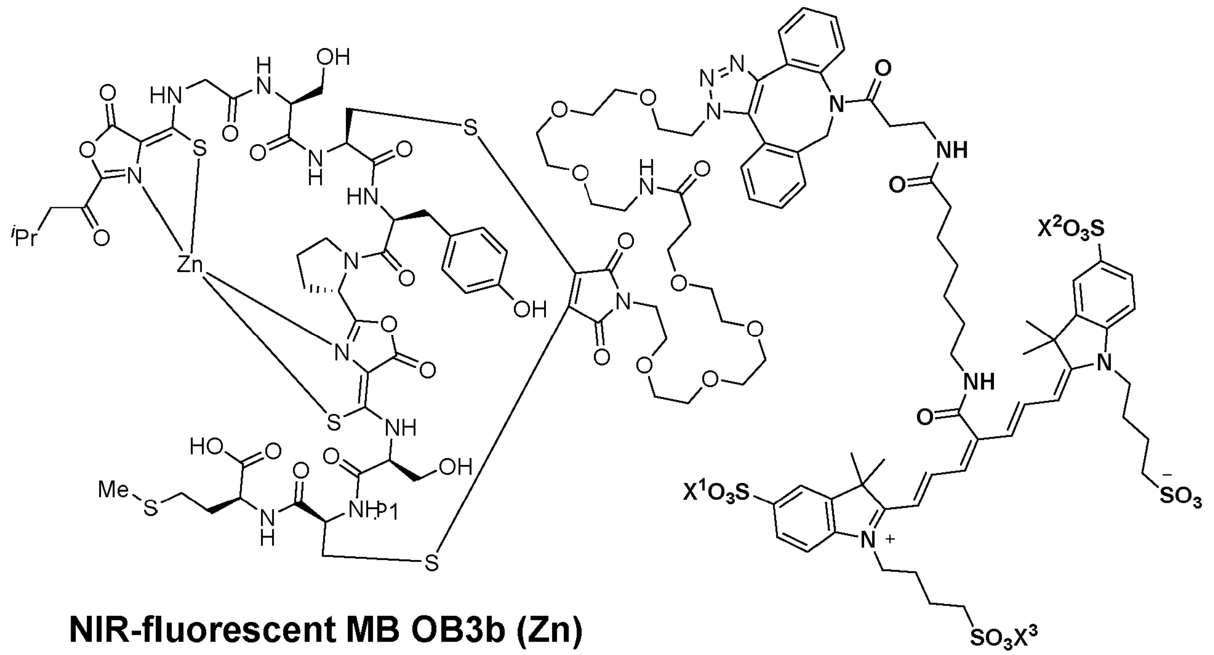

**NIR-fluorescent MB OB3b (Zn)**

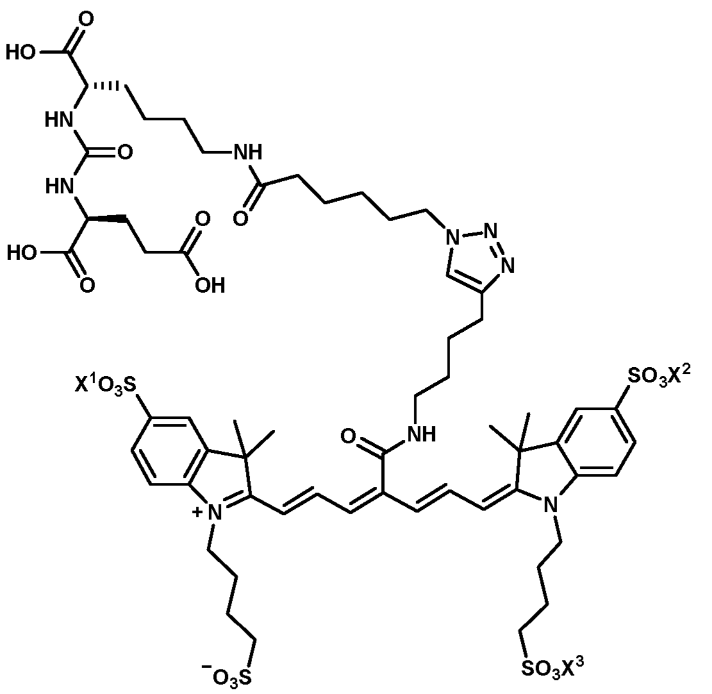

**NIR-fluorescent KUE Ligand I**

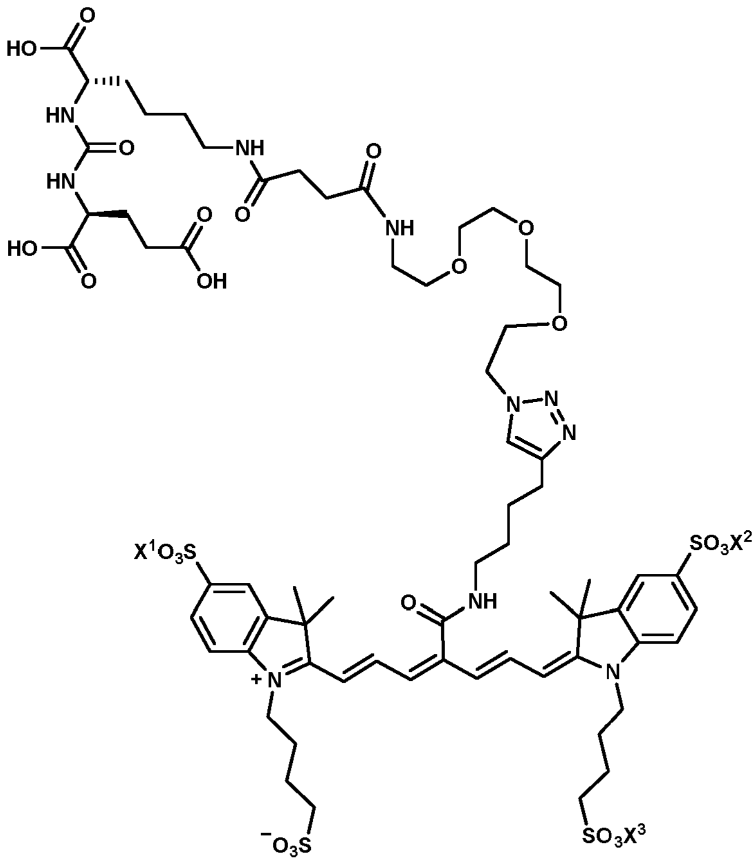

**NIR-fluorescent KUE Ligand II**

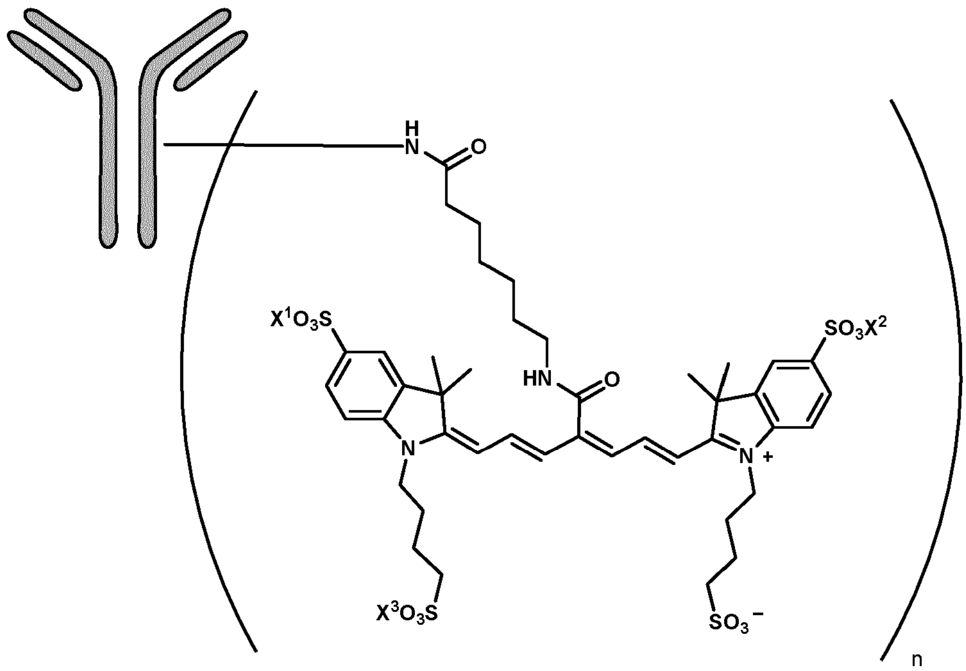

**NIR-fluorescent Panitumumab**

wherein the antibody shown symbolically in the formula ("NIR-fluorescent Panitumumab") is Panitumumab,

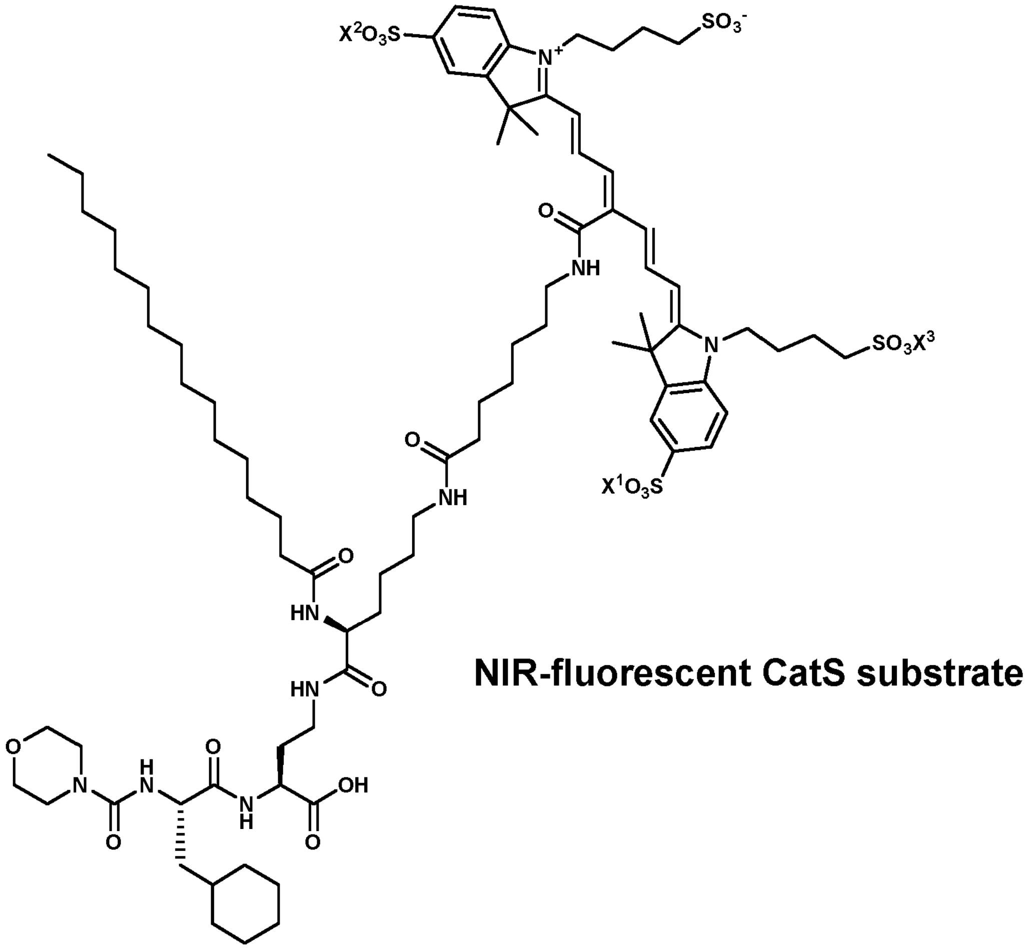

NIR-fluorescent CatS substrate

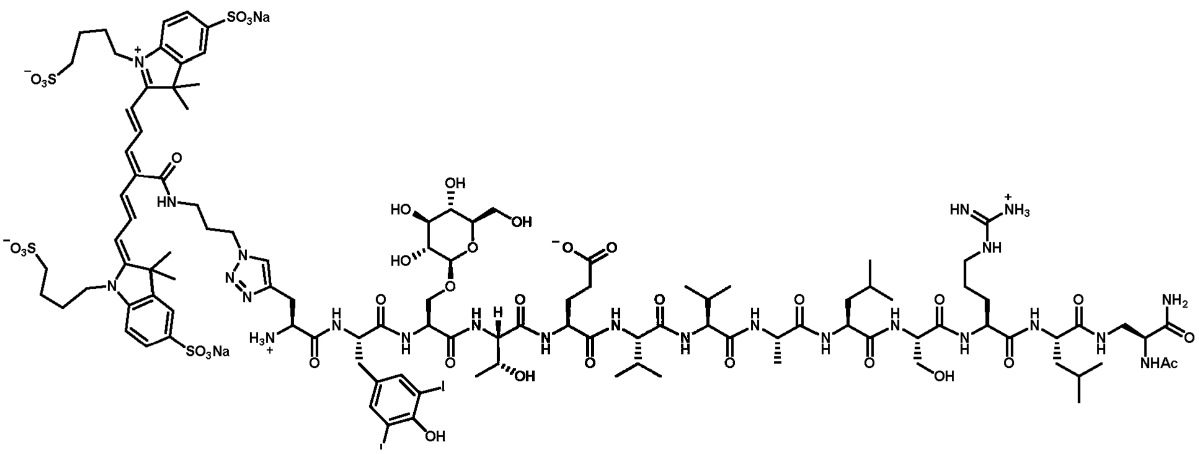

NIR-fluorecent leptin-receptor-agonist-I

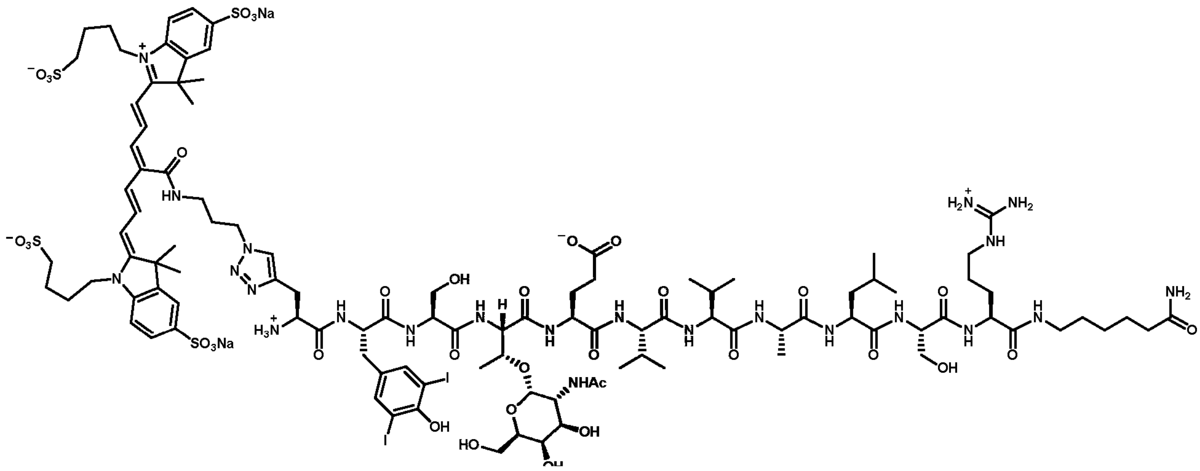

**NIR-fluorecent leptin-receptor-agonist-II**

**10.** The compound of claims 1 to 9 wherein

i) the peak emission in PBS is between 400 nm to 1200 nm, preferably from 500 nm to 900 nm, more preferably from 790 to 820 nm and/or
ii) the absorption crosssection in PBS is > 5,000 1/(M*cm), preferably > 50,000 1/(M*cm), more preferably >200,000 1/(M*cm) and/or
iii) the peak absorption in PBS is from 400 nm to 1200 nm, preferably from 600 nm to 1000 nm, more preferably from 750 nm to 850 nm.

**11.** Use of the compound of claims 1 to 10 as fluorescent dye.

**12.** The compound of claims 1 to 10; , wherein the compound is a contrast agent.

**13.** The compound of claims 1 to 12 for use in diagnosis or for intraoperative surgery.

**14.** The compound for use according to claim 13, wherein the indication to be diagnosed or treated by intraoperative surgery is selected from the group consisting of inflammatory diseases, infectious diseases and cancer.

**15.** The compound for use according to claim 14 , wherein

i) the inflammatory disease is selected from the group consisting of otitis media, allergic rhinitis and chronic rhinosinusitis, chronic inflammation of tonsils, adenoids, and laryngitis;
ii) the infectious disease is selected from the group consisting of implant infection (e.g. hip, dental), secondary infections of the inner ear, e.g. accompanying cholesteatoma, preferably caused by gram positive or gram negative bacteria, responding to antibiotic treatment, resistant or multi-drug resistant, in particular by ESKAPE strains *Enterococcus faecium, Staphylococcus aureus, Klebsiella pneumoniae, Acinetobacter baumannii, Pseudomonas aeruginosa,* and *Enterobacter spp.*.
iii) diagnosing of cancer or treating cancers by intraoperative surgery relates to visualizing the primary tumor and/or metastatic lesions in situ during surgery selected from the group meningioma, glioblastoma, NSCLC, pancreatic cancer, neuroendocrine tumors, lung cancer, breast cancer, and meningiomas.

## Patentansprüche

**1.** Verbindung gemäß Formel (I),

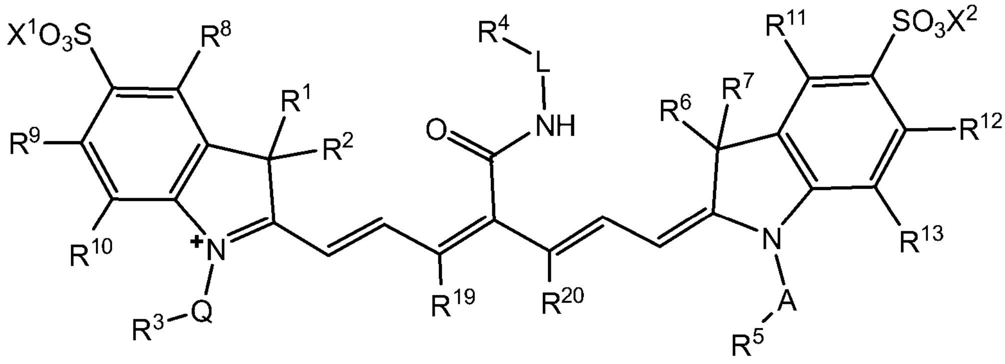

(I)

wobei

$R^1$, $R^2$, $R^6$, $R^7$, $R^{14}$, $R^{15}$ $R^{16}$, $R^{17}$und $R^{18}$unabhängig voneinander aus der Gruppe ausgewählt sind, bestehend aus H und ($C_1$-$C_6$)-Alkyl, vorzugsweise Methyl;
$R^3$ -$SO_3$ $X^3$ oder COOH ist;
$R^4$ ist ausgewählt aus der Gruppe bestehend aus -Ethinyl, -Azid,

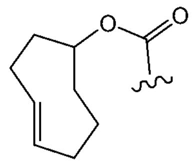

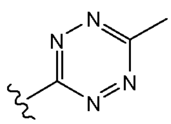

,

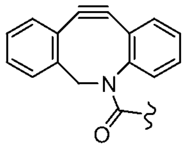

,

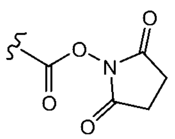

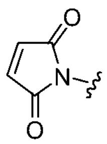

,

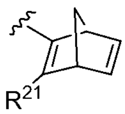

,

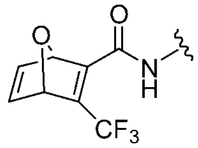

-COOH, -$NH_2$, Cl, Br, I, - $OSO_2$ $CF_3$, -$COSO_2$ $CH_3$ , -OH, Vinyl, -SH;
$R^5$ ist -$SO_3$ $X^4$;
$R^8$, $R^9$, $R^{10}$, $R^{11}$, $R^{12}$ und $R^{13}$ sind unabhängig voneinander ausgewählt aus der Gruppe bestehend aus H, -CN, -COO($C_1$ -$C_6$)Alkyl, -CONH($C_1$ -$C_6$)Alkyl, Cl, Br, I, F, -$CF_3$ , -O($C_1$ -$C_6$ ), -$NR^{14}$ $R^{15}$ , -NHC(O)($C_1$ -$C_6$)Alkyl, -NHC(O)NH($C_1$ -$C_6$)Alkyl), -$SO_3$ H, -$SO_2$ $NH_2$, -($C_1$ -$C_6$ )Alkyl, vorzugsweise H;
$R^{19}$ und $R^{20}$ sind unabhängig voneinander aus der Gruppe ausgewählt, die aus H, -($C_1$ -$C_6$ )Alkyl, -O($C_1$ -$C_6$ ) Alkyl, Cl, F, Br, CN; C(O)$NH_2$ , vorzugsweise H, besteht;
$R^{21}$ ist H, -($C_1$ -$C_{10}$ )Alkyl;
L ist eine Gruppe, bestehend aus einem oder mehreren und/oder jeweils einem oder mehreren der Elemente, ausgewählt aus der Gruppe: -$(CH_2)_{n1}$-, -($C_1$-$C_6$)cycloalkylen-, -($C_1$-$C_6$)heterocycloalkylen-, -O-, -$NR^{16}$ -, -NH-, -NHC(O)-, -C(O)$NR^{17}$ -, -CH(OH)- und -CH($OR^{18}$ )-;
$n^1$ ist eine ganze Zahl zwischen 1 und 30, vorzugsweise zwischen 1 und 20, noch bevorzugter zwischen 1 und 10;
$X^1$, $X^2$, $X^3$ und $X^4$ sind unabhängig voneinander ausgewählt aus einem Kation, einem Proton oder sind nicht vorhanden, wobei das Kation vorzugsweise ein einwertiges Kation oder ein zweiwertiges Kation ist, noch bevorzugter ein einwertiges Kation, am meisten bevorzugt ein Alkalimetallkation oder ein Ammoniumkation, besonders bevorzugt ist das Kation unabhängig ausgewählt aus der Gruppe bestehend aus $Na^+$ , $K^+$ ; und $NH_4{}^+$ ;
Q ist -$(CH_2)_{n2}$ -,
wobei optional eines oder mehrere der H-Atome in -$(CH_2)_{n2}$ - durch -($C_1$ -$C_{10}$ )Alkyl ersetzt sein können;
$n^2$ eine ganze Zahl zwischen 1 und 10, vorzugsweise zwischen 1 und 6, noch bevorzugter zwischen 1 und 4 ist;
A ist -$(CH_2)_{n3}$,
wobei optional eines oder mehrere der H-Atome in -$(CH_2)_{n3}$ - durch -($C_1$ -$C_{10}$ )Alkyl ersetzt sein können;
$n^3$ eine ganze Zahl zwischen 1 und 10, vorzugsweise zwischen 1 und 6, noch bevorzugter zwischen 1 und 4 ist.

**2.** Verbindungen nach Anspruch 1, wobei

$R^1$, $R^2$ , $R^6$, $R^7$ Methyl sind;
$R^3$ $-SO_3$ $X^3$ ist;
$R^4$ ist ausgewählt aus der Gruppe bestehend aus -Ethinyl, -Azid,

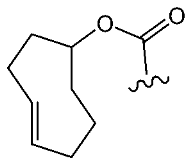 , 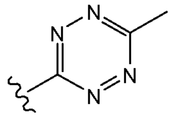

, 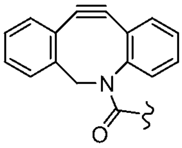 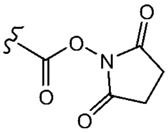 ., 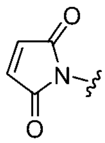 , 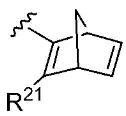 , 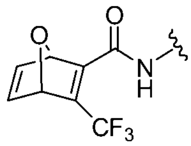

-COOH und $NH_2$, vorzugsweise Ethinyl, -Azid,

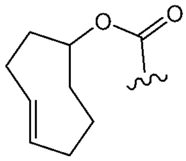 , 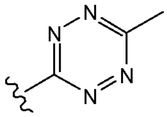 , 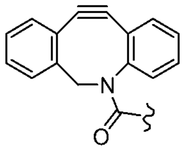 , 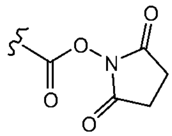 ;

$R^5$ ist $-SO_3$ $X^4$;
$R^8$, $R^9$, $R^{10}$, $R^{11}$, $R^{12}$und $R^{13}$sind H;
$R^{16}$, $R^{17}$und $R^{18}$sind unabhängig voneinander aus der Gruppe ausgewählt, die aus H und ($C_1$-$C_6$)-Alkyl, vorzugsweise Methyl, besteht;
$R^{19}$ und $R^{20}$ sind H;
$R^{21}$ ist H, -($C_1$ -$C_{10}$ )Alkyl;
L ist eine Gruppe, bestehend aus einem oder mehreren und/oder jeweils einem oder mehreren der Elemente, ausgewählt aus der Gruppe: $-(CH_2)_{n1}-$, -O-, $-NR^{16}-$, -NHC(O)-, $-C(O)NR^{17}$ -, - CH(OH)- und $-CH(OR^{18}$ )-; vorzugsweise $-(CH_2)_{n1}$ - und -O-;
$n^1$ ist eine ganze Zahl zwischen 1 und 30, vorzugsweise zwischen 1 und 20, noch bevorzugter zwischen 1 und 10;
$X^1$, $X^2$, $X^3$ und $X^4$ sind unabhängig voneinander ausgewählt aus einem Kation, einem Proton oder sind nicht vorhanden; vorzugsweise ist das Kation ein einwertiges Kation oder ein zweiwertiges Kation; noch bevorzugter ist das Kation ein einwertiges Kation, am meisten bevorzugt ein Alkalimetallkation oder ein Ammoniumkation; besonders bevorzugt ist das Kation unabhängig ausgewählt aus der Gruppe bestehend aus $Na^+$ , $K^+$ ; und $NH_4^+$ ;
Q ist $-(CH_2)_2-$
wobei wahlweise eines oder mehrere der H-Atome in $-(CH_2)_n{}^2$ durch -($C_1$-$C_{10}$)Alkyl ersetzt sein können;
$n^2$ eine ganze Zahl zwischen 1 und 10, vorzugsweise zwischen 1 und 6, noch bevorzugter zwischen 1 und 4 ist;
A ist $-(CH_2)_{n3}$,
wobei optional eines oder mehrere der H-Atome in $-(CH_2)n^3-$ durch -($C_1$-$C_{10}$)Alkyl ersetzt sein können;
$n^3$ eine ganze Zahl zwischen 1 und 10, vorzugsweise zwischen 1 und 6, noch bevorzugter zwischen 1 und 4 ist.

**3.** Verbindung nach Anspruch 1, wobei die Verbindung ausgewählt ist aus

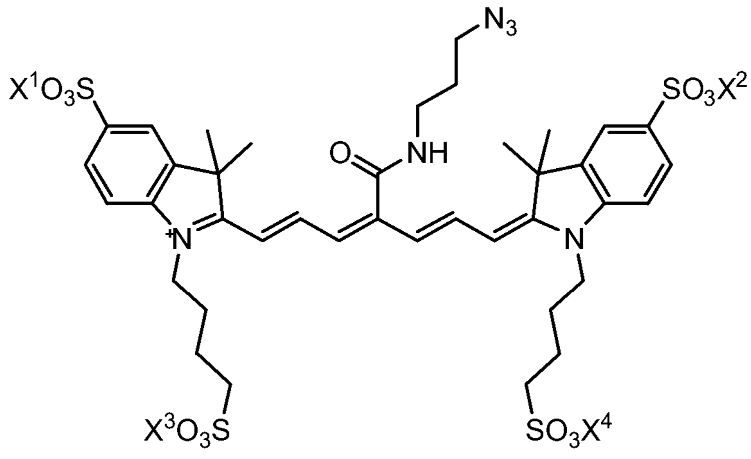

und ,

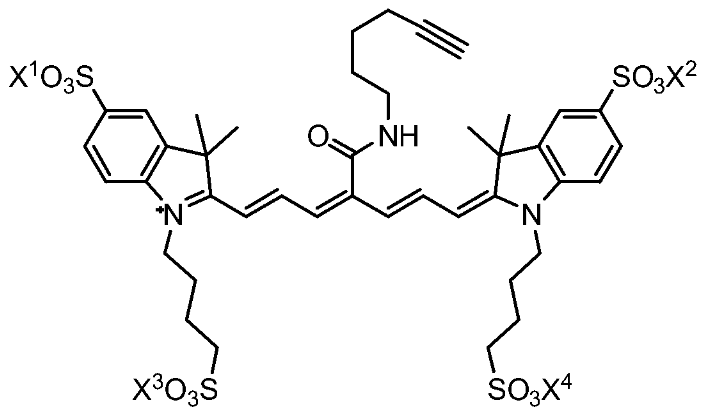

,

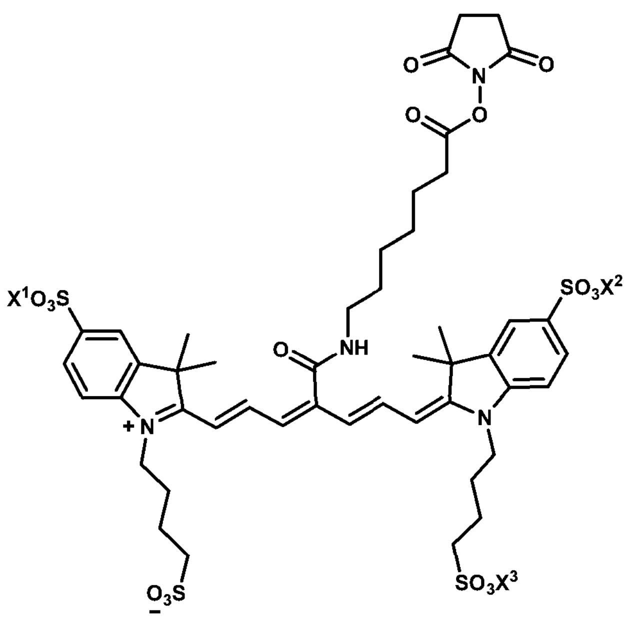

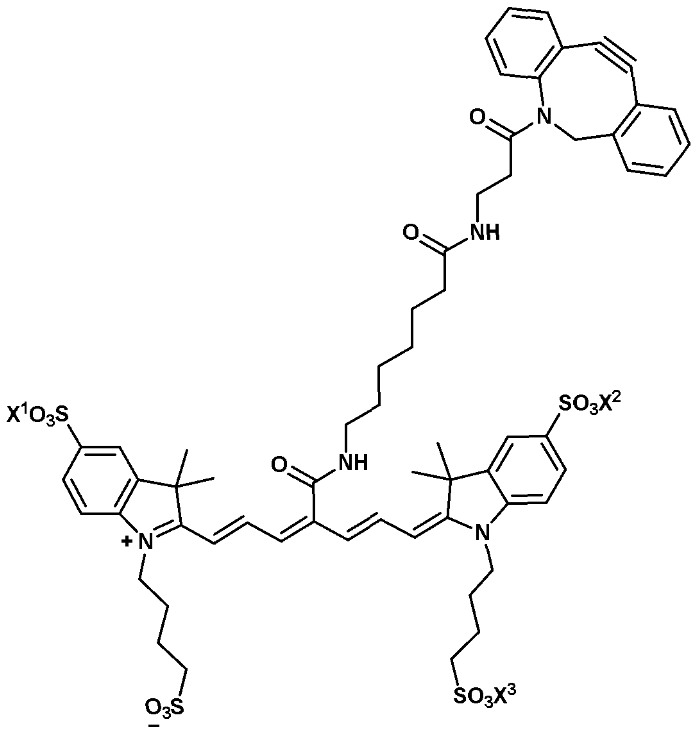

**4.** Eine Verbindung gemäß Formel (II)

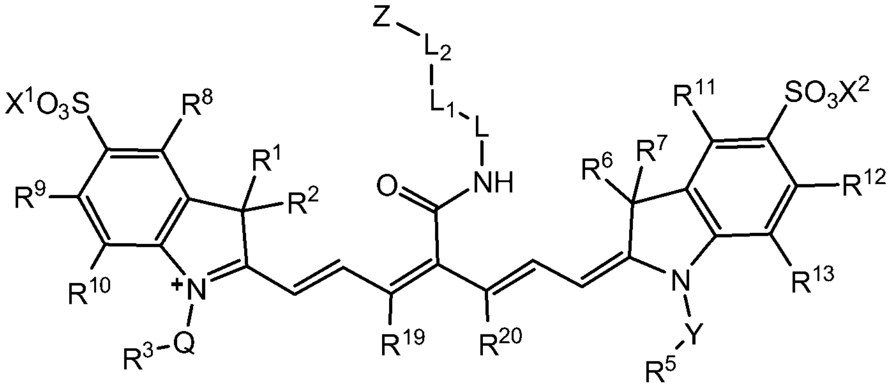

(II)

wobei

$R^1$, $R^2$, $R^6$, $R^7$, $R^{14}$, $R^{15}$, $R^{16}$, $R^{17}$und $R^{18}$ unabhängig voneinander aus der Gruppe ausgewählt sind, bestehend aus H und $(C_1$-$C_6)$-Alkyl, vorzugsweise Methyl;
$R^3$ $-SO_3$ $X^3$ oder COOH ist;
Z ist ein Protein, ein ein- oder mehrwertiges Saccharid, ein Peptid, ein kleines Molekül, ein Antikörper, ein Oligonukleotid, ein Nanokörper oder ein Polymer;
$R^5$ ist $-SO_3$ $X^4$ ;
$R^8$, $R^9$, $R^{10}$, $R^{11}$, $R^{12}$und $R^{13}$sind unabhängig voneinander aus der Gruppe ausgewählt, die aus H, CN, $-COO(C_1$ $-C_6)$Alkyl, $-CONH(C_1$ $-C_6)$Alkyl, Cl, Br, I, F, $-CF_3$, $-O(C_1$-$C_6)$, $-NR^{14}R^{15}$ , - $NHC(O)(C_1$-$C_6)$alkyl, -NHC(O) $NH(C_1$-$C_6)$alkyl), $-SO_3$ H, $-SO_2$, $-NH_2$, $-(C_1$-$C_6)$alkyl, vorzugsweise H;
$R^{19}$ und $R^{20}$ sind unabhängig voneinander aus der Gruppe ausgewählt, bestehend aus H, $-(C_1$ $-C_6$ )Alkyl, $-O(C_1$ $-C_6$ )Alkyl, Cl, F, Br, CN; $C(O)NH_2$ , vorzugsweise H;
$R^{21}$ ist H, $-(C_1$ $-C_{10}$ )Alkyl;
$X^1$, $X^2$, $X^3$ und $X^4$ sind unabhängig voneinander ausgewählt aus einem Kation, einem Proton oder sind nicht

vorhanden, vorzugsweise ist das Kation ein einwertiges Kation oder ein zweiwertiges Kation, noch bevorzugter ist das Kation ein einwertiges Kation, am meisten bevorzugt ein Alkalimetallkation oder ein Ammoniumkation, besonders bevorzugt ist das Kation unabhängig voneinander ausgewählt aus der Gruppe bestehend aus $Na^+$, $K^+$ ; und $NH_4^+$ ;
Q ist $-(CH_2)_{n2}-$,
wobei optional eines oder mehrere der H-Atome in $-(CH_2)_{n2}-$ durch $-(C_1-C_{10})$Alkyl ersetzt sein können;
$n^2$ eine ganze Zahl zwischen 1 und 10, vorzugsweise zwischen 1 und 6, noch bevorzugter zwischen 1 und 4 ist;
Y ist $-(CH_2)_{n3}-$,
wobei wahlweise eines oder mehrere der H-Atome in $-(CH_2)_{n3}$ durch $-(C_1-C_{10})$Alkyl ersetzt sein können;
$n^3$ eine ganze Zahl zwischen 1 und 10, vorzugsweise zwischen 1 und 6, noch bevorzugter zwischen 1 und 4 ist;
L eine Gruppe ist, bestehend aus einem oder mehreren und/oder jeweils einem oder mehreren der Elemente, ausgewählt aus der Gruppe: $-(CH_2)_{n1}-$, $-(C_1-C_6)$cycloalkylen-, $-(C_1-C_6)$heterocycloalkylen-, -O-, $-NR^{16}-$, -NH-, -NHC(O)-, $-C(O)NR^{17}-$, -CH(OH)- und $-CH(OR^{18})-$;
$n^1$ ist eine ganze Zahl zwischen 1 und 30, vorzugsweise zwischen 1 und 20, noch bevorzugter zwischen 1 und 10;
$L_1$ ist ausgewählt aus der Gruppe bestehend aus

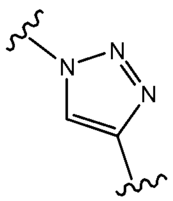 , 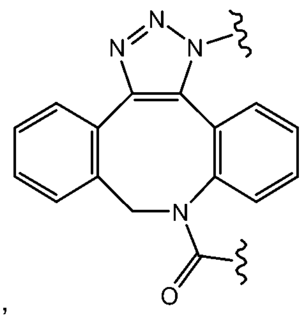 , 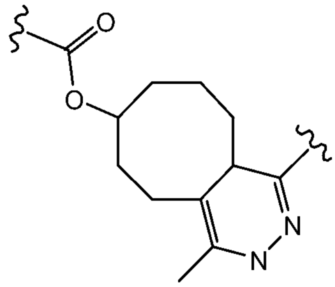 , 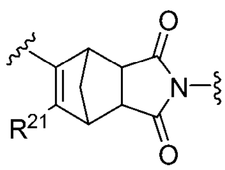 ,

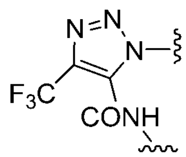 , 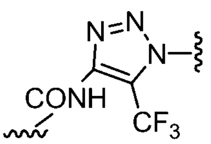 , –CO-;

$L_2$ ist eine Gruppe, bestehend aus einem oder mehreren und/oder jeweils einem oder mehreren der Elemente, ausgewählt aus der Gruppe: $-(CH_2)_{n1}-$, $-(C_1-C_6)$Cycloalkylen-, $-(C_1-C_6)$Heterocycloalkylen-, -O-, $-NR^{16}-$, -NH-, -NHC(O)-, $-C(O)NR^{17}-$, -CH(OH)- und $-CH(OR^{18})-$.

**5.** Verbindung nach Anspruch 4, wobei

$R^1$, $R^2$, $R^6$ und $R^7$ Methyl sind;
$R^3$ $-SO_3X^3$ ist;
$R^5$ $-SO_3X^4$ ist;
$R^8$, $R^9$, $R^{10}$, $R^{11}$, $R^{12}$ und $R^{13}$ sind H;
$R^{16}$, $R^{17}$ und $R^{18}$ sind unabhängig voneinander aus der Gruppe ausgewählt, die aus H und $(C_1-C_6)$-Alkyl, vorzugsweise Methyl, besteht;
$R^{19}$ und $R^{20}$ sind H;
$X^1$, $X^2$, $X^3$ und $X^4$ sind unabhängig voneinander ausgewählt aus einem Kation, einem Proton oder sind nicht vorhanden, wobei das Kation vorzugsweise ein einwertiges Kation oder ein zweiwertiges Kation ist, noch bevorzugter ist das Kation ein einwertiges Kation, am meisten bevorzugt ein Alkalimetallkation oder ein Ammoniumkation, besonders bevorzugt ist das Kation unabhängig ausgewählt aus der Gruppe bestehend aus $Na^+$ , $K^+$ ; und $NH_4^+$ ;
Q ist $-(CH_2)_{n2}-$,
wobei optional eines oder mehrere der H-Atome in $-(CH_2)_{n2}-$ durch $-(C_1-C_{10})$Alkyl ersetzt sein können;
$n^2$ eine ganze Zahl zwischen 1 und 10, vorzugsweise zwischen 1 und 6, noch bevorzugter zwischen 1 und 4 ist;
Y ist $-(CH_2)_{n3}$,
wobei optional eines oder mehrere der H-Atome in $-(CH_2)_{n3}-$ durch $-(C_1-C_{10})$Alkyl ersetzt sein können;
$n^3$ eine ganze Zahl zwischen 1 und 10, vorzugsweise zwischen 1 und 6, noch bevorzugter zwischen 1 und 4 ist.

L ist eine Gruppe, bestehend aus einem oder mehreren und/oder jeweils einem oder mehreren der Elemente, ausgewählt aus der Gruppe: $-(CH_2)_{n1}-$, -O-, $-NR_{16}-$, -NHC(O)-, $-C(O)NR^{17}-$, - CH(OH)-, und $-CH(OR^{18})-$; vorzugsweise $-(CH_2)_{n1}-$, und -O-;
$n^1$ ist eine ganze Zahl zwischen 1 und 30, vorzugsweise zwischen 1 und 20, noch bevorzugter zwischen 1 und 10;
$n^4$ ist eine ganze Zahl zwischen 1 und 10, vorzugsweise zwischen 1 und 6, noch bevorzugter zwischen 1 und 4
$L_1$ wird ausgewählt aus der Gruppe bestehend aus

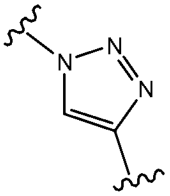 , 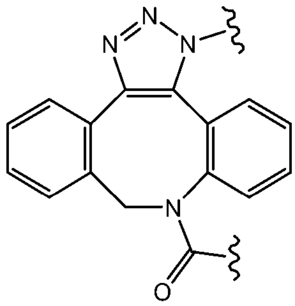 , 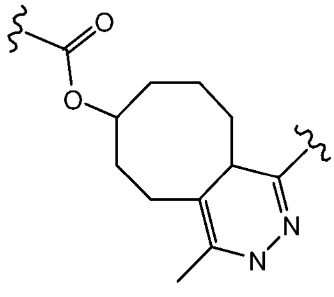 , 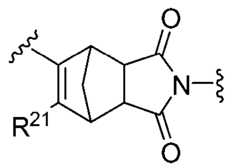 , ,

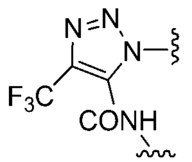 , 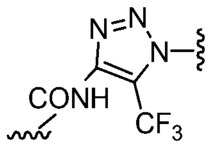 , –CO-, `

$R^{21}$ ist H, $-(C_1-C_{10})$Alkyl.

6. Verbindung nach Anspruch 4, wobei Z ausgewählt ist aus der Gruppe bestehend aus Octreotid, Octreotat, TOC, TATE, NP41, Leptin, Insulin, Glucagon, GLP-1, Methanobactin OB3b, PSMA (Prostata-spezifisches Membranantigen), PSMA-bindende Liganden wie KUE, Antikörper wie EGFR-gerichtete Antikörper wie Cetuximab, Panitumumab, HER2-gerichtete Antikörper wie Trastuzumab, CEA-gerichtete Antikörper, VEGFR-gerichtete Antikörper wie Bevatuzumab, $\alpha v\beta 6$-Integrin-Binder wie Trivehexin.

7. Verbindung nach Anspruch 4 oder 6, wobei $L_1$ aus der Gruppe ausgewählt ist, bestehend aus

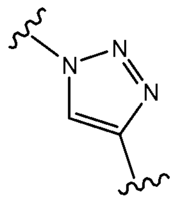 –CO-, `

$n^4$ eine ganze Zahl zwischen 1 und 10, vorzugsweise zwischen 1 und 6, noch bevorzugter zwischen 1 und 4 ist.

8. Verbindung nach Anspruch 1 bis 7, wobei L aus der Gruppe ausgewählt ist, die aus $-CH_2(CH_2OCH_2)_{n1}CH_2-$, $-(CH_2)_{n2}-$, $-CH_2(CH_2NHCH_2)_{n1}CH_2-$, $-CH_2(CH_2NR^{16}CH_2)_{n1}CH_2-$, $-CH_2(CH_2OCH_2)_{n1}CH_2C(O)(CH_2)_{n5}-$, $-CH_2(CH_2NHCH_2)_{n1}CH_2C(O)(CH_2)_{n5}-$, $-CH_2(CH_2NR^{16}CH_2)_{n1}CH_2C(O)(CH_2)_{n5}-$;

$n^1$ ist eine ganze Zahl zwischen 1 und 30, vorzugsweise zwischen 1 und 20, noch bevorzugter zwischen 1 und 10;
$n^2$ ist eine ganze Zahl zwischen 1 und 10, vorzugsweise zwischen 1 und 6, noch bevorzugter zwischen 1 und 4;
$n^5$ ist eine ganze Zahl zwischen 1 und 10, vorzugsweise zwischen 1 und 6, noch bevorzugter zwischen 1 und 4.

9. Verbindung nach Anspruch 4, wobei die Verbindung

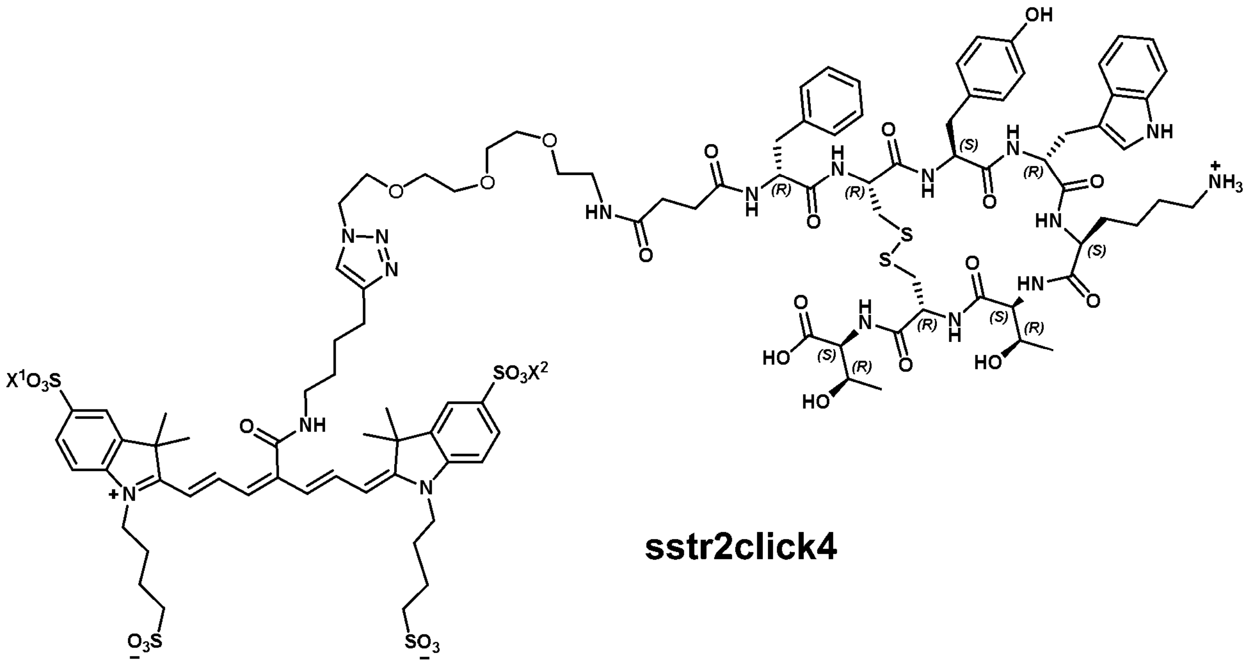

sstr2click4

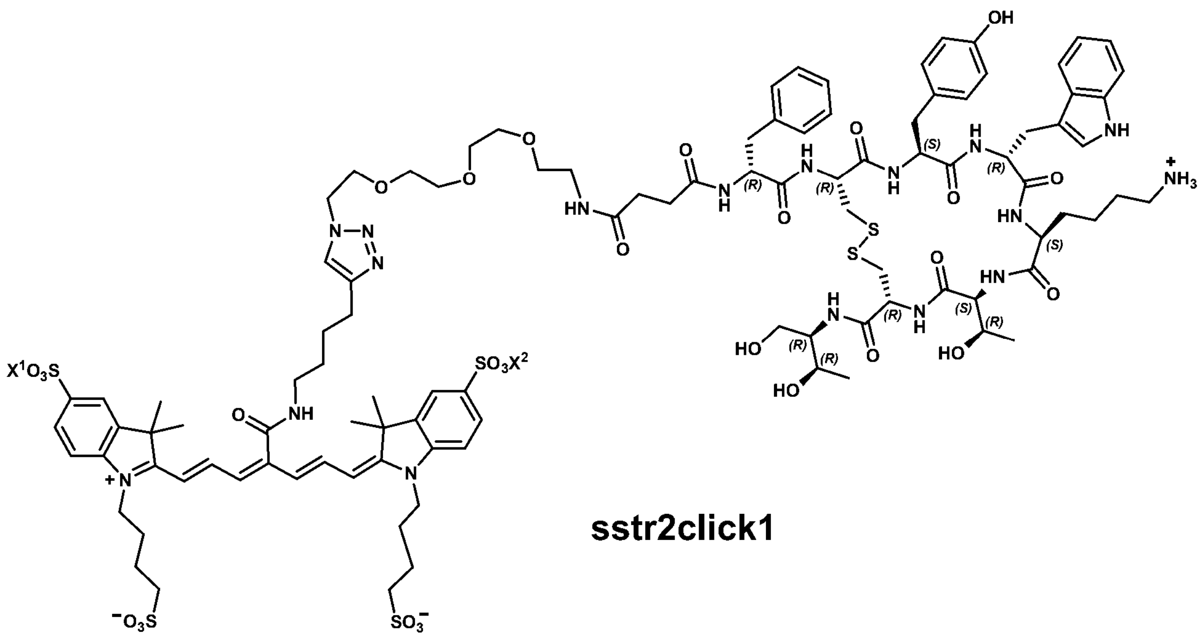

sstr2click1

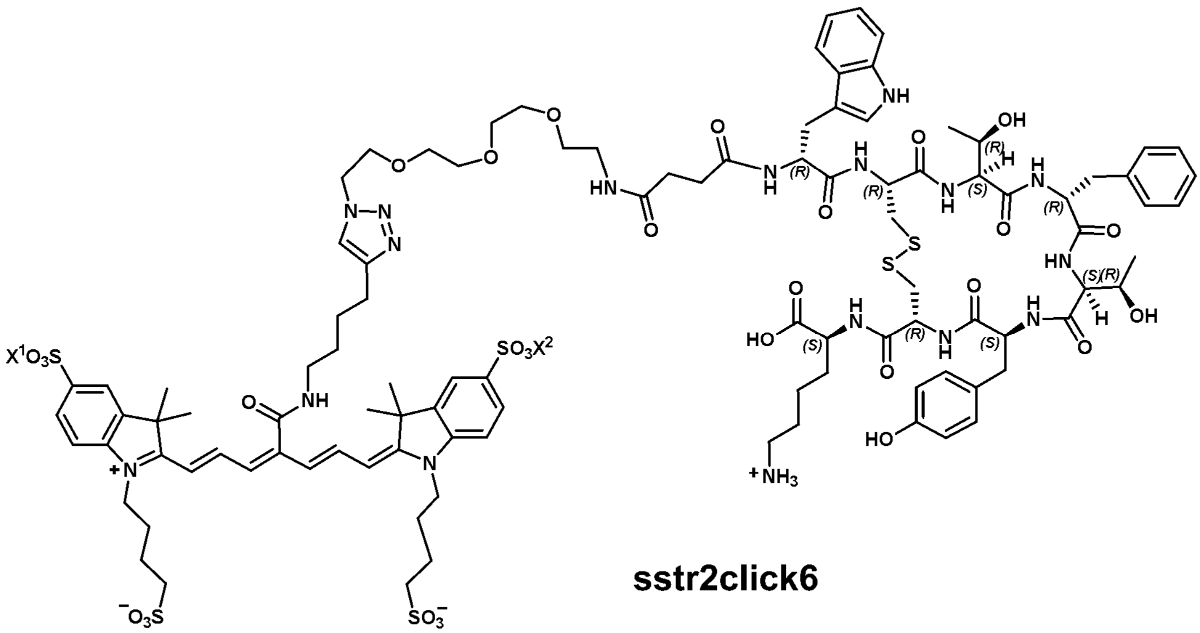

**sstr2click6**

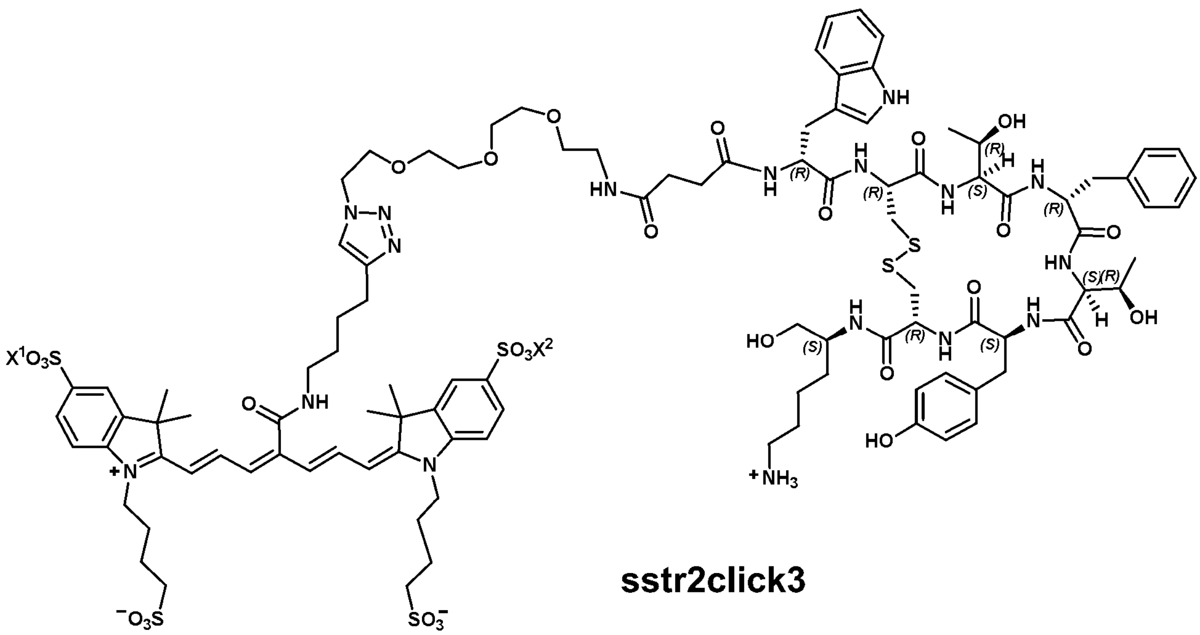

**sstr2click3**

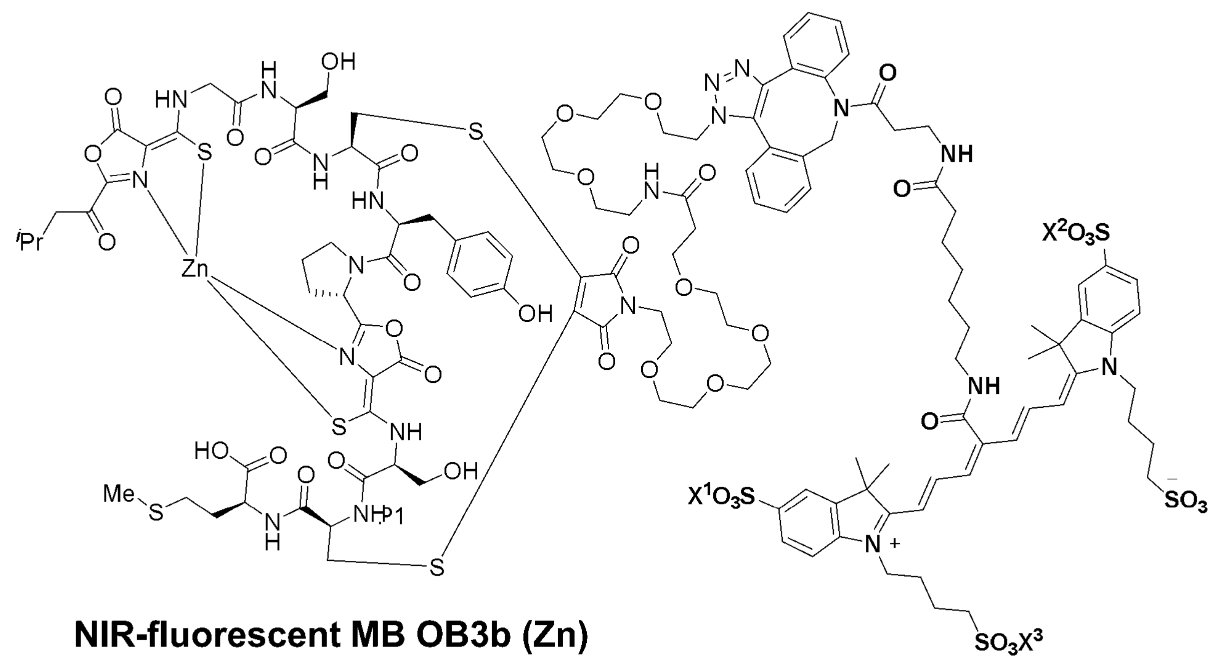

**NIR-fluorescent MB OB3b (Zn)**

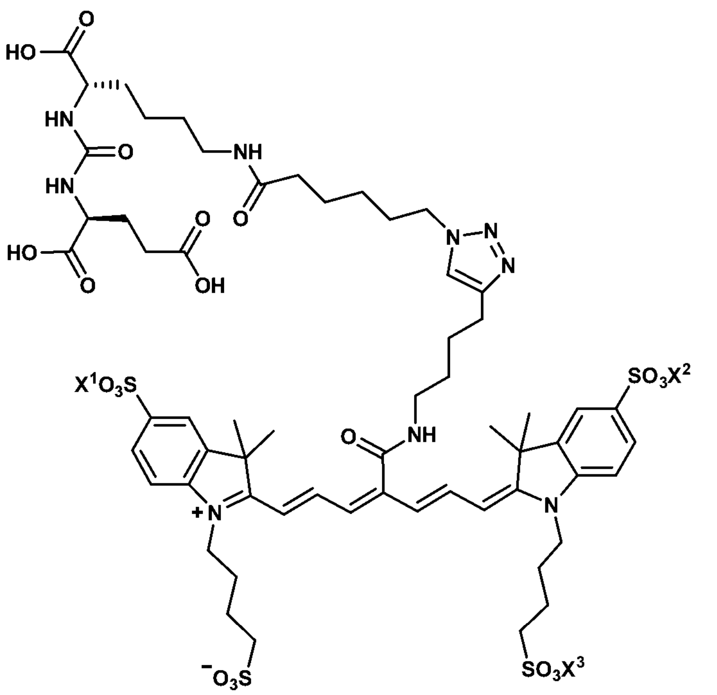

**NIR-fluorescent KUE Ligand I**

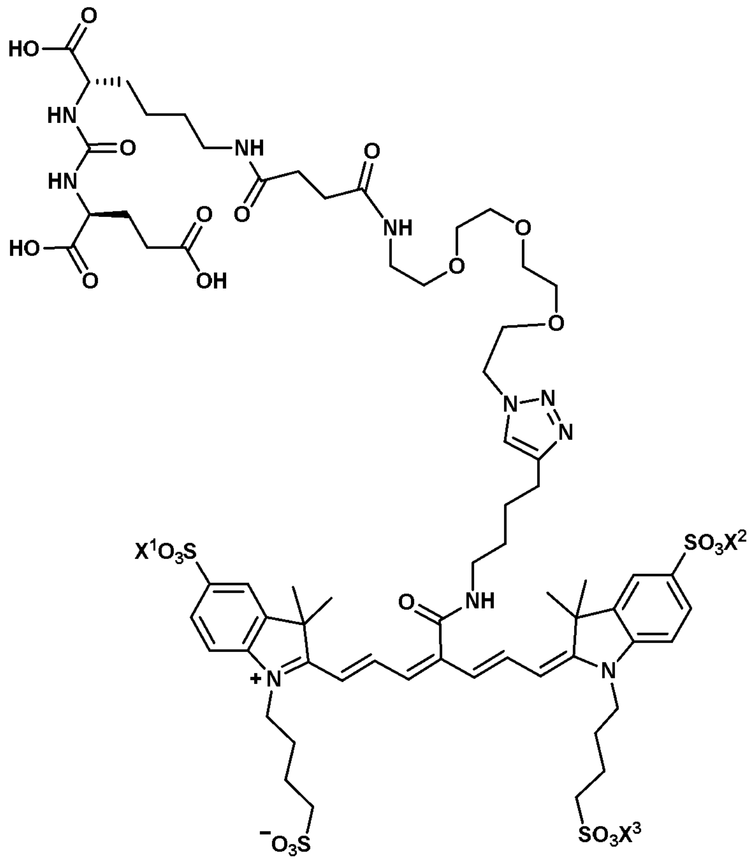

**NIR-fluorescent KUE Ligand II**

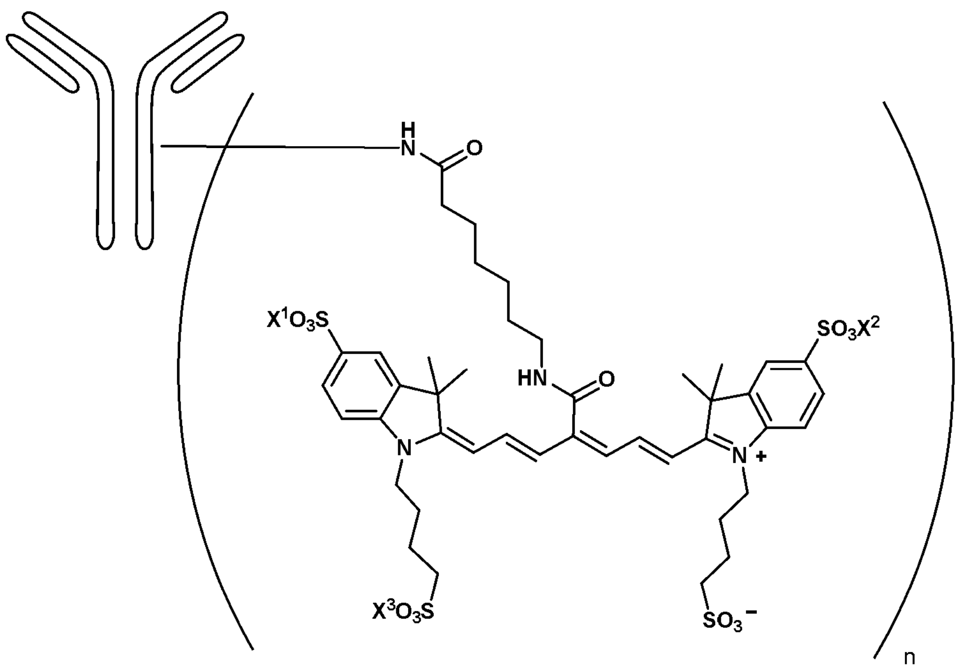

**NIR-fluorescent Panitumumab**

wobei der in der Formel symbolisch dargestellte Antikörper ("NIR-fluoreszierendes Panitumumab") Panitumumab ist,

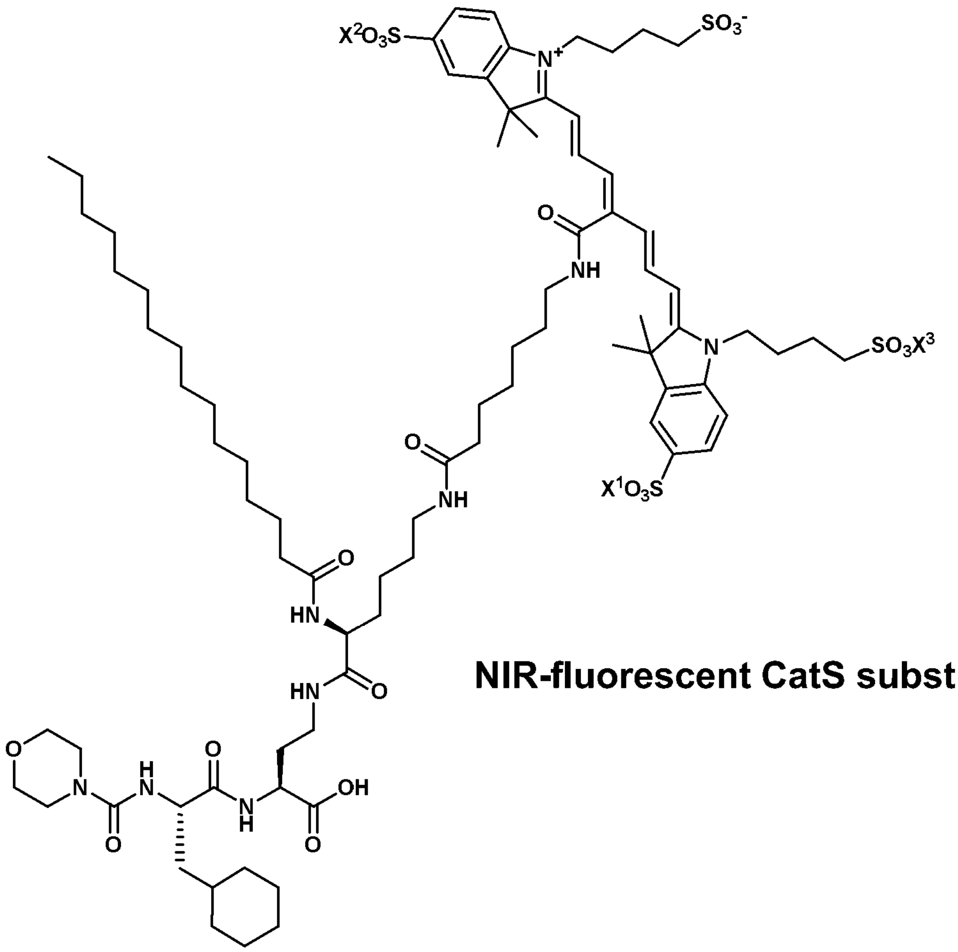

**NIR-fluorescent CatS substrate**

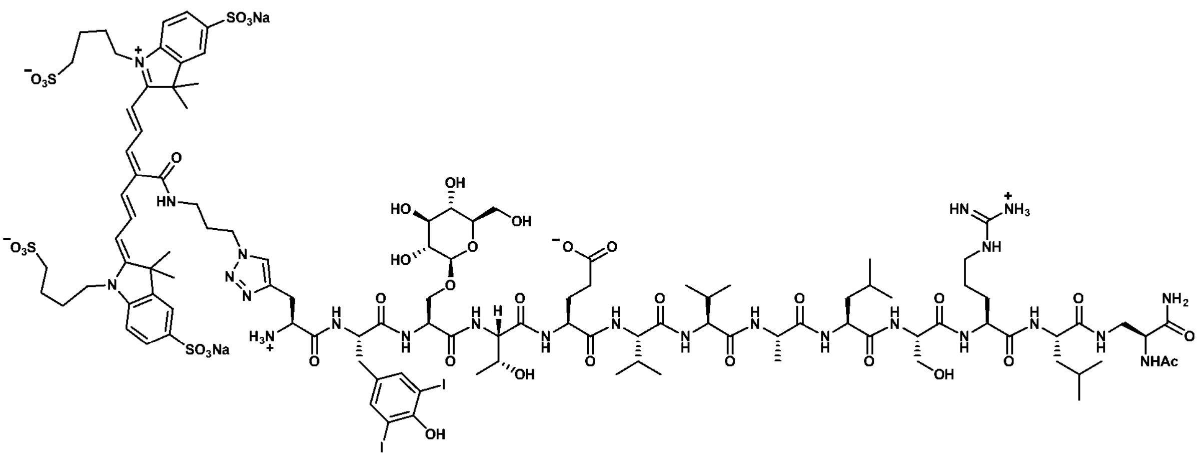

**NIR-fluorecent leptin-receptor-agonist-I**

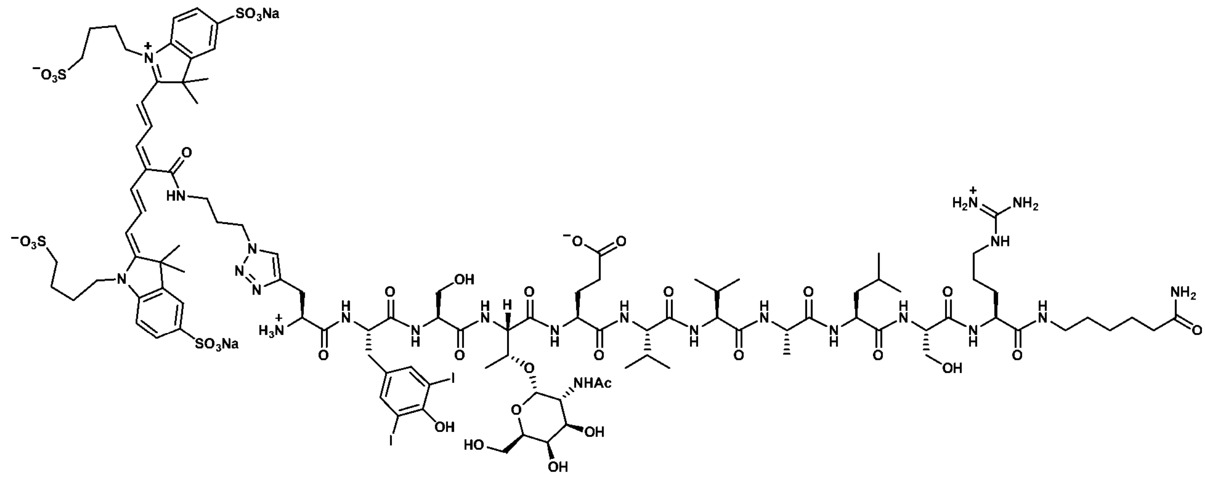

# NIR-fluorecent leptin-receptor-agonist-II

**10.** Die Verbindung nach den Ansprüchen 1 bis 9, wobei

i) die Emissionsspitze in PBS zwischen 400 nm und 1200 nm liegt, vorzugsweise zwischen 500 nm und 900 nm, noch bevorzugter zwischen 790 nm und 820 nm, und/oder
ii) der Absorptionsquerschnitt in PBS > 5.000 1/(M*cm), vorzugsweise > 50.000 1/(M*cm), noch bevorzugter > 200.000 1/(M*cm) beträgt und/oder
iii) die Absorptionsspitze in PBS liegt zwischen 400 nm und 1200 nm, vorzugsweise zwischen 600 nm und 1000 nm, noch bevorzugter zwischen 750 nm und 850 nm.

**11.** Verwendung der Verbindung nach den Ansprüchen 1 bis 10 als Fluoreszenzfarbstoff.

**12.** Die Verbindung nach den Ansprüchen 1 bis 10, wobei die Verbindung ein Kontrastmittel ist.

**13.** Die Verbindung nach den Ansprüchen 1 bis 12 zur Verwendung in der Diagnostik oder für intraoperative Eingriffe.

**14.** Verbindung zur Verwendung nach Anspruch 13, wobei die durch einen intraoperativen chirurgischen Eingriff zu diagnostizierende oder zu behandelnde Indikation aus der Gruppe ausgewählt ist, die aus entzündlichen Erkrankungen, Infektionskrankheiten und Krebs besteht.

**15.** Die Verbindung zur Verwendung nach Anspruch 14, wobei

i) die entzündliche Erkrankung aus der Gruppe ausgewählt ist, die aus Mittelohrentzündung, allergischer Rhinitis und chronischer Rhinosinusitis, chronischer Entzündung der Mandeln, Polypen und Laryngitis besteht;
ii) die Infektionskrankheit aus der Gruppe ausgewählt ist, die aus Implantatinfektionen (z. B. Hüfte, Zahn), Sekundärinfektionen des Innenohrs, z. B. im Zusammenhang mit einem Cholesteatom, besteht, die vorzugsweise durch grampositive oder gramnegative Bakterien verursacht werden, auf eine Antibiotikabehandlung ansprechen, resistent oder multiresistent sind, insbesondere durch ESKAPE-Stämme *wie Enterococcus faecium, Staphylococcus aureus, Klebsiella pneumoniae, Acinetobacter baumannii, Pseudomonas aeruginosa* und *Enterobacter spp.;*
iii) die Diagnose von Krebs oder die Behandlung von Krebserkrankungen durch intraoperative Chirurgie bezieht sich auf die Darstellung des Primärtumors und/oder metastatischer Läsionen in situ während der Operation, ausgewählt aus der Gruppe Meningeom, Glioblastom, NSCLC, Bauchspeicheldrüsenkrebs, neuroendokrine Tumoren, Lungenkrebs, Brustkrebs und Meningeome.

## Revendications

**1.** Composé selon la formule (I),

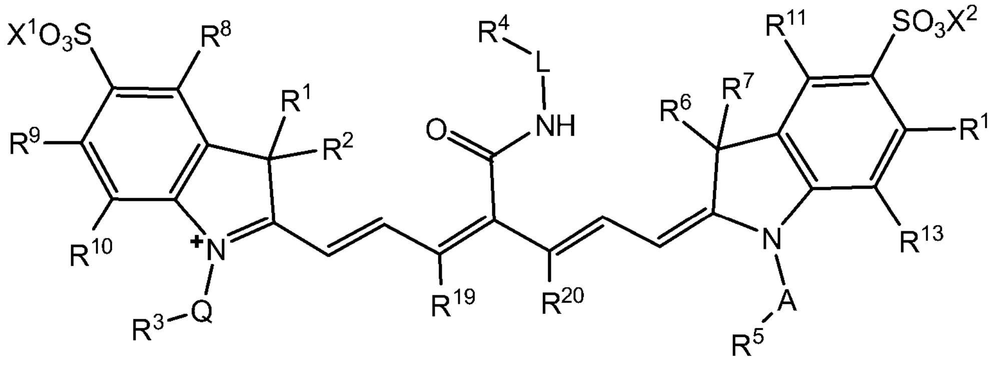

(I)

dans laquelle

$R^1$, $R^2$, $R^6$, $R^7$, $R^{14}$, $R^{15}$, $R^{16}$, $R^{17}$ et $R^{18}$ sont choisis indépendamment dans le groupe constitué par H et un groupe alkyle en $C_1$ à $C_6$, de préférence méthyle ;
$R^3$ est $-SO_4\,X^3$ ou COOH ;
$R^4$ est choisi parmi le groupe constitué de -éthynyle, -azide,

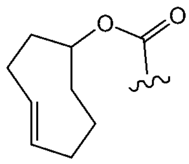, 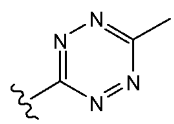,

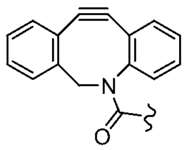 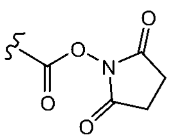, 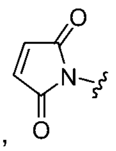 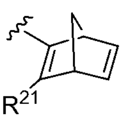, 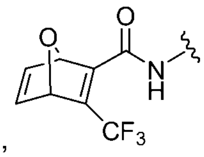

-COOH, $NH_2$, Cl, Br, I, - $OSO_2\,CF_3$ , $-COSO_2\,CH_3$ , -OH, vinyle, -SH ;
$R^5$ est $-SO_3\,X^4$ ;
$R^8$, $R^9$, $R^{10}$, $R^{11}$, $R^{12}$ et $R^{13}$ sont choisis indépendamment parmi le groupe constitué de H, CN, - $COO(C_1\text{-}C_6)$ alkyle, $-CONH(C_1\text{-}C_6)$alkyle, Cl, Br, I, F, $-CF_3$, $-O(C_4\text{-}C_6)$, $-NR^{14}R^{15}$ , $-NHC(O)(C_1\text{-}C_6)$alkyle, $-NHC(O)NH(C_1\text{-}C_6)$alkyle), $-SO_3\,H$, $-SO_2\,NH_2$ , $-(C_1\text{-}C_6)$alkyle, de préférence H ;
$R^{19}$ et $R^{20}$ sont choisis indépendamment parmi le groupe constitué de H, d'un groupe $(C_1\text{-}C_6)$ alkyle, d'un groupe $-O(C_1\text{-}C_6)$alkyle, de Cl, de F, de Br, de CN ; $C(O)NH_2$ , de préférence H ;
$R^{21}$ est H, un groupe $(C_1\text{-}C_{10})$alkyle;
L est un groupe constitué d'un ou plusieurs et/ou d'un ou plusieurs de chacun des éléments choisis parmi le groupe : $-(CH_2)_{n1}$ -, $-(C_1\text{-}C_6)$cycloalkylène-, $-(C_1\text{-}C_6)$hétérocycloalkylène-, -O-, $-NR^{16}$ -, -NH-, -NHC(O)-, $-C(O)NR^{17}$-, -CH(OH)-, et $-CH(OR^{18})$- ;
$n^1$ est un nombre entier compris entre 1 et 30, de préférence entre 1 et 20, plus préférablement entre 1 et 10;
$X^1$, $X^2$, $X^3$ et $X^4$ sont choisis indépendamment parmi un cation, un proton ou l'absence de cation ; de préférence, le cation est un cation monovalent ou un cation divalent ; de manière plus préférentielle, le cation est un cation monovalent; de manière encore plus préférentielle, le cation est un cation de métal alcalin ou un cation ammonium; de manière particulièrement préférée, le cation est choisi indépendamment dans le groupe constitué de $Na^+$ , $K^+$ ; et $NH_4^+$;
Q est $-(CH_2)_{n2}$ -,
dans lequel, éventuellement, un ou plusieurs des H présents dans $-(CH_2)_{n2}$ - peuvent être remplacés par un groupe $-(C_1\text{-}C_{10})$alkyle ;
$n^2$ est un nombre entier compris entre 1 et 10, de préférence entre 1 et 6, plus préférablement entre 1 et 4 ;
A représente $-(CH_2)n_3$-,
dans lequel, éventuellement, un ou plusieurs des atomes d'hydrogène (H) dans $-(CH_2)_{n3}$ - peuvent être remplacés par un groupe $(C_1\text{-}C_{10})$alkyle;

$n^3$ est un nombre entier compris entre 1 et 10, de préférence entre 1 et 6, plus préférablement entre 1 et 4.

**2.** Composés selon la revendication 1, dans lesquels

$R^1$, $R^2$, $R^6$, $R^7$ sont des groupes méthyle ;
$R^3$ est $-SO_3$ $X^3$ ;
$R^4$est choisi parmi le groupe constitué de -éthynyle, -azide,

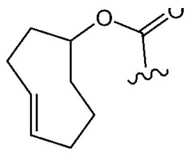, 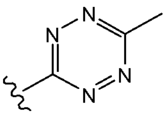,

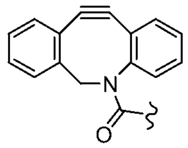 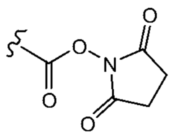., 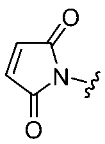, 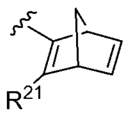, 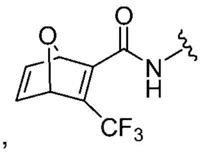

-COOH et $NH_2$, de préférence éthynyle, -azide,

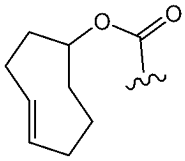, 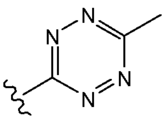, 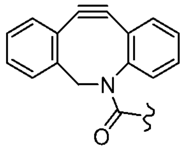 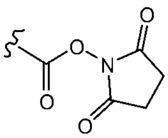;

$R^5$ est $-SO_3$ $X^4$ ;
$R^8$, $R^9$, $R^{10}$, $R^{11}$, $R^{12}$et $R^{13}$ sont H;
$R^{16}$, $R^{17}$et $R^{18}$sont choisis indépendamment parmi le groupe constitué de H et d'un groupe $(C_1-C_6)$alkyle, de préférence un groupe méthyle ;
$R^{19}$ et $R^{20}$ sont H;
$R^{21}$ est H, un groupe $(C_1-C_6)$alkyle;
L est un groupe constitué d'un ou plusieurs et/ou d'un ou plusieurs de chacun des éléments choisis parmi le groupe: $-(CH_2)_{n1}$-, -O-, $-NR^{16}$-, -NHC(O)-, $-C(O)NR^{17}$-, -CH(OH)-, et - $CH(OR^{18})$- ; de préférence $-(CH_2)_{n1}$-, et -O- ;
$n^1$ est un nombre entier compris entre 1 et 30, de préférence entre 1 et 20, plus préférablement entre 1 et 10 ;
$X^1$ , $X^2$ , $X^3$ et $X^4$ sont choisis indépendamment parmi un cation, un proton ou l'absence de cation; de préférence, le cation est un cation monovalent ou un cation divalent ; de manière plus préférentielle, le cation est un cation monovalent ; de manière encore plus préférentielle, le cation est un cation de métal alcalin ou un cation ammonium ; de manière particulièrement préférée, le cation est choisi indépendamment dans le groupe constitué de $Na^+$ , $K^+$ ; et $NH_4^+$;
Q est $-(CH_2)_{n2}$-,
dans laquelle, éventuellement, un ou plusieurs des atomes d'hydrogène (H) présents dans le groupe $-(CH_2)_{n2}$ peut être remplacé par un groupe alkyle $-(C_1-C_{10})$,
$n^2$ est un nombre entier compris entre 1 et 10, de préférence entre 1 et 6, plus préférablement entre 1 et 4;
A représente $-(CH_2)n_3$-,
dans lequel, éventuellement, un ou plusieurs atomes d'hydrogène H, dans $-(CH_2)_{n3}$- peuvent être remplacés par un groupe $(C_1-C_{10})$alkyle;
$n^3$ est un nombre entier compris entre 1 et 10, de préférence entre 1 et 6, plus préférablement entre 1 et 4.

**3.** Composé selon la revendication 1, dans lequel le composé est choisi parmi

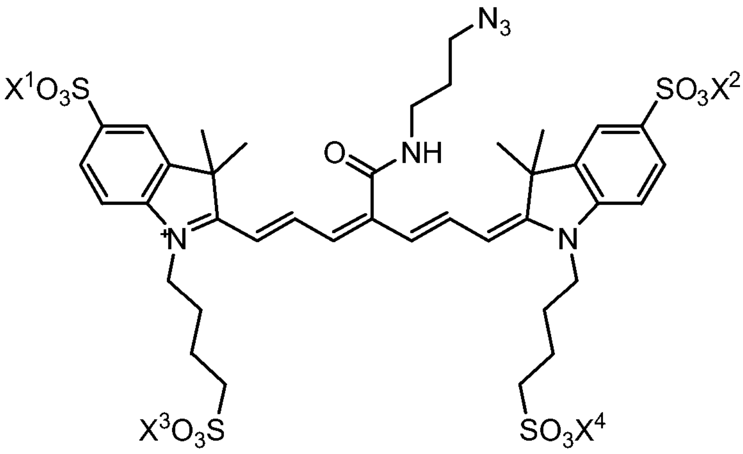

et ,

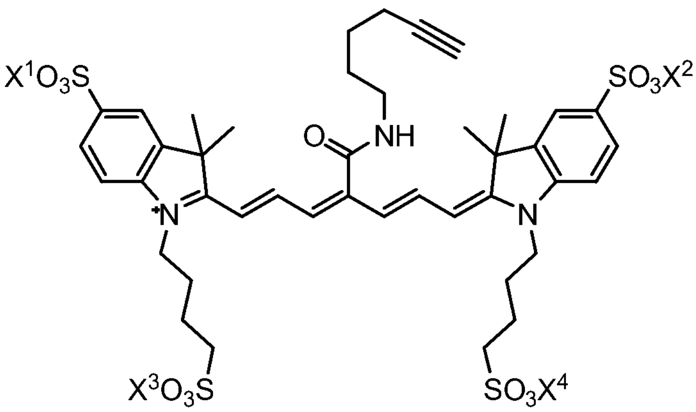

,

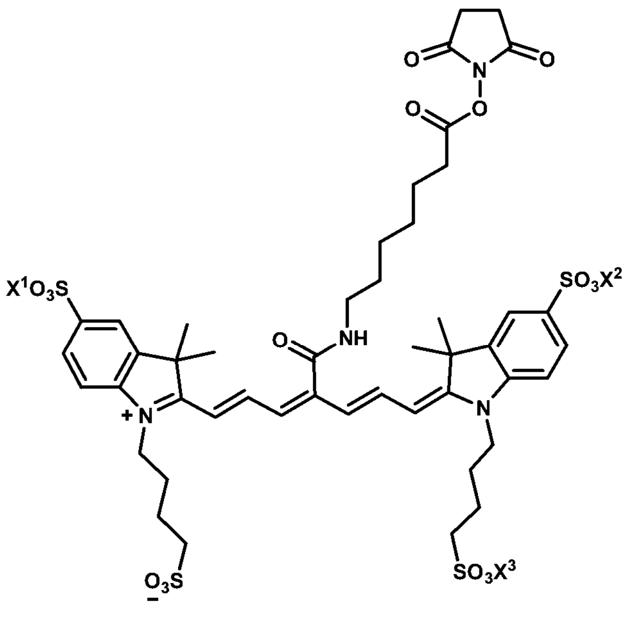

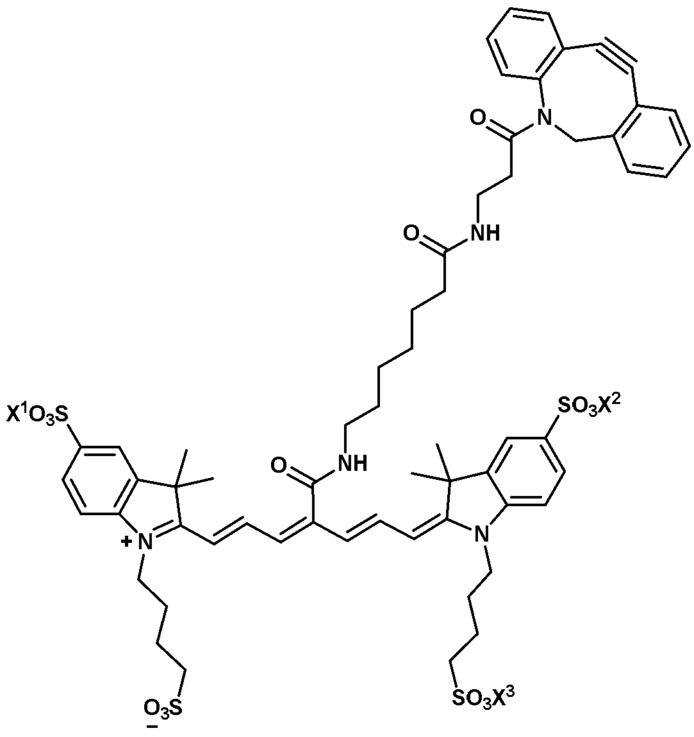

**4.** Composé selon la formule (II)

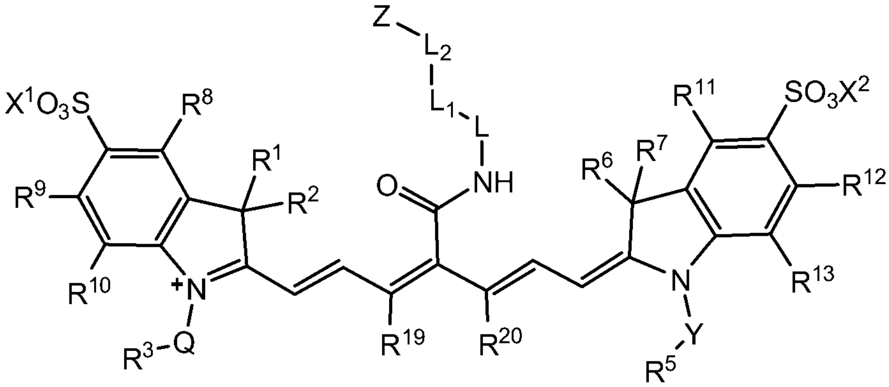

(II)

dans laquelle

$R^1$, $R^2$, $R^6$, $R^7$, $R^{14}$, $R^{15}$, $R^{16}$, $R^{17}$ et $R^{18}$ sont choisis indépendamment dans le groupe constitué par H et un groupe $(C_1-C_6)$ alkyle, de préférence méthyle ;
$R^3$ est $-SO_3\ X^3$ ou COOH;
Z est une protéine, un saccharide mono- ou polyvalent, un peptide, une petite molécule, un anticorps, un oligonucléotide, un nanocorps ou un polymère;
$R^5$ est $-SO_3\ X^4$;
$R^8$, $R^9$, $R^{10}$, $R^{11}$, $R^{12}$et $R^{13}$ sont choisis indépendamment parmi le groupe constitué de H, CN, - $COO(C_1 -C_6)$ alkyle, $-CONH(C_1 -C_6)$alkyle, Cl, Br, I, F, $-CF_3$, $-O(C_4 -C_6)$, $-NR^{14} R^{15}$ , $-NHC(O)(C_1 -C_6)$alkyle, $-NHC(O)NH(C_1 -C_6)$alkyle), $-SO_3 H$, $-SO_2 NH_2$ , $-(C_1 -C_6)$alkyle, de préférence H ;
$R^{19}$, $R^{20}$ sont choisis indépendamment dans le groupe constitué de H, $-(C_1 -C_6)$alkyle, $-O(C_1 -C_6)$alkyle, Cl, F, Br, CN ; $-C(O)NH_2$, de préférence H ;
$R^{21}$ est H, un groupe $(C1-C_{10})$alkyle;
$X^1$, $X^2$, $X^3$ et $X^4$ sont choisis indépendamment parmi un cation, un proton ou l'absence de cation, de préférence le

cation est un cation monovalent ou un cation divalent, plus préférablement le cation est un cation monovalent, de manière encore plus préférable un cation de métal alcalin ou un cation ammonium, particulièrement préférablement le cation est choisi indépendamment dans le groupe constitué de $Na^+$ , $K^+$ ; et $NH_4^+$ ;

Q est $-(CH_2)_{n2}-$,

dans lequel, éventuellement, un ou plusieurs des atomes d'hydrogène (H) dans $-(CH_2)_{n2}-$peuvent être remplacés par un groupe $(C_1-C_{10})$alkyle;

$n^2$ est un nombre entier compris entre 1 et 10, de préférence entre 1 et 6, plus préférablement entre 1 et 4 ;

Y est $-(CH_2)_{n3}-$,

dans laquelle, éventuellement, un ou plusieurs des atomes d'hydrogène (H) présents dans le groupe $-(CH_2)n_3$ peuvent être remplacés par un groupe $-(C_1-C_{10})$alkyle;

$n^3$ est un nombre entier compris entre 1 et 10, de préférence entre 1 et 6, plus préférablement entre 1 et 4 ;

L est un groupe constitué d'un ou plusieurs et/ou d'un ou plusieurs de chacun des éléments choisis parmi le groupe : $-(CH_2)_{n1}-$, $-(C_1-C_6)$cycloalkylène-, $-(C_1-C_6)$hétérocycloalkylène-, -O-, $-NR^{16}$ -, -NH-, -NHC(O)-, -C(O)$NR^{17}$ -, -CH(OH)-, et $-CH(OR^{18})$- ;

$n^1$ est un nombre entier compris entre 1 et 30, de préférence entre 1 et 20, plus préférablement entre 1 et 10 ;

$L_1$ est choisi parmi le groupe constitué de

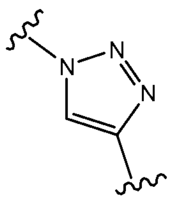 , 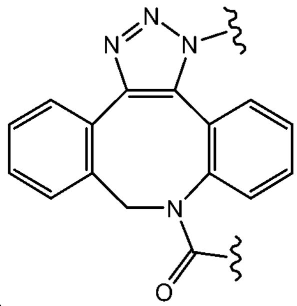 , 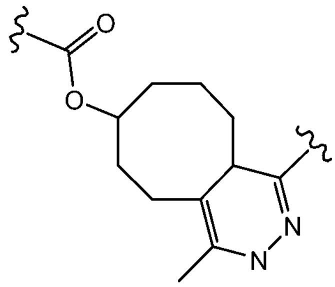 , 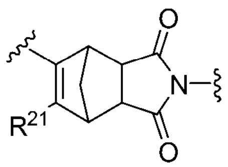 ,

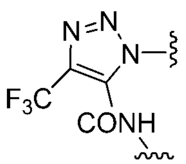 , 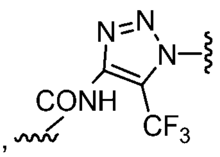 , –CO- ;

$L_2$ est un groupe constitué d'un ou plusieurs et/ou d'un ou plusieurs de chacun des éléments choisis parmi le groupe : $-(CH_2)_{n1}$ -, $-(C_1-C_6)$cycloalkylène-, $-(C_1-C_6)$hétérocycloalkylène-, - O-, $-NR^{16}$ -, -NH-, -NHC(O)-, -C(O)$NR^{17}$ -, -CH(OH)-, et $-CH(OR^{18})$-.

**5.** Composé selon la revendication 4, dans lequel

$R^1$, $R^2$, $R^6$ et $R^7$représentent un groupe méthyle;

$R^3$ est $-SO_3X^3$ ;

$R^5$ est $-SO_3X^4$ ;

$R^8$, $R^9$, $R^{10}$, $R^{11}$, $R^{12}$et $R^{13}$représentent H;

$R^{16}$, $R^{17}$et $R^{18}$ sont choisis indépendamment dans le groupe constitué par H et un groupe (C1-$C_6$)alkyle, de préférence un groupe méthyle ;

$R^{19}$ et $R^{20}$ représentent H ;

$X^1$, $X^2$ , $X^3$et $X^4$ sont choisis indépendamment parmi un cation, un proton ou l'absence de cation, de préférence le cation est un cation monovalent ou un cation divalent, plus préférablement le cation est un cation monovalent, de manière encore plus préférable un cation de métal alcalin ou un cation ammonium, particulièrement préférablement le cation est choisi indépendamment parmi le groupe constitué de $Na^+$ , $K^+$ ; et $NH_4^+$ ;

Q est $-(CH_2)_{n2}$ -,

dans lequel, éventuellement, un ou plusieurs des H présents dans $-(CH_2)_{n2}$ - peuvent être remplacés par un groupe $-(C_1-C_{10})$alkyle ;

$n^2$ est un nombre entier compris entre 1 et 10, de préférence entre 1 et 6, plus préférablement entre 1 et 4 ;

Y est $-(CH_2)n_3$-,

dans lequel, éventuellement, un ou plusieurs des atomes d'hydrogène dans $-(CH_2)_{n3}$ - peuvent être remplacés

par un groupe ($C_1$-$C_{10}$)alkyle;
$n^3$ est un nombre entier compris entre 1 et 10, de préférence entre 1 et 6, plus préférablement entre 1 et 4.
L est un groupe constitué d'un ou plusieurs et/ou d'un ou plusieurs de chacun des éléments choisis parmi le groupe : $-(CH_2)_{n1}-$, -O-, $-NR^{16}-$, -NHC(O)-, $-C(O)NR^{17}-$, -CH(OH)-, et $-CH(OR^{18})-$ ; de préférence $-(CH_2)_{n1}-$, et -O- ;
$n^1$ est un nombre entier compris entre 1 et 30, de préférence entre 1 et 20, plus préférablement entre 1 et 10 ;
$n^4$ est un nombre entier compris entre 1 et 10, de préférence entre 1 et 6, plus préférablement entre 1 et 4
$L_1$ est choisi parmi le groupe constitué de

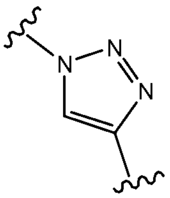 , 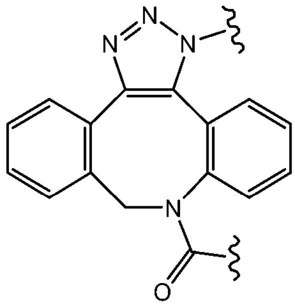 , 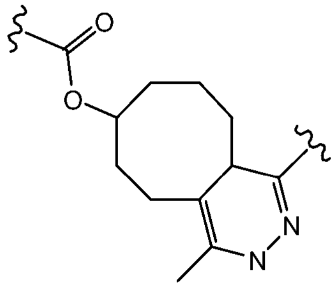 , 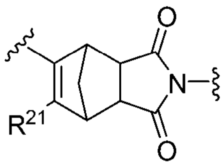 , ,

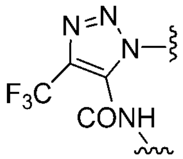 , 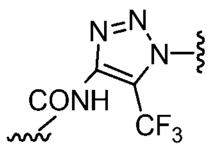 , –CO-, `

$R^{21}$ est H, un groupe alkyle en $C_1$ à $C_{10}$.

6. Composé selon la revendication 4, dans lequel Z est choisi parmi le groupe constitué de l'octréotide, l'octréotate, le TOC, le TATE, le NP41, la leptine, l'insuline, le glucagon, le GLP-1, la méthanobactine OB3b, le PSMA (antigène membranaire spécifique de la prostate), des ligands de liaison au PSMA tels que KUE, des anticorps tels que des anticorps ciblant l'EGFR comme le cétuximab, le panitumumab, des anticorps ciblant HER2 tels que le trastuzumab, des anticorps ciblant le CEA, des anticorps ciblant le VEGFR tels que le bevatuzumab, des agents de liaison à l'intégrine $\alpha v\beta 6$ tels que la trivehexine.

7. Composé selon la revendication 4 ou 6, dans lequel $L_1$ est choisi parmi le groupe constitué de

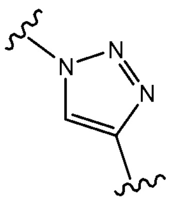 , –CO-, `

$n^4$ est un nombre entier compris entre 1 et 10, de préférence entre 1 et 6, et plus préférablement entre 1 et 4.

8. Composé selon l'une des revendications 1 à 7, dans lequel L est choisi parmi le groupe constitué de $-CH_2(CH_2OCH_2)_{n1}CH_2-$, $-(CH_2)_{n2}-$, $-CH_2(CH_2NHCH_2)_{n1}CH_2-$, $-CH_2(CH_2NR^{16}CH_2)_{n1}CH_2-$, $-CH_2(CH_2OCH_2)_{n1}CH_2C(O)(CH_2)_{n5}-$, $-CH_2(CH_2NHCH_2)_{n1}CH_2C(O)(CH_2)_{n5}-$, $-CH_2(CH_2NR^{16}CH_2)_{n1}CH_2C(O)(CH_2)_{n5}-$;

$n^1$ est un nombre entier compris entre 1 et 30, de préférence entre 1 et 20, plus préférablement entre 1 et 10 ;
$n^2$ est un nombre entier compris entre 1 et 10, de préférence entre 1 et 6, plus préférablement entre 1 et 4 ;
$n^5$ est un nombre entier compris entre 1 et 10, de préférence entre 1 et 6, et plus préférablement entre 1 et 4.

9. Composé selon la revendication 4, dans lequel le composé est

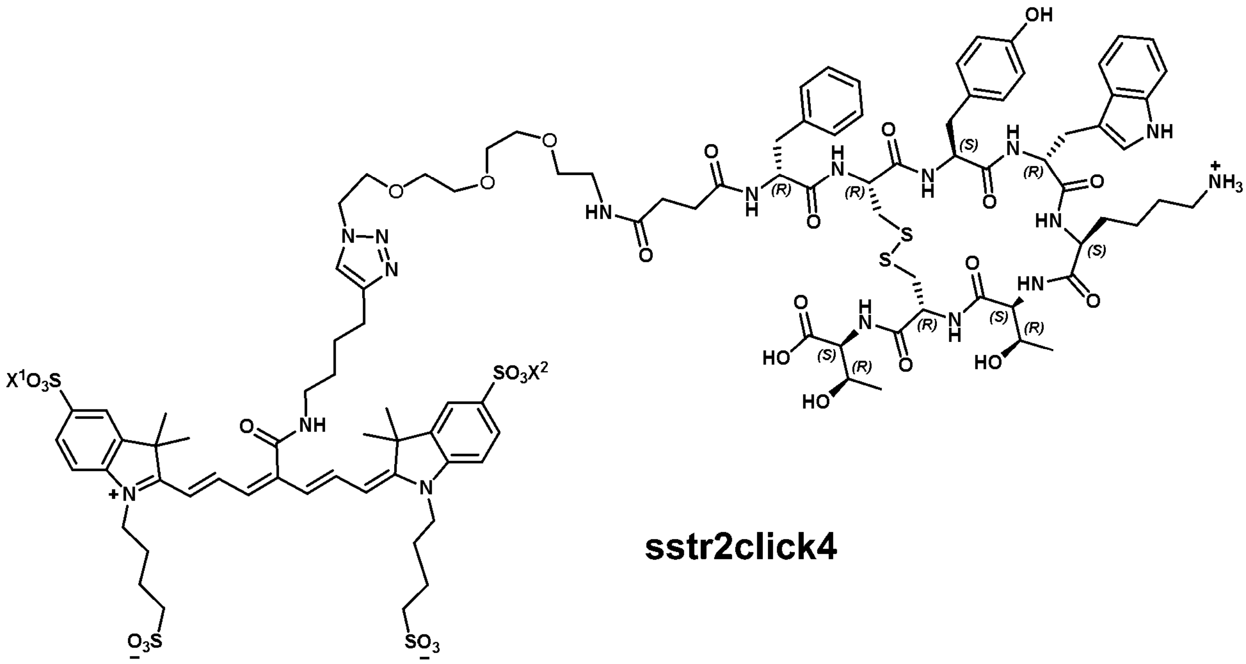

sstr2click4

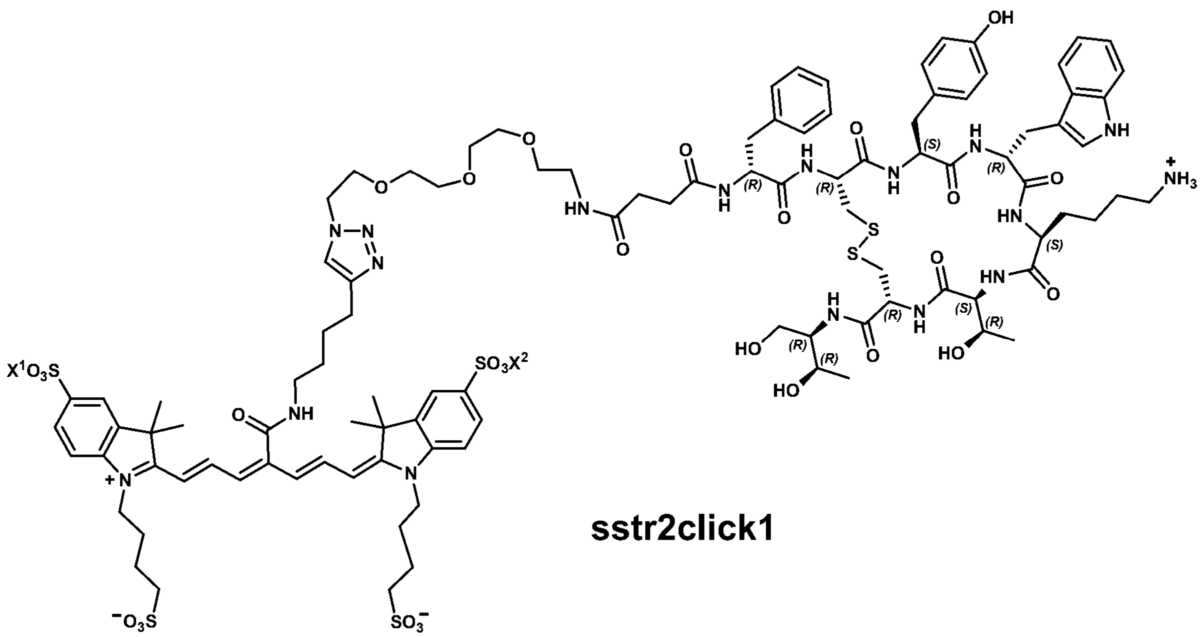

sstr2click1

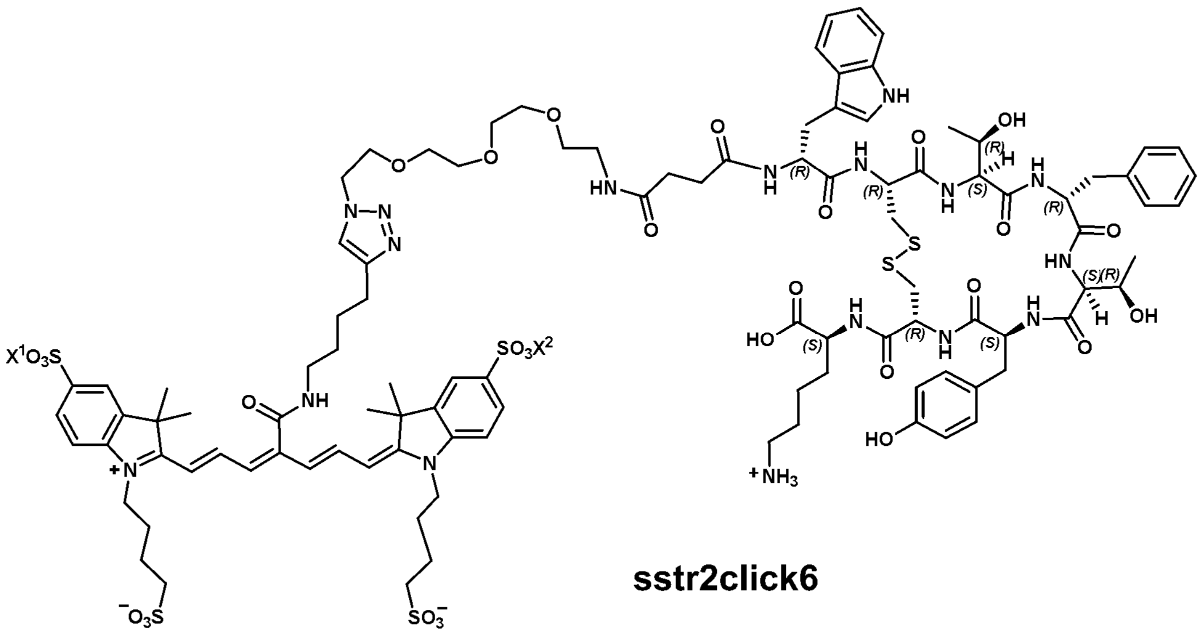

sstr2click6

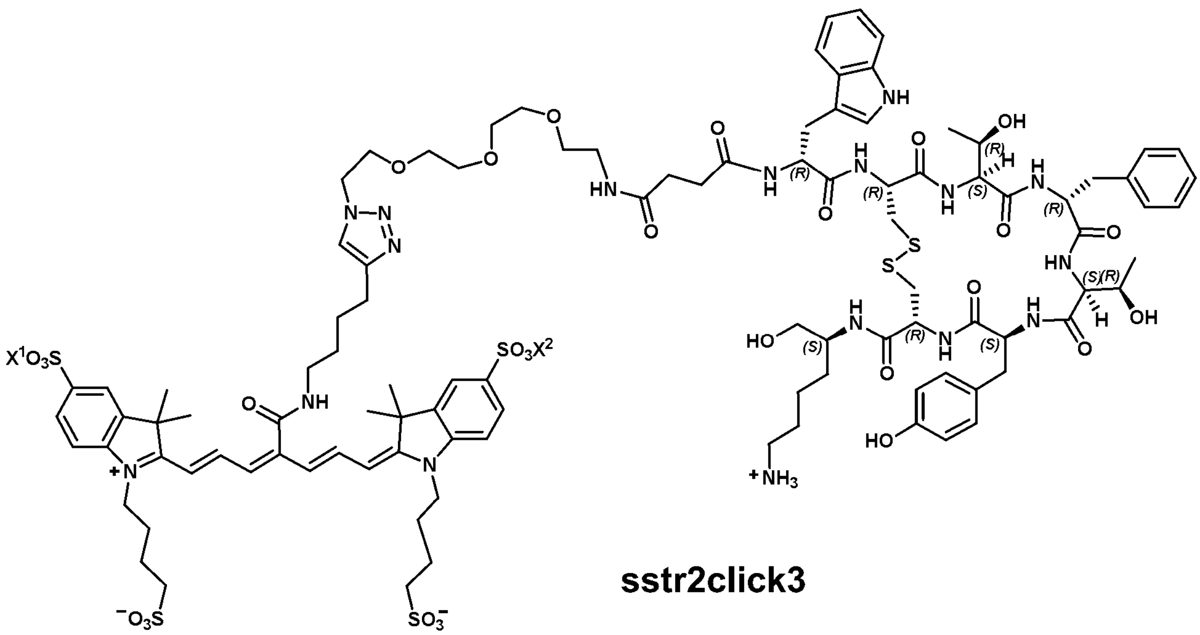

sstr2click3

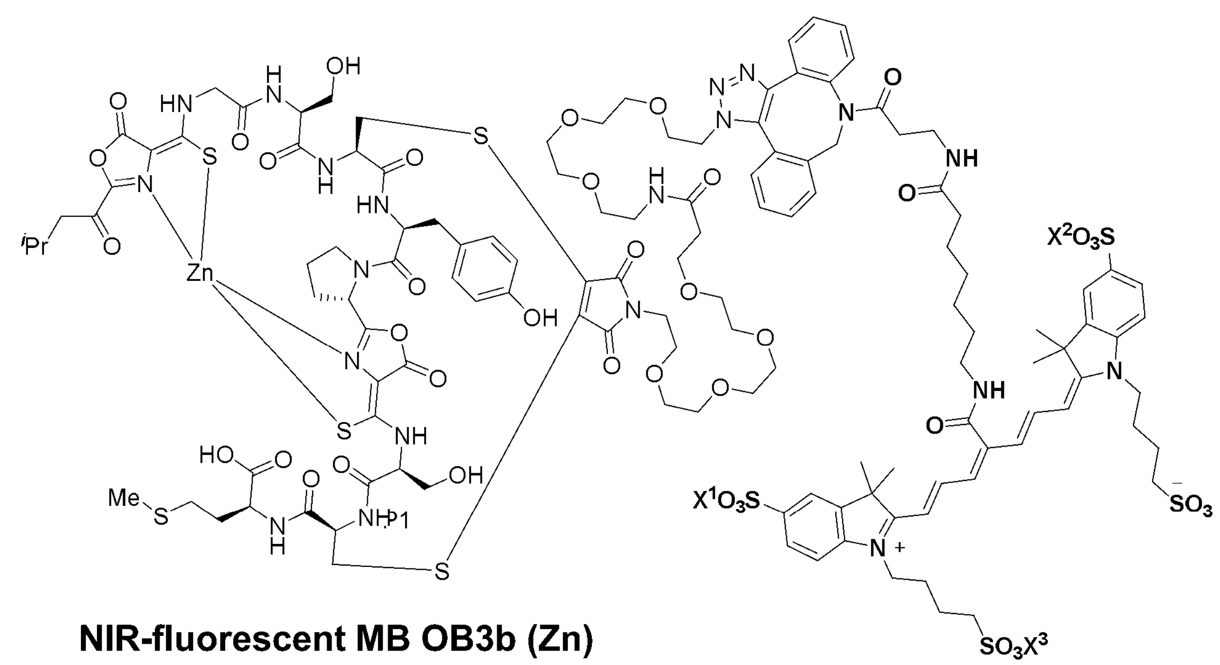

NIR-fluorescent MB OB3b (Zn)

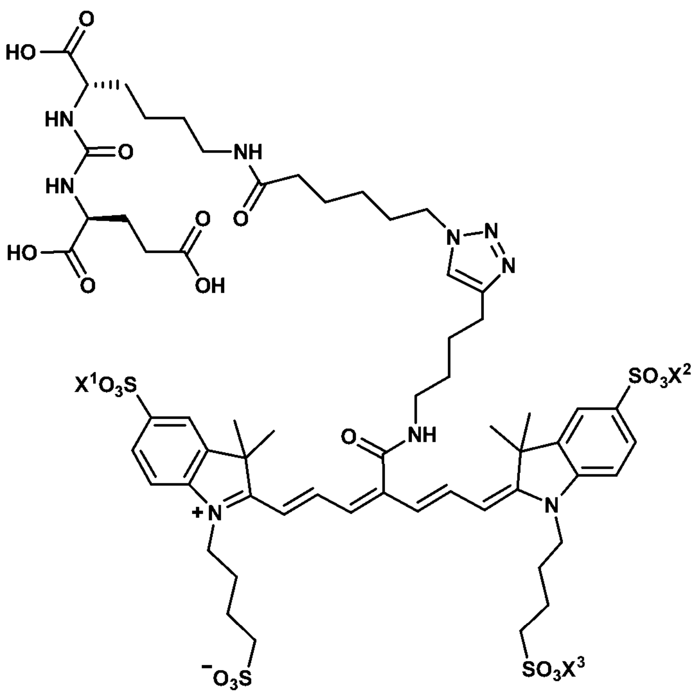

NIR-fluorescent KUE Ligand I

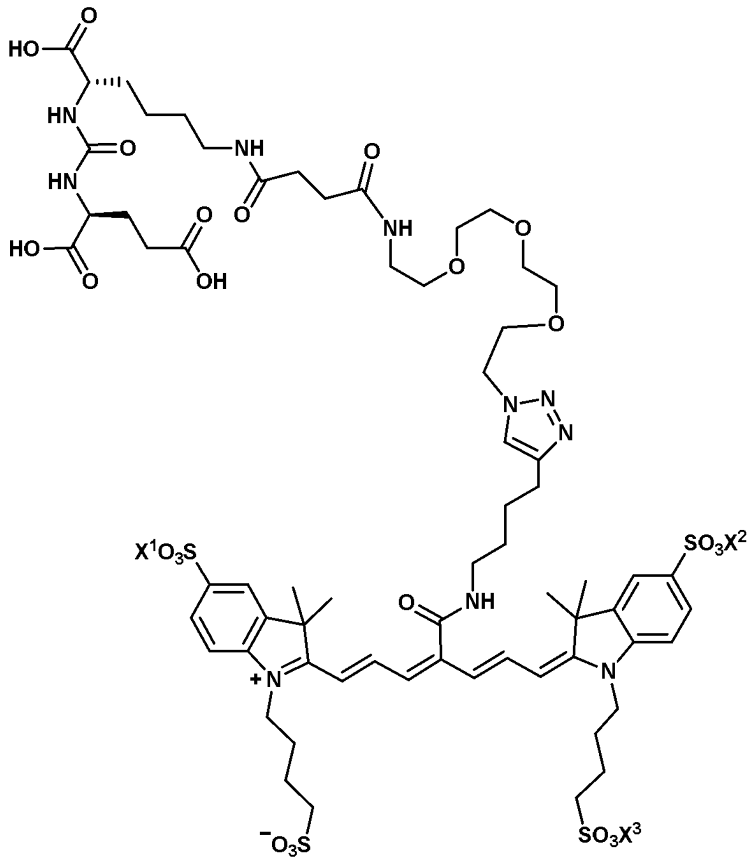

**NIR-fluorescent KUE Ligand II**

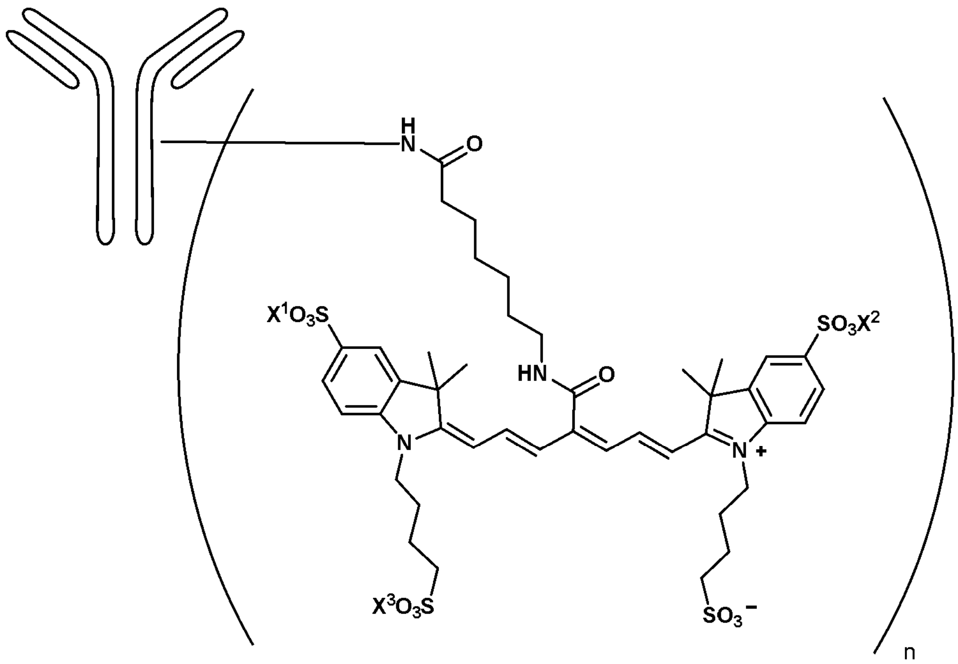

**NIR-fluorescent Panitumumab**

dans lequel l'anticorps représenté symboliquement dans la formule (« Panitumumab NIR-fluorescent ») est le Panitumumab,

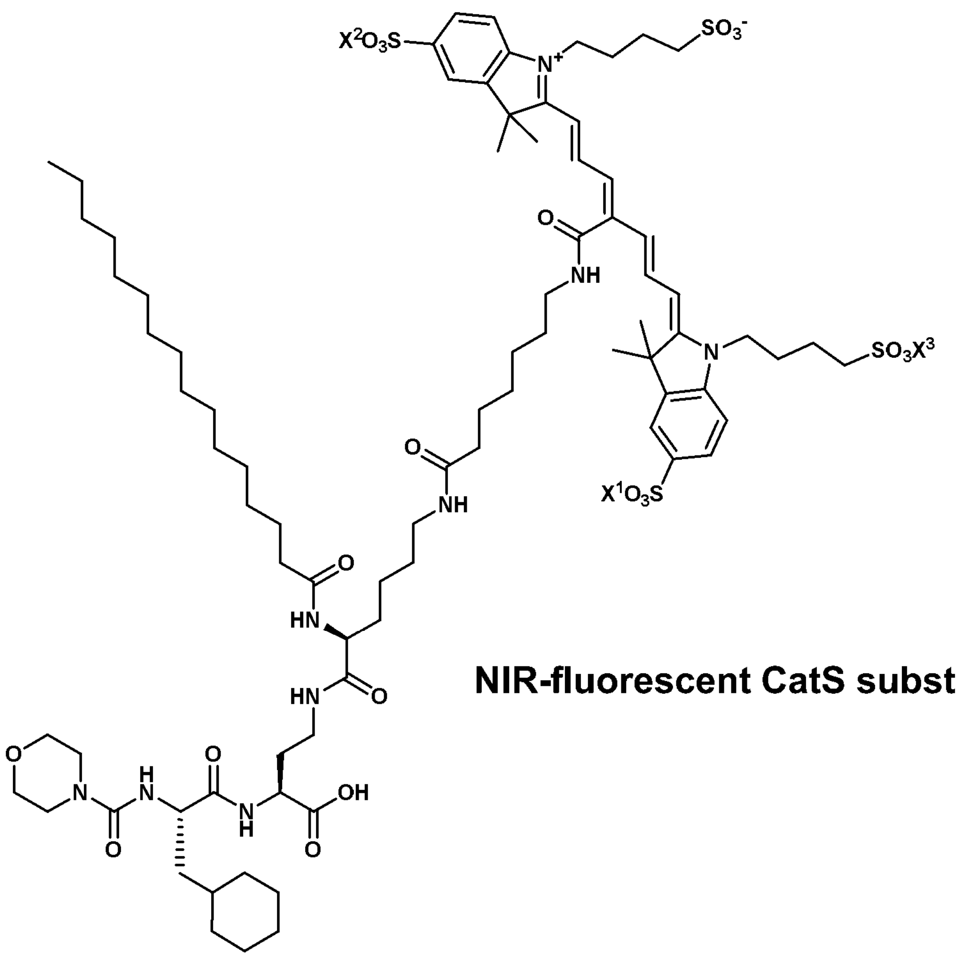

NIR-fluorescent CatS substrate

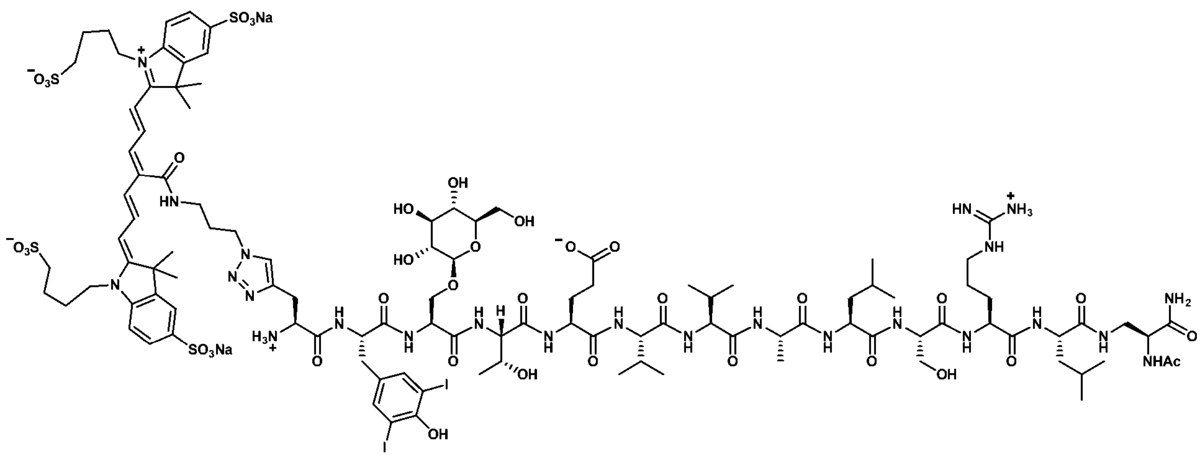

NIR-fluorecent leptin-receptor-agonist-I

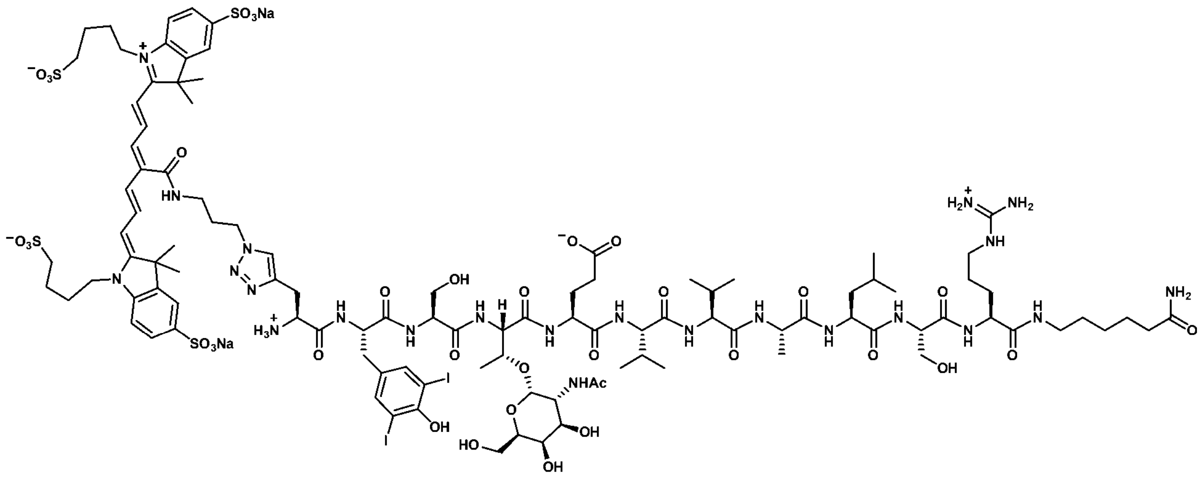

**NIR-fluorecent leptin-receptor-agonist-II**

10. Composé selon les revendications 1 à 9, dans lequel

i) le pic d'émission dans le PBS est compris entre 400 nm et 1 200 nm, de préférence entre 500 nm et 900 nm, plus préférablement entre 790 nm et 820 nm et/ou
ii) la section efficace d'absorption dans le PBS est > 5 000 1/(M*cm), de préférence > 50 000 1/(M*cm), plus préférablement > 200 000 1/(M*cm) et/ou
iii) le pic d'absorption dans le PBS est compris entre 400 nm et 1 200 nm, de préférence entre 600 nm et 1 000 nm, plus préférablement entre 750 nm et 850 nm.

11. Utilisation du composé selon les revendications 1 à 10 en tant que colorant fluorescent.

12. Le composé selon les revendications 1 à 10, dans lequel le composé est un agent de contraste.

13. Le composé selon les revendications 1 à 12 destiné à être utilisé en diagnostic ou en chirurgie peropératoire.

14. Composé destiné à être utilisé selon la revendication 13, dans lequel l'indication devant faire l'objet d'un diagnostic ou d'un traitement par chirurgie peropératoire est choisie parmi le groupe comprenant les maladies inflammatoires, les maladies infectieuses et le cancer.

15. Composé destiné à être utilisé selon la revendication 14, dans lequel

i) la maladie inflammatoire est choisie parmi le groupe comprenant l'otite moyenne, la rhinite allergique et la rhinosinusite chronique, l'inflammation chronique des amygdales, des végétations adénoïdes et la laryngite ;
ii) la maladie infectieuse est choisie parmi le groupe comprenant les infections d'implants (par exemple de la hanche, dentaires), les infections secondaires de l'oreille interne, par exemple associées à un cholestéatome, de préférence causées par des bactéries à Gram positif ou à Gram négatif, sensibles au traitement antibiotique, résistantes ou multirésistantes, en particulier par les souches ESKAPE *Enterococcus faecium, Staphylococcus aureus, Klebsiella pneumoniae, Acinetobacter baumannii, Pseudomonas aeruginosa* et *Enterobacter spp.*
iii) le diagnostic du cancer ou le traitement des cancers par chirurgie peropératoire concerne la visualisation in situ, pendant l'intervention chirurgicale, de la tumeur primaire et/ou des lésions métastatiques, choisies parmi le groupe comprenant le méningiome, le glioblastome, le CPNPC, le cancer du pancréas, les tumeurs neuroendocrines, le cancer du poumon, le cancer du sein et les méningiomes.

**Figure 1**

**A**

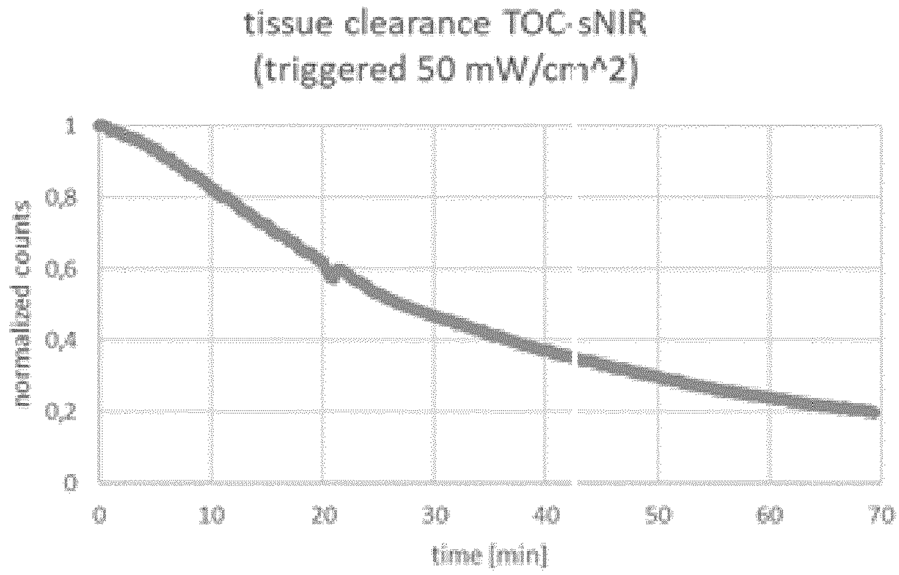
tissue clearance TOC-sNIR
(triggered 50 mW/cm^2)
normalized counts
1
0,8
0,6
0,4
0,2
0
0
10
20
30
40
50
60
70
time [min]

**B**

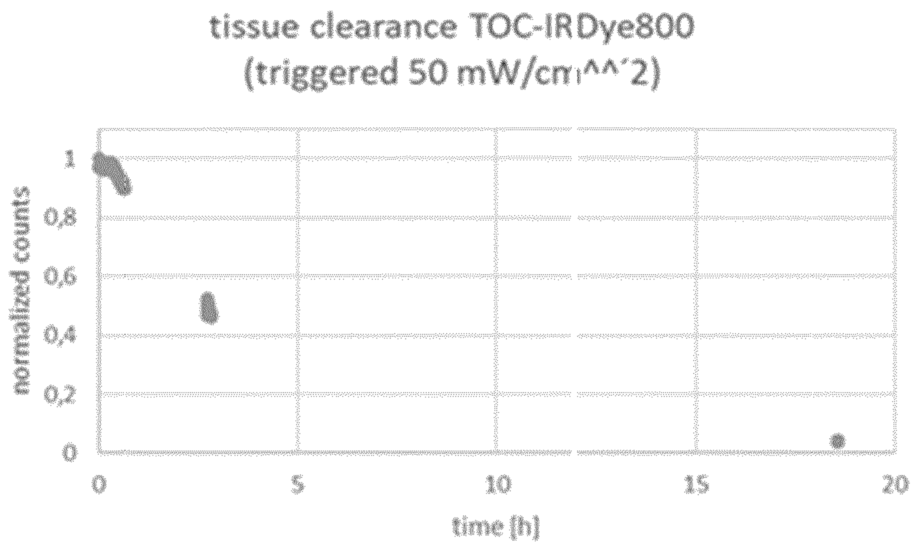
tissue clearance TOC-IRDye800
(triggered 50 mW/cm^^'2)
normalized counts
1
0,8
0,6
0,4
0,2
0
0
5
10
15
20
time [h]

**Figure 1 continued**

C

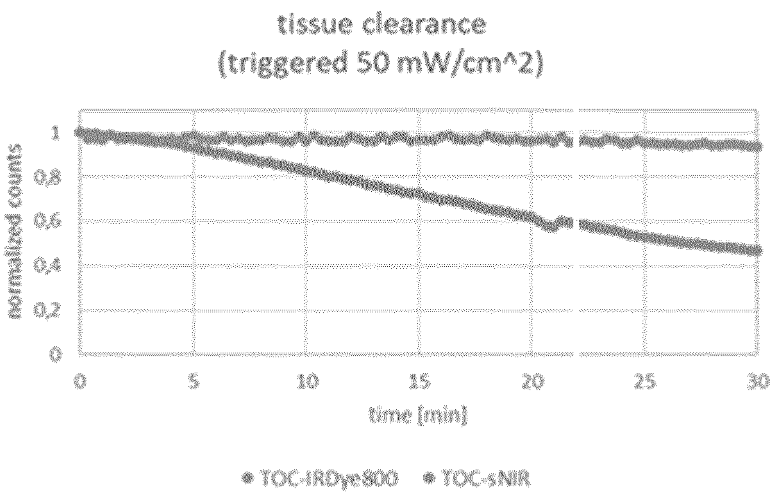


D

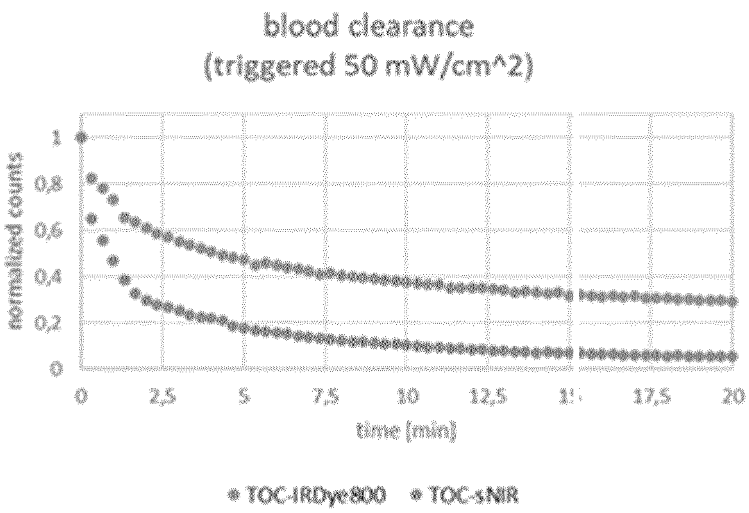

## Figure 2

### A

5 nmol TOC-sNIR i.v. injected, 3 h PI start bleaching
(continous 785 nm laser, 1.68A, 200 mW/cm^2, 1 frame/10 sec)

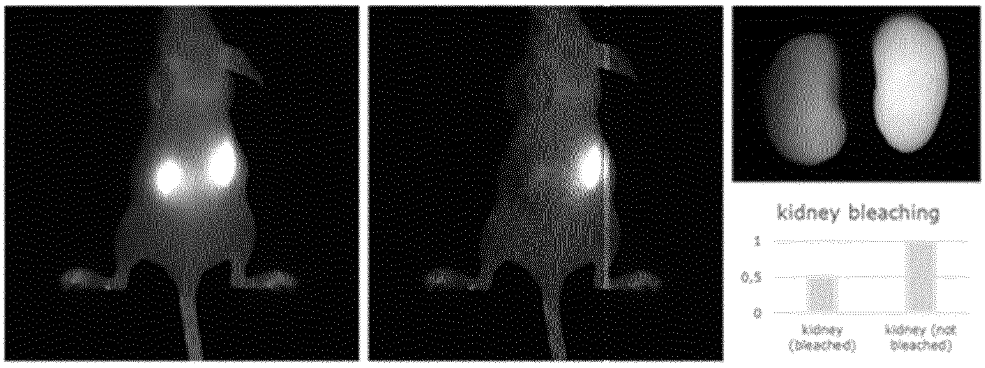


30 min → bleaching not complete

### B

5 nmol TOC-IRDye800 i.v. injected, 3 h PI start bleaching
(continous 785 nm laser, 1.68A, 200 mW/cm^2, 1 frame/10 sec)

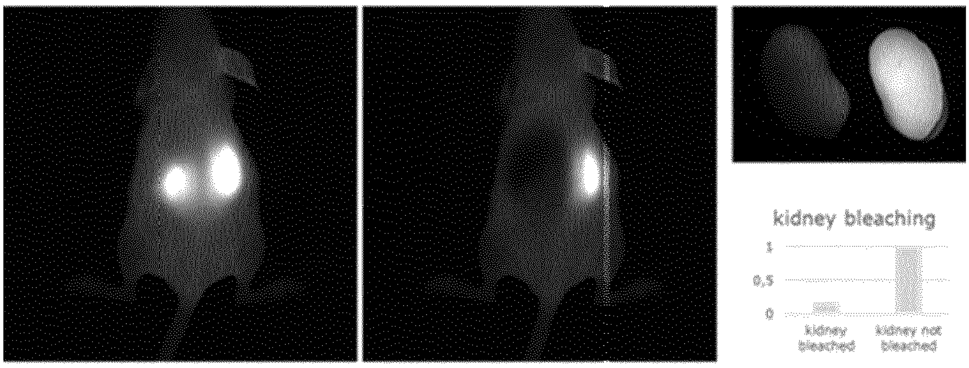


30 min → bleaching complete

## Figure 2 continued

C

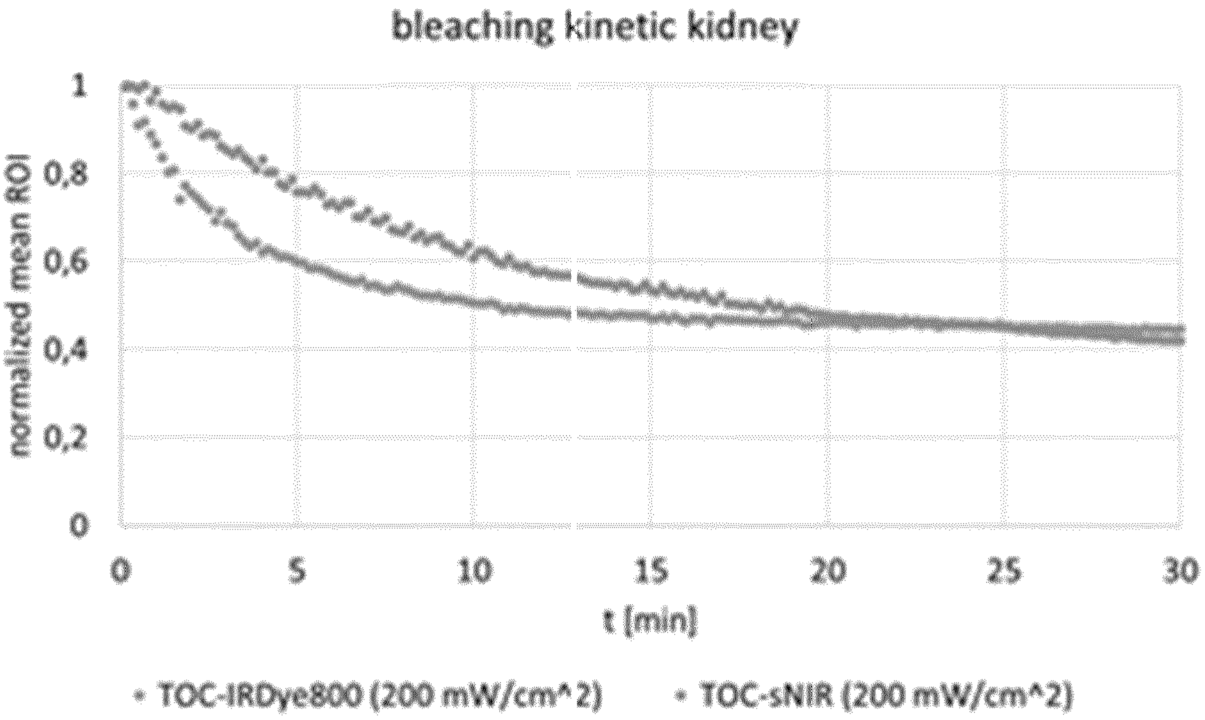


D

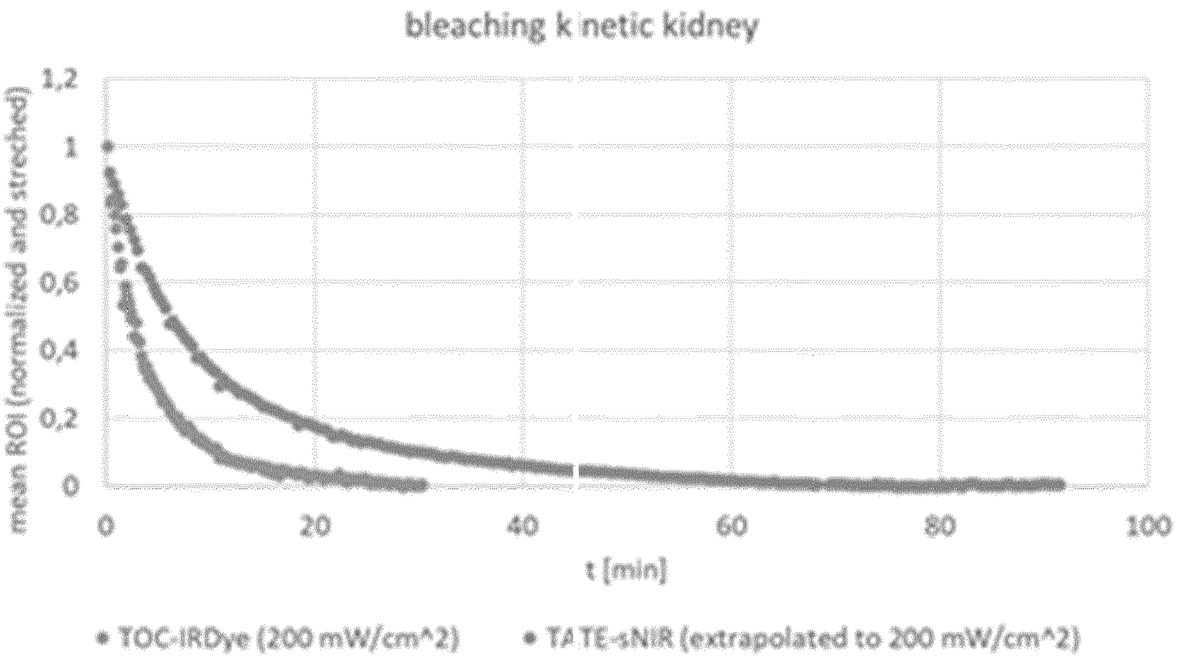

## Figure 3

**A**

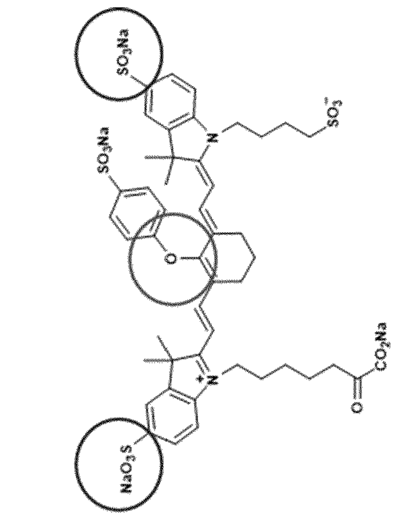

**IRDye800®CW**

$C_{47}H_{50}N_2Na_4O_{16}S_4$

M [g/mol] = 1119.1141

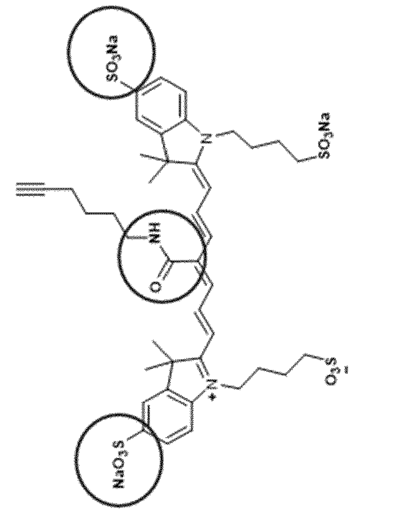

**sNIR**

$C_{42}H_{50}N_3Na_3O_{13}S_4$

M [g/mol] = 1002,0793

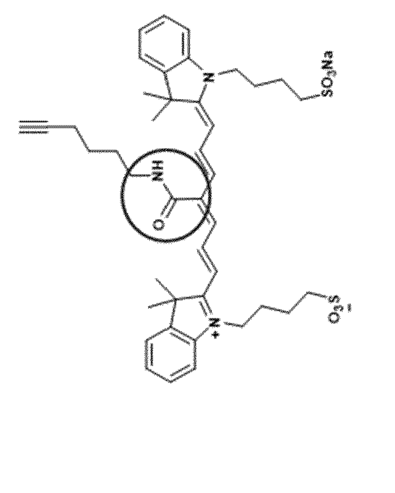

**NIR**

$C_{42}H_{52}N_3NaO_7S_2$

M [g/mol] = 798,0018

**Figure 3 continued**

**B**

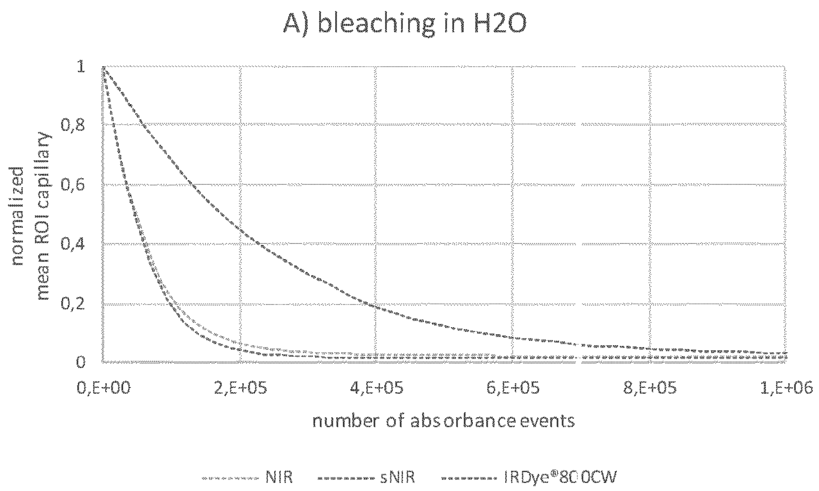


**C**

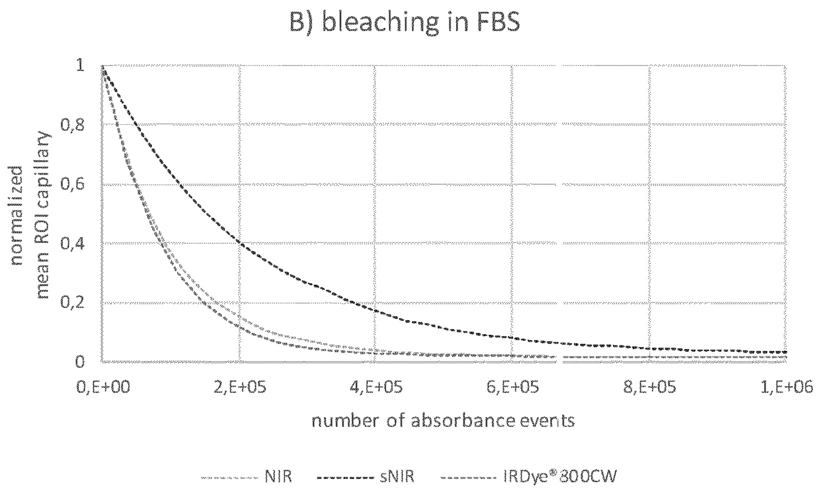

**Figure 4**

**A**

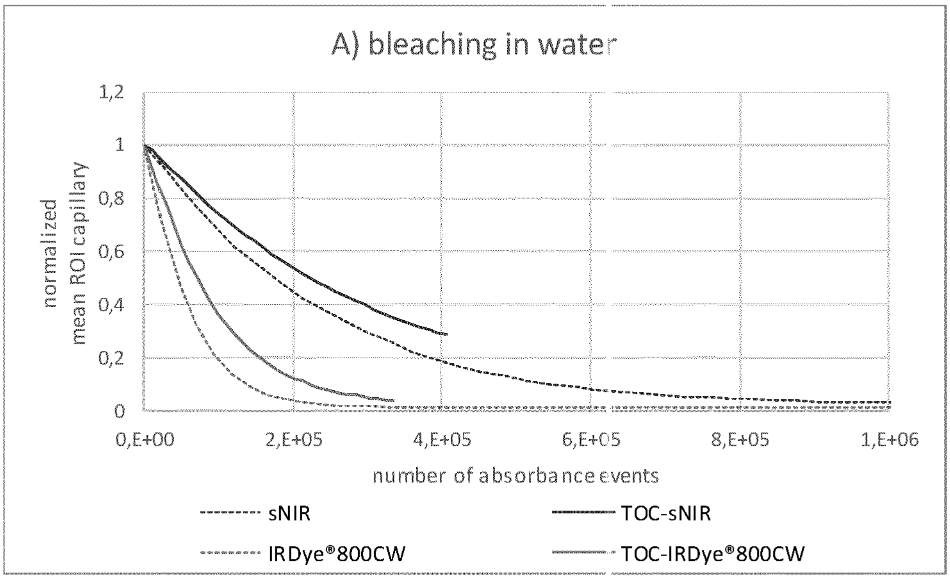
A) bleaching in water
1,2
1
0,8
0,6
0,4
0,2
0
normalized
mean ROI capillary
0,E+00
2,E+05
4,E+05
6,E+05
8,E+05
1,E+06
number of absorbance events
sNIR
TOC-sNIR
IRDye®800CW
TOC-IRDye®800CW

**B**

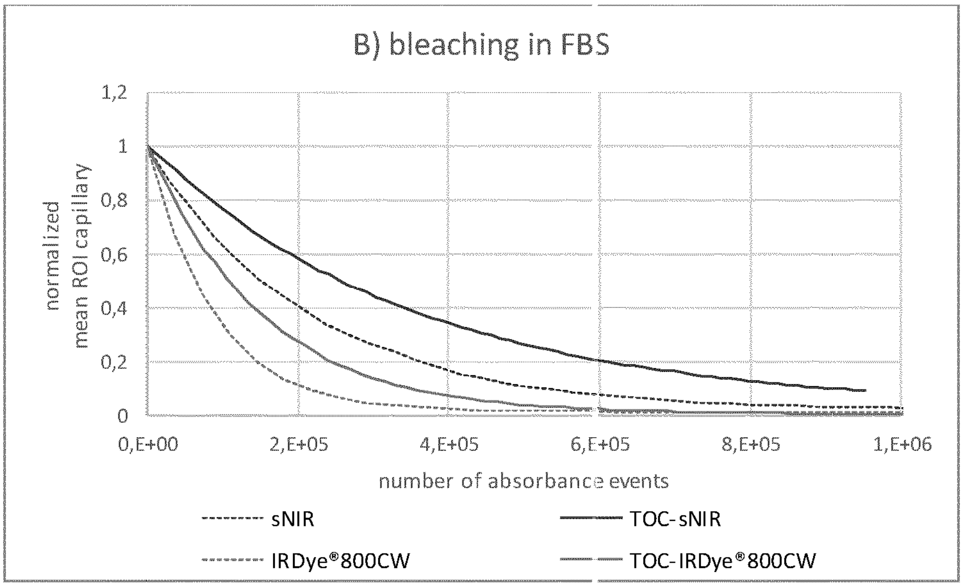
B) bleaching in FBS
1,2
1
0,8
0,6
0,4
0,2
0
normalized
mean ROI capillary
0,E+00
2,E+05
4,E+05
6,E+05
8,E+05
1,E+06
number of absorbance events
sNIR
TOC-sNIR
IRDye®800CW
TOC-IRDye®800CW

**Figure 4 continued**

**C**

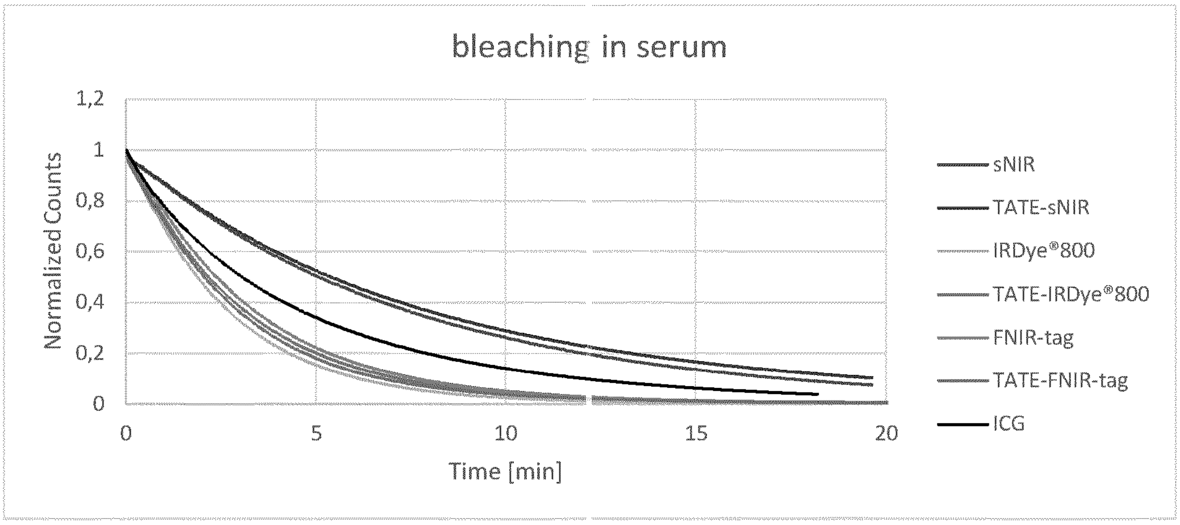

Figure 5
A

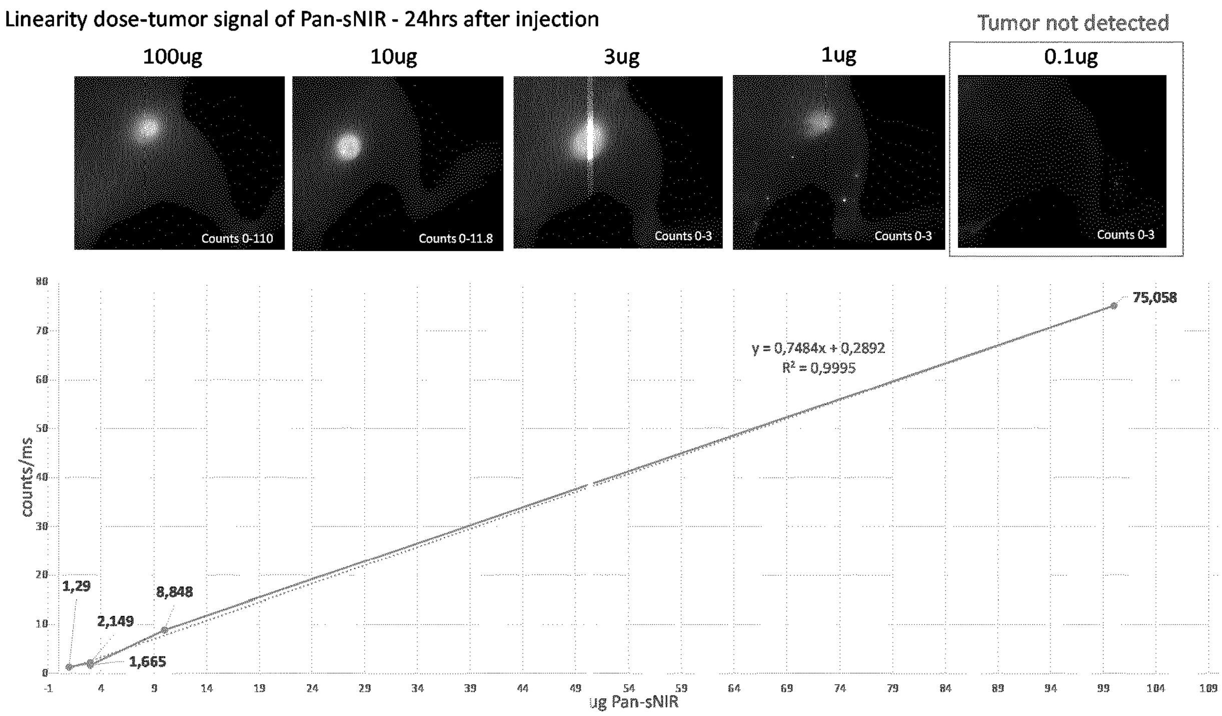

Linearity dose-tumor signal of Pan-sNIR - 24hrs after injection
Tumor not detected
100ug
10ug
3ug
1ug
0.1ug
Counts 0-110
Counts 0-11.8
Counts 0-3
Counts 0-3
Counts 0-3
75,058
y = 0,7484x + 0,2892
R² = 0,9995
counts/ms
1,29
2,149
8,848
1,665
ug Pan-sNIR

**Figure 5 continued**

**B**

Tumor to liver ratio (TLR) Pan-sNIR vs Pan-800CW - 24hrs after injection

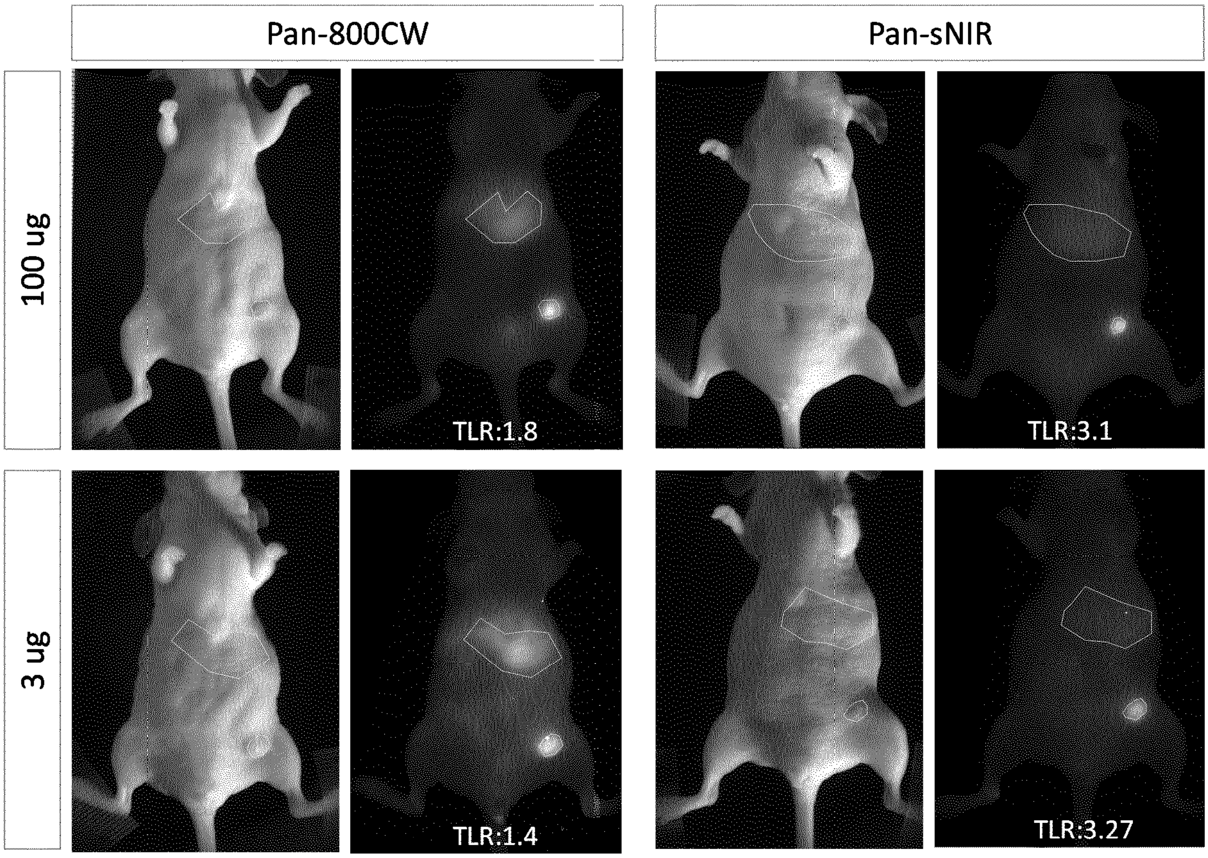

## Figure 6

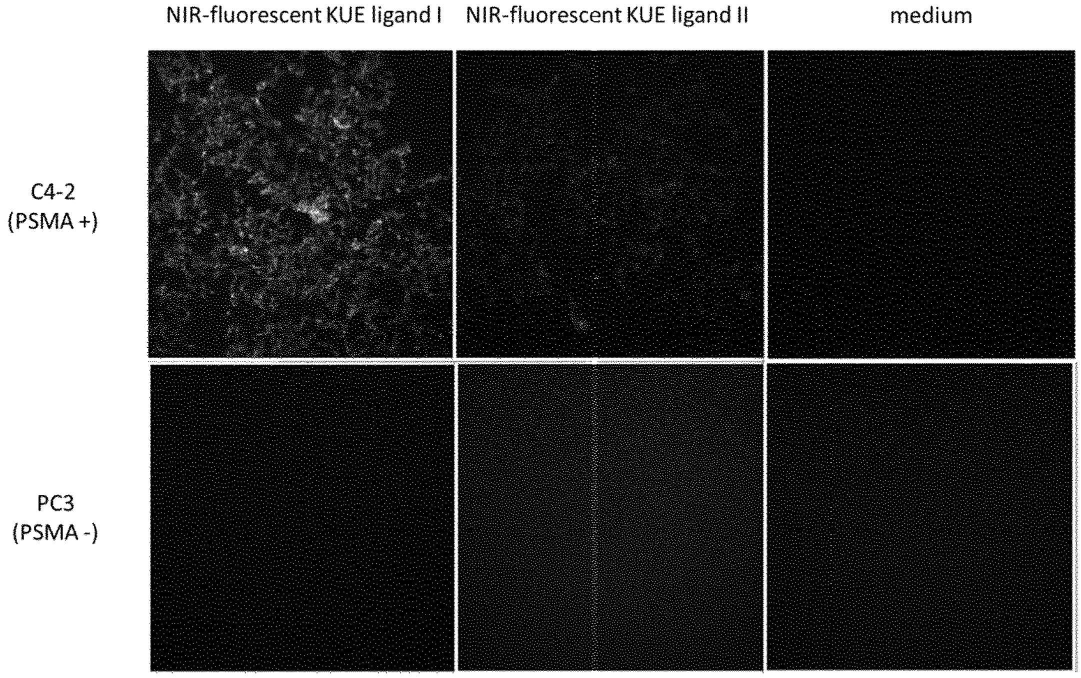
NIR-fluorescent KUE ligand I
NIR-fluorescent KUE ligand II
medium
C4-2
(PSMA +)
PC3
(PSMA -)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 0221512 A1
- WO 2011116142 A1
- WO 2020020905 A1
- WO 0071162 A2
- US 20080233050 A1
- US 10441607 B1


### Non-patent literature cited in the description


- *ACS Nano*, 24 May 2016, vol. 10 (5), 5015-5026
- *Clin Cancer Res*, 15 October 2012, vol. 18 (20)
- **ROGER R. et al.** *Org. Lett.*, 2015, vol. 17, 302-305
- **LI et al.** *Angew. Chem. Int. Ed.*, 2020, vol. 59, 12154-12161
- **LUCIANO et al.** *ACS Chem. Biol.*, 2019, vol. 14, 934-940
- **VARGAS, S. H. et al.** *Clin Cancer Res*, vol. 25 (14), 201
- **DOMMERHOLT, J. ; RUTJES, F. P. J. T. ; VAN DELFT, F. L.** Strain-Promoted 1,3-Dipolar Cycloaddition of Cycloalkynes and Organic Azides. *Top. Curr. Chem.*, 2016, vol. 374, 1-20
- **LIU, Z. ; DEROSA, J. ; ENGLE, K. M.** Palladium(II)-Catalyzed Regioselective syn-Hydroarylation of Disubstituted Alkynes Using a Removable Directing Group. *J. Am. Chem. Soc.*, 2016, vol. 138, 13076-13081
- **CAO, H. et al.** A red Cy3-based biarsenical fluorescent probe targeted to a complementary binding peptide. *J. Am. Chem. Soc.*, 2007, vol. 129, 8672-8673
- **SCHOTTELIUS, M. ; WESTER, H. J. ; REUBI, J. C. ; SENEKOWITSCH-SCHMIDTKE, R. ; SCHWAIGER, M.** Improvement of pharmacokinetics of radioiodinated Tyr3-octreotide by conjugation with carbohydrates. *Bioconjug. Chem.*, 2002, vol. 13, 1021-1030
- **JADHAV, S. V. ; BANDYOPADHYAY, A. ; BENKE, S. N. ; MALI, S. M. ; GOPI, H. N.** A facile synthesis and crystallographic analysis of N-protected β-amino alcohols and short peptaibols. *Org. Biomol. Chem.*, 2011, vol. 9, 4182-4187
- **WEINEISEN et al.** *EJNMMI Research*, 2014, vol. 4, 63
- *Angew. Chem. Int. Ed.*, 2014, vol. 53, 7669-7673
- *Biochimica et Biophysica Acta*, 2008, vol. 1783, 1745-1754
- *Diabetes, Obesity and Metabolism*, 2010, vol. 12, 393-402